# EUROPEAN PATENT SPECIFICATION

(11) **EP 2 776 409 B1**
(45) Date of publication and mention of the grant of the patent: **03.02.2016**
(21) Application number: 12787341.2
(22) Date of filing: 09.11.2012
(51) Int. Cl.: C07D 249/04, A61P 35/00

(54) **MACROCYCLIC COMPOUNDS FOR INHIBITION OF INHIBITORS OF APOPTOSIS**
MAKROCYCLISCHE VERBINDUNGEN ZUR HEMMUNG VON APOPTOSEINHIBITOREN
COMPOSÉS MACROCYCLIQUES POUR L'INHIBITION D'INHIBITEURS DE L'APOPTOSE

(30) Priority: 09.11.2011 US 201161557823 P
(43) Date of publication of application: 17.09.2014
(73) Proprietor: Ensemble Therapeutics Corporation, Cambridge MA 02139 (US); Bristol-Myers Squibb Company, Princeton, NJ 08543 (US)
(72) Inventor: SEIGAL, Benjamin, Adam, Newton, MA 02461 (US); TERRETT, Nicholas, Cambridge, MA 02139 (US); CARTER, Percy, H., Princeton, NJ 08543 (US); BORZILLERI, Robert, M., Princeton, NJ 08543 (US); ZHANG, Yong, Princeton, NJ 08543 (US); MILLER, Michael, M., Princeton, New Jersey 08543 (US)
(74) Representative: Cole, William Gwyn
(86) International application number: PCT/US2012/064342
(87) International publication number: WO 2013/071035

(56) References cited:
- WO-A2-2010/022249
- SHLEZINGER, N. ET AL.: "Apoptosis-like programmed cell death in the grey mould fungus Botrytis cinerea: genes and their role in pathogenicity", BIOCHEMICAL SOCIETY TRANSACTIONS, vol. 39, no. 5, 1 October 2011 (2011-10-01), pages 1493-1498, XP002690064,
- SMOLEWSKI, P., ROBAK, T.: "Inhibitors of apoptosis proteins (IAPs) as potential molecular targets for therapy of hematological malignancies", CURRENT MOLECULAR MEDICINE, vol. 11, no. 8, 1 November 2011 (2011-11-01), XP009166078,

## Description

### CROSS-REFERENCE TO RELATED APPLICATION

This application claims the benefit of priority to U.S. Provisional Patent Application No. 61/557823, filed on November 9, 2011. The content of this application is incorporated herein by reference in its entirety.

### FIELD OF THE INVENTION

The invention relates generally to macrocyclic compounds and their therapeutic use. More particularly, the invention relates to macrocyclic compounds that modulate the activity of inhibitors of apoptosis (IAPs) and/or are useful in the treatment of medical conditions, such as cancer.

### BACKGROUND OF THE INVENTION

Apoptosis or programmed cell death is a genetically and biochemically regulated mechanism that plays an important role in development and homeostasis in invertebrates as well as vertebrates.

Aberrancies in apoptosis that lead to premature cell death have been linked to a variety of developmental disorders. Deficiencies in apoptosis that result in the lack of cell death have been linked to cancer and chronic viral infections (Thompson et al., (1995) Science 267, 1456-1462).

Caspases are cysteine-containing aspartate specific proteases that play a key role in effecting apoptosis. Once activated from their inactive zymogen form by proteolytic processing, caspases digest vital cell proteins from within the cell. Since caspases are such strong proteases, tight control of this family of proteins is necessary to prevent premature cell death. In addition to proteolytic processing, caspases are also regulated by a family of molecules known as Inhibitors of Apoptosis (IAP) (Deveraux et al., J Clin Immunol (1999), 19:388-398). IAPs are found in all organisms ranging from Drosophila to human and are known to be overexpressed in many human cancers. IAPs always comprise one to three Baculovirus IAP repeat (BIR) domains, and most forms also possess a carboxyl-terminal RING finger motif. The BIR domain itself is a zinc binding domain of about 70 residues comprising 4 alpha-helices and 3 beta strands, with cysteine and histidine residues that coordinate the zinc ion (Hinds et al., (1999) Nat. Struct. Biol. 6, 648-651). The BIR domain is believed to cause the anti-apoptotic effect by inhibiting the caspases.

As an example, human X-chromosome linked IAP (XIAP) inhibits caspase 3, caspase 7 and the Apaf-1 -cytochrome C mediated activation of caspase 9 (Deveraux et al., (1998) EMBO J. 17, 2215-2223). Caspases 3 and 7 are inhibited by the BIR2 domain of XIAP, while the BIR3 domain of XIAP is responsible for the inhibition of caspase 9 activity. XIAP is expressed ubiquitously in most adult and fetal tissues (Liston et al., Nature, 1996, 379(6563):349), and is overexpressed in a number of tumor cell lines of the NCI 60 cell line panel (Fong et al., Genomics, 2000, 70:113; Tamm et al., Clin. Cancer Res. 2000, 6(5): 1796). Overexpression of XIAP in tumor cells has been demonstrated to confer protection of the tumor cells against a variety of pro-apoptotic stimuli and promotes resistance to chemotherapy (LaCasse et al., Oncogene, 1998, 17(25):3247). Consistent with this, a strong correlation between XIAP protein levels and survival has been demonstrated for patients with acute myelogenous leukemia (Tamm et al., supra).

In normal cells signaled to undergo apoptosis, the IAP-mediated inhibition is removed in part performed by the mitochondrial protein Smac (second mitochondrial activator of caspases; also known as DIABLO). Smac is synthesized as a precursor molecule of 239 amino acids; the N-terminal 55 residues serve as the mitochondria targeting sequence that is removed after import. The mature form of Smac resides in the inter-membrane space of mitochondria. At the time of apoptosis induction, Smac is released from mitochondria into the cytosol where, together with cytochrome c, it binds to IAPs, and eliminates the inhibitory effect of IAPs on caspases. Smac interacts with essentially all IAPs that have been examined to date and thus appears to be a master regulator of apoptosis in mammals.

Down-regulation of XIAP expression by antisense oligonucleotides has been shown to sensitize tumor cells to death induced by a wide range of pro-apoptotic agents, both in vitro and in vivo. Smac/DIABLO-derived peptides have also been demonstrated to sensitize a number of different tumor cell lines to apoptosis induced by a variety of pro-apoptotic drugs. Because IAP inhibition appears to be a viable mechanism for promoting apoptosis and treating diseases and conditions that are sensitive to apoptosis, there is a continuing need to develop compounds that can inhibit IAP.

### SUMMARY

The present invention provides macrocyclic compounds, methods of modulating the activity of IAP, and methods for treating various medical conditions using such compounds. In one aspect, the invention provides a compound represented by Formula I: 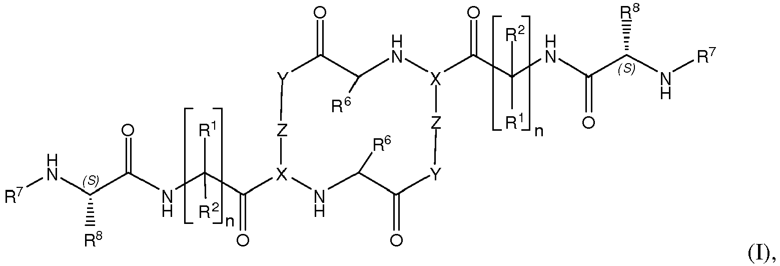 including pharmaceutically acceptable salts thereof, wherein the variables are as defined in the detailed description.

In another aspect, the invention provides compounds of formula I for use in a method of treating a patient suffering from or susceptible to a medical condition that is sensitive to apoptosis. A number of medical conditions can be treated. The method comprises administering to the patient a therapeutically effective amount of a composition comprising a macrocyclic compound described herein. For example, the compounds described herein may be used to treat or prevent infections, proliferative diseases (e.g., cancer), and autoimmune diseases.

In another aspect, the invention provides compounds of formula I for use in a method of inhibiting the activity of an IAP in a cell, thus promoting apoptosis. The method comprises exposing the cell to a compound described herein.

The foregoing and other aspects and embodiments of the invention may be more fully understood by reference to the following detailed description and claims.

### DETAILED DESCRIPTION OF THE INVENTION

The present invention provides macrocyclic compounds, methods of modulating the activity of IAP and in particular XIAP, and methods for treating various medical conditions, especially cancer, using such compounds. The practice of the present invention employs, unless otherwise indicated, conventional techniques of organic chemistry, pharmacology, and biochemistry. For example, procedures for synthesizing organic compounds are described in the literature, such as "Comprehensive Organic Synthesis" (B.M. Trost & I. Fleming, eds., 1991-1992). Various aspects of the invention are set forth below in sections; however, aspects of the invention described in one particular section are not to be limited to any particular section. Further, when a variable is not accompanied by a definition, the previous definition of the variable controls.

### I. Macrocyclic Compounds

In one aspect, the invention provides a compound represented by Formula I: 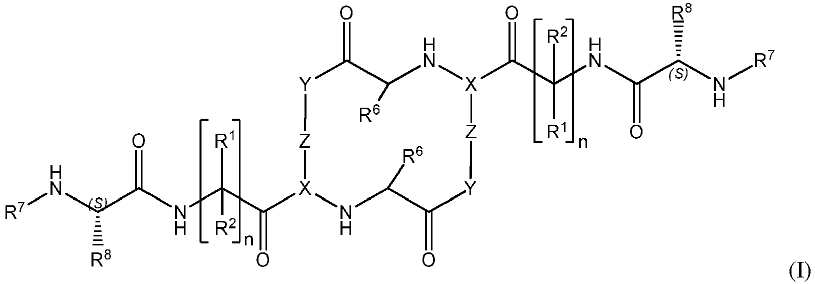 or a pharmaceutically acceptable salt thereof, wherein:
each n is independently 1 or 2;
each R¹ is independently selected from hydrogen, cycloalkyl and -(C₁-C₄ alkylene)-R⁴, wherein each R⁴ is independently selected from hydrogen, aryl, and cycloalkyl, wherein at least one R¹ is other than hydrogen; and
each R² is hydrogen; or
R¹ and R² are taken together to the carbon atom to which they are commonly bound to form a cycloalkyl;
each R⁶ is independently -(C₁-C₄ alkylene)-R⁹, wherein each R⁹ is independently selected from hydrogen, aryl, heteroaryl and cycloalkyl; wherein any aryl, heteroaryl or cycloalkyl portion of R⁶ is optionally substituted with up to two substituents independently selected from halo, CF₃, OH, C₁-C₄ alkoxy, C₁-C₄ alkenyloxy, phenyl, phenyloxy, and phenylmethyloxy; and wherein one -CH₂- in the -(C₁-C₄ alkylene)- portion of R⁶ is optionally replaced with -O-;
each R⁷ is independently selected from hydrogen and methyl;
each R⁸ is independently selected from methyl and ethyl;
each X is independently selected from: 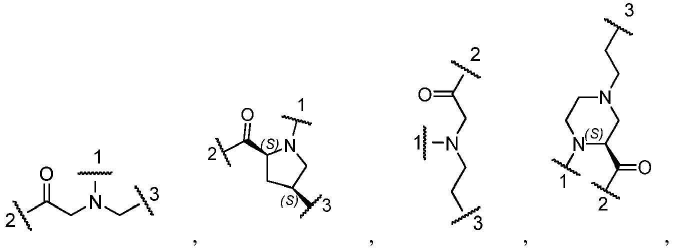 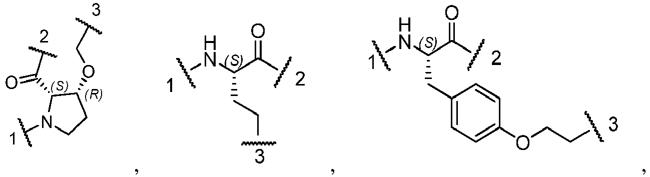 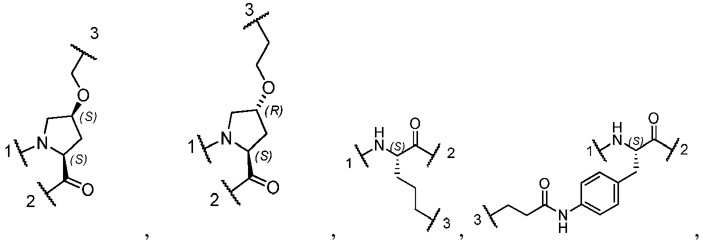 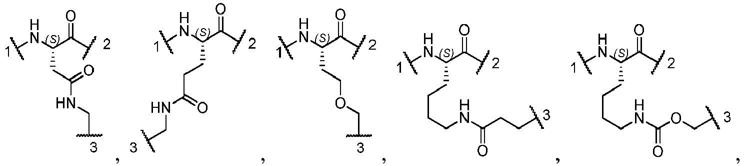 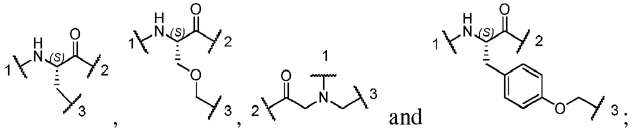
Z is 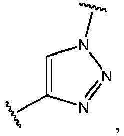 wherein each 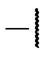 represents a point of attachment to the compound; and
each Y is independently selected from: 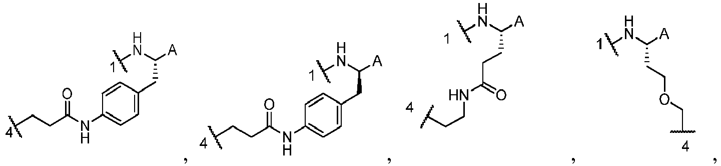 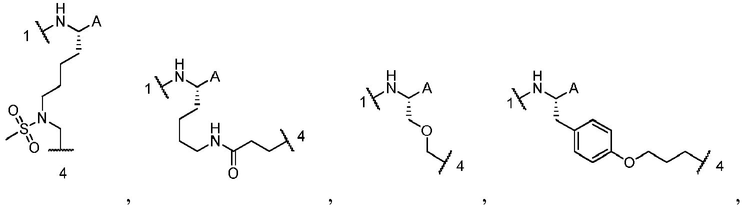 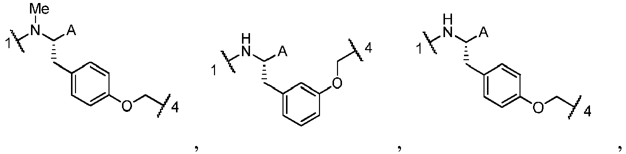 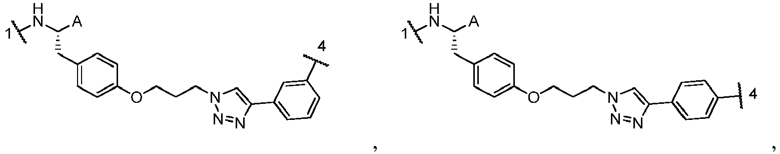 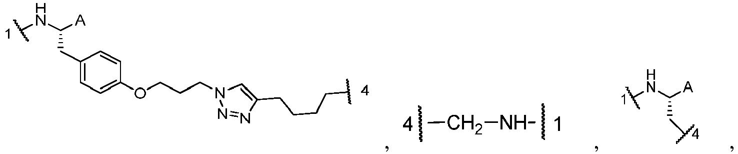 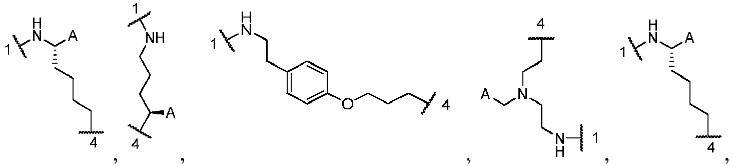 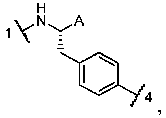 and 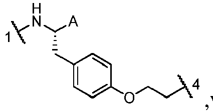 wherein: 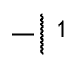
   represents a point of attachment to a -C=O portion of the compound; 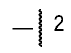
   represents a point of attachment to a -NH portion of the compound; 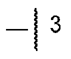
   represents a first point of attachment to Z; 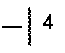
   represents a second point of attachment to Z; and
A is selected from -C(O)R³ or 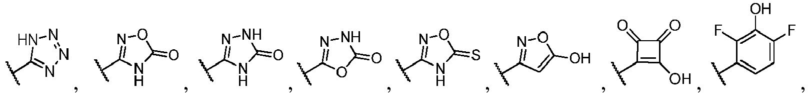 or a tautomeric form of any of the foregoing, wherein:
   R³ is OH, NHCN, NHSO₂R¹⁰, NHOR¹¹ or N(R¹²)(R¹³);
   R¹⁰ and R¹¹ are selected from -C₁-C₄ alkyl, cycloalkyl, aryl, heteroaryl, or heterocycloalkyl, any of which are optionally substituted, and hydrogen;
   each of R¹² and R¹³ are independently selected from hydrogen, -C₁-C₄ alkyl, -(C₁-C₄ alkylene)-NH-(C₁-C₄ alkyl), -(C₁-C₄ alkylene)-O-(C₁-C₄ alkyl), and -(C₁-C₄ alkylene)-O-(C₁-C₄ hydroxyalkyl), or R¹² and R¹³ are taken together with the nitrogen atom to which they are commonly bound to form a saturated heterocyclyl optionally comprising one additional heteroatom selected from N, O and S, and wherein the saturated heterocycle is optionally substituted with methyl.

In a broader aspect of Formula I, X is additionally selected from

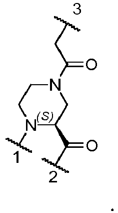

In another broader aspect of Formula I, Y is additionally selected from

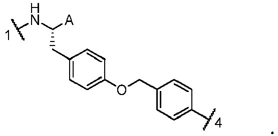

In still another broader aspect of Formula I, R³ is additionally selected from -O-(C1-C4 alkyl).

In still another broader aspect of Formula I, A is additionally selected from 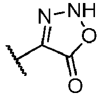 and its tautomer.

In certain embodiments of Formula I, R⁷ is methyl and R⁸ is methyl.

In certain embodiments of Formula I, the portion of the compound represented by -NH-[C(R¹)(R²)]ₙ-C(O)- is selected from: 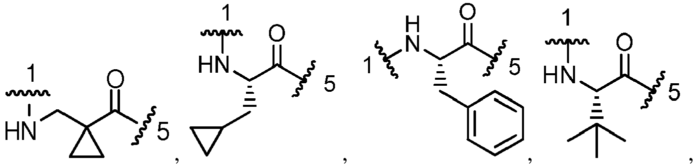 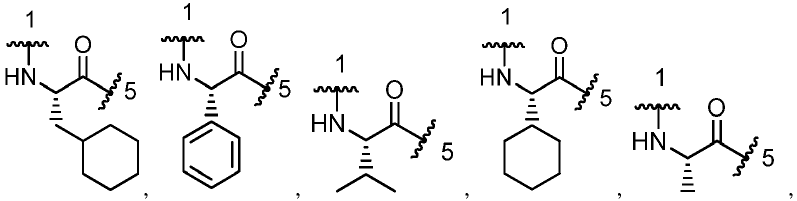 and 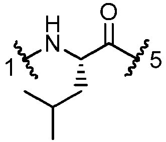 wherein: 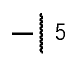
represents a point of attachment to the amino portion of X. In more specific aspects of these embodiments, X is

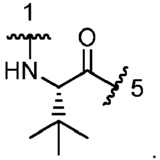

In certain embodiments of Formula I, the portion of the compound represented by -NH-[C(R¹)(R²)]ₙ-C(O)- is selected from: 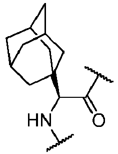 and

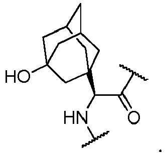

In certain embodiments of Formula I, the portion of the compound represented by -NH-CH(R⁶)-C(O)- is selected from: 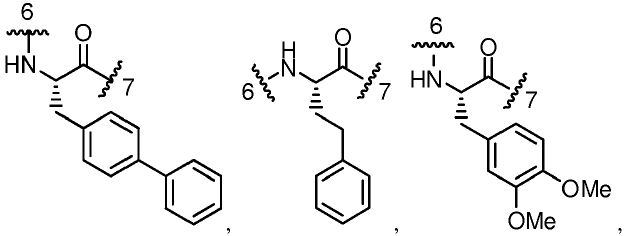 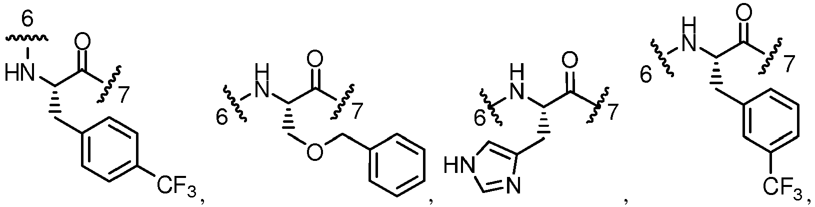 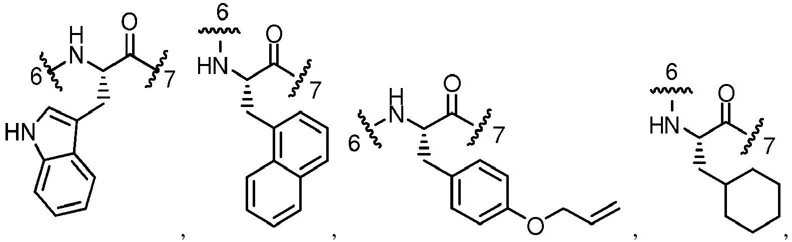 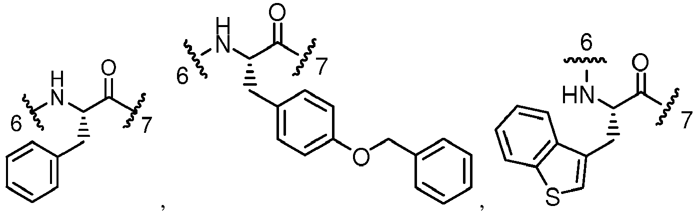 and 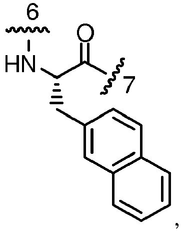 wherein: 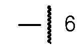
represents a point of attachment to the -C=O portion of X; and 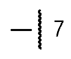
represents a point of attachment to the amino portion of Y. In a more specific aspect of these embodiments, the portion of the compound represented by -NH-CH(R⁶)-C(O)- is

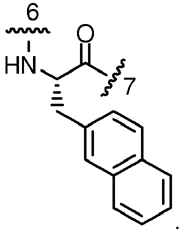

In certain embodiments of Formula I, X is selected from: 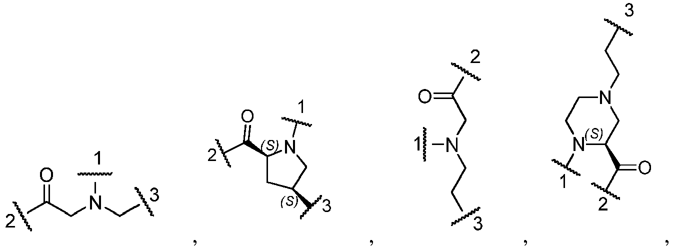 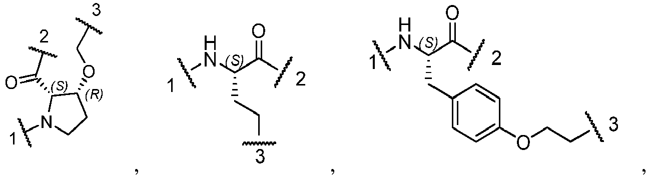 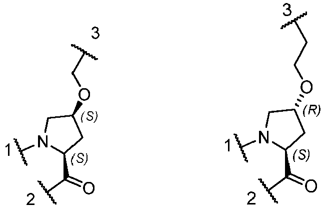 , and . In one aspect of these embodiments, X is selected from: 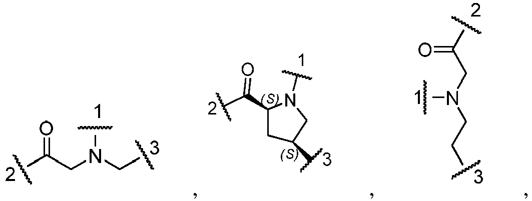 and 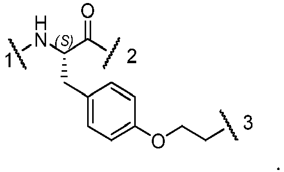 In an alternate aspect of these embodiments, X is selected from 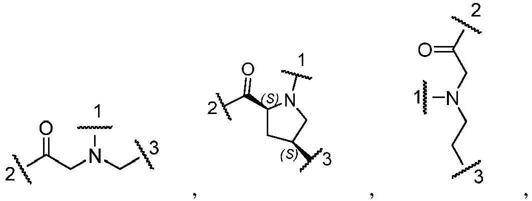 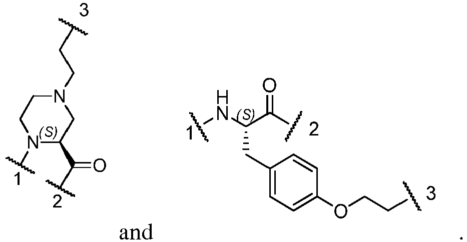 In a more specific aspect of these embodiments, X is

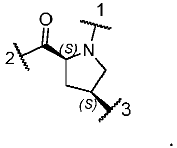

In certain embodiments of Formula I, Y is selected from 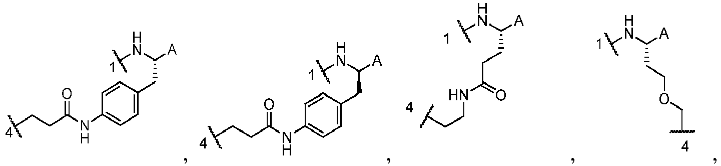 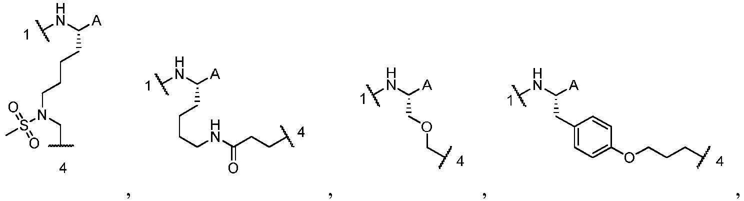 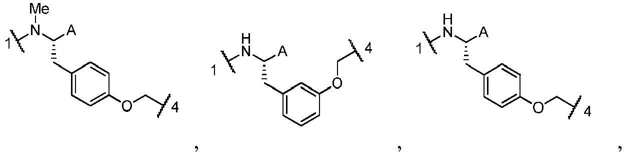 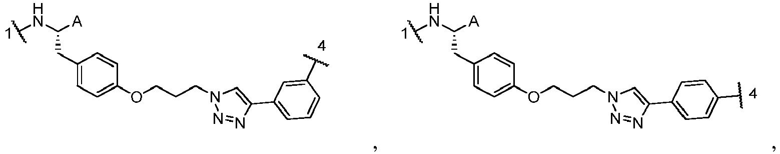 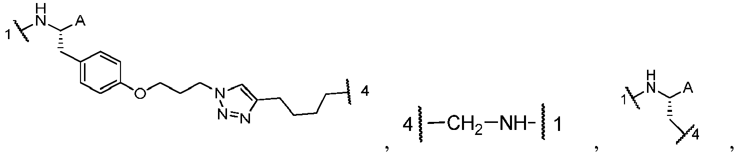 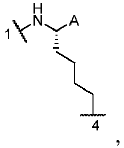 and 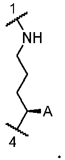 In one aspect of these embodiments, X is

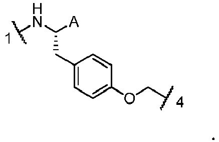

In certain embodiments of Formula I, each n is 1.

In certain embodiments of Formula I, the compound is a homodimer (e.g., each R¹ is the same; each R² is the same; each R⁶ is the same; each R⁷ is the same; each R⁸ is the same; each X is the same; each Y is the same; each Z is the same; and each n is the same).

Exemplary compounds of Formula I are set forth in the table below.

**TABLE 1 -- Compounds of Formula I**

| **Cmpd No.** | **Structure** | **Predicted MS** | **Observed MS** |
|---|---|---|---|
| 100 | 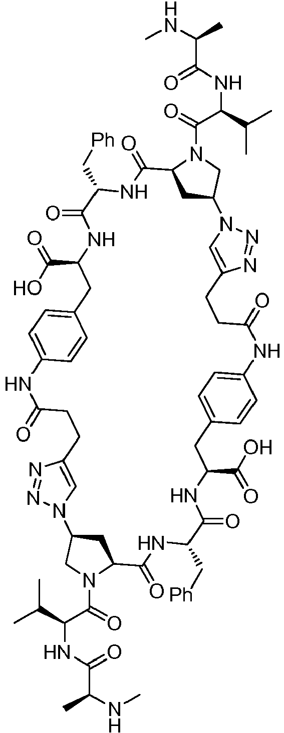 | 1459.65 | 730.25 |
| 101 | 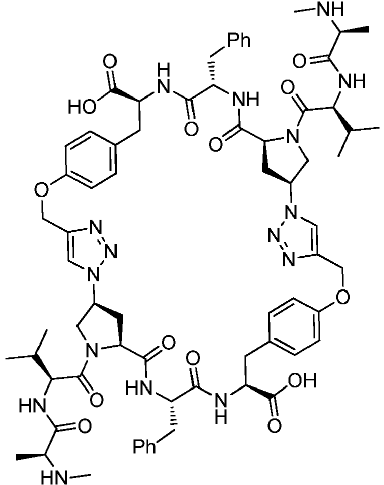 | 1377.5465 | 689.88 |
| 102 | 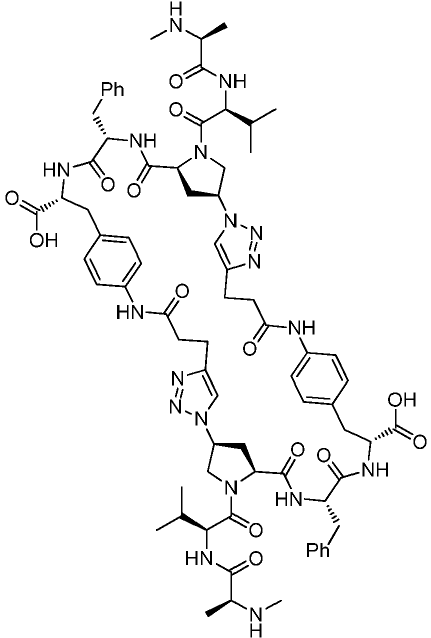 | 1459.6504 | 730.32 |
| 103 | 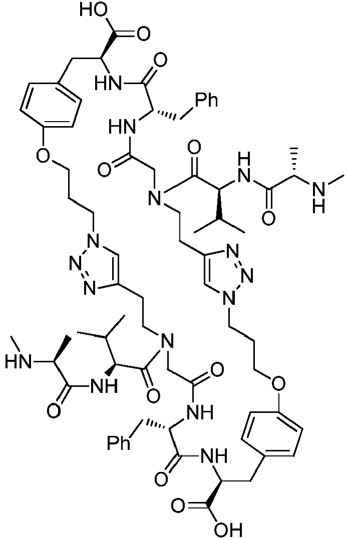 | 1409.6315 | 705.34 |
| 104 | 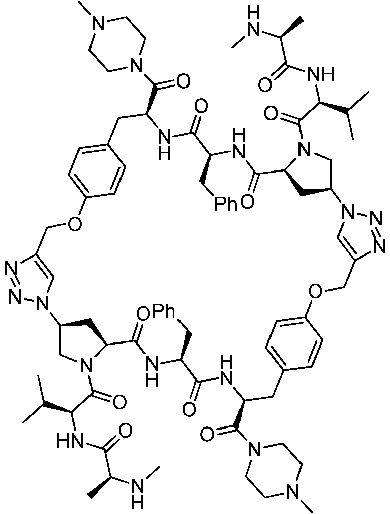 | 1541.8403 | 771.18 |
| 105 | 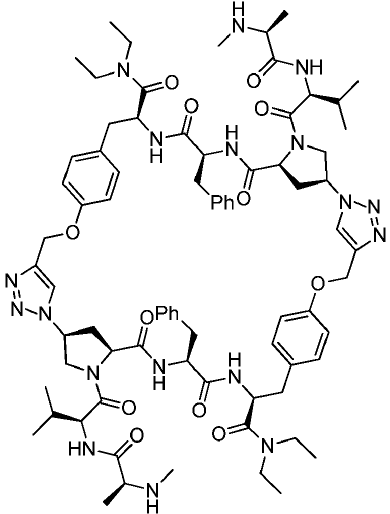 | 1487.7897 | 744.41 |
| 106 | 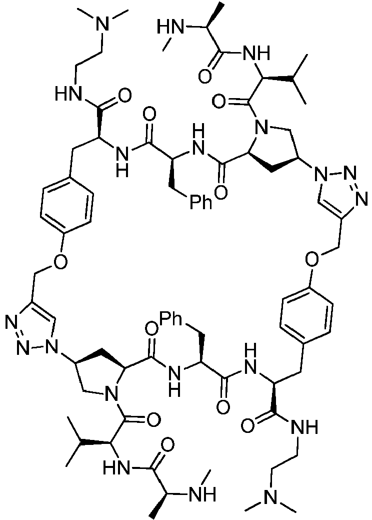 | 1517.819 | 759.21 |
| 107 | 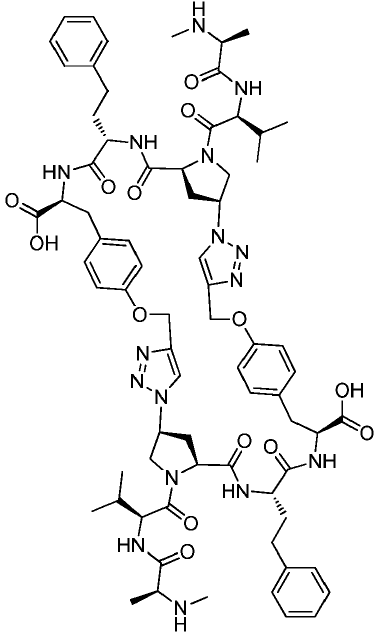 | 1405.5997 | 703.07 |
| 108 | 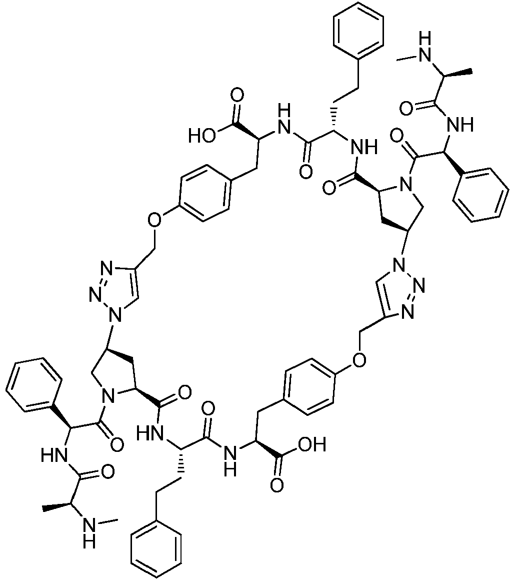 | 1473.6321 | 737.31 |

| **Cmp No.** | **Structure** | **Predicted MS** | **Observed MS** |
|---|---|---|---|
| 109 | 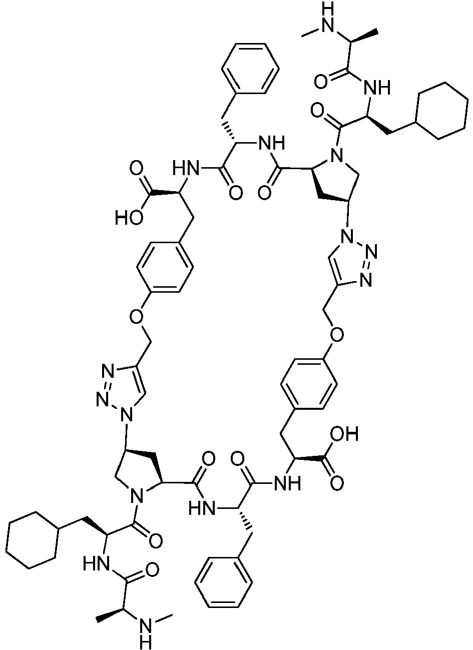 | 1485.7274 | 743.15 |
| 110 | 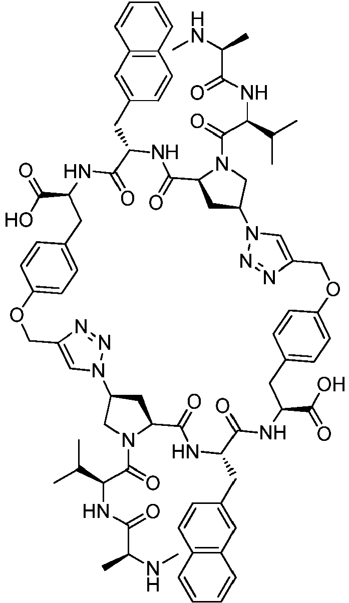 | 1477.6639 | 739.08 |

| **Cmpd No.** | **Structure** | **Predicted MS** | **Observed MS** |
|---|---|---|---|
| 111 | 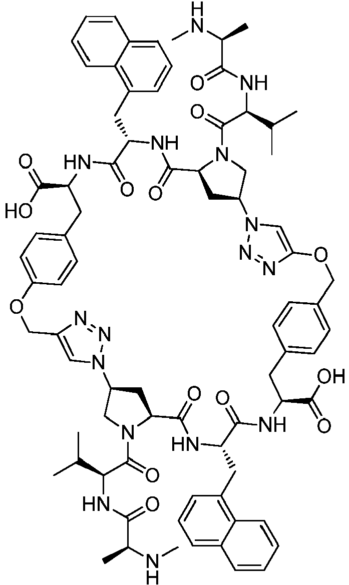 | 1477.6639 | 739.01 |
| 112 | 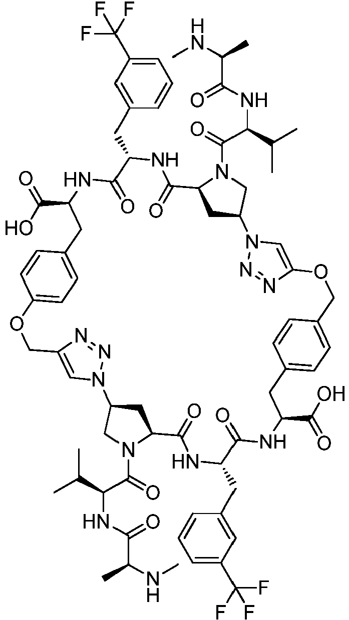 | 1513.5425 | 756.98 |
| 113 | 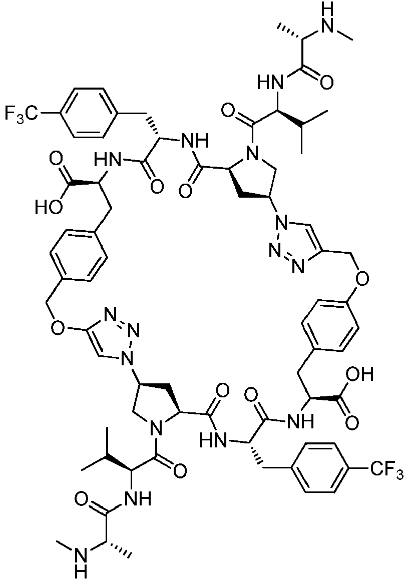 | 1513.5425 | 756.98 |
| 114 | 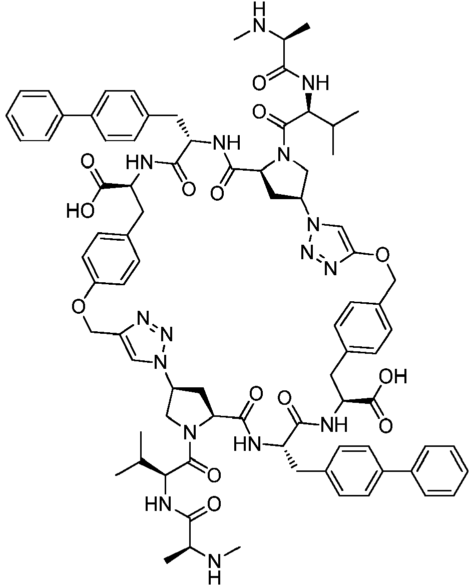 | 1529.7384 | 765.48 |
| 115 | 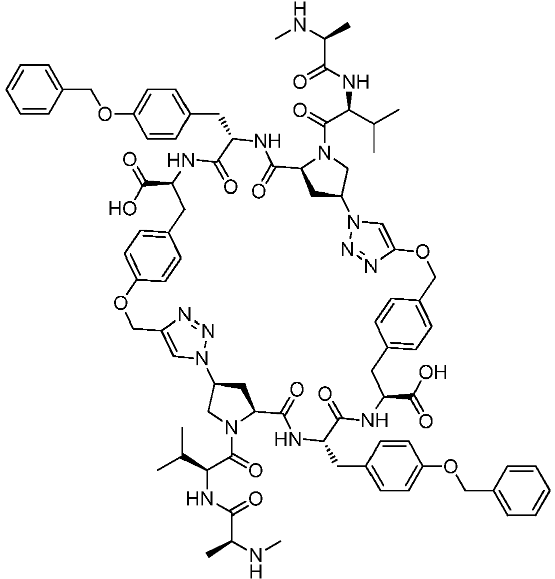 | 1589.7904 | 795.43 |
| 116 | 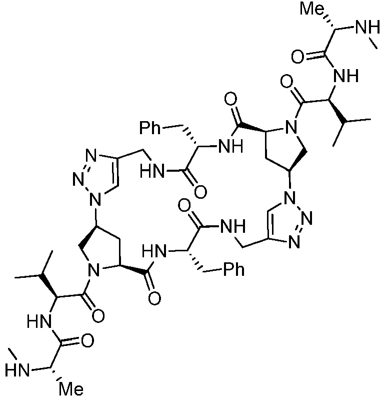 | 1049.2305 | 1050.00 |
| 117 | 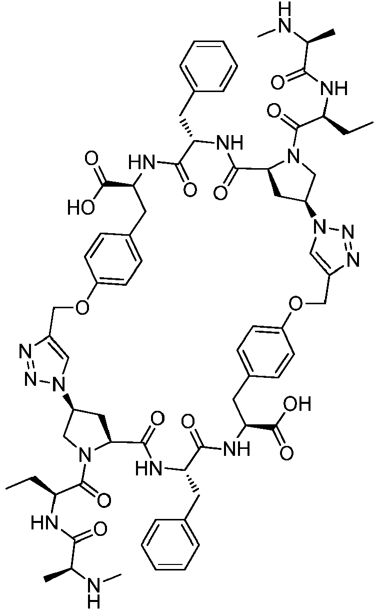 | 1349.4934 | 675.01 |
| 118 | 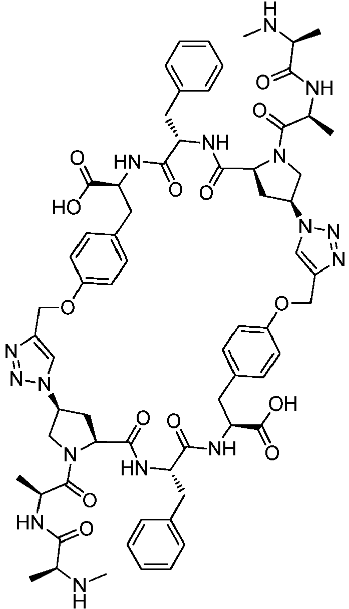 | 1321.4402 | 661.55 |
| 119 | 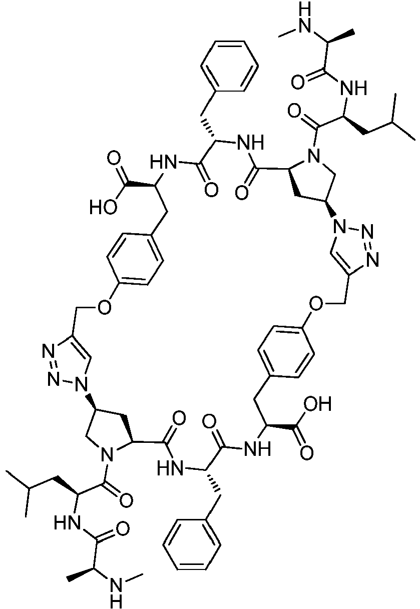 | 1405.5997 | 703.34 |
| 120 | 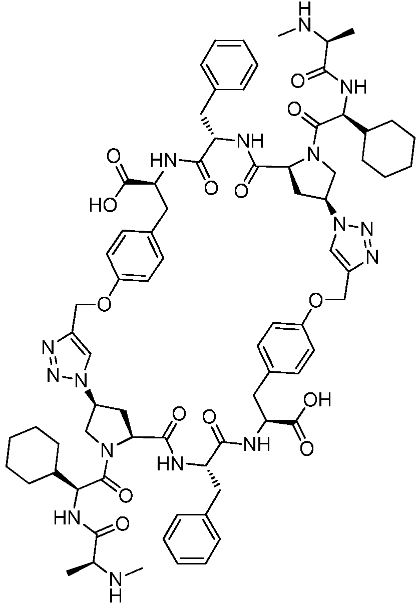 | 1457.6742 | 729.53 |
| 121 | 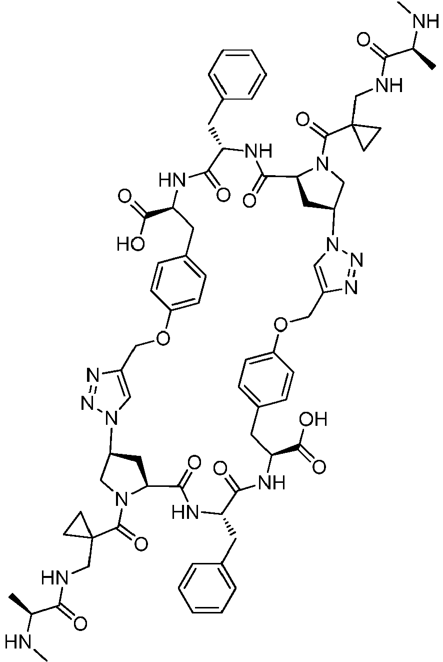 | 1373.5148 | 687.81 |
| 122 | 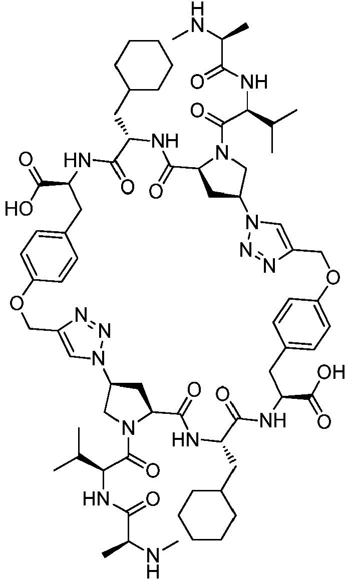 | 1389.6418 | 695.95 |
| 123 | 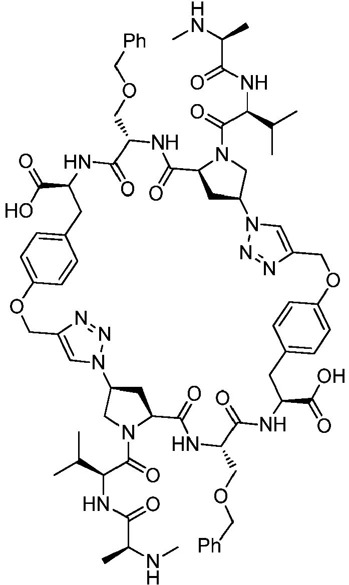 | 1437.5984 | 719.51 |
| 124 | 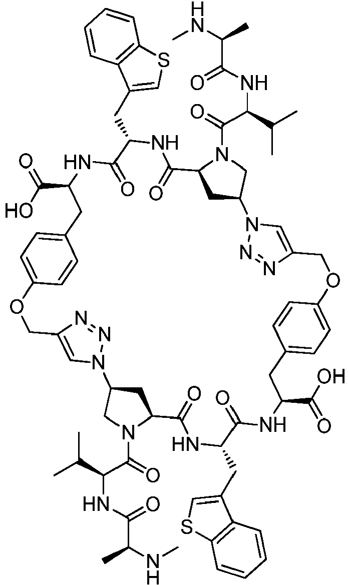 | 1489.7194 | 745.34 |
| 125 | 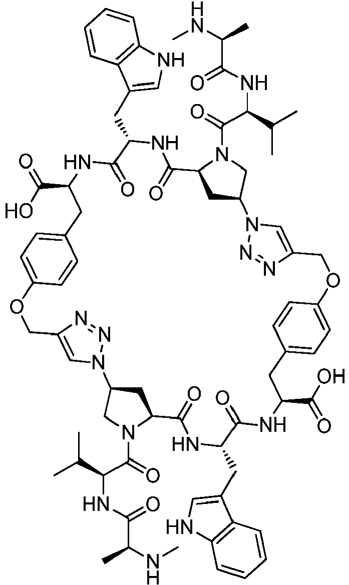 | 1455.6187 | 728.62 |
| 126 | 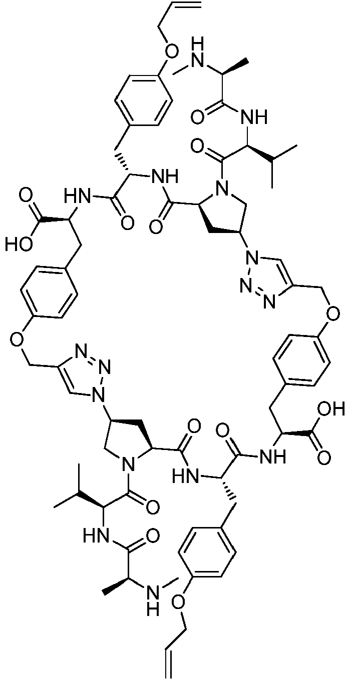 | 1489.6731 | 745.73 |
| 127 | 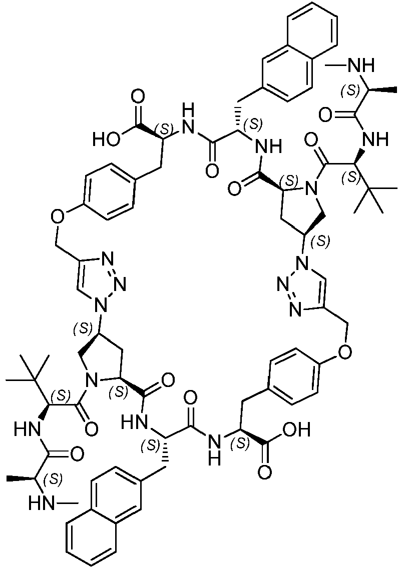 | 1505.717 | 753.52 |
| 128 | 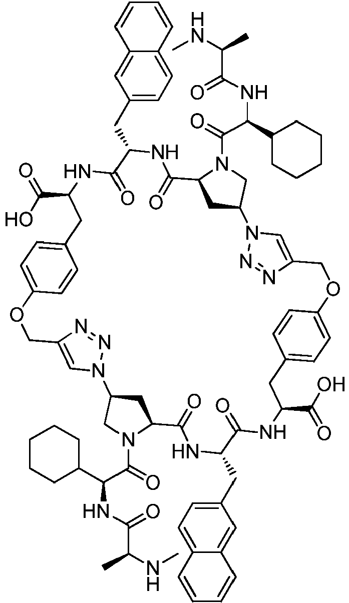 | 1557.7916 | 779.69 |
| 129 | 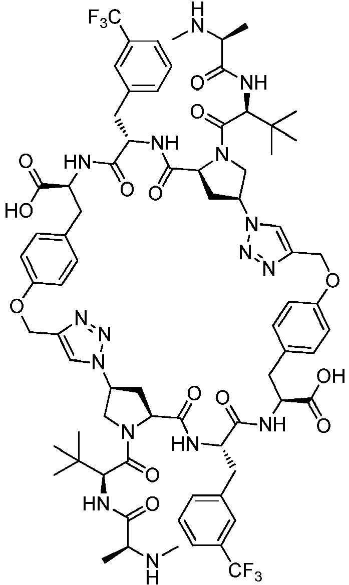 | 1541.5956 | 771.70 |
| 130 | 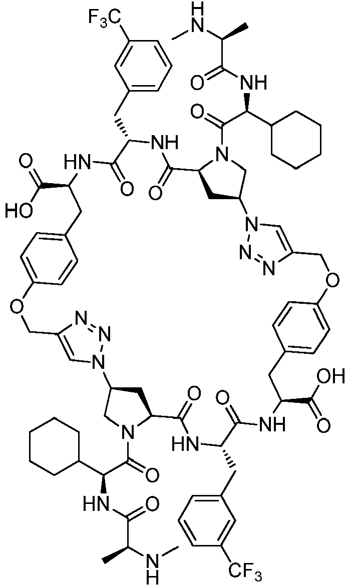 | 1593.6701 | 797.66 |
| 131 | 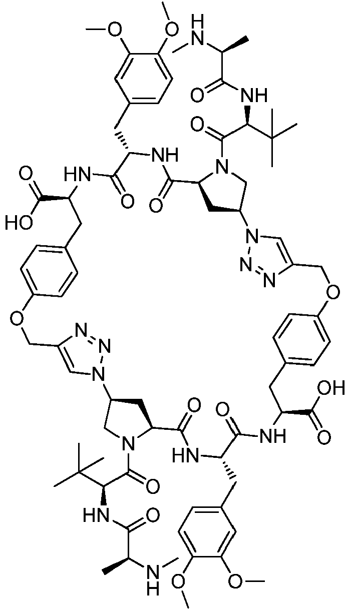 | 1525.7036 | 763.71 |
| 132 | 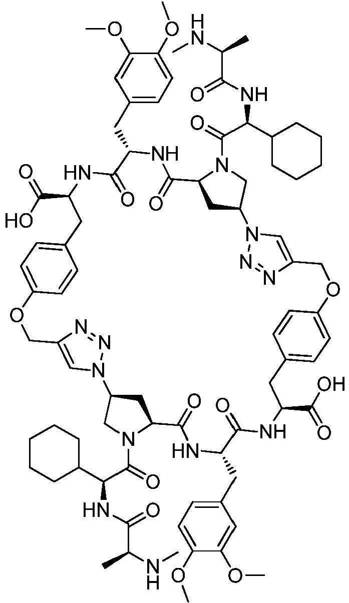 | 1577.7781 | 789.97 |
| 133 | 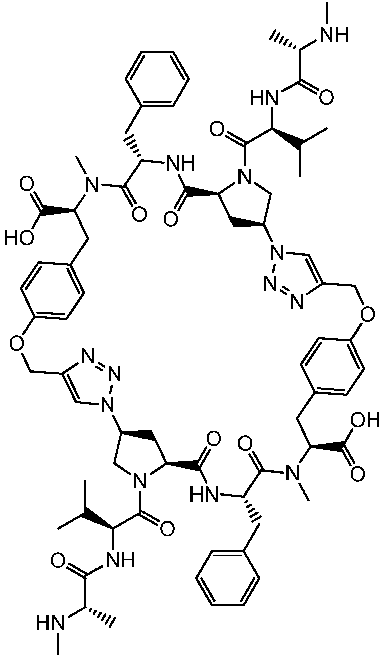 | 1405.5997 | 703.79 |
| 134 | 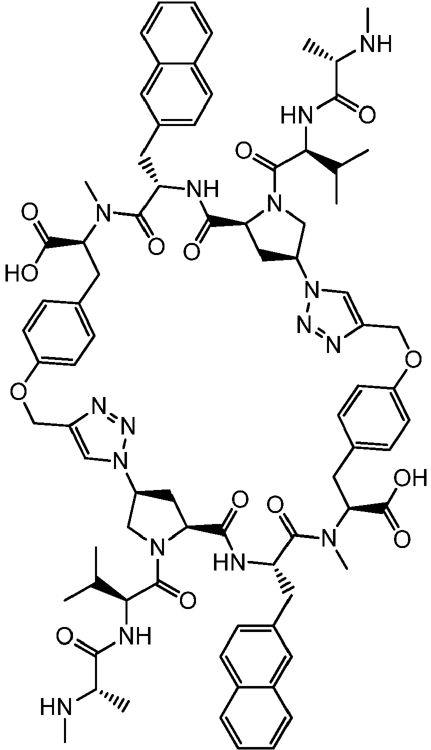 | 1505.717 | 753.79 |
| 135 | 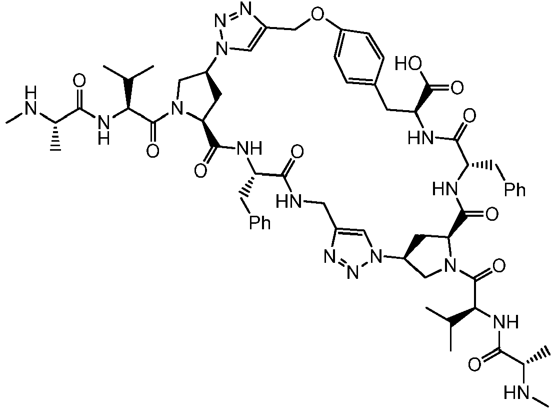 | 1213.3885 | 1214.15 |
| 136 | 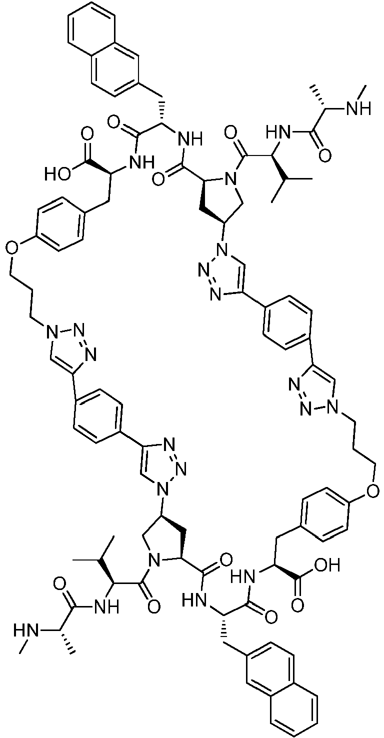 | 1820.061 | 910.89 |
| 137 | 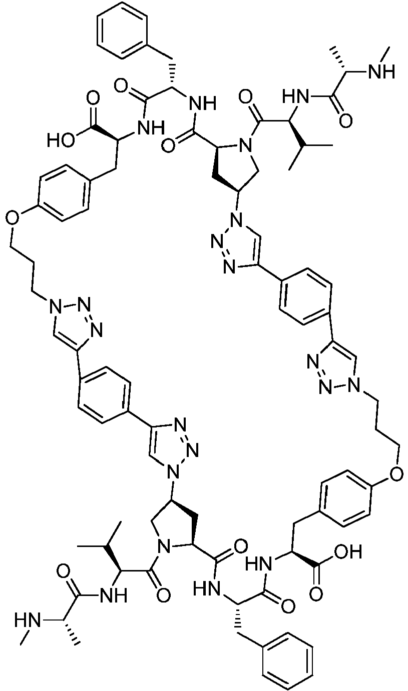 | 1719.9436 | 860.82 |
| 138 | 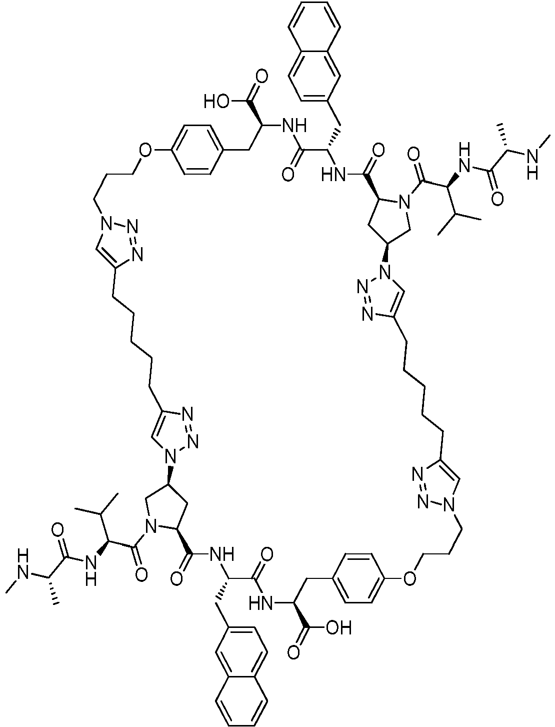 | 1780.0817 | 890.60 |
| 139 | 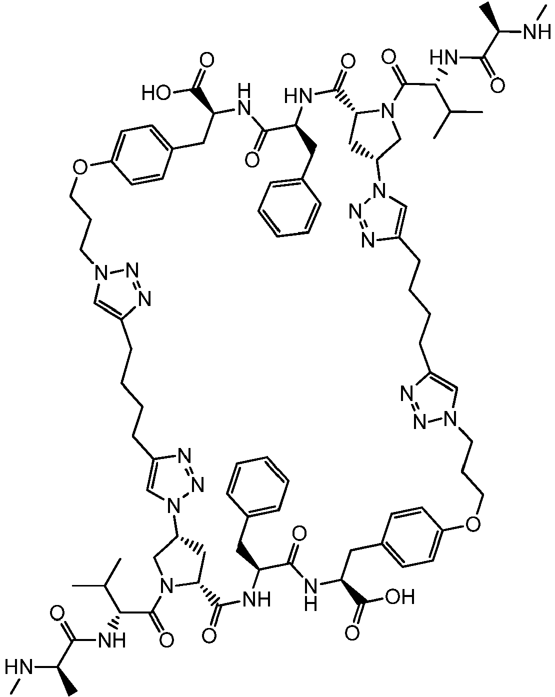 | 1679.9644 | 841.29 |
| 140 | 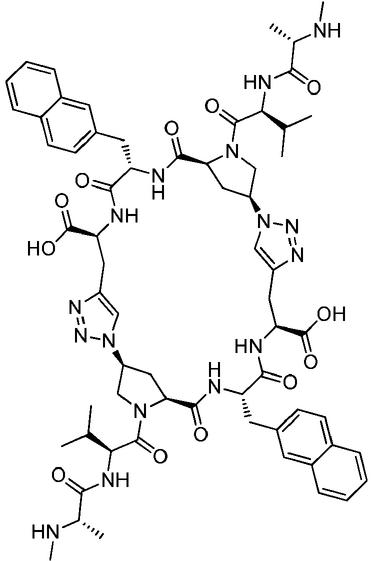 | 1265.42 | 633.81 |
| 141 | 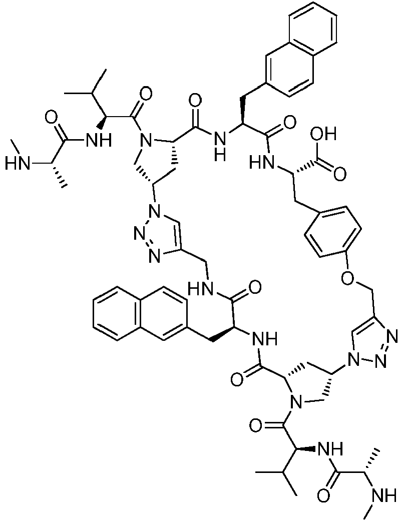 | 1313.5059 | 657.85 |
| 142 | 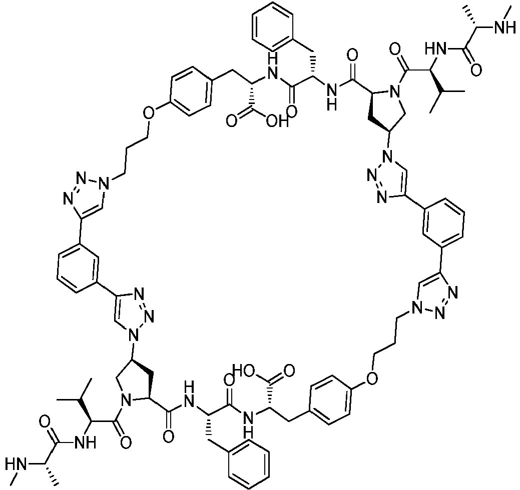 | 1719.9436 | 860.74 |
| 143 | 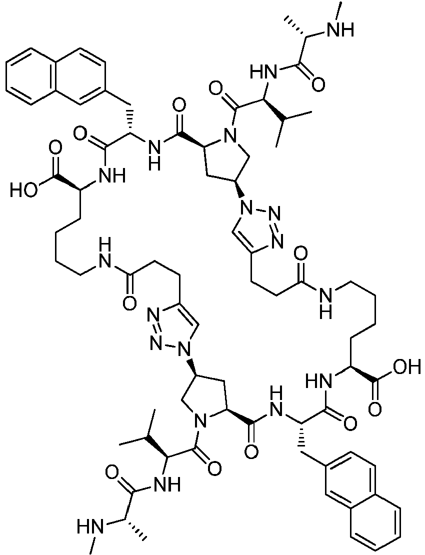 | 1491.7352 | 746.40 |
| 144 | 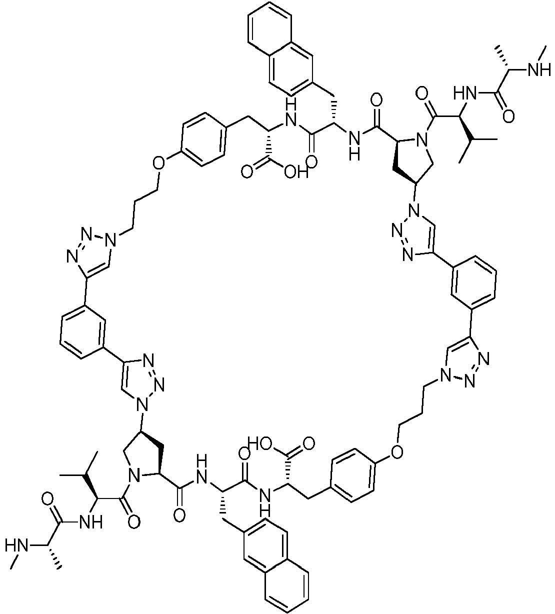 | 1820.061 | 910.98 |
| 145 | 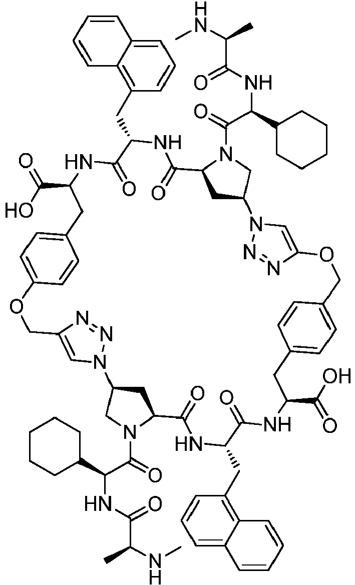 | 1557.7916 | 779.76 |
| 146 | 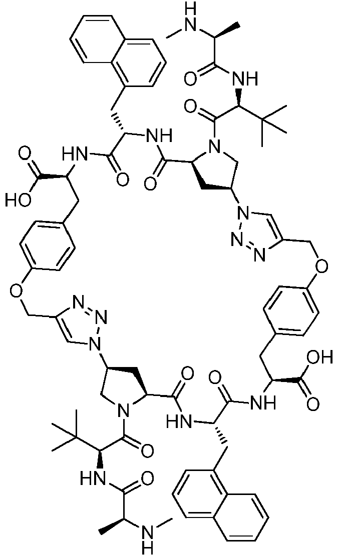 | 1505.717 | 753.73 |
| 147 | 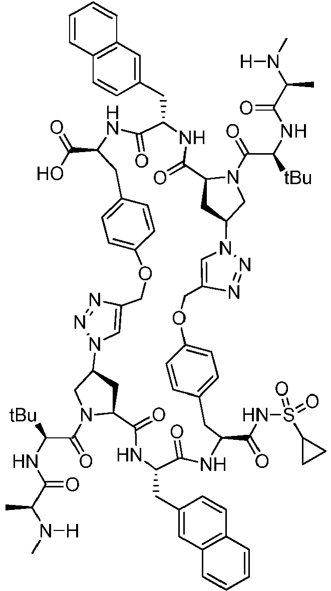 | 1607.738 | 1608.6 |
| 148 | 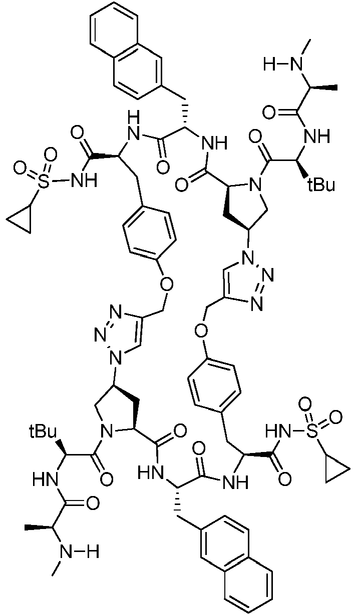 | 1710.748 | 1711.9 |
| 149 | 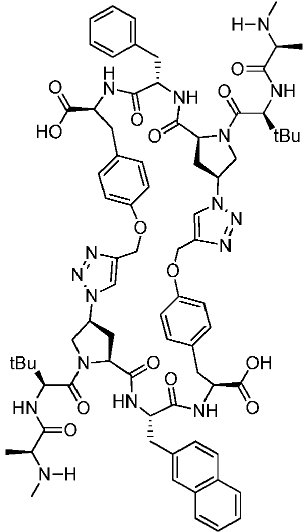 | 1454.714 | 1456.2 |
| 150 | 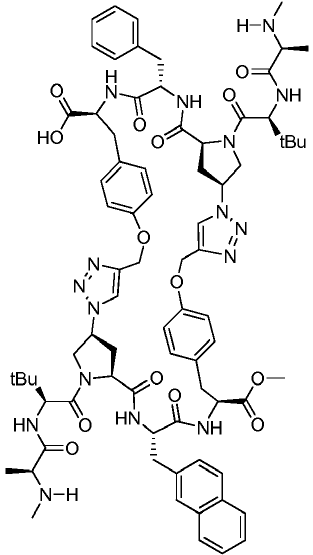 | 1468.729 | 1469.9 |
| 1000 | 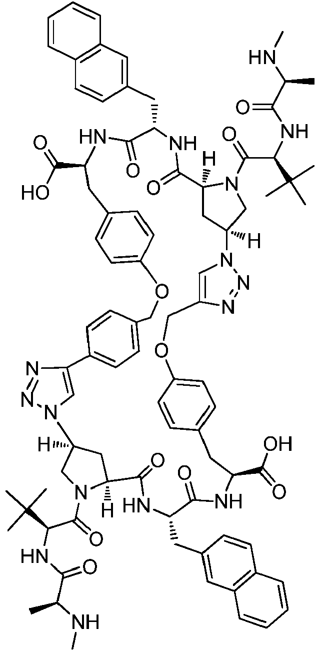 | 1580.8 | 792.0 |
| 1001 | 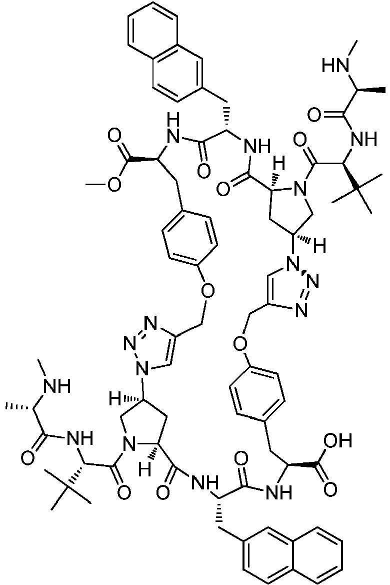 | 1518.8 | 760.4 |
| 1002 | 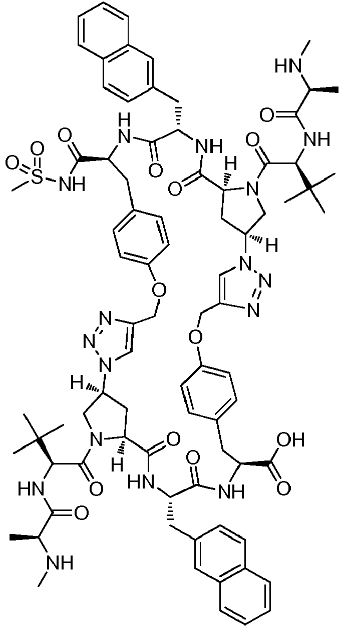 | 1581.7 | 792.7 |
| 1003 | 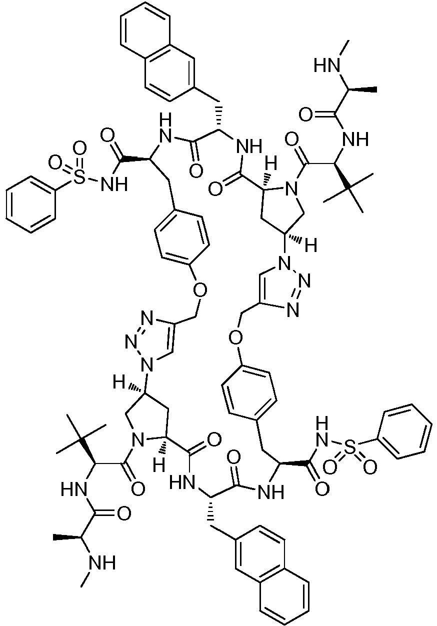 | 1782.8 | 892.9 |
| 1004 | 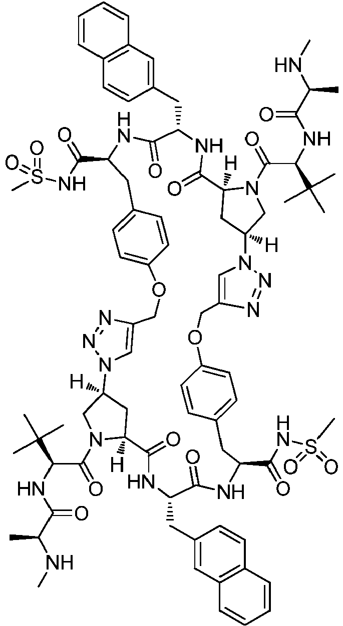 | 1658.7 | 830.4 |
| 1005 | 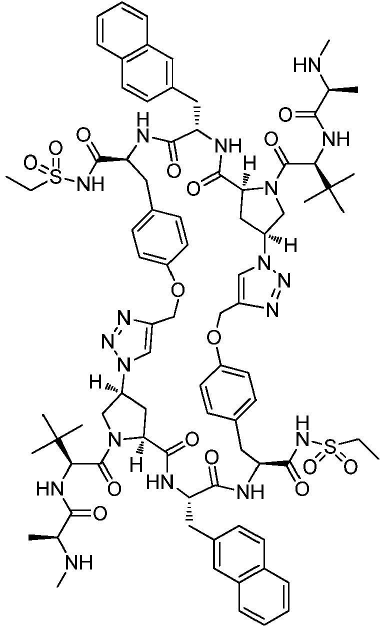 | 1686.8 | 844.1 |
| 1006 | 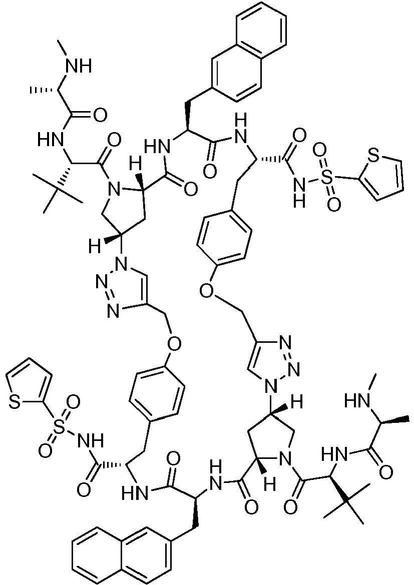 | 1794.7 | 898.9 |
| 1007 | 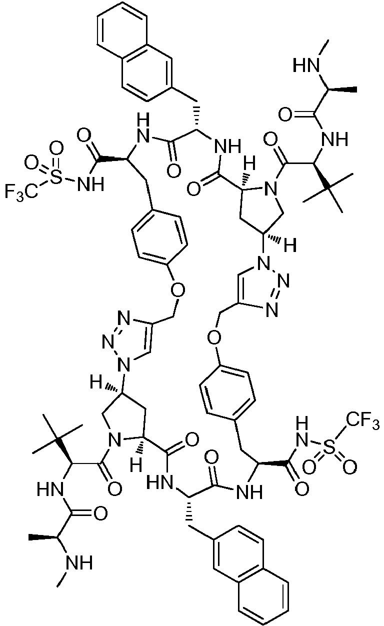 | 1766.7 | 884.5 |
| 1008 | 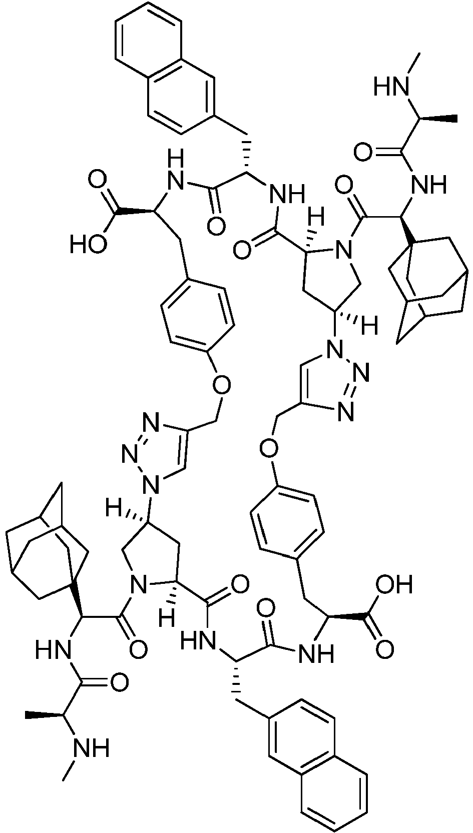 | 1660.8 | 831.5 |
| 1009 | 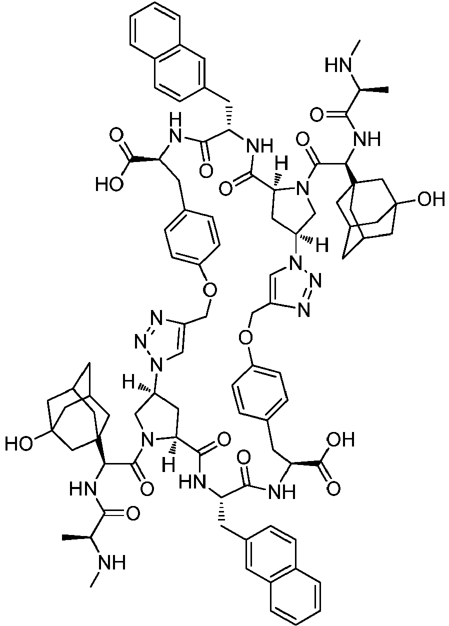 | 1692.8 | 847.6 |
| 1010 | 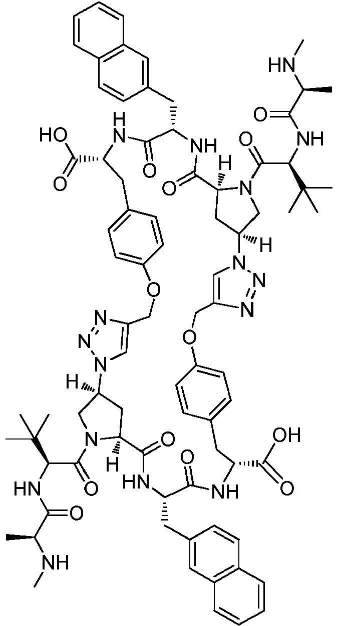 | 1504.7 | 753.3 |
| 1011 | 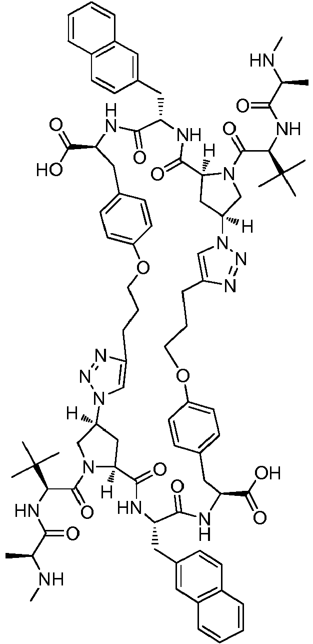 | 1560.8 | 781.6 |
| 1012 | 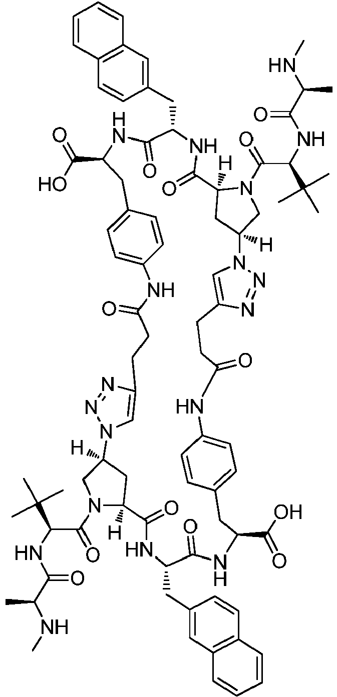 | 1587.8 | 794.3 |
| 1013 | 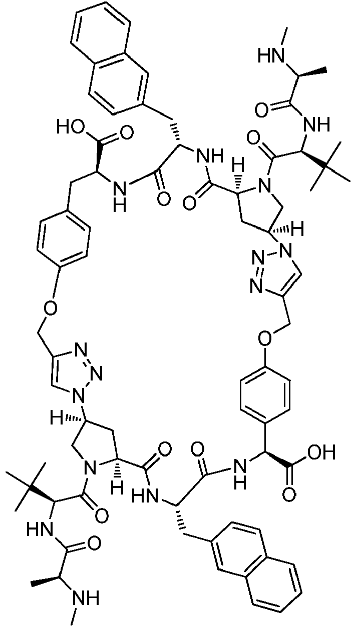 | 1490.7 | 746.5 |
| 1014 | 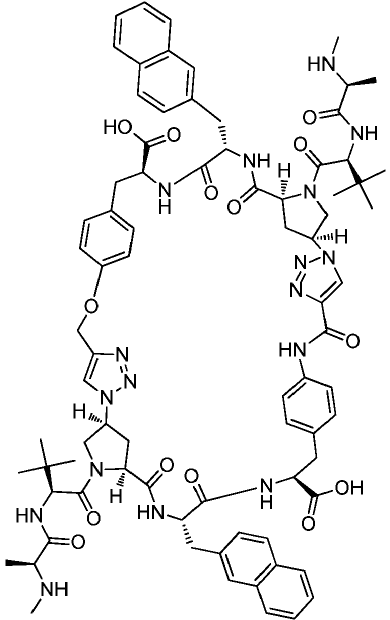 | 1517.7 | 760.2 |
| 1015 | 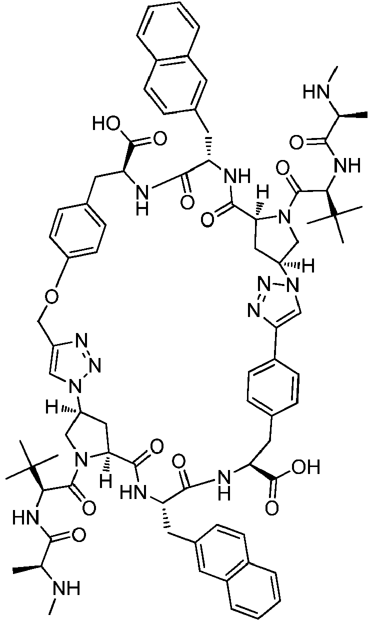 | 1474.7 | 738.5 |
| 1016 | 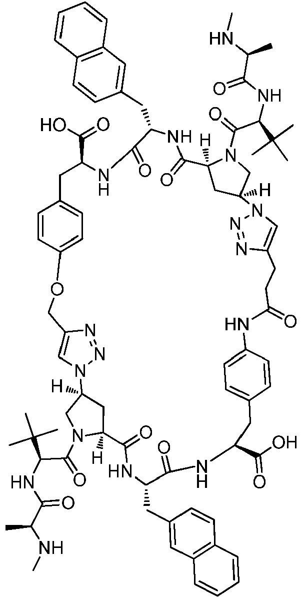 | 1545.8 | 774.0 |
| 1017 | 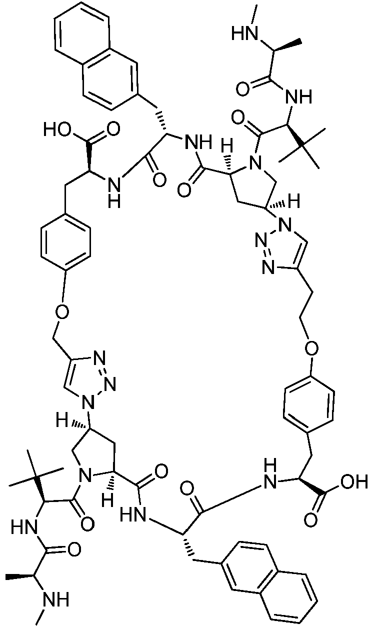 | 1518.7 | 760.5 |
| 1018 | 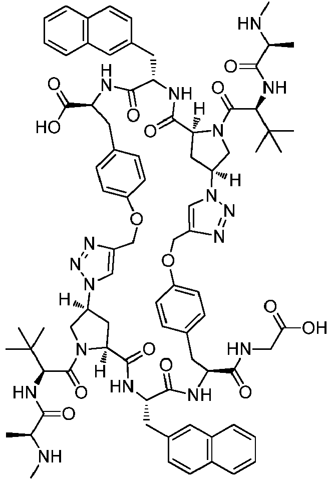 | 1562.8 | 1562.65 |
| 1019 | 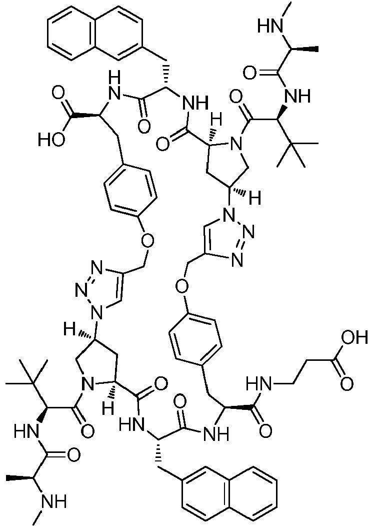 | 1576.8 | 1576.55 |
| 1020 | 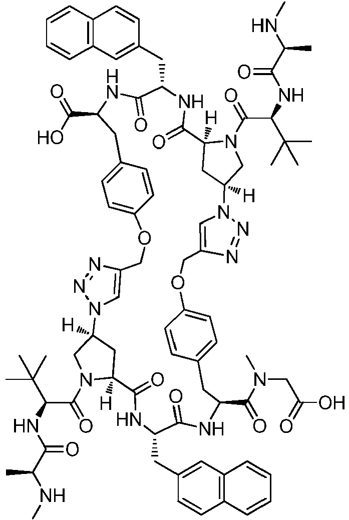 | 1576.8 | 1576.82 |
| 1021 | 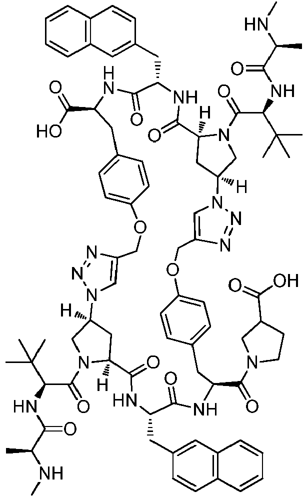 | 1602.9 | 1602.92 |
| 1022 | 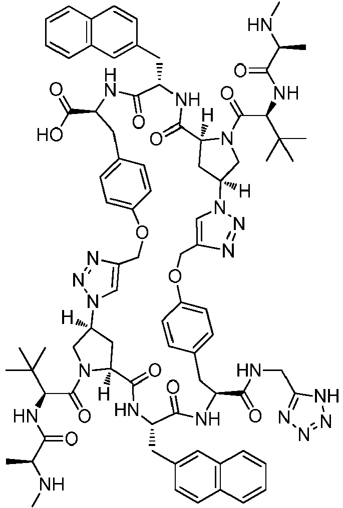 | 1586.8 | 1586.81 |
| 1023 | 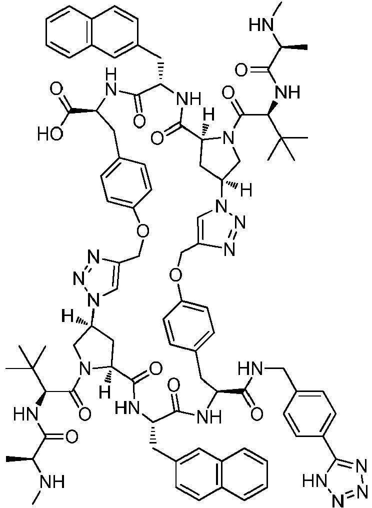 | 1662.9 | 1662.95 |
| 1024 | 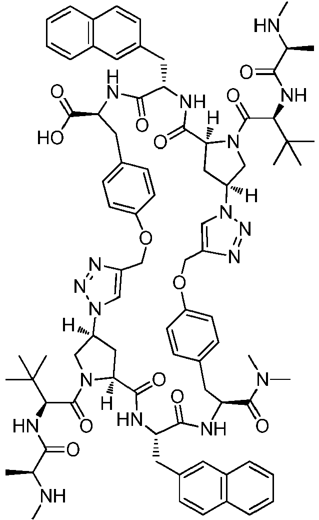 | 1532.8 | 1532.82 |
| 1025 | 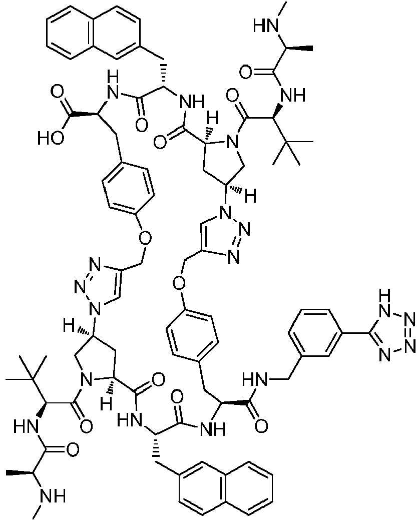 | 1662.9 | 1663.02 |
| 1026 | 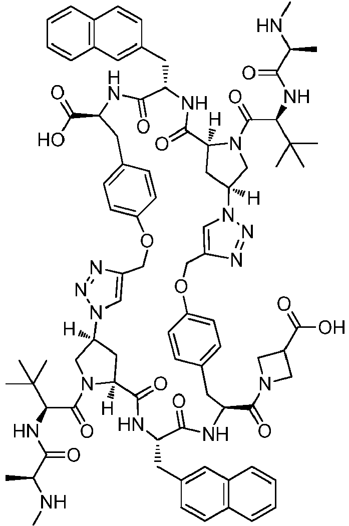 | 1588.8 | 1588.98 |
| 1027 | 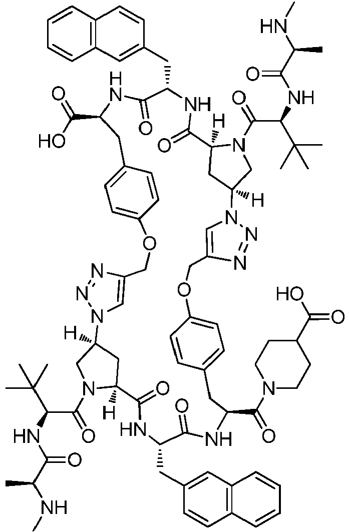 | 1616.9 | 1616.75 |
| 1028 | 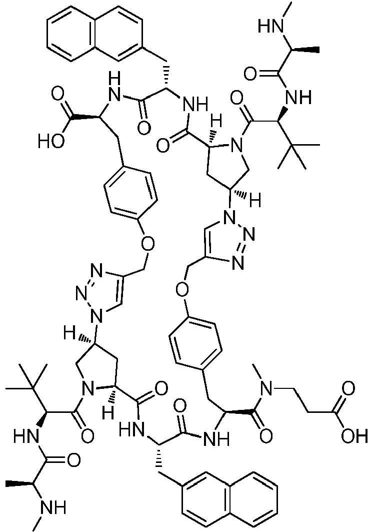 | 1590.8 | 1591.01 |
| 1029 | 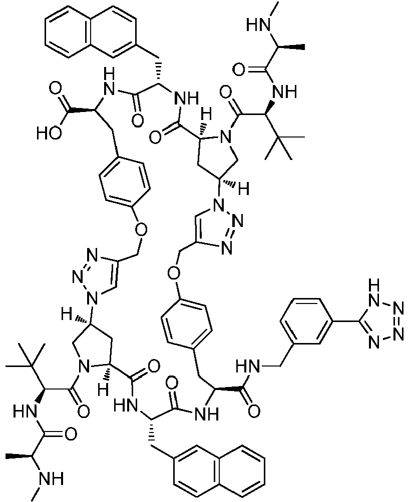 | 1662.9 | 1663.00 |
| 1030 | 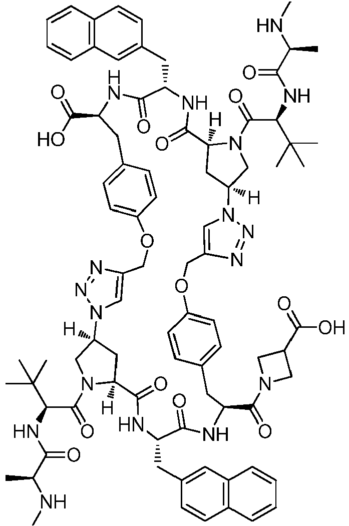 | 1588.8 | 1589.05 |
| 1031 | 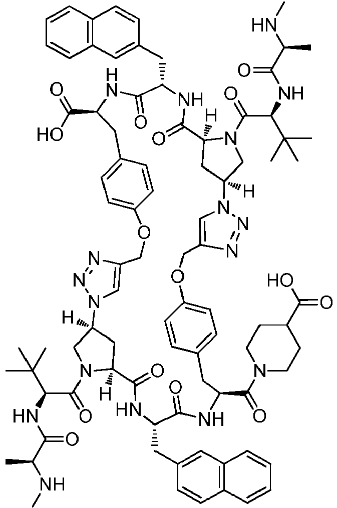 | 1616.9 | 1616.95 |
| 1032 | 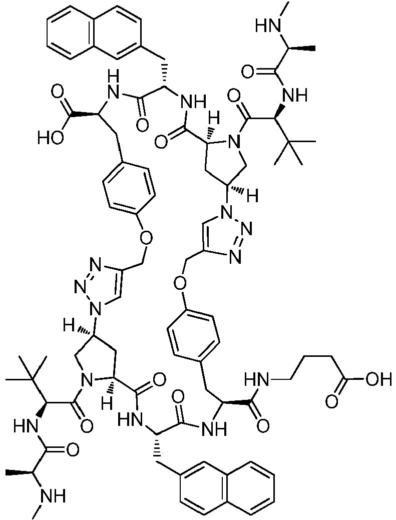 | 1590.8 | 1590.50 |
| 1033 | 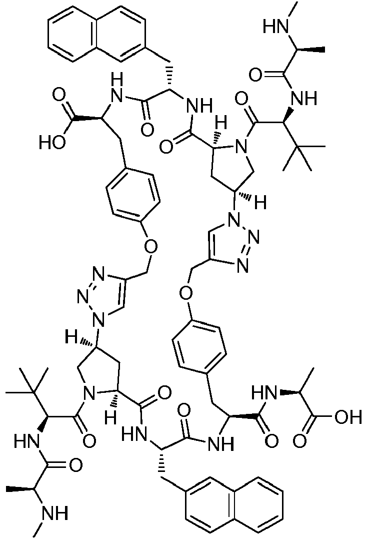 | 1576.8 | 789.15 M+2 |
| 1034 | 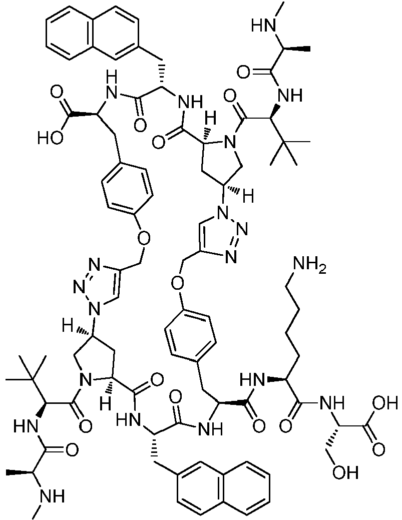 | 1720.99 | 1720.55 |
| 1035 | 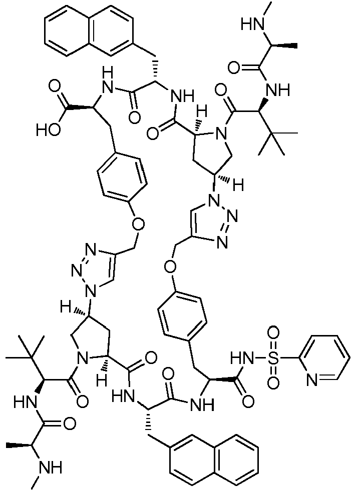 | 1645.9 | 1645.45 |
| 1036 | 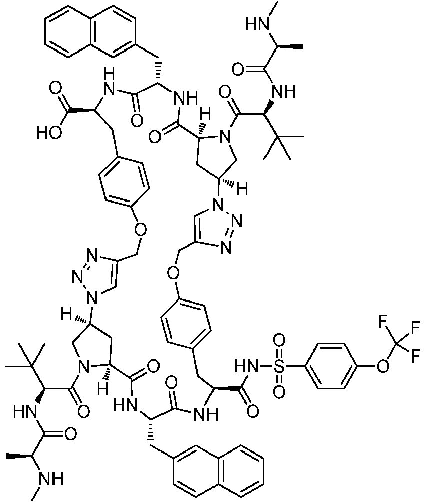 | 1728.9 | 1728.40 |
| 1037 | 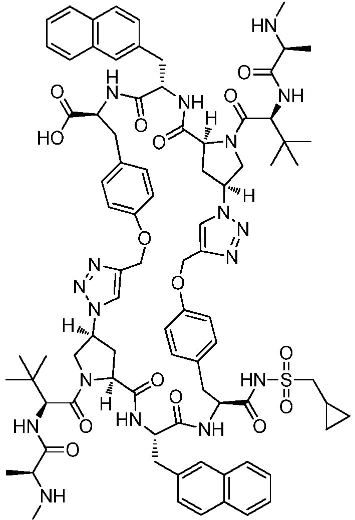 | 1622.9 | 811.84 M+2 |
| 1038 | 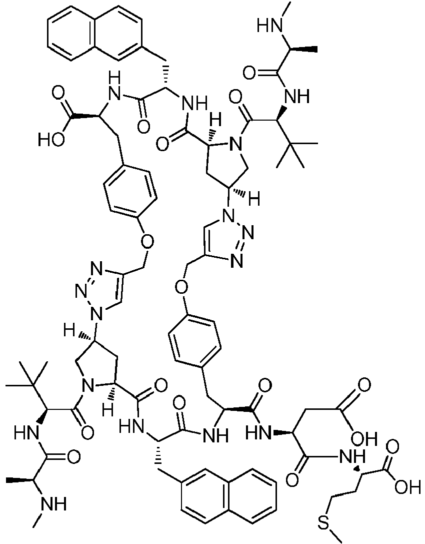 | 1752.0 | 875.85 M+2 |
| 1039 | 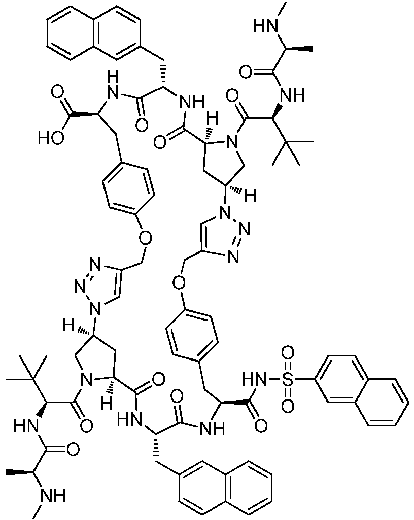 | 1694.97 | 847.85 M+2 |
| 1040 | 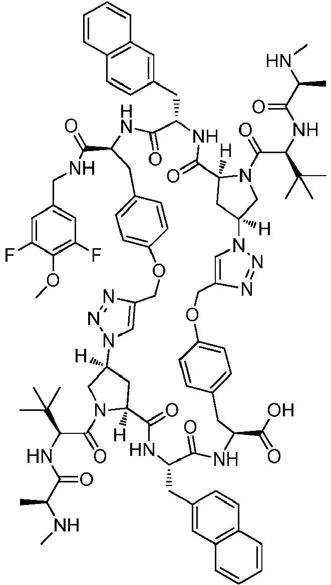 | 1660.9 | 831.4 M+2 |
| 1041 | 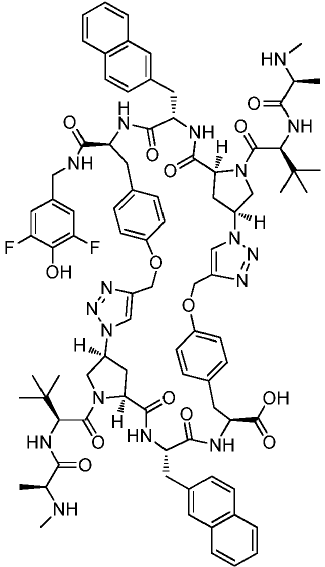 | 1646.9 | 824.1 M+2 |
| 1042 | 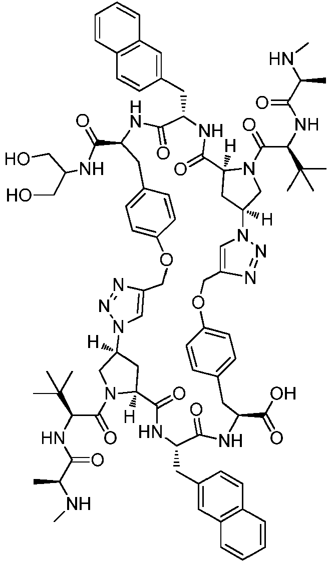 | 1578.8 | 790.6 M+2 |
| 1043 | 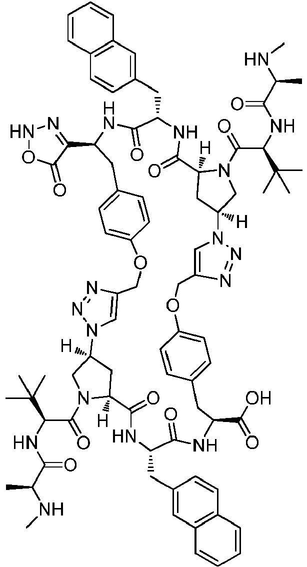 | 1545.8 | 1546.6 |
| 1044 | 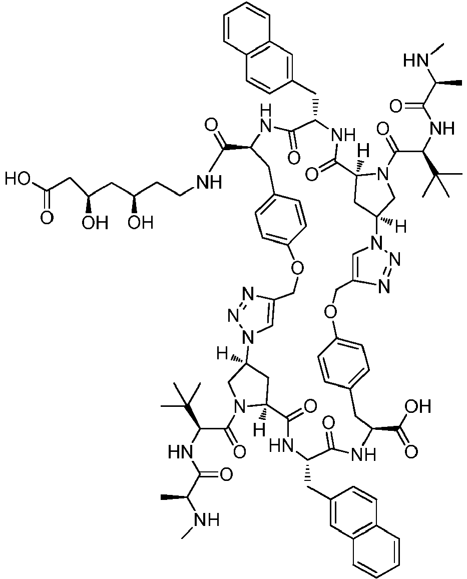 | 1664.9 | 824.1 M+2 |
| 1045 | 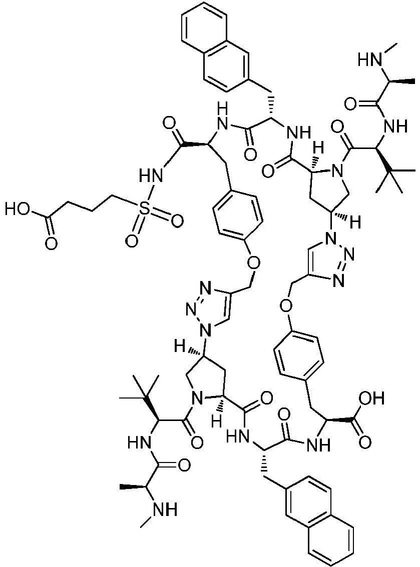 | 1654.9 | 828.5 M+2 |
| 1046 | 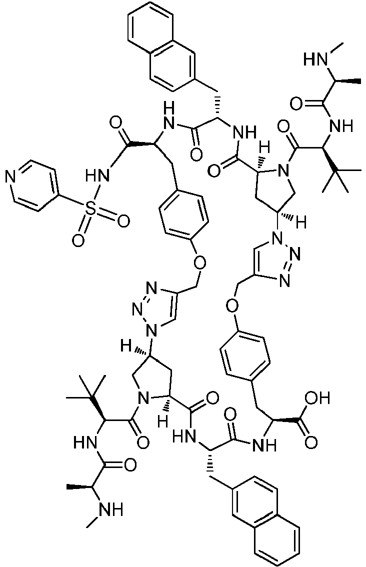 | 1645.9 | 823.6 M+2 |
| 1047 | 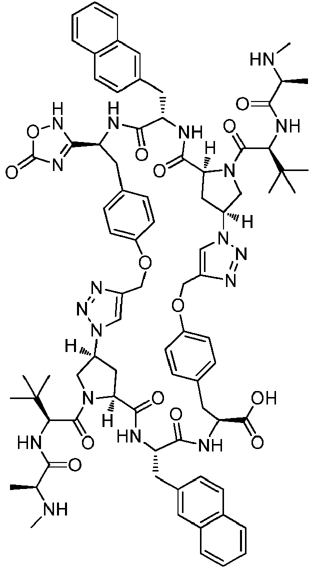 | 1545.8 | 773.8 M+2 |
| 1048 | 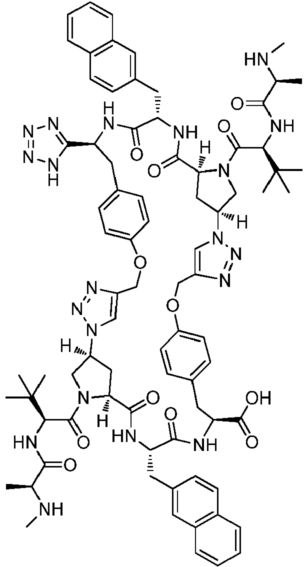 | 1529.7 | 765.7 M+2 |
| 1049 | 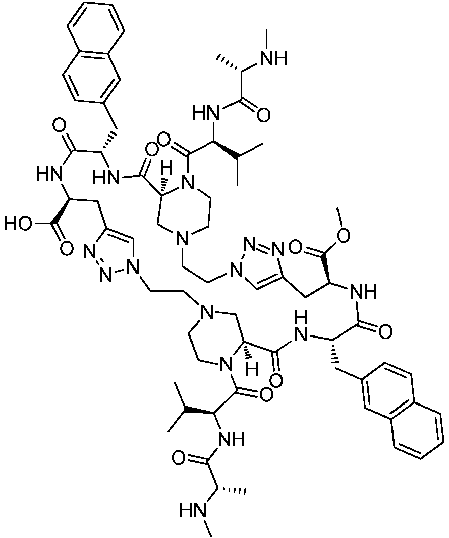 | 1365.6 | 1366.3 |
| 1050 | 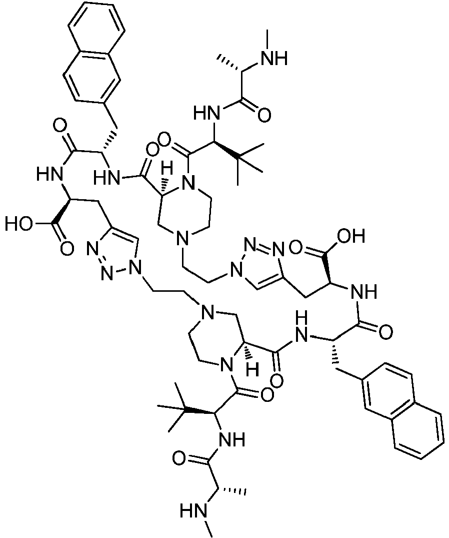 | 1379.6 | 1380.2 |
| 1051 | 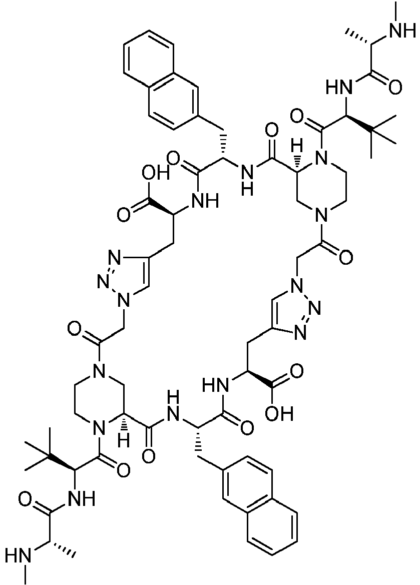 | 1407.6 | 1408.2 |
| 1052 | 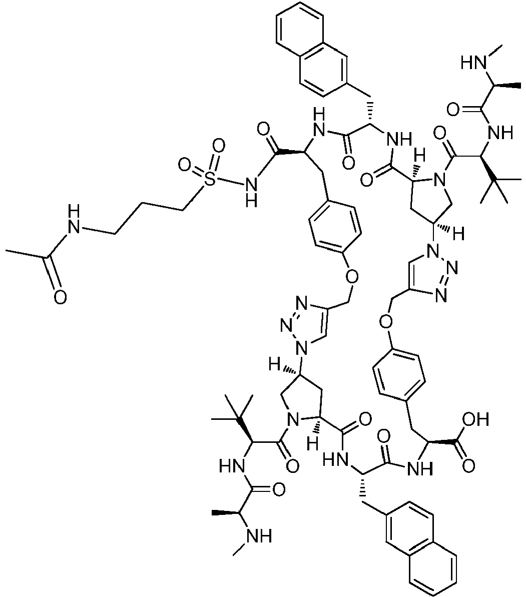 | 1667.9 | 834.8 |

In one embodiment, the compound of Formula I is selected from any one of the compounds set forth in Table 1.

The compounds of the present invention can be prepared using an iterative peptide coupling procedure as illustrated in following synthetic schemes. Exemplary general synthetic protocols are presented in Schemes 1 through 6. The schemes and accompanying description of synthetic procedures are given for the purpose of illustrating the invention, and should not be construed as limiting the scope or spirit of the invention.

Abbreviations as used herein include *O*-(7-azabenzotriazol-1-yl)-*N,N,N',N*'-tetramethyluronium hexafluorophosphate (HATU); diisopropylethylamine (DIPEA); dimethylaminopyridine (DMAP); dimethylformamide (DMF); 9-fluorenylmethoxycarbonyl (Fmoc); methanol (MeOH); methylene chloride (DCM); tert-butoxycarbonyl (Boc); *tert*-butyl (tBu); tetrahydrofuran (THF); trifluoroacetic acid (TFA); 1,8-diazobicyclo [5.4.0]-undec-7-ene (DBU); 1,1'-dicarbonyldiimidazole (CDI); 1,1'-thiocarbonyldiimidazole (TCDI), *tert*-butyldimethylsilyl (TBS); 1-ethyl-3-(3-dimethylaminopropyl)carbodiimide (EDC), 1-hydroxy-7-azabenzotriazole (HOAt), phenyl (Ph), and copper(II) (Z)-2,2,6,6-tetramethyl-5-oxohept-3-en-3-olate (Cu(thd)₂).

In each of the Schemes, each P is an independently selected protecting group, such as Fmoc or Boc. Y' is a derivative of any of the Y moieties defined in the specification, wherein the A group has been removed. The P group depicted as bonded to Y' in the schemes is bound to the free amino group present at the bond designated as "1" in Y. The alkyne depicted as bonded to Y' in the schemes is bound at the bond designated as "4" in Y. X is meant to designate any of the X moieties set forth in the specification. Any -OH group depicted as bonded to X in the schemes is bound to the terminal -C(O) of X at the bond designated as "2". Any P group depicted as bonded to X in the schemes is bound to the secondary amine residue of X at the bond designated as "1".

Scheme 1 depicts a general synthesis method for compounds of Formula I, wherein the compound is a homodimer (e.g., each R¹ is the same; each R² is the same; each R⁶ is the same; each R⁷ is the same; each R⁸ is the same; each X is the same; each Y is the same; each Z is the same; and each n is the same). In Scheme 1, each P is an independently selected protecting group, such as Fmoc or Boc; and Y' is a derivative of Y (as defined above), wherein the A group has been removed.

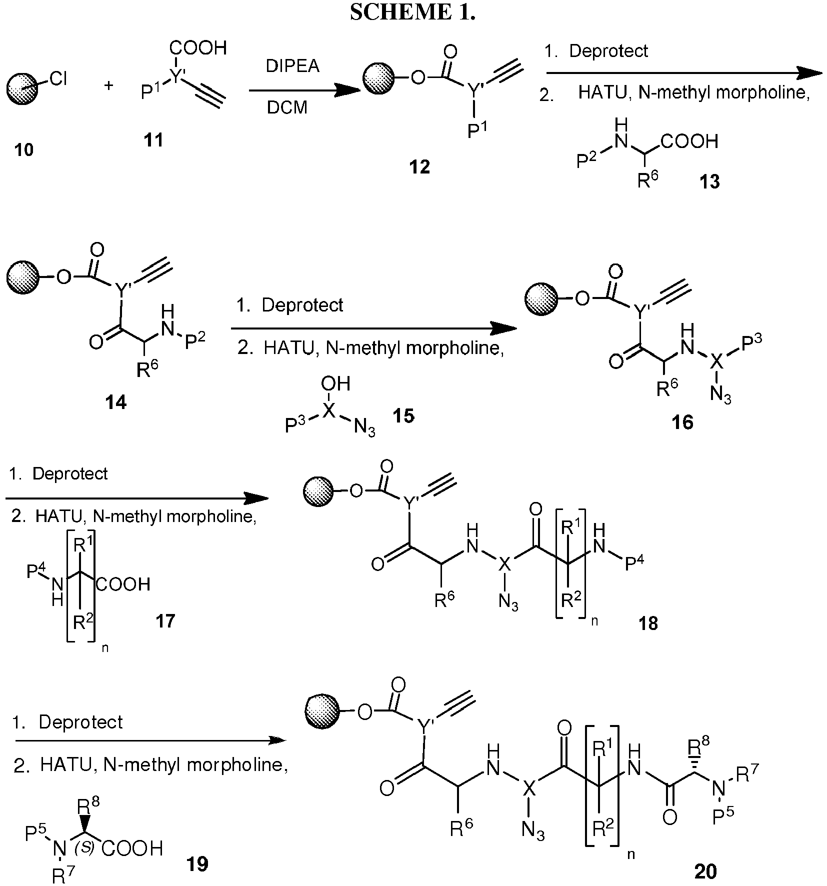

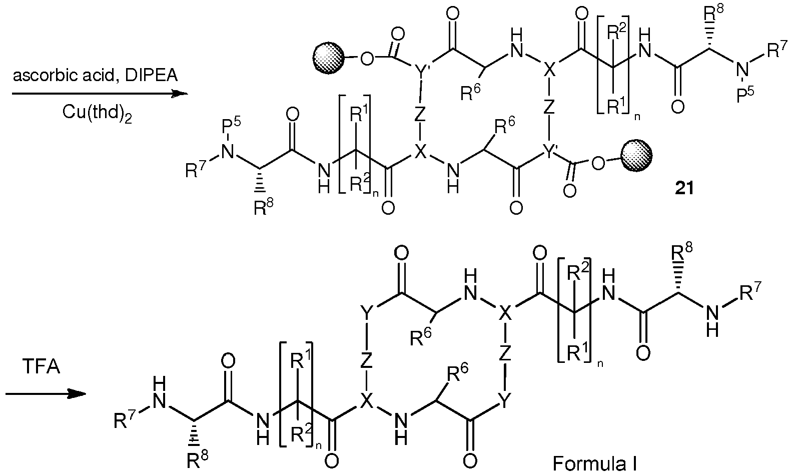

In Scheme 1, a chlorinated resin **10** is reacted with the protected alkyne derivative of Y' **11** in the presence of DCM and diisopropylethylamine to form resin-linked compound **12.** The compound **12** is deprotected (e.g., by treatment with DBU and piperidine in DMF) and then reacted with the protected amino acid **13** in the presence of HATU and N-methyl morpholine to produce resin-linked compound **14.** After deprotection, the protected azide amino acid **15** is added to produce resin-linked compound **16.** Protected amino acid **17** is added after deprotection of **16** to produce **18.** Another round of deprotection is followed by the addition of amino acid **19** to produce resin-linked compound **20.** The azide group and alkyne group are reacted with one another to form triazole Z, using a C^{u2+} reagent in the presence of DIPEA, ascorbic acid, and 2,6-dimethylpyridine to produce the resin-linked cyclic dimer **21,** which is deprotected and cleaved from the resin with TFA to produce a compound of Formula I.

Scheme 2 depicts a general synthesis method for compounds of Formula I, wherein the compound is a heterodimer. In Scheme 2, each P is an independently selected protecting group, such as Fmoc or Boc, but in this scheme P⁵ must be different and differentially cleavable from P¹ (e.g., one is Fmoc and the other is Boc); and Y' is a derivative of Y (as defined above), wherein the A group has been removed.

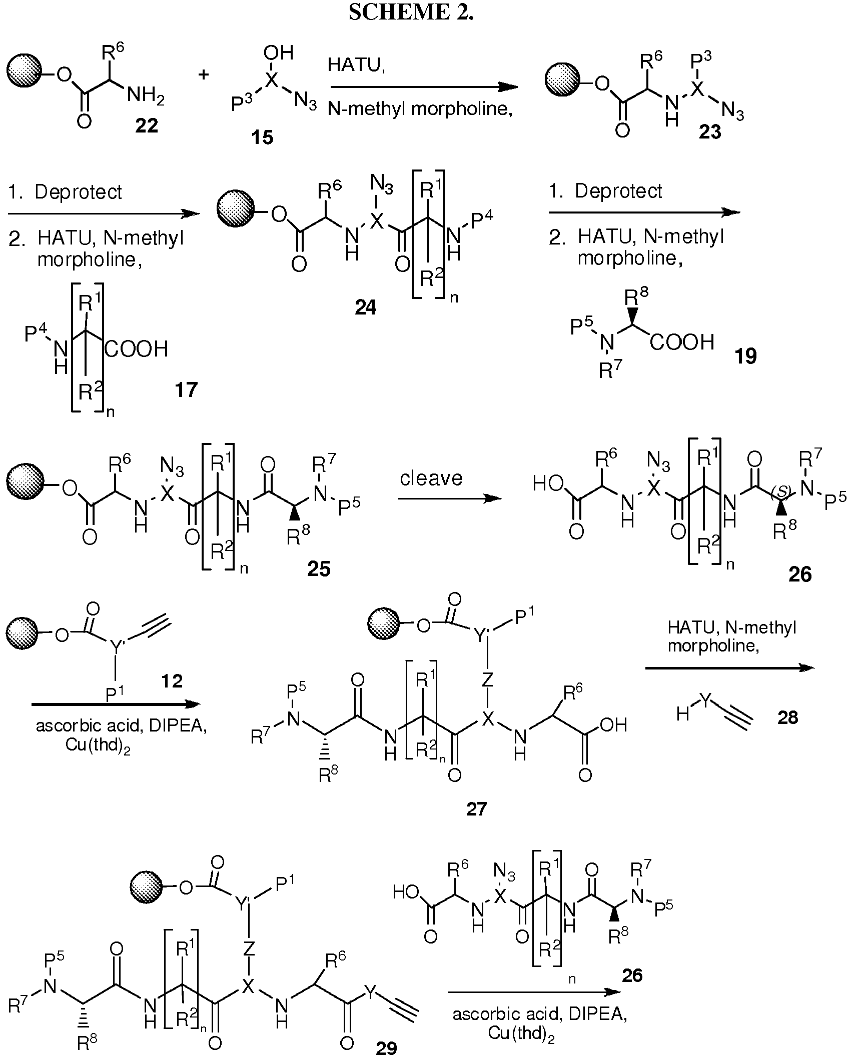

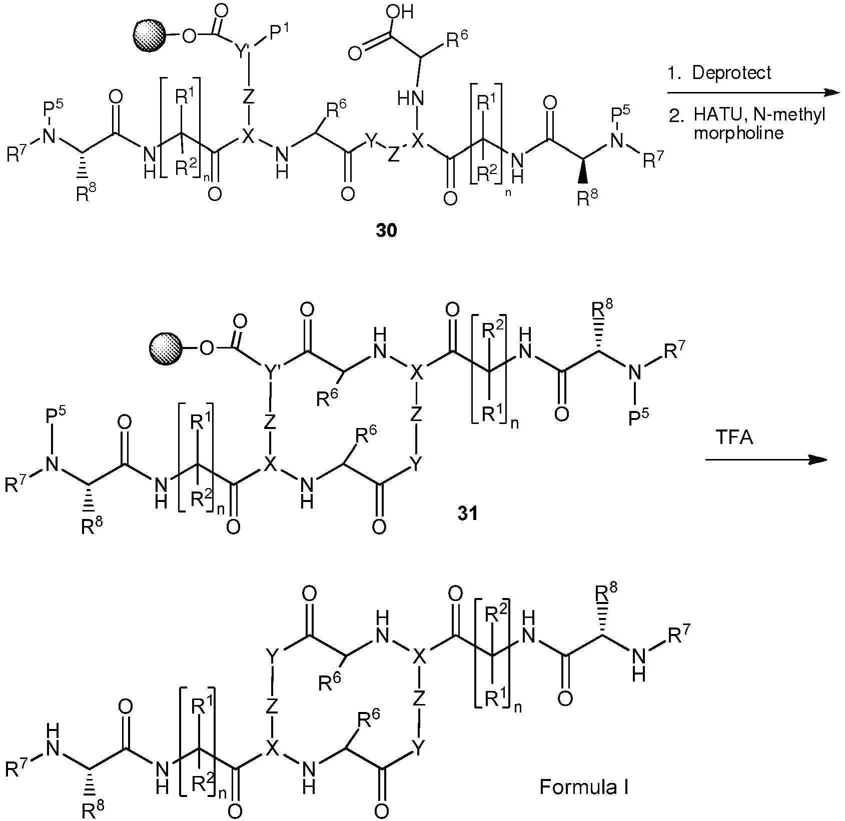

In Scheme 2, a commercially available resin-linked amino acid **22** is reacted with a protected azide amino acid **15** in the presence of HATU and N-methyl morpholine to produce resin-linked compound **23.** Alternatively, a chlorinated resin **10** may be coupled to an amino acid to produce **23.** After deprotection of **23,** protected amino acid **17** is added in the presence of HATU and N-methyl morpholine to form resin-linked compound **24.** Protected amino acid **19** is added in the same manner to produce resin-linked compound **25,** which is then cleaved from the resin to produce the amino-protected, azide-containing peptide **26.**

Compound **26** is then reacted with a resin-linked, amino-protected alkyne **12** in the presence of a Cu²⁺ reagent, DIPEA, ascorbic acid, and 2,6-dimethylpyridine to form resin-linked triazole compound **27.** Compound **27** is then coupled to an alkyne derivative of Y **28** to form resin-linked compound **29.** Compound **29** is then reacted with amino acid-protected, azide-containing peptide **26** in the presence of a Cu²⁺ reagent, DIPEA, ascorbic acid, and 2,6-dimethylpyridine to form resin-linked triazole compound **30,** which is then deprotected to remove P¹, but not P⁵, and cyclized with HATU and N-methyl morpholine. The resulting resin-linked compound **31** is then treated with TFA to remove P⁵ and cleave the compound from the resin to produce a compound of Formula I

Scheme 3 depicts a general synthesis for compounds of Formula I, wherein the compound is either a homodimer or heterodimer. In Scheme 3, each P is an independently selected protecting group, such as Fmoc or Boc; and Y' is a derivative of Y (as defined above), wherein the A group has been removed. Compound **32,** which can be derived by the methods depicted in Schemes 1 or 2, is reacted with 1,1'-dicarbonyldiimidazole (CDI), followed by an appropriately substituted sulfonamide **33** in the presence of DBU to give a separable mixture of mono- and bisacylsulfonamide derivatives **34** and **35.** Removal of the P⁵ protecting group of **34** and **35,** with for example, TFA affords **36** and **37,** respectively.

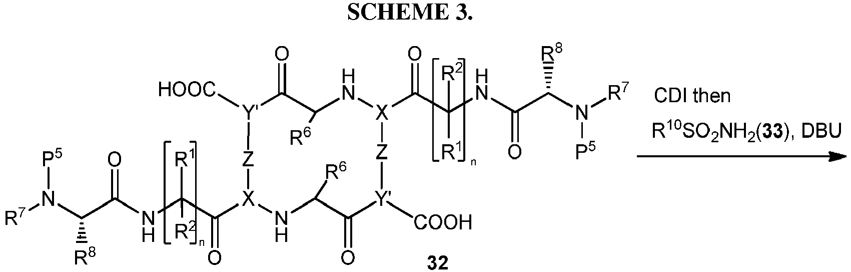

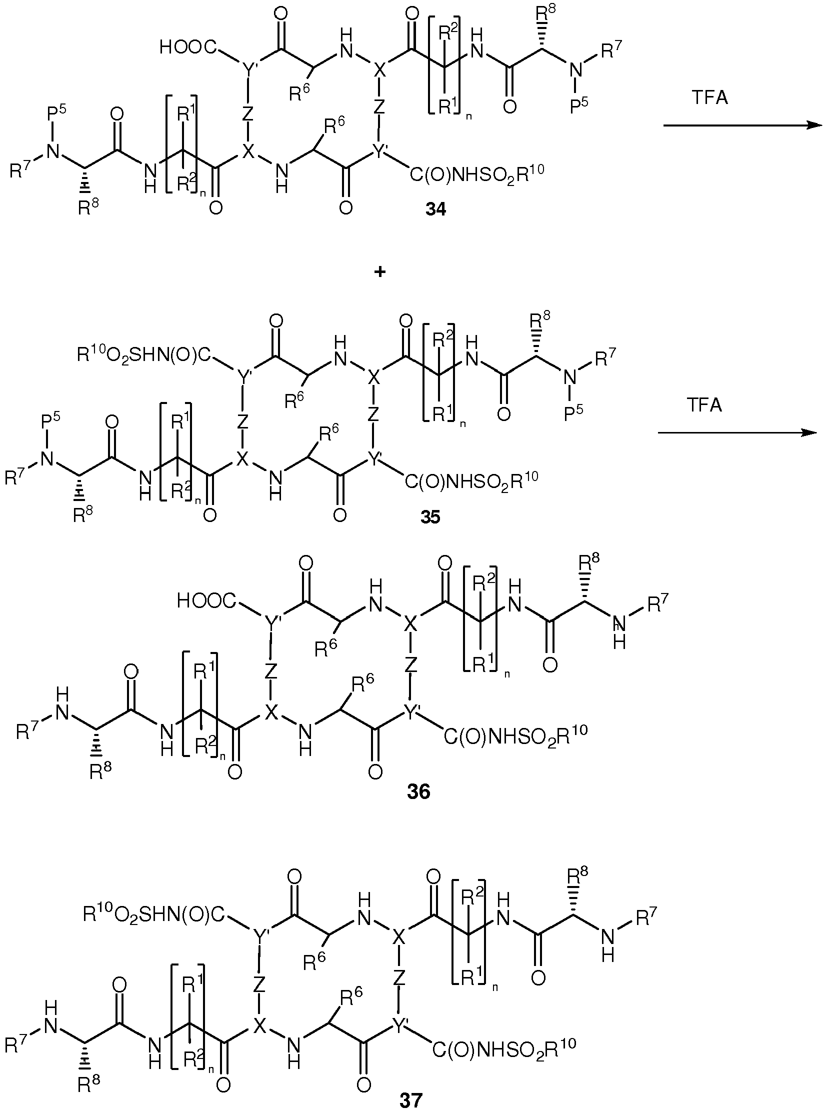

Scheme 4 depicts a general synthesis method for compounds of Formula I, wherein the compound is a heterodimer. In Scheme 4, each P is an independently selected protecting group, such that P¹, P⁵ and P⁶ are different and differentially cleavable (e.g., one is Fmoc, one is Boc, and the other is TBS). Y' is a derivative of Y (as defined above), wherein the A group has been removed.

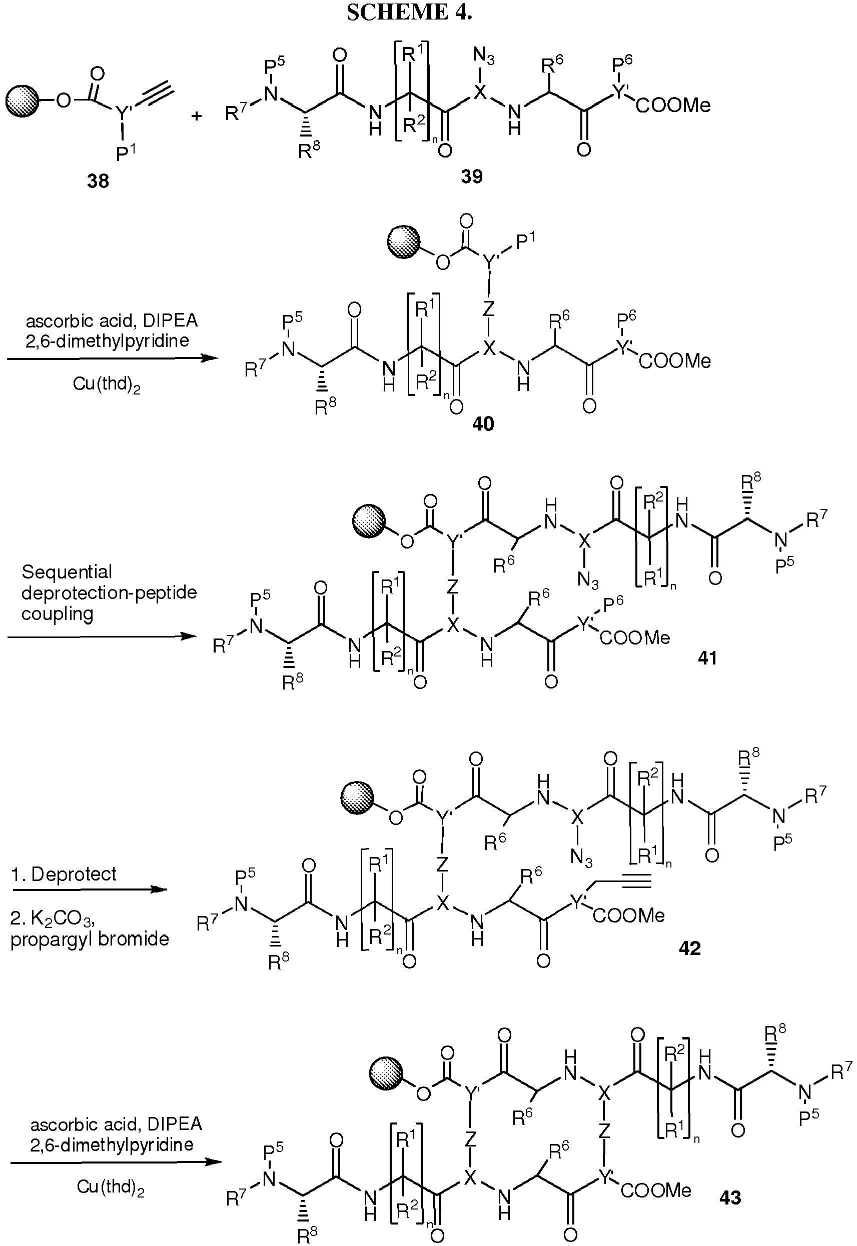

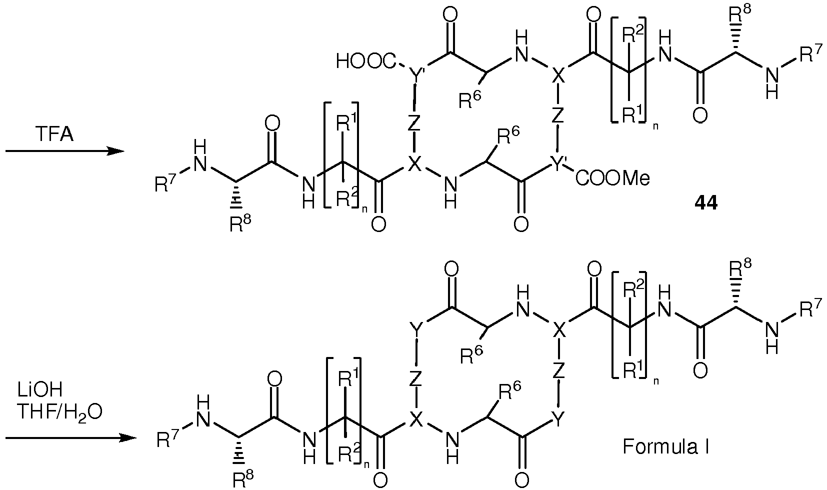

In Scheme 4, linear peptide **39** can be prepared using standard peptide coupling methods (EDC, HOAt, Boc-protected amino acid) and a suitable solvent (e.g., DMF). A resin-linked, amino-protected alkyne **38** is reacted with compound **39** in the presence of a Cu²⁺ reagent, DIPEA, ascorbic acid and 2,6-dimethylpyridine to form resin-linked triazole compound **40.** Intermediate **40** is then sequentially coupled with suitably protected amino acids to give resin-linked peptide compound **41.** Remove of P⁶ in the presence of P⁵, followed by alkylation with propargyl bromide provides resin-linked alkyne **42.** Cyclization of **42** in the presence of a Cu²⁺ reagent, DIPEA, ascorbic acid and 2,6-dimethylpyridine provides resin-linked macrocycle **43.** Removal of the P⁵ group of **43,** with for example TFA furnishes monoester **44.** Based-promoted hydrolysis of **44,** with, for example lithium hydroxide affords a compound of Formula I.

Scheme 5 depicts a general synthesis method for compounds of Formula **I,** wherein the compound is a homodimer and A is a carboxylic acid isostere such as a tetrazole. In Scheme 5, each P is an independently selected protecting group, such as Fmoc or Boc; and Y' is a derivative of Y (as defined above), wherein the A group has been removed.

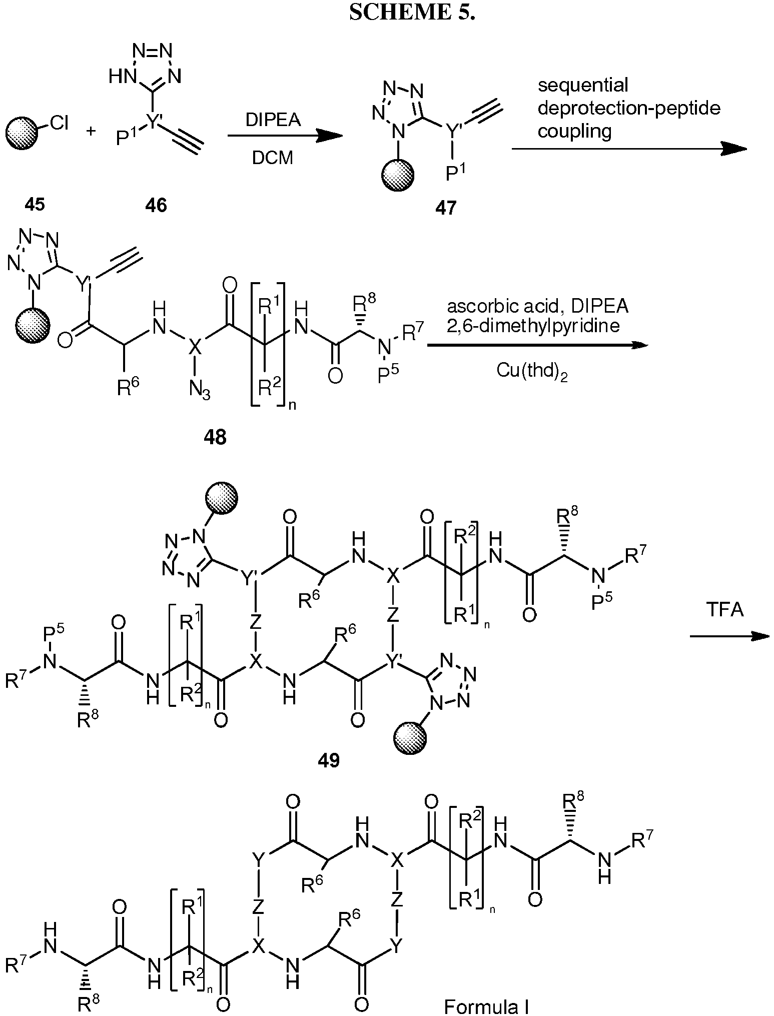

In Scheme 5, a chlorinated resin **45** is reacted with the protected tetrazole derivative of Y' **46** in the presence of dichloromethane and diisopropylethylamine to form resin-linked compound **47.** Several rounds of sequential deprotection and peptide coupling reactions are performed to produce resin-linked compound **48.** Copper(II) -promoted formation of the triazole Z in the presence of DIPE, ascorbic acid, and 2,6-dimethylpyridine led to the resin-linked cyclic dimer **49.** Intermediate **49** can then be deprotected and cleaved from the resin with, for example, TFA to produce a compound of Formula I.

The synthesis method depicted in Scheme 5 can also be utilized to produce compounds of Formula I with alternative carboxylic acid isosteres by substituting intermediate **46** with amino acid derivatives, such as compounds **53, 54, 57, 59, 60, 63, 66,** and **70** (Scheme 6). These intermediates can be prepared according to general methods known to one skilled in the art, including those found in the following references: Synlett, 2007, 17, 2643; Tetrahedron, 2009, 65, 9536; Tetrahedron Lett. 2005, 46, 311***;*** Tetrahedron Lett, 2007, 48, 1479.

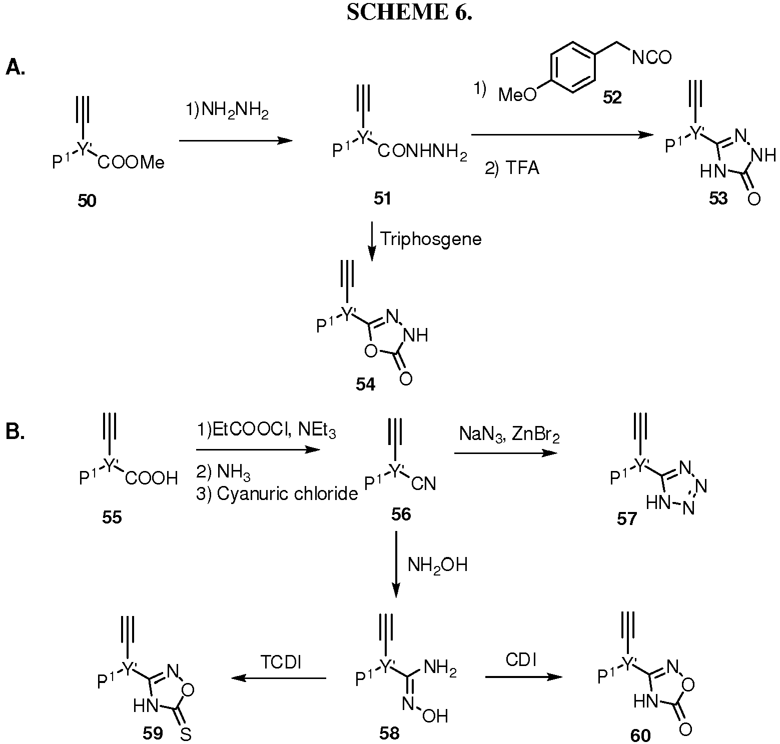

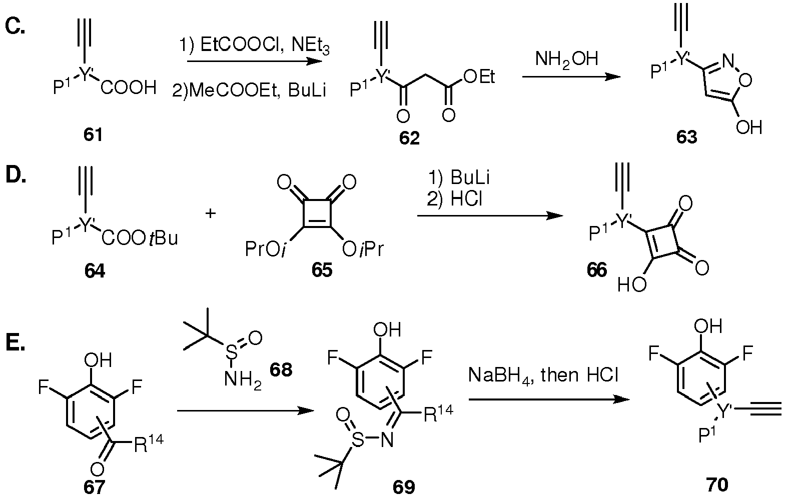

### II. Therapeutic Applications

The compounds and pharmaceutical compositions of the present invention are useful in treating or preventing any disease or conditions that are sensitive to apoptosis. These include infections (e.g. skin infections, GI infection, urinary tract infections, genito-urinary infections, systemic infections), proliferative diseases (e.g., cancer), and autoimmune diseases (e.g., rheumatoid arthritis, lupus). In certain embodiments, the compound or pharmaceutical composition is administered orally. In other embodiments, the compound or pharmaceutical composition is administered parenterally.

In one embodiment, the compounds of this invention can be used for the treatment of any cancer type that fails to undergo apoptosis in a patient. This includes, but is not limited to: solid tumors, including but not limited to carcinomas; sarcomas including Kaposi's sarcoma; erythroblastoma; glioblastoma; meningioma; astrocytoma; melanoma; and myoblastoma. Treatment or prevention of non-solid tumor cancers, such as leukemia, is also contemplated by this invention.

Types of cancers that may be treated with the compounds of this invention include, but are not limited to, brain cancers, skin cancers, bladder cancers, ovarian cancers, breast cancers, gastric cancers, pancreatic cancers, prostate cancers, colon cancers, blood cancers, lung cancers and bone cancers. Examples of such cancer types include neuroblastoma, intestine carcinoma such as rectum carcinoma, colon carcinoma, familiar adenomatous polyposis carcinoma and hereditary non-polyposis colorectal cancer, esophageal carcinoma, labial carcinoma, larynx carcinoma, hypopharynx carcinoma, tong carcinoma, salivary gland carcinoma, gastric carcinoma, adenocarcinoma, medullary thyroid carcinoma, papillary thyroid carcinoma, renal carcinoma, kidney parenchymal carcinoma, ovarian carcinoma, cervix carcinoma, uterine corpus carcinoma, endometrium carcinoma, chorion carcinoma, pancreatic carcinoma, prostate carcinoma, testis carcinoma, breast carcinoma, urinary carcinoma, melanoma, brain tumors such as glioblastoma, astrocytoma, meningioma, medulloblastoma and peripheral neuroectodermal tumors, Hodgkin lymphoma, non-Hodgkin lymphoma, Burkitt lymphoma, acute lymphatic leukemia (ALL), chronic lymphatic leukemia (CLL), acute myeloid leukemia (AML), chronic myeloid leukemia (CML), adult T-cell leukemia lymphoma, diffuse large B-cell lymphoma (DLBCL), hepatocellular carcinoma, gall bladder carcinoma, bronchial carcinoma, small cell lung carcinoma, non-small cell lung carcinoma, multiple myeloma, basalioma, teratoma, retinoblastoma, choroid melanoma, seminoma, rhabdomyosarcoma, craniopharyngioma, osteosarcoma, chondrosarcoma, myosarcoma, liposarcoma, fibrosarcoma, Ewing sarcoma and plasmocytoma.

In addition to apoptosis defects found in tumors, defects in the ability to eliminate self- reactive cells of the immune system due to apoptosis resistance are considered to play a key role in the pathogenesis of autoimmune diseases. Autoimmune diseases are characterized in that the cells of the immune system produce antibodies against its own organs and molecules or directly attack tissues resulting in the destruction of the latter. A failure of those self-reactive cells to undergo apoptosis leads to the manifestation of the disease. Defects in apoptosis regulation have been identified in autoimmune diseases such as systemic lupus erythematosus or rheumatoid arthritis.

Thus, according to another embodiment, the invention relates to the treatment of autoimmune diseases. Examples of such autoimmune diseases include, but are not limited to, collagen diseases such as rheumatoid arthritis, systemic lupus erythematosus. Sharp's syndrome, CREST syndrome (calcinosis, Raynaud's syndrome, esophageal dysmotility, telangiectasia), dermatomyositis, vasculitis (Morbus Wegener's) and Sjogren's syndrome, renal diseases such as Goodpasture's syndrome, rapidly-progressing glomerulonephritis and membrano-proliferative glomerulonephritis type II, endocrine diseases such as type-I diabetes, autoimmune polyendocrinopathy-candidiasis-ectodermal dystrophy (APECED), autoimmune parathyroidism, pernicious anemia, gonad insufficiency, idiopathic Morbus Addison's, hyperthyreosis, Hashimoto's thyroiditis and primary myxedema, skin diseases such as pemphigus vulgaris, bullous pemphigoid, herpes gestationis, epidermolysis bullosa and erythema multiforme major, liver diseases such as primary biliary cirrhosis, autoimmune cholangitis, autoimmune hepatitis type-1, autoimmune hepatitis type-2, primary sclerosing cholangitis, neuronal diseases such as multiple sclerosis, myasthenia gravis, myasthenic Lambert-Eaton syndrome, acquired neuromyotomy, Guillain-Barre syndrome (Muller-Fischer syndrome), stiff-man syndrome, cerebellar degeneration, ataxia, opsoclonus, sensoric neuropathy and achalasia, blood diseases such as autoimmune hemolytic anemia, idiopathic thrombocytopenic purpura (Morbus Werlhof), infectious diseases with associated autoimmune reactions such as AIDS, Malaria and Chagas disease.

Compounds of the invention are useful for sensitizing cells to apoptotic signals. Thus, in one embodiment, the compounds of the invention are coadministered with radiation therapy or a second therapeutic agent with cytostatic or antineoplastic activity. Suitable cytostatic chemotherapy compounds include, but are not limited to (i) antimetabolites, such as cytarabine, fludarabine, 5- fluoro-2'-deoxyuiridine, gemcitabine, hydroxyurea or methotrexate; (ii) DNA-fragmenting agents, such as bleomycin, (iii) DNA-crosslinking agents, such as chlorambucil, cisplatin, cyclophosphamide or nitrogen mustard; (iv) intercalating agents such as adriamycin (doxorubicin) or mitoxantrone; (v) protein synthesis inhibitors, such as L-asparaginase, cycloheximide, puromycin or diphtheria toxin; (vi) topoisomerase I poisons, such as camptothecin or topotecan; (vii) topoisomerase II poisons, such as etoposide (VP-16) or teniposide; (viii) microtubule-directed agents, such as colcemid, colchicine, paclitaxel, vinblastine or vincristine; (ix) kinase inhibitors such as flavopiridol, staurosporin, STI571 (CPG 57148B) or UCN-O1 (7-hydroxystaurosporine); (x) miscellaneous investigational agents such as thioplatin, PS-341, phenylbutyrate, ET-18- OCH3, or farnesyl transferase inhibitors (L-739749, L-744832); polyphenols such as quercetin, resveratrol, piceatannol, epigallocatechin gallate, theaflavins, flavanols, procyanidins, betulinic acid and derivatives thereof; (xi) hormones such as glucocorticoids or fenretinide; and (xii) hormone antagonists, such as tamoxifen, finasteride or LHRH antagonists. In one aspect of this embodiment, compounds of the present invention are coadministered with a cytostatic compound selected from the group consisting of cisplatin, doxorubicin, taxol, taxotere and mitomycin C. In a more specific aspect, the cytostatic compound is doxorubicin.

The combination therapy is intended to embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single dosage form having a fixed ratio of each therapeutic agent or in multiple, single dosage forms for each of the therapeutic agents. Sequential or substantially simultaneous administration of each therapeutic agent can be effected by any appropriate route including, but not limited to, oral routes, intravenous routes, intramuscular routes, and direct absorption through mucous membrane tissues. The therapeutic agents can be administered by the same route or by different routes. For example, a first therapeutic agent of the combination selected may be administered by intravenous injection while the other therapeutic agents of the combination may be administered orally. Alternatively, for example, all therapeutic agents may be administered orally or all therapeutic agents may be administered by intravenous injection. Combination therapy also can embrace the administration of the therapeutic agents as described above in further combination with other biologically active ingredients and non-drug therapies (e.g., surgery or radiation treatment.) Where the combination therapy further comprises a non-drug treatment, the non-drug treatment may be conducted at any suitable time so long as a beneficial effect from the co-action of the combination of the therapeutic agents and non-drug treatment is achieved. For example, in appropriate cases, the beneficial effect is still achieved when the non-drug treatment is temporally removed from the administration of the therapeutic agents, perhaps by days or even weeks.

### III. Pharmaceutical Compositions and Dosing

The invention also provides pharmaceutically acceptable compositions which comprise a therapeutic ally-effective amount of one or more of the macrocyclic compounds of Formula I, formulated together with one or more pharmaceutically acceptable carriers (additives) and/or diluents, and optionally, one or more additional therapeutic agents described above. As described in detail below, the pharmaceutical compositions of the present invention may be specially formulated for administration in solid or liquid form, including those adapted for the following: (1) oral administration, for example, drenches (aqueous or non-aqueous solutions or suspensions), tablets, e.g., those targeted for buccal, sublingual, and systemic absorption, boluses, powders, granules, pastes for application to the tongue; (2) parenteral administration, for example, by subcutaneous, intramuscular, intravenous or epidural injection as, for example, a sterile solution or suspension, or sustained-release formulation; (3) topical application, for example, as a cream, ointment, or a controlled-release patch or spray applied to the skin; (4) intravaginally or intrarectally, for example, as a pessary, cream or foam; (5) sublingually; (6) ocularly; (7) transdermally; or (8) nasally.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically-acceptable carrier" as used herein means a pharmaceutically-acceptable material, composition or vehicle, such as a liquid or solid filler, diluent, excipient, manufacturing aid (e.g., lubricant, talc magnesium, calcium or zinc stearate, or steric acid), or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; and (22) other non-toxic compatible substances employed in pharmaceutical formulations.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Formulations of the present invention include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient which can be combined with a carrier material to produce a single dosage form will generally be that amount of the compound which produces a therapeutic effect. Generally, out of one hundred percent, this amount will range from about 0.1 percent to about ninety-nine percent of active ingredient, preferably from about 5 percent to about 70 percent, most preferably from about 10 percent to about 30 percent.

In certain embodiments, a formulation of the present invention comprises an excipient selected from the group consisting of cyclodextrins, celluloses, liposomes, micelle forming agents, e.g., bile acids, and polymeric carriers, e.g., polyesters and polyanhydrides; and a compound of the present invention. In certain embodiments, an aforementioned formulation renders orally bioavailable a compound of the present invention.

Methods of preparing these formulations or compositions include the step of bringing into association a compound of the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A compound of the present invention may also be administered as a bolus, electuary or paste.

In solid dosage forms of the invention for oral administration (capsules, tablets, pills, dragees, powders, granules, trouches and the like), the active ingredient is mixed with one or more pharmaceutically-acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds and surfactants, such as poloxamer and sodium lauryl sulfate; (7) wetting agents, such as, for example, cetyl alcohol, glycerol monostearate, and non-ionic surfactants; (8) absorbents, such as kaolin and bentonite clay; (9) lubricants, such as talc, calcium stearate, magnesium stearate, solid polyethylene glycols, sodium lauryl sulfate, zinc stearate, sodium stearate, stearic acid, and mixtures thereof; (10) coloring agents; and (11) controlled release agents such as crospovidone or ethyl cellulose. In the case of capsules, tablets and pills, the pharmaceutical compositions may also comprise buffering agents. Solid compositions of a similar type may also be employed as fillers in soft and hard-shelled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be formulated for rapid release, e.g., freeze-dried. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions of the invention for rectal or vaginal administration may be presented as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active compound.

Formulations of the present invention which are suitable for vaginal administration also include pessaries, tampons, creams, gels, pastes, foams or spray formulations containing such carriers as are known in the art to be appropriate.

Dosage forms for the topical or transdermal administration of a compound of this invention include powders, sprays, ointments, pastes, creams, lotions, gels, solutions, patches and inhalants. The active compound may be mixed under sterile conditions with a pharmaceutically-acceptable carrier, and with any preservatives, buffers, or propellants which may be required.

The ointments, pastes, creams and gels may contain, in addition to an active compound of this invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of this invention, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of a compound of the present invention to the body. Such dosage forms can be made by dissolving or dispersing the compound in the proper medium. Absorption enhancers can also be used to increase the flux of the compound across the skin. The rate of such flux can be controlled by either providing a rate controlling membrane or dispersing the compound in a polymer matrix or gel.

Ophthalmic formulations, eye ointments, powders, solutions and the like, are also contemplated as being within the scope of this invention.

Pharmaceutical compositions of this invention suitable for parenteral administration comprise one or more compounds of the invention in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain sugars, alcohols, antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms upon the subject compounds may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally-administered drug form is accomplished by dissolving or suspending the drug in an oil vehicle.

Injectable depot forms are made by forming microencapsuled matrices of the subject compounds in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissue.

When the compounds of the present invention are administered as pharmaceuticals, to humans and animals, they can be given per se or as a pharmaceutical composition containing, for example, 0.1 to 99% (more preferably, 10 to 30%) of active ingredient in combination with a pharmaceutically acceptable carrier.

Regardless of the route of administration selected, the compounds of the present invention, which may be used in a suitable hydrated form, and/or the pharmaceutical compositions of the present invention, are formulated into pharmaceutically-acceptable dosage forms by conventional methods known to those of skill in the art.

Actual dosage levels of the active ingredients in the pharmaceutical compositions of this invention may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular compound of the present invention employed, or the ester, salt or amide thereof, the route of administration, the time of administration, the rate of excretion or metabolism of the particular compound being employed, the rate and extent of absorption, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved.

In general, a suitable daily dose of a compound of the invention will be that amount of the compound which is the lowest dose effective to produce a therapeutic effect. Such an effective dose will generally depend upon the factors described above. Generally, oral, intravenous, intracerebroventricular and subcutaneous doses of the compounds of this invention for a patient will range from about 0.01 to about 50 mg per kilogram of body weight per day.

If desired, the effective daily dose of the active compound may be administered as two, three, four, five, six or more sub-doses administered separately at appropriate intervals throughout the day, optionally, in unit dosage forms. In certain aspects of the invention, dosing is one administration per day.

While it is possible for a compound of the present invention to be administered alone, it is preferable to administer the compound as a pharmaceutical formulation (composition).

### IV. Definitions

To facilitate an understanding of the present invention, a number of terms and phrases are defined below.

The term "alkyl" is art-recognized and refers to a saturated straight or branched hydrocarbon, such as a straight or branched group of 1-12, 1-10, or 1-6 carbon atoms, referred to herein as C₁-C₁₂alkyl, C₁-C₁₀alkyl, and C₁-C₆alkyl, respectively. Exemplary alkyl groups include, but are not limited to, methyl, ethyl, propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, pentyl, isopentyl, neopentyl, hexyl, heptyl, octyl, etc.

The term "cycloalkyl" is art-recognized and refers to a monovalent saturated cyclic, bicyclic, or bridged cyclic (e.g., adamantyl) hydrocarbon group of 3-10, 3-8, 4-8, or 4-6 carbons, referred to herein, e.g., as "C₄₋₈cycloalkyl," derived from a cycloalkane. Exemplary cycloalkyl groups include, but are not limited to, cyclohexane, cyclopentane, cyclobutane, and cyclopropane.

The term "substituted" refers to the replacement of a hydrogen atom in a moiety with a functional group. Unless otherwise indicated, an "optionally substituted" group may have a suitable substituent at each substitutable position of the group, and when more than one position in any given structure may be substituted with more than one substituent selected from a specified group, the substituent may be either the same or different at each position. Combinations of substituents envisioned under this invention are preferably those that result in the formation of stable or chemically feasible compounds. The term "stable", as used herein, refers to compounds that are not substantially altered when subjected to conditions to allow for their production, detection, and, in certain embodiments, their recovery, purification, and use for one or more of the purposes disclosed herein.

Suitable monovalent substituents on a substitutable carbon atom of an "optionally substituted" group (such as an alkyl, alkenyl, alkynyl, alkylene, alkenylene, alkynylene or the carbon atom of a carbocyclyl, aryl, heterocyclyl or heteroaryl) are independently halogen; -(CH₂)₀₋₄R°; -(CH₂)₀₋₄OR°; -O-(CH₂)₀₋₄C(O)OR°; -(CH₂)₀₋₄CH(OR°)₂; -(CH₂)₀₋₄SR°; -(CH₂)₀₋₄Ph, which may be substituted with R°; -(CH₂)₀₋₄O(CH₂)₀₋₁Ph which may be substituted with R°; -CH=CHPh, which may be substituted with R°; -NO₂; -CN; -N₃; -(CH₂)₀₋₄N(R°)₂; -(CH₂)₀₋₄N(R°)C(O)R°; -N(R°)C(S)R°; -(CH₂)₀₋₄N(R°)C(O)NR°₂; -N(R°)C(S)NR°₂; -(CH₂)₀₋₄N(R°)C(O)OR°; -N(R°)N(R°)C(O)R°; -N(R°)N(R°)C(O)NR°₂; -N(R°)N(R°)C(O)OR°; -(CH₂)₀₋₄C(O)R°; -C(S)R°; -(CH₂)₀₋₄C(O)OR°; -(CH₂)₀₋₄C(O)SR°; -(CH₂)₀₋₄C(O)OSiR°₃; -(CH₂)₀₋₄OC(O)R°; -OC(O)(CH₂)₀₋₄SR-, SC(S)SR°; -(CH₂)₀₋₄SC(O)R°; -(CH₂)₀₋₄C(O)NR°₂; -C(S)NR°₂; -C(S)SR°; -(CH₂)₀₋₄OC(O)NR°₂; -C(O)N(OR°)R°; -C(O)C(O)R°; -C(O)CH₂C(O)R°; -C(NOR°)R°; -(CH₂)₀₋₄SSR°; -(CH₂)₀₋₄S(O)₂R°; -(CH₂)₀₋₄S(O)₂OR°; -(CH₂)₀₋₄OS(O)₂R°; -S(O)₂NR°₂; -(CH₂)₀₋₄S(O)R°; -N(R°)S(O)₂NR°₂; -N(R°)S(O)₂R°; -N(OR°)R°; -C(NH)NR°₂; -P(O)₂R°; -P(O)R°₂; -OP(O)R°₂; -OP(O)(OR°)₂; -SiR°₃; -(C₁₋₄ straight or branched alkylene)O-N(R°)₂; or -(C₁₋₄ straight or branched alkylene)C(O)O-N(R°)₂, wherein each R° may be substituted as defined below and is independently hydrogen, C₁₋₆ aliphatic, -CH₂Ph, -O(CH₂)₀₋₁Ph, or a 5-6-membered saturated, partially unsaturated, or aryl ring having 0-4 heteroatoms independently selected from nitrogen, oxygen, or sulfur, or, notwithstanding the definition above, two independent occurrences of R°, taken together with their intervening atom(s), form a 3-12-membered saturated, partially unsaturated, or aryl mono- or bicyclic ring having 0-4 heteroatoms independently selected from nitrogen, oxygen, or sulfur, which may be substituted as defined below.

Suitable monovalent substituents on R° (or the ring formed by taking two independent occurrences of R° together with their intervening atoms), are independently halogen, -(CH₂)₀₋₂R^{●}, -(haloR^{●}), -(CH₂)₀₋₂OH, -(CH₂)₀₋₂OR^{●}, -(CH₂)₀₋₂CH(OR^{●})₂; -O(haloR^{●}), -CN, -N₃, -(CH₂)₀₋₂C(O)R^{●}, -(CH₂)₀₋₂C(O)OH, -(CH₂)₀₋₂C(O)OR^{●}, -(CH₂)₀₋₂SR^{●}, -(CH₂)₀₋₂SH, -(CH₂)₀₋₂NH₂, -(CH₂)₀₋₂NHR^{●}, -(CH₂)₀₋₂NR^{●}₂, -NO₂, -SiR^{●}₃, -OSiR^{●}₃, -C(O)SR^{●}, -(C₁₋₄ straight or branched alkylene)C(O)OR^{●}, or -SSR^{●} wherein each R^{●} is unsubstituted or where preceded by "halo" is substituted only with one or more halogens, and is independently selected from C₁₋₄ aliphatic, -CH₂Ph, -O(CH₂)₀₋₁Ph, or a 5-6-membered saturated, partially unsaturated, or aryl ring having 0-4 heteroatoms independently selected from nitrogen, oxygen, or sulfur. Suitable divalent substituents on a saturated carbon atom of R° include =O and =S.

Suitable divalent substituents on a saturated carbon atom of an "optionally substituted" group include the following: =O, =S, =NNR^{*}₂, =NNHC(O)R^{*}, =NNHC(O)OR^{*}, =NNHS(O)₂R^{*}, =NR^{*}, =NOR^{*}, -O(C(R^{*}₂))₂₋₃O-, or -S(C(R^{*}₂))₂₋₃S-, wherein each independent occurrence of R^{*} is selected from hydrogen, C₁₋₆ aliphatic which may be substituted as defined below, or an unsubstituted 5-6-membered saturated, partially unsaturated, or aryl ring having 0-4 heteroatoms independently selected from nitrogen, oxygen, or sulfur. Suitable divalent substituents that are bound to vicinal substitutable carbons of an "optionally substituted" group include: -O(CR^{*}₂)₂₋₃O-, wherein each independent occurrence of R^{*} is selected from hydrogen, C₁₋₆ aliphatic which may be substituted as defined below, or an unsubstituted 5-6-membered saturated, partially unsaturated, or aryl ring having 0-4 heteroatoms independently selected from nitrogen, oxygen, or sulfur.

Suitable substituents on the aliphatic group of R^{*} include halogen, -R^{●}, -(haloR^{●}), -OH, -OR^{●}, -O(haloR^{●}), -CN, -C(O)OH, -C(O)OR^{●}, -NH₂, -NHR^{●}, -NR^{●}₂, or -NO₂, wherein each R^{●} is unsubstituted or where preceded by "halo" is substituted only with one or more halogens, and is independently C₁₋₄ aliphatic, -CH₂Ph, -O(CH₂)₀₋₁Ph, or a 5-6-membered saturated, partially unsaturated, or aryl ring having 0-4 heteroatoms independently selected from nitrogen, oxygen, or sulfur.

Suitable substituents on a substitutable nitrogen of an "optionally substituted" group include -R^{†}, -NR^{†}₂, -C(O)R^{†}, -C(O)OR^{†}, -C(O)C(O)R^{†}, -C(O)CH₂C(O)R^{†}, -S(O)₂R^{†}, -S(O)₂NR^{†}₂, -C(S)NR^{†}₂, -C(NH)NR^{†}₂, or -N(R^{†})S(O)₂R^{†}; wherein each R^{†} is independently hydrogen, C₁₋₆ aliphatic which may be substituted as defined below, unsubstituted -OPh, or an unsubstituted 5-6-membered saturated, partially unsaturated, or aryl ring having 0-4 heteroatoms independently selected from nitrogen, oxygen, or sulfur, or, notwithstanding the definition above, two independent occurrences of R^{†}, taken together with their intervening atom(s) form an unsubstituted 3-12-membered saturated, partially unsaturated, or aryl mono- or bicyclic ring having 0-4 heteroatoms independently selected from nitrogen, oxygen, or sulfur.

Suitable substituents on the aliphatic group of R^{†} are independently halogen, -R^{●}, -(haloR^{●}), -OH, -OR^{●}, -O(haloR^{●}), -CN, -C(O)OH, -C(O)OR^{●}, -NH₂, -NHR^{●}, -NR^{●}₂, or -NO₂, wherein each R^{●} is unsubstituted or where preceded by "halo" is substituted only with one or more halogens, and is independently C₁₋₄aliphatic, -CH₂Ph, -O(CH₂)₀₋₁Ph, or a 5-6-membered saturated, partially unsaturated, or aryl ring having 0-4 heteroatoms independently selected from nitrogen, oxygen, or sulfur.

The term "moiety" refers to a portion of a compound of this invention comprising at least one hydrogen atom and at least one carbon atom.

The term "alkylene" refers to the diradical of an alkyl group.

The term "haloalkyl" refers to an alkyl group that is substituted with at least one halogen. For example, -CH₂F, -CHF₂, -CF₃, -CH₂CF₃, -CF₂CF₃, and the like.

The term "aralkyl" refers to an alkyl group substituted with an aryl group.

The term "heteroaralkyl" refers to an alkyl group substituted with a heteroaryl group.

The terms "alkenyl" and "alkynyl" are art-recognized and refer to unsaturated aliphatic groups analogous in length to the alkyls described above, but that contain at least one double or triple bond, respectively.

The term "aryl" is art-recognized and refers to a carbocyclic aromatic group. Representative aryl groups include phenyl, naphthyl, anthracenyl, and the like. The term "aryl" also includes polycyclic ring systems having two or more carbocyclic rings in which two or more carbons are common to two adjoining rings (the rings are "fused rings") wherein at least one of the rings is aromatic, e.g., the other cyclic rings may be cycloalkyls, cycloalkenyls, and/or aryls.

The term "arylene" refers to the diradical of an aryl group.

The term "heteroaryl" is art-recognized and refers to aromatic groups that include at least one ring heteroatom. In certain instances, a heteroaryl group contains 1, 2, 3, or 4 ring heteroatoms. Representative examples of heteroaryl groups include pyrrolyl, furanyl, thiophenyl, imidazolyl, oxazolyl, thiazolyl, triazolyl, pyrazolyl, pyridinyl, pyrazinyl, pyridazinyl and pyrimidinyl, and the like. The term "heteroaryl" also includes polycyclic ring systems having two or more rings in which two or more carbons are common to two adjoining rings (the rings are "fused rings") wherein at least one of the rings is heteroaromatic, e.g., the other cyclic rings may be cycloalkyls, cycloalkenyls, and/or aryls.

The term "saturated heterocyclyl" refers to a saturated cyclic group that includes at least one ring heteroatom. The heteroatoms can be the same or different from each other. Examples of heteroatoms include, but are not limited to, nitrogen, oxygen and sulfur. Examples of oxygen-containing saturated heterocyclic rings include, but are not limited to, tetrahydrofuran and tetrahydro-2H-pyran. Examples of nitrogen-containing saturated heterocyclic rings include, but are not limited to, pyrrolidine and piperidine.

The terms "amine" and "amino" are art-recognized and refer to both unsubstituted and substituted amines, e.g., a moiety that may be represented by the general formula: 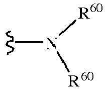 wherein each R⁶⁰ independently represent hydrogen or alkyl.

The terms "alkoxyl" or "alkoxy" are art-recognized and refer to an alkyl group, as defined above, having an oxygen radical attached thereto. Representative alkoxyl groups include methoxy, ethoxy, propyloxy, tert-butoxy and the like. An "ether" is two hydrocarbons covalently linked by an oxygen. Term "alkenyloxy" is art-recognized and refers to an alkenyl group, as defined above, having an oxygen radical attached thereto.

Certain compounds of the present invention may exist in particular geometric or stereoisomeric forms. In addition, polymers of the present invention may also be optically active. The present invention contemplates all such compounds, including cis- and trans-isomers, R- and S-enantiomers, diastereomers, (D)-isomers, (L)-isomers, the racemic mixtures thereof, and other mixtures thereof, as falling within the scope of the invention. Additional asymmetric carbon atoms may be present in a substituent such as an alkyl group. All such isomers, as well as mixtures thereof, are intended to be included in this invention.

If, for instance, a particular enantiomer of compound of the present invention is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carboxyl, diastereomeric salts are formed with an appropriate optically-active acid or base, followed by resolution of the diastereomers thus formed by fractional crystallization or chromatographic means well known in the art, and subsequent recovery of the pure enantiomers.

Unless otherwise indicated, when a disclosed compound is named or depicted by a structure without specifying the stereochemistry and has one or more chiral centers, it is understood to represent all possible stereoisomers of the compound, as well as enantiomeric mixtures thereof.

As used herein, the term "patient" refers to organisms to be treated by the methods of the present invention. Such organisms preferably include, but are not limited to, mammals (e.g., murines, simians, equines, bovines, porcines, canines, felines, and the like), and most preferably includes humans.

As used herein, the term "effective amount" refers to the amount of a compound *(e.g.,* a compound of the present invention) sufficient to effect beneficial or desired results. An effective amount can be administered in one or more administrations, applications or dosages and is not intended to be limited to a particular formulation or administration route. As used herein, the term "treating" includes any effect, *e.g.,* lessening, reducing, modulating, ameliorating or eliminating, that results in the improvement of the condition, disease, disorder, and the like, or ameliorating a symptom thereof.

As used herein, the term "pharmaceutical composition" refers to the combination of an active agent with a carrier, inert or active, making the composition especially suitable for diagnostic or therapeutic use *in vivo* or *ex vivo.*

As used herein, the term "pharmaceutically acceptable salt" refers to any pharmaceutically acceptable salt (*e.g.,* acid or base) of a compound of the present invention which, upon administration to a subject, is capable of providing a compound of this invention or an active metabolite or residue thereof. As is known to those of skill in the art, "salts" of the compounds of the present invention may be derived from inorganic or organic acids and bases. Examples of acids include, but are not limited to, hydrochloric, hydrobromic, sulfuric, nitric, perchloric, fumaric, maleic, phosphoric, glycolic, lactic, salicylic, succinic, toluene-p-sulfonic, tartaric, acetic, citric, methanesulfonic, ethanesulfonic, formic, benzoic, malonic, naphthalene-2-sulfonic, benzenesulfonic acid, and the like. Other acids, such as oxalic, while not in themselves pharmaceutically acceptable, may be employed in the preparation of salts useful as intermediates in obtaining the compounds of the invention and their pharmaceutically acceptable acid addition salts.

Examples of bases include, but are not limited to, alkali metals *(e.g.,* sodium) hydroxides, alkaline earth metals *(e.g.,* magnesium), hydroxides, ammonia, and compounds of formula NW₄⁺, wherein W is C₁₋₄ alkyl, and the like.

Examples of salts include, but are not limited to: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, flucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, palmoate, pectinate, persulfate, phenylpropionate, picrate, pivalate, propionate, succinate, tartrate, thiocyanate, tosylate, undecanoate, and the like. Other examples of salts include anions of the compounds of the present invention compounded with a suitable cation such as Na⁺, NH₄⁺, and NW₄⁺ (wherein W is a C₁₋₄ alkyl group), and the like.

For therapeutic use, salts of the compounds of the present invention are contemplated as being pharmaceutically acceptable. However, salts of acids and bases that are non-pharmaceutically acceptable may also find use, for example, in the preparation or purification of a pharmaceutically acceptable compound.

### EXAMPLES

The invention now being generally described, will be more readily understood by reference to the following examples, which are included merely for purposes of illustration of certain aspects and embodiments of the present invention, and are not intended to limit the invention.

### EXAMPLE 1 -- SYNTHESIS OF COMPOUND 127

The linear precursor for Compound 127 was prepared using standard Fmoc chemistry on the Protein Technologies' Prelude peptide synthesizer (the "Prelude") (Protein Technologies, Inc., Tucson, AZ U.S.A.).

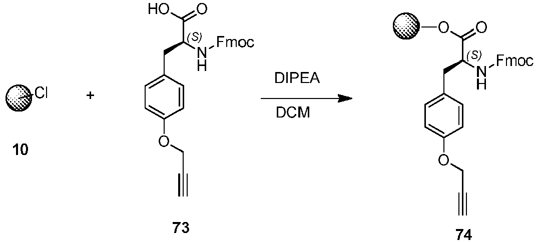

Fmoc-L-Tyrosine propargyl ether, **(73;** 0.5 mmol, 220 mg) and DIPEA (10 equiv., 5 mmol, 646 mg, 0.873 mL) in DCM (8 mL) was added to 2-chlorotrityl resin **(10;** Chemical and Biopharmaceutical Laboratories, 1.58 mmol/g, 3 equiv, 1.5 mmol, 0.95 g) in a Biorad (Bio-Rad Laboratories Hercules, CA, USA) prep column. The resin was rocked for 2 h and MeOH (2 mL) was added followed by rocking for an additional 30 minutes. The solvent was removed by filtration, and the solid resin **74** was transferred to a Prelude reaction vessel.

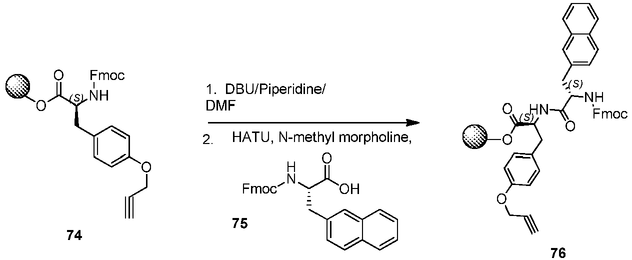

The resin **74** (0.5 mmol) was swelled with DMF (15 mL x 5 min) and mixed with a gentle stream of N₂ every 30 seconds. The solvent was drained and the Fmoc group was removed from the resin-supported building block by washing the resin twice with a solution of 2% DBU, 2% piperidine in DMF (15 mL and 5 minutes per wash) and mixing with a gentle stream of N₂ every 30 seconds. The resin was washed six times with DMF (15 mL and 30 seconds per wash). (S)-2-((((9H-fluoren-9-yl)methoxy)carbonyl)amino)-3-(naphthalen-2-yl)propanoic acid **(75;** 0.1 M solution in DMF, 15 mL, 3 equiv, 1.5 mmol) was then added, followed by HATU (0.2M solution in DMF, 7.5 mL, 3 equiv, 1.5 mmol) and *N*-methyl morpholine (1.0 M in DMF, 2.5 mL, 5 equiv, 2.5 mmol). The reaction mixture was agitated by a gentle stream of nitrogen for 30 min. The reagents were drained from the reaction vessel, and the resin was washed with DMF (15 mL x 5 min).

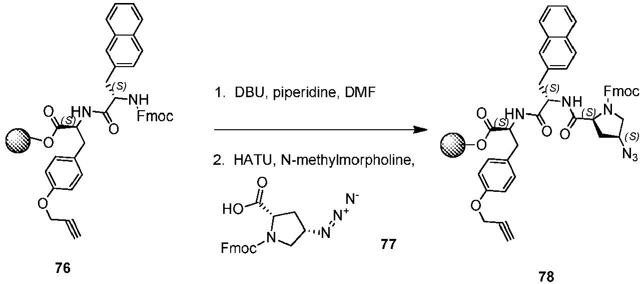

The Fmoc group was removed from the resin-supported building block **76** by washing the resin twice with a solution of 2% DBU, 2% piperidine in DMF (15 mL and 5 minutes per wash) and mixing with a gentle stream of N₂ every 30 seconds. The resin was washed six times with DMF (15 mL and 30 seconds per wash). (2S,4S)-1-(((9H-fluoren-9-yl)methoxy)carbonyl)-4-azidopyrrolidine-2-carboxylic acid **(77;** 0.1 M solution in DMF, 15 mL, 3 equiv, 1.5 mmol) was added, followed by HATU (0.2M solution in DMF, 7.5 mL, 3 equiv, 1.5 mmol) and *N*-methyl morpholine (1.0 M in DMF, 2.5 mL, 5 equiv, 2.5 mmol). The reaction mixture was agitated by a gentle stream of nitrogen for 30 min. The reagents were drained from the reaction vessel, and the resin was washed with DMF (15 mL x 5 min).

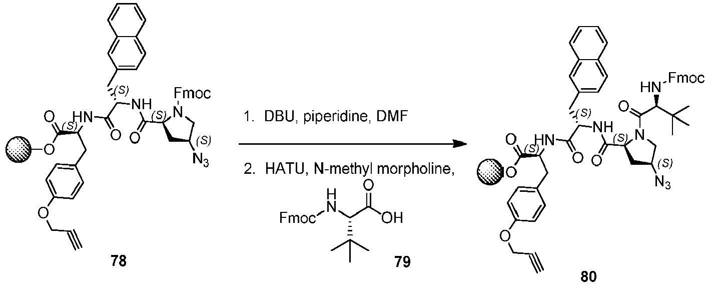

The Fmoc group was removed from the resin-supported building block **78** by washing the resin twice with a solution of 2% DBU, 2% piperidine in DMF (15 mL and 5 minutes per wash) and mixing with a gentle stream of N₂ every 30 seconds. The resin was washed six times with DMF (15 mL and 30 seconds per wash). (S)-2-((((9H-fluoren-9-yl)methoxy)carbonyl)amino)-3,3-dimethylbutanoic acid **(79;** 0.1 M solution in DMF, 15 mL, 3 equiv, 1.5 mmol) was added, followed by HATU (0.2M solution in DMF, 7.5 mL, 3 equiv, 1.5 mmol) and *N*-methyl morpholine (1.0 M in DMF, 2.5 mL, 5 equiv, 2.5 mmol). The reaction mixture was agitated by a gentle stream of nitrogen for 5 hr. The reagents were drained from the reaction vessel, and the resin was washed with DMF (15 mL x 5 min).

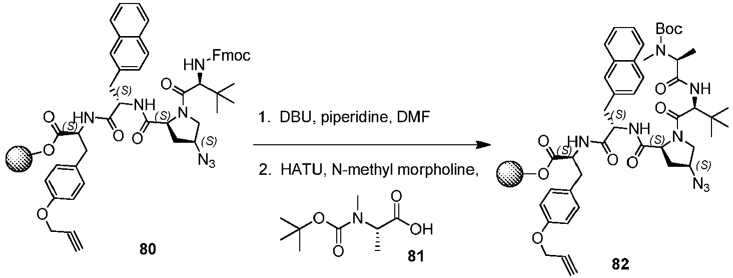

The Fmoc group was removed from the resin-supported building block **80** by washing the resin twice with a solution of 2% DBU, 2% piperidine in DMF (15 mL and 5 minutes per wash) and mixing with a gentle stream of N₂ every 30 seconds. The resin was washed six times with DMF (15 mL and 30 seconds per wash). (S)-2-((tert-butoxycarbonyl)(methyl)amino)propanoic acid **(81;** 0.1 M solution in DMF, 15 mL, 3 equiv, 1.5 mmol) was added, followed by HATU (0.2M solution in DMF, 7.5 mL, 3 equiv, 1.5 mmol) and *N*-methyl morpholine (1.0 M in DMF, 2.5 mL, 5 equiv, 2.5 mmol). The reaction mixture was agitated by a gentle stream of nitrogen for 30 min. The reagents were drained from the reaction vessel, and the resin was washed with DMF (15 mL x 5 min).

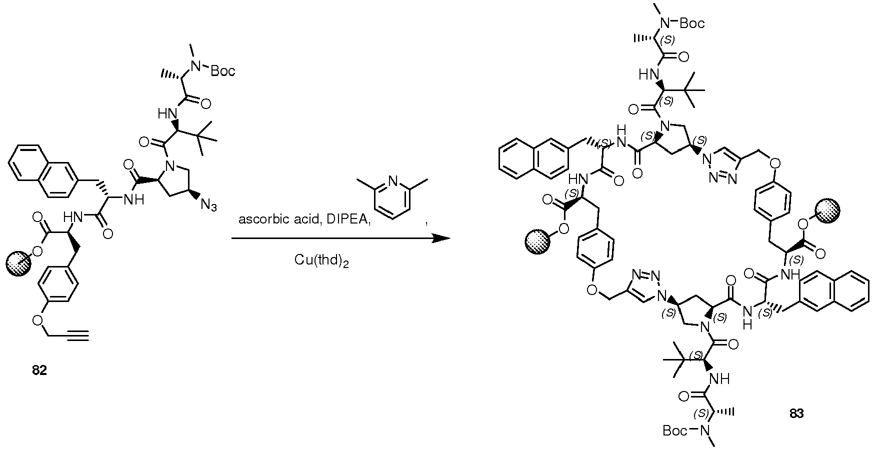

A freshly made solution of copper(II) (Z)-2,2,6,6-tetramethyl-5-oxohept-3-en-3-olate (0.5 equiv, 0.25 mmol, 108 mg), ascorbic acid (3 equiv, 1.5 mmol, 264 mg), DIPEA (10 equiv, 5 mmol, 0.9 mL), 2,6-dimethylpyridine (10 equiv, 5 mmol, 0.6 mL) in DMF (7 mL) and THF (7 mL) was added to the linear peptide on resin **82.** The reaction mixture was agitated by a gentle stream of nitrogen for 30 min. The reagents were drained from the reaction vessel, and the resin was washed with DMF (15 mL x 5 min) to produce the protected dimeric macrocycle **83.** The corresponding monomeric macrocycle **84** was produced as a by-product of this reaction.

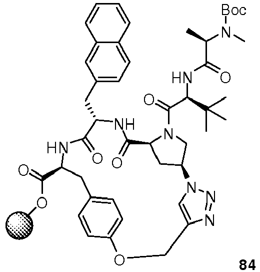

The resulting resin-bound cyclized product **83** was washed with DMF (15 mL x 6; 30 seconds per wash), and DCM (15 mL x 6; 30 seconds per wash) and then deprotected and cleaved from the resin with 5% trifluoroacetic acid (TFA) in DCM (10mL x 5 min, 10 mL x 30 sec, 10 mL x 5 min, 10 mL x 30 sec). The TFA washing was automated on the Prelude using the cleave and collect method. Solvent was removed by evaporation in a Genevac EZ2.2 evaporator with the lamp off on low/medium BP until the crude reaction mixture (containing **127)** formed a very thick oil or a dry solid.

Separation of Compound **127** from the corresponding monomeric macrocycle was achieved on a Gilson HPLC containing a GX 281 liquid handler, UV/VIS 155 detector at 220 nm and 254 nm, and a 321 pump (Gilson, Inc., Middleton, WI, USA) running at 20 mL/min with an Waters Xterra Prep MS C8, 5 um, 19 x 100 mm column PN 186001935 (Waters Corporation, Milford Massachusetts USA), using a solvent gradient from 5% acetonitrile/water with 0.1% TFA increasing to 35% acetonitrile/water with 0.1% TFA, followed by a rapid column flush with 100% acetonitrile/water with 0.1% TFA.

Other homodimeric macrocycles of Formula I were similarly prepared substituting the appropriate Fmoc- or Boc-protected building block at each step.

### EXAMPLE 2 -- SYNTHESIS OF COMPOUND 135 & OTHER EXEMPLARY MACROCYCLIC COMPOUNDS

The linear precursor for Compound **135** was built using standard Fmoc chemistry on the Prelude.

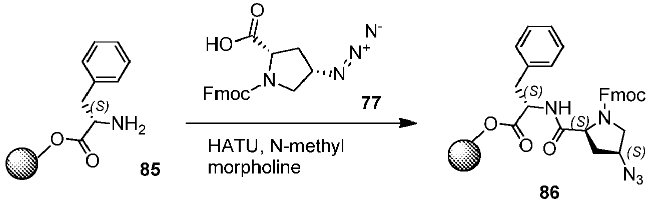

Phe-substituted resin **85** (Chemical and Biopharmaceutical Laboratories) was washed with and then suspended in DMF (15 mL x 5 min) and mixed with a gentle stream of N₂ every 30 seconds. (2S,4S)-1-(((9H-fluoren-9-yl)methoxy)carbonyl)-4-azidopyrrolidine-2-carboxylic acid **(77,** 0.1 M solution in DMF, 15 mL, 3 equiv, 1.5 mmol), followed by HATU (0.2M solution in DMF, 7.5 mL, 3 equiv, 1.5 mmol) and *N*-methyl morpholine (1.0 M in DMF, 2.5 mL, 5 equiv, 2.5 mmol) were added to the resin **85** (0.5 mmol). The reaction mixture was agitated by a gentle stream of nitrogen for 30 min. The reagents were drained from the reaction vessel, and the resin **86** was washed with DMF (15 mL x 5 min).

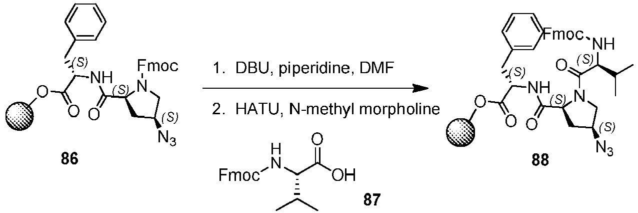

The Fmoc group was removed from the resin-supported building block **86** by mixing the resin twice with a solution of 2% DBU, 2% piperidine in DMF (15 mL and 5 minutes per wash) while agitating with a gentle stream of N₂ every 30 seconds. The resin was washed six times with DMF (15 mL and 30 seconds per wash). (S)-2-((((9H-fluoren-9-yl)methoxy)carbonyl)amino)-3-methylbutanoic acid **(87;** 0.1 M solution in DMF, 15 mL, 3 equiv, 1.5 mmol), followed by HATU (0.2 M solution in DMF, 7.5 mL, 3 equiv, 1.5 mmol) and *N*-methyl morpholine (1.0 M in DMF, 2.5 mL, 5 equiv, 2.5 mmol) were added to the resin **86** (0.5 mmol). The reaction mixture was agitated by a gentle stream of nitrogen for 5 hr. The reagents were drained from the reaction vessel, and the resin **88** was washed with DMF (15 mL x 5 min).

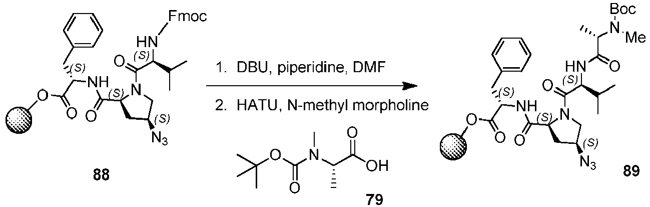

The Fmoc group was removed from the resin-supported building block **88** by mixing the resin twice with a solution of 2% DBU, 2% piperidine in DMF (15 mL and 5 minutes per wash) while agitating with a gentle stream of N₂ every 30 seconds. The resin was washed six times with DMF (15 mL and 30 seconds per wash). (S)-2-((tert-butoxycarbonyl)(methyl)amino)propanoic acid **(79;** 0.1 M solution in DMF, 15 mL, 3 equiv, 1.5 mmol), followed by HATU (0.2M solution in DMF, 7.5 mL, 3 equiv, 1.5 mmol) and *N*-methyl morpholine (1.0 M in DMF, 2.5 mL, 5 equiv, 2.5 mmol) were added to the resin (0.5mmol). The reaction mixture was agitated by a gentle stream of nitrogen for 30 min. The reagents were drained from the reaction vessel, and the resin **89** was washed with DMF (15 mL x 5 min).

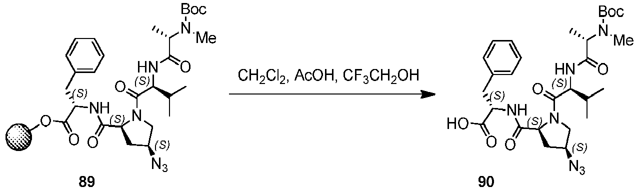

The resin **89** was washed six times with DMF (15 mL and 30 seconds per wash). The resin was washed six times with CH₂Cl₂ (15 mL and 30 seconds per wash), and then with a mixture of CH₂Cl₂:AcOH: CF₃CH₂OH (3:1:1, 20 mL) The reaction mixture was agitated by a gentle stream of nitrogen for 2 h, followed by a second wash with CH₂Cl₂:AcOH: CF₃CH₂OH (3:1:1, 20 mL) for 30 seconds. The AcOH washings were combined and the solvents were removed *in vacuo*. The product **90** was purified on a Biotage Isolera 1 system running at 50 mL/min with an Biotage snap 60g C18 column (Biotage AB, Uppsala, Sweden), using a solvent gradient from 15% acetonitrile/water with 0.1% DIPEA increasing to 60% acetonitrile/water with 0.1% DIPEA, followed by a rapid column flush with 100% acetonitrile with 0.1% DIPEA.

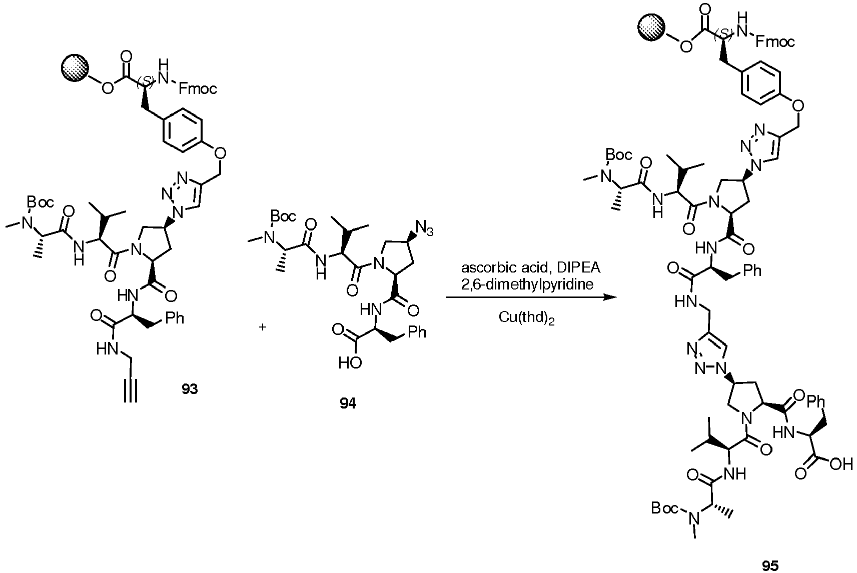

A freshly made solution of copper(II) (Z)-2,2,6,6-tetramethyl-5-oxohept-3-en-3-olate (0.5 equiv, 0.075 mmol, 32 mg), ascorbic acid (3 equiv, 0.45 mmol, 79 mg), DIPEA (10 equiv, 1.5 mmol, 0.160 g), 2,6-dimethylpyridine (10 equiv, 1.5 mmol, 0.194 g) and linear peptide precursor **(90;** 1 equiv, 0.15 mmol, 66 mg) in DMF (2 mL) and THF (2 mL) was added to Fmoc amino acid resin **74.** The reaction mixture was agitated by a gentle stream of nitrogen for 30 min. The reagents were drained from the reaction vessel, and the resin **91** was washed with DMF (15 mL x 5 min).

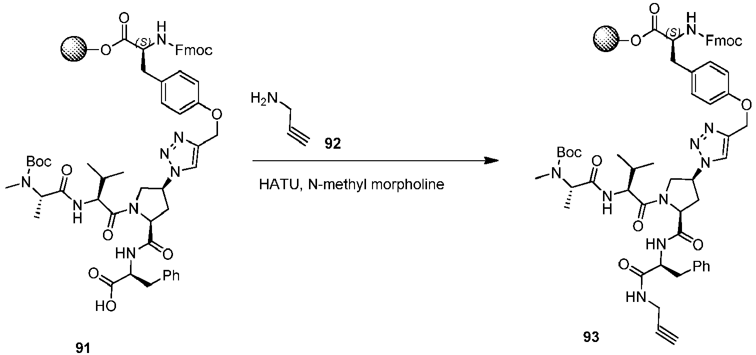

Propargyl amine **(92;** 0.091 mL, 10 equiv, 1.5 mmol), followed by HATU (0.2M solution in DMF, 4.5 mL, 6 equiv, 0.9 mmol) and *N*-methyl morpholine (1.0 M in DMF, 1.5 mL, 10 equiv, 1.5 mmol) were added to the peptide on resin **91** (0.15 mmol). The reaction mixture was agitated by a gentle stream of nitrogen for 30 min. The reagents were drained from the reaction vessel, and the resin **93** was washed with DMF (15 mL x 5 min).

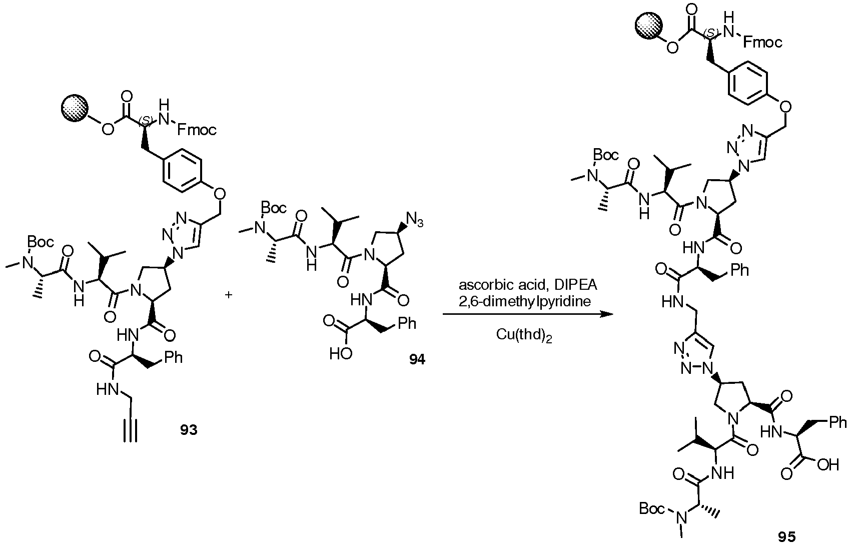

A freshly made solution of copper(II) (Z)-2,2,6,6-tetramethyl-5-oxohept-3-en-3-olate (0.5 equiv, 0.075 mmol, 32 mg), ascorbic acid (3 equiv, 0.45 mmol, 79 mg), DIPEA (10 equiv, 1.5 mmol, 0.160 g), 2,6-dimethylpyridine (10 equiv, 1.5 mmol, 0.194 g) and linear peptide **94** (1 equiv, 0.15 mmol, 66 mg) in DMF (2 mL) and THF (2 mL) was added to the linear peptide resin **93** (0.15 mmol). The reaction mixture was agitated by a gentle stream of nitrogen for 30 min. The reagents were drained from the reaction vessel, and the resin **95** was washed with DMF (15 mL x 5 min).

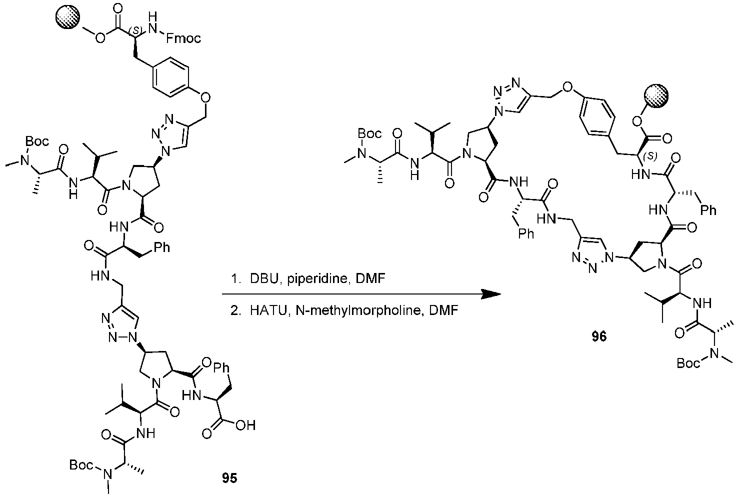

The Fmoc group was removed from the resin-supported building block **95** by mixing the resin twice with a solution of 2% DBU, 2% piperidine in DMF (15 mL and 5 minutes per wash) while agitating with a gentle stream of N₂ every 30 seconds. The resin was washed six times with DMF (15 mL and 30 seconds per wash). HATU (0.2M solution in DMF, 2.25 mL, 3 equiv, 0.45 mmol) and *N*-methyl morpholine (1.0 M in DMF, 1.5 mL, 10 equiv, 2.5 mmol) were added to resin (0.15 mmol). The reaction mixture was agitated by a gentle stream of nitrogen for 30 min. The reagents were drained from the reaction vessel, and the resin **96** was washed with DMF (15 mL x 5 min).

The resulting resin-bound cyclized product **96** was washed with DMF (15 mL x 6; 30 seconds per wash), and CH₂Cl₂ (15 mL x 6; 30 seconds per wash) and then treated with 5% TFA in CH₂Cl₂ (10mL x 5 min, 10 mL x 30 sec, 10 mL x 5 min, 10 mL x 30 sec). The TFA washing was automated on the Prelude using the cleave and collect method. The TFA washings were combined and solvent was removed by evaporation using a Genevac EZ2.2 evaporator with the lamp off on low/medium BP until the crude reaction mixture formed a very thick oil or a dry solid.

Purification of compound **135** was achieved on a Gilson HPLC containing a GX 281 liquid handler, UV/VIS 155 detector at 220 nm and 254 nm. The 321 pump was running at 20 mL/min with an Waters Xterra Prep MS C8, 5 um, 19 x 100 mm column PN 186001935, using a solvent gradient from 5% acetonitrile/water with 0.1% TFA increasing to 35% acetonitrile/water with 0.1% TFA, followed by a rapid column flush with 100% acetonitrile/water with 0.1% TFA.

### EXAMPLE 3 -- SYNTHESIS OF COMPOUNDS 147, 148, AND OTHER EXEMPLARY MACROCYCLIC COMPOUNDS

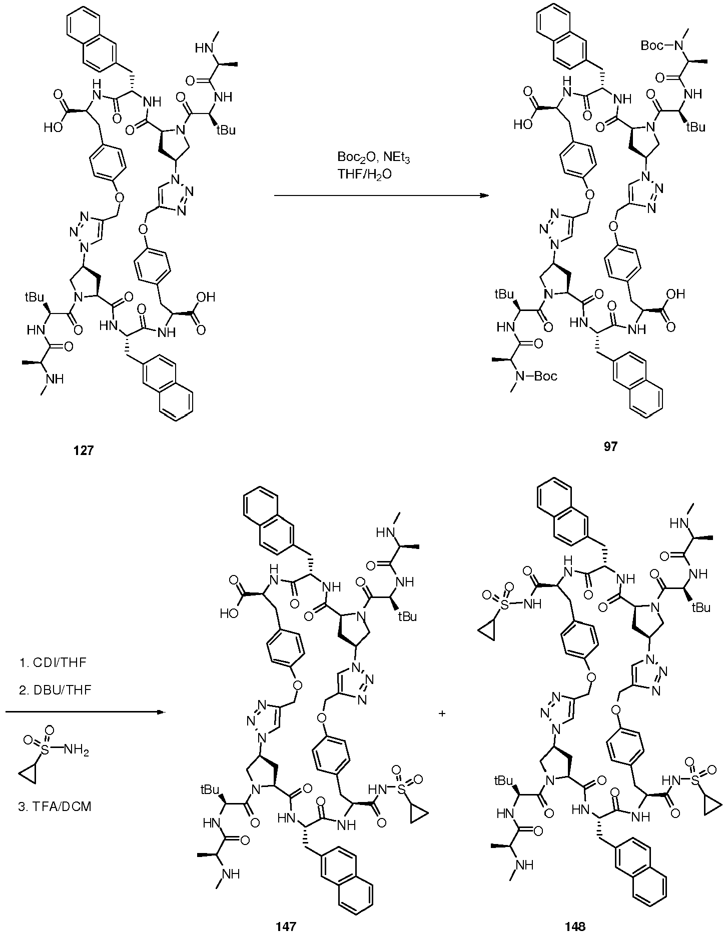

To a solution of Compound **127** (62 mg, 0.04 mmol) in THF/H₂O (2 mL, 3:1) was added triethylamine (20 mg, 0.2 mmol) and di-*tert*-butyl dicarbonate (23 mg, 0.1 mmol). The reaction was stirred at room temperature for 15 h, and was then concentrated *in vacuo*. The residue was triturated with sat. aq. Na₂CO₃ (0.5 mL). The white precipitate was washed sequentially with H₂O (0.5 mL) and ether (0.5 mL) and dried under high vacuum to give Compound **97** as a white solid (62 mg, 88%). MS(ESI⁺) m/z 1705.5 (M + H)⁺.

Compound **97** (15 mg, 0.009 mmol) and CDI (7 mg, 0.04 mmol) were dissolved in THF (0.7 mL) and stirred at room temperature for 2 h. A solution of cyclopropylsulfonamide (Aldrich, 11.4 mg, 0.094 mmol) in THF (0.3 mL) and DBU (0.014 mL, 0.094 mmol) was then added. The resulting solution was stirred at room temperature for 12 h, and was then concentrated *in vacuo* to give a yellow oil. A solution of TFA (1mL) in DCM (1 mL) was added to the oil and the resulting mixture was stirred for 30 minutes. The reaction was then concentrated *in vacuo*.

Separation of compound **147** (5.5 mg, 31%) from **148** (2.4 mg, 14%) was achieved on a Shimadzu HPLC with a Phenomenex AXIA 5 um, 21.2 x 100 nm column, using a solvent gradient from 10% acetonitrile/water with 0.1 % TFA increasing to 45% acetonitrile/water with 0.1% TFA, followed by a rapid column flush with 100% acetonitrile/water with 0.1% TFA.

### EXAMPLES 4 -- SYNTHESIS OF COMPOUNDS 149, 150 AND OTHER EXEMPLARY MACROCYCLIC COMPOUNDS

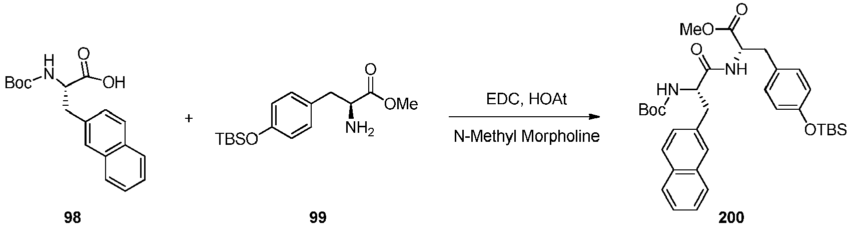

To a solution of (*S*)-2-((*tert*-butoxycarbonyl)amino)3-(naphthalen-2-yl)propanoic acid **(98,** Chem-Impex International, 820 mg, 2.6 mmol) in DMF (20 mL) was added EDC·HCl(498 mg, 2.6 mmol) and HOAt (354 mg, 2.6 mmol). The resulting solution was stirred at 0 °C for 15 min and treated with (*S*)-methyl 2-amino-3-(4-((*tert*-butyldimethylsilyl)oxy)phenyl)propanoate **(99,** 885 mg, 2.86 mmol, prepared as described in Hansen, D. W., Pilipauskas, D. J. Org. Chem. 1985, 50, 945-950) and *N*-methylmorpholine (789 mg , 7.89 mmol). The reaction mixture was allowed to warm to room temperature and stirred for 30 min. The reaction was then quenched with 10% aq. lithium hydroxide solution and extracted with ethyl acetate. The organic layers were concentrated *in vacuo* and the resulting residue was purified by flash chromatography (ISCO; 80 g column eluting with 10-60% EtOAc / hexanes) to give the title compound (1.42 g, 90%) as a white solid. MS(ESI⁺) m/z 607.5 (M + H)⁺.

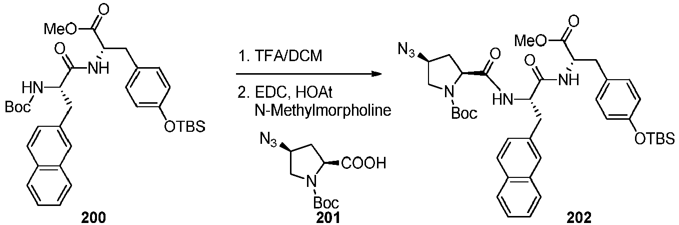

To a solution of (*S*)-methyl 2-((*S*)-2-((*tert*-butoxycarbonyl)amino)-3-naphthalen-2-yl)propanamido)-3-(4-((*tert*-butyldimethylsilyl)oxy)phenyl)propanoate (200, Chem-Impex International, 1.21 g, 2 mmol) in DCM (20 mL) was added 30% TFA in DCM (20 mL). The resulting solution was stirred at room temperature for 1.5 h and then was quenched with sat. aq. sodium bicarbonate solution and extracted with DCM. The organic layers were washed with brine, dried over sodium sulfate, and concentrated *in vacuo* to give the free amine of **200** which was used directly without further purification.

To a solution of (2*S*, 4*S*)-4 azido-1-(*tert*-butoxycarbonyl)pyrrolidine-2-carboxylic acid **(201,** Chem-Impex International, 615 mg, 2.4 mmol) in DMF(15 mL) was added EDC.HCl (460 mg, 2.4 mmol) and HOAt (136 mg, 2.4 mmol). The resulting solution was stirred at 0 °C for 30 minutes. A solution of the free amine and *N*-methylmorpholine (607 mg , 6 mmol) was then added. The reaction mixture was allowed to warm to room temperature and stirred for 1 hour. The reaction was then quenched with 10% aq. lithium hydroxide solution and extracted with ethyl acetate. The organic layers were washed with brine, dried over sodium sulfate, and concentrated *in vacuo*. The residue was purified by flash chromatography (ISCO; 80 g column eluting with 20-60% EtOAc / hexanes) to give compound **202** (1.3 g, 87%) as a white solid. MS(ESI⁺) m/z 745.6 (M + H)⁺.

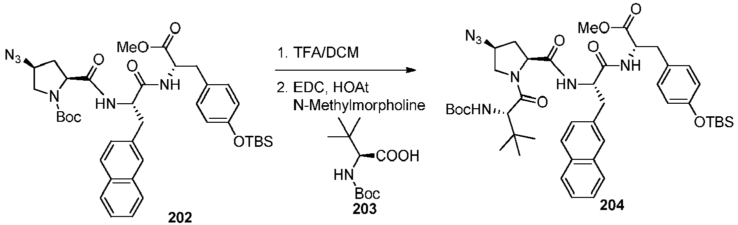

Following a procedure analogous to that for the synthesis of Compound **202** of Example 4, Compound **202** (745 mg, 1 mmol) was converted to compound **204** and purified by flash chromatography (ISCO; 80 g column eluting with 10-60% EtOAc / hexanes) to give the title compound (0.78 g, 91%) as a white foam. MS(ESI⁺) m/z 858.6 (M + H)⁺.

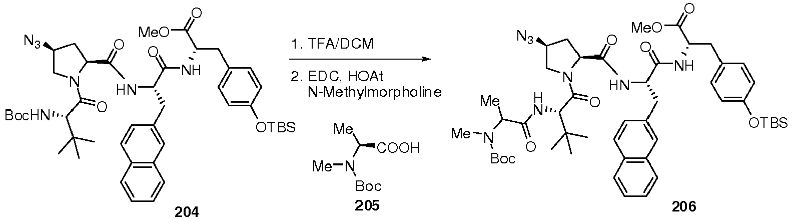

Following a procedure analogous to that for the synthesis of Compound **202** of Example 4, Compound **204** (857 mg, 1 mmol) was converted to compound **206** and purified by flash chromatography (ISCO; 80 g column eluting with 10-100% EtOAc / hexanes) to give the title compound (0.827 g, 83%) as a white foam. MS(ESI⁺) m/z 943.8 (M + H)⁺. 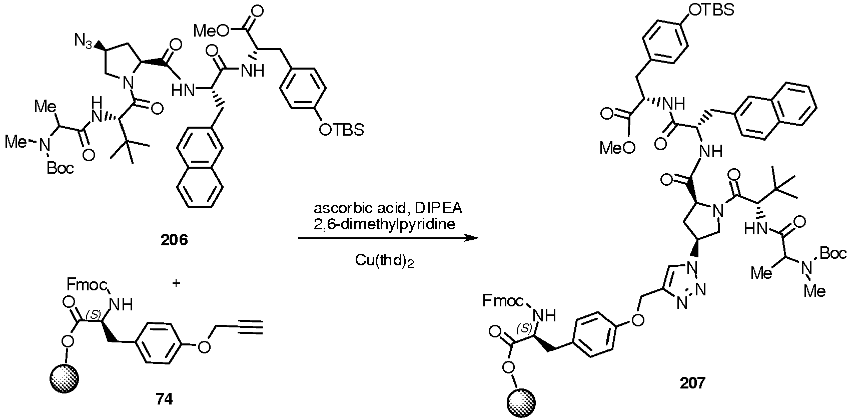 A freshly made solution of copper(II) (Z)-2,2,6,6-tetramethyl-5-oxohept-3-en-3-olate (Aldrich, 0.002 mmol, 1 mg), ascorbic acid (0.015 mmol, 2.5 mg), DIPEA (0.05 mmol, 5 mg), 2,6-dimethylpyridine (0.05 mmol, 6 mg) and linear peptide precursor **(206, 0.01** mmol, 10 mg) in DMF (1 mL) and THF (1 mL) was added to Fmoc amino acid resin **74** (10 mg). The reaction mixture was agitated by a gentle stream of nitrogen for 12 h. The reagents were drained from the reaction vessel, and the resin **207** was washed with DMF (3 x 2 mL) and then DCM (2 x 3 mL).

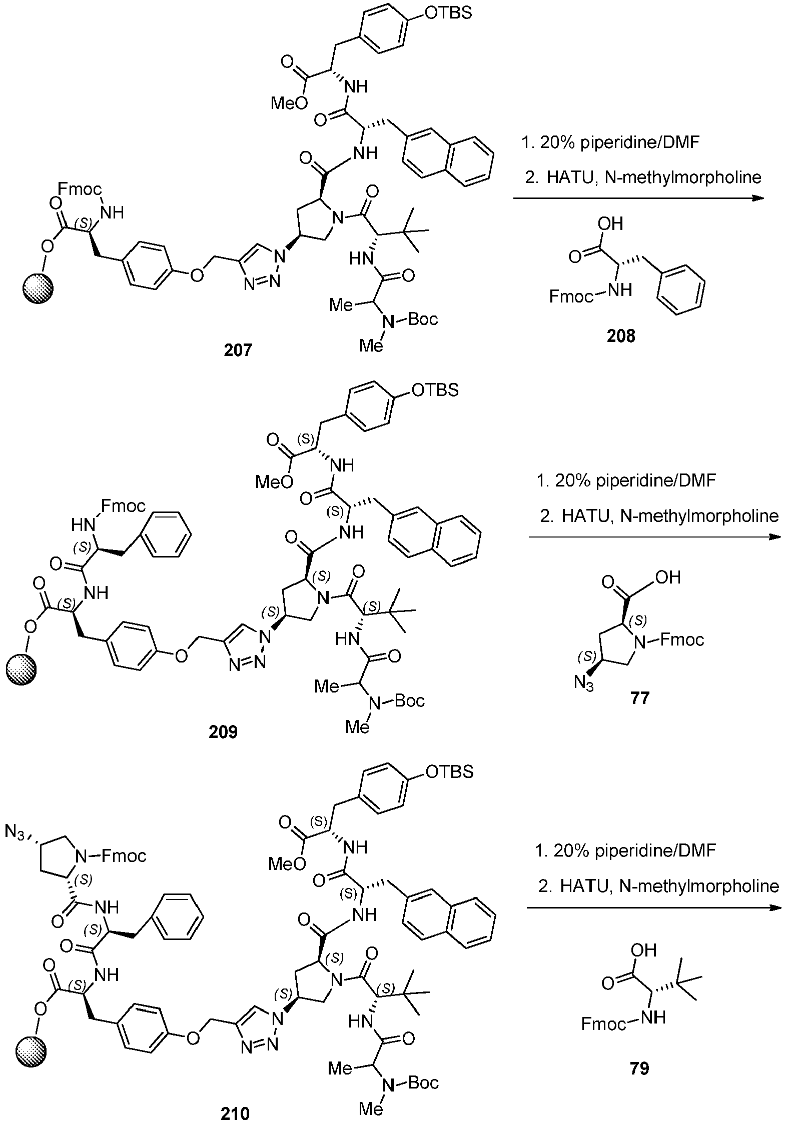

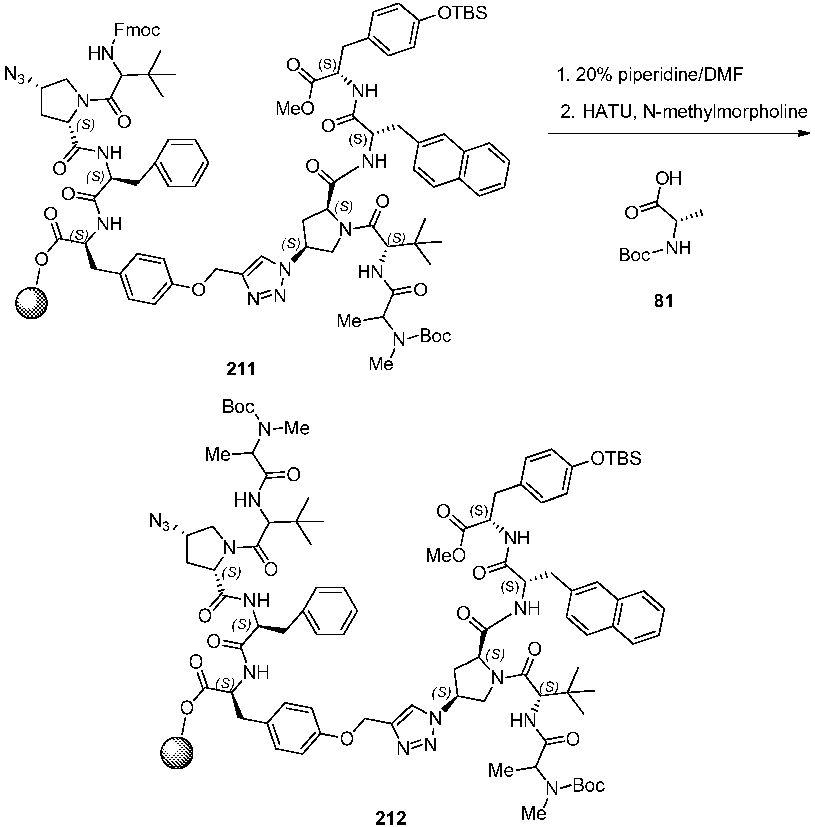

Following a procedure analogous to that for the synthesis of Compound **82** of Example 1, resin **207** (30 mg) was converted to the resin-linked peptide **212** using standard Fmoc chemistry on the Protein Technologies' Prelude peptide synthesizer.

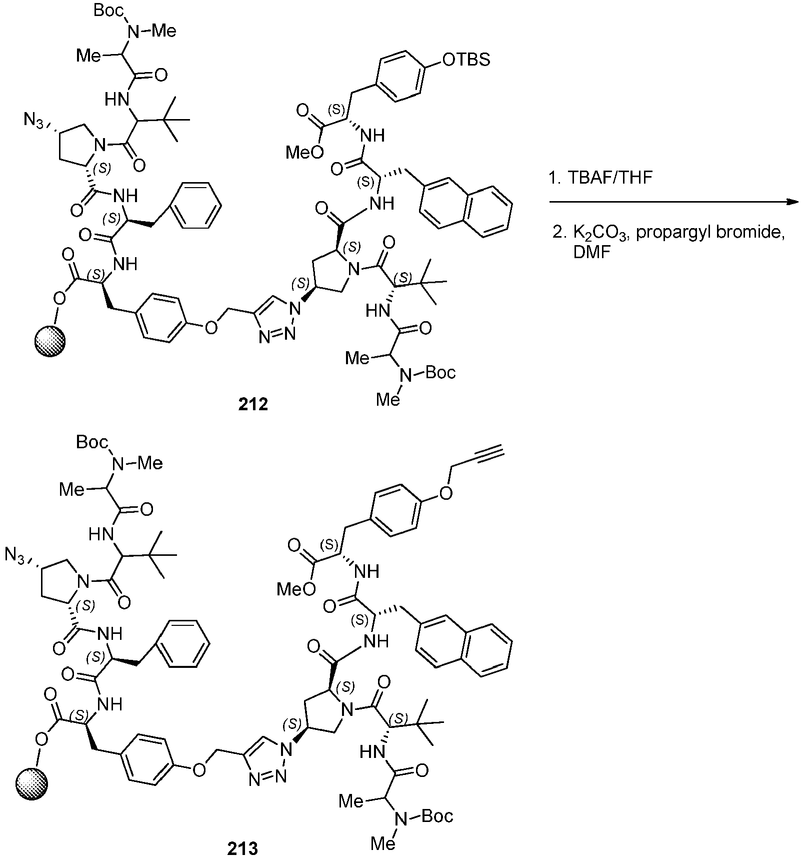

To the resin **212** was added a solution of tetrabutylammonium fluoride (TBAF) in THF (1M, 0.5 mL). The reaction mixture was agitated by a gentle stream of nitrogen for 1 h. The reagents were drained from the reaction vessel, and the resin was washed sequentially with THF (2 mL) and DCM (2 x 3 mL). DMF (1.5 mL), K₂CO₃ (80 mg) and propargyl bromide (0.3 mL, Aldrich, 80% in toluene) were then added to the solution and the resulting mixture was agitated at room temperature for 16 h. The reagents were drained from the reaction vessel, and the resin **213** was washed sequentially with DMF (2 x 3 mL), H₂O (2 x 3 mL), DMF (2 x 3 mL) and DCM (2 x 3 mL).

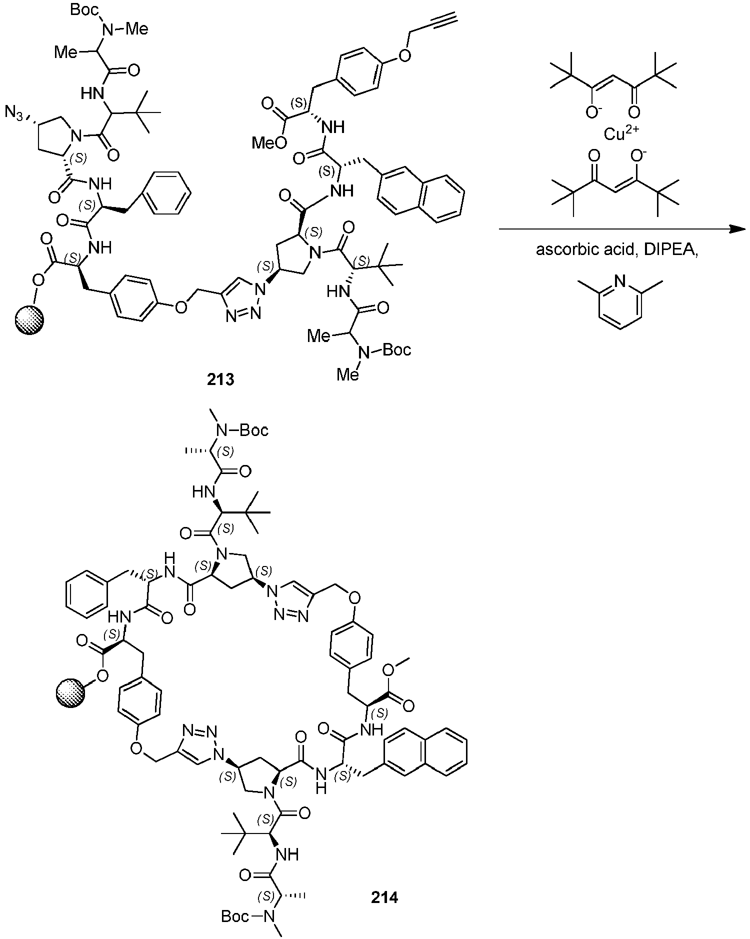

A freshly made solution of copper(II) (Z)-2,2,6,6-tetramethyl-5-oxohept-3-en-3-olate (0.004 mmol, 2 mg), ascorbic acid (0.03 mmol, 5 mg), DIPEA (0.1 mmol, 10 mg), 2,6-dimethylpyridine (0.1 mmol, 12 mg) in DMF (0.8 mL) and THF (0.8 mL) was added to resin **213.** The reaction mixture was agitated by a gentle stream of nitrogen for 12 h. The reagents were drained from the reaction vessel, and the resin **214** was washed with DMF (2 x 3 mL) and then DCM (2 x 3 mL).

The resulting resin-bound cyclized product **214** was deprotected and cleaved from the resin with 30 % TFA in DCM (4 mL, 30 min). The TFA solution was then concentrated *in vacuo* to give a thick oil which was purified on a Shimadzu HPLC with a Phenomenex AXIA 5 um, 21.2 x 100 nm column, using a solvent gradient from 10% acetonitrile/water with 0.1 % TFA increasing to 40% acetonitrile/water with 0.1% TFA, followed by a rapid column flush with 100% acetonitrile/water with 0.1% TFA. Compound **150** was isolated as a white solid.

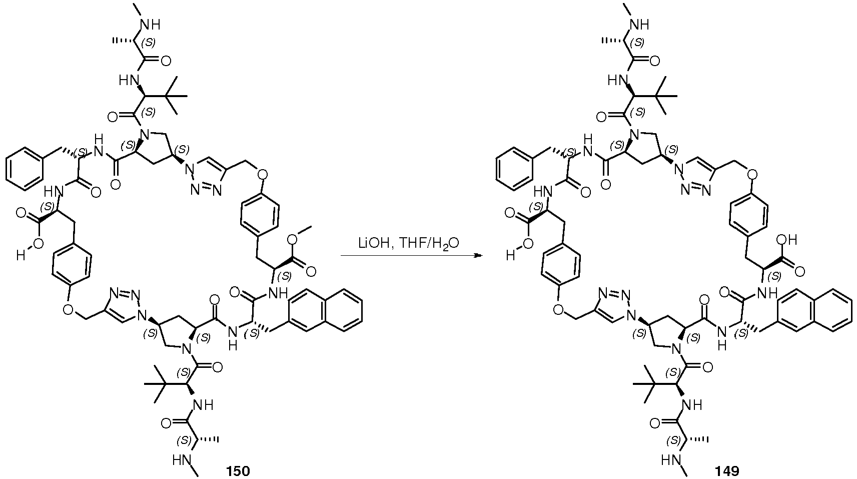

To a suspension of Compound **150** (6 mg) in THF (0.5 mL) was added aq. LiOH solution (1M, 0.3 mL). The reaction was stirred at room temperature for 3 h and then concentrated *in vacuo*. The residue was purified on a Shimadzu HPLC with a Phenomenex AXIA 5 um, 21.2 x 100 nm column, using a solvent gradient from 10% acetonitrile/water with 0.1 % TFA increasing to 40% acetonitrile/water with 0.1% TFA, followed by a rapid column flush with 100% acetonitrile/water with 0.1% TFA. Compound **149** was isolated as a white solid (4.6 mg, 83%).

### EXAMPLE 5- EVALUATION OF BIOLOGICAL ACTIVITY

Exemplary compounds were tested for inhibition of XIAP BIR2 and BIR3 and cIAP BIR3 activity. Experimental procedures and results are provided below.

### Experimental Procedures:

### A. XIAP-BIR2 / SMAC Peptide AlphaScreen Assay

Assays were performed in white, flat-bottom, 384-well ProxiPlates (Perkin Elmer). The final assay volume was 10 µL prepared from additions of His-BIR2 (124-240/C202A/C213G), Biotinylated SMAC peptide, and test compounds in assay buffer consisting of 25 mM Hepes, 100 mM NaCl, 0.1% BSA, and 5 mM CaCl₂. The reaction was incubated at room temperature for 60 minutes. After 60 minutes, 2.5 µL of Alphascreen detection reagent (Perkin Elmer) was added to the reaction mixture and incubated at room temperature in the dark for 120 minutes. The Alphascreen signal generated by the reaction was detected on the Envision Plate Reader. Inhibition data were calculated from an Alphascreen signal generated by the no protein control reactions for 100% inhibition and vehicle-only reactions for 0% inhibition. The final concentration of reagents in the assay was 50 nM His-BIR2 (124-240/C202A/C213G), 50 nM Biotinylated SMAC peptide, 4 µg/mL Alphascreen detection reagents, and 0.5% DMSO. Dose response curves were generated to determine the concentration required for inhibiting 50% of kinase activity (IC₅₀). Compounds were dissolved at 10 mM in dimethylsulfoxide (DMSO) and evaluated at eleven concentrations. IC₅₀ values were derived by non-linear regression analysis.

### B. XIAP-BIR3 SMAC Peptide Fluorescence Polarization Assay (FPA)

Assays were performed in black, flat-bottom, 384-well plates. The final assay volume was 50 µL prepared from additions of N-His-Tb-BIR3(241-356, XIAP), fluoresceinated modified SMAC peptide, and test compounds in assay buffer consisting of 20 mM Sodium Phosphate, 1 mM EDTA, 50 mM NaCl, and 0.05% Pluronic F68. The reaction was incubated at room temperature for 60 minutes and fluorescence polarization of the reaction was detected on the LJL Plate Reader. Inhibition data were calculated from mP values generated by the no protein control reactions for 100% inhibition and vehicle-only reactions for 0% inhibition. The final concentration of reagents in the assay was 130 nM N-His-Tb-BIR3(241-356, XIAP), 1.4 nM fluoresceinated modified SMAC peptide, and 1% DMSO. Dose response curves were generated to determine the concentration required for inhibiting 50% of polarization activity (IC₅₀). Compounds were dissolved at 10 mM in dimethylsulfoxide (DMSO) and evaluated at eleven concentrations. IC₅₀ values were derived by non-linear regression analysis.

### Results:

Results of the BIR2 and BIR3 assays are shown in Table 2 below. BIR2 IC₅₀ values are reported as follows: "A" indicates an IC₅₀ value of less than 2 µM; "B" indicates an IC₅₀ value of 2 µM to 12 µM; and "C" indicates an IC₅₀ value of greater than 12 µM. "NT" means that the compound was not tested in the assay. BIR3 IC₅₀ values are reported as follows: "A" indicates an IC₅₀ value of less than 50 nM; "B" indicates an IC₅₀ value of 50 nM to 100 nM; and "C" indicates an IC₅₀ value of greater than 100 nM. "NT" means that the compound was not tested in the assay.

**TABLE 2 -- BIR2 and BIR3 Inhibitory Activity of Select Compounds of Formula I**

| **Compound No.** | **CIR2 IC₅₀** | **BIR3 IC₅₀** |
|---|---|---|
| 100 | A | B |
| 101 | A | B |
| 102 | A | B |
| 103 | B | C |
| 104 | C | B |
| 105 | C | B |
| 106 | C | B |
| 107 | A | C |
| 108 | C | C |
| 109 | A | A |
| 110 | A | A |
| 111 | A | A |
| 112 | B | A |
| 113 | C | B |
| 114 | A | A |
| 115 | B | A |
| 116 | A | B |
| 117 | A | A |
| 118 | C | B |
| 119 | A | A |
| 120 | A | A |
| 121 | C | C |
| 122 | B | C |
| 123 | B | C |
| 124 | A | A |
| 125 | A | A |
| 126 | B | B |
| 127 | A | A |
| 128 | A | B |
| 129 | A | A |
| 130 | B | A |
| 131 | B | C |
| 132 | C | C |
| 133 | A | A |
| 134 | A | B |
| 135 | A | A |
| 136 | NT | NT |
| 137 | A | A |
| 138 | NT | NT |
| 139 | NT | NT |
| 140 | A | A |
| 141 | NT | NT |
| 142 | NT | NT |
| 143 | NT | NT |
| 144 | NT | NT |
| 145 | NT | NT |
| 146 | NT | NT |

### C. cIAP1-BIR3 SMAC Peptide Fluorescence Polarization Assay (FPA)

Assays were performed in black, round-bottom, 96-well plates. The final assay volume was 30 µL prepared from additions of N-His-Tb-BIR3(262-352, cIAP1), fluoresceinated modified SMAC peptide, and test compounds in assay buffer consisting of 20 mM Sodium Phosphate, 1 mM EDTA, 50 mM NaCl, and 0.05% Pluronic F68. The reaction was incubated at room temperature for 60 minutes and fluorescence polarization of the reaction was detected on the LJL Plate Reader. Inhibition data were calculated from mP values generated by the no protein control reactions for 100% inhibition and vehicle-only reactions for 0% inhibition. The final concentration of reagents in the assay was 36.1 nM N-His-Tb-BIR3(262-352, cIAP1), 1.4 nM fluoresceinated modified SMAC peptide, and 1% DMSO. Dose response curves were generated to determine the concentration required for inhibiting 50% of polarization activity (IC₅₀). Compounds were dissolved at 10 mM in dimethylsulfoxide (DMSO) and evaluated at eight concentrations. IC₅₀ values were derived by non-linear regression analysis.

Results of this assay are shown in Table 3. Only certain compounds were tested. IC₅₀ values are reported as follows: "A" indicates an IC₅₀ value of less than 50 nM; "B" indicates an IC₅₀ value of 50 nM to 100 nM; and "C" indicates an IC₅₀ value of greater than 100 nM.

**TABLE 3. BIR3 Inhibitory Activity of Select Compounds of Formula I Measured by cIAP1-BIR3 SMAC Peptide Fluorescence Polarization Assay**

| **Compound No.** | **BIR3 IC₅₀** |
|---|---|
| 111 | A |
| 110 | A |
| 137 | A |

### EXAMPLE 6 -- SYNTHESIS OF INTERMEDIATE 312, COMPOUND 1003 & OTHER EXEMPLARY MACROCYCLIC COMPOUNDS

The solution phase synthesis of key tetrapeptide intermediate **312** was carried out using the synthetic sequence outlined below.

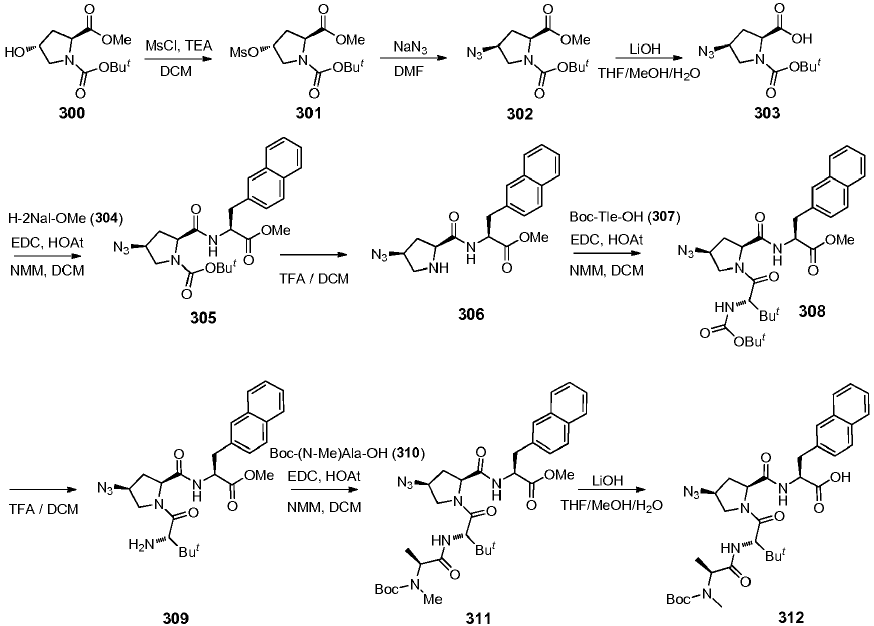

**(2S,4S)-1-*tert*-Butyl 2-methyl 4-((methylsulfonyl)oxy)pyrrolidine-1,2-dicarboxylate**. To a solution of (2S,4R)-1-*tert*-butyl 2-methyl 4-hydroxypyrrolidine-1,2-dicarboxylate **(300;** 53.0 mmol, 13.0 g) in DCM (100 mL) was added triethylamine (85 mmol, 11.8 mL) and the resulting solution was cooled to 0 °C. While monitoring the internal temperature with a thermocouple, methanesulfonyl chloride (58.3 mmol, 4.55 mL) was added at such a rate as to not allow the temperature to exceed 3.5 °C and the reaction mixture was permitted to stir at 0 °C for 2 h. The reaction was quenched with IN HCl and extracted with DCM (3x). The combined organics were washed with water, dried over MgSO₄, filtered and concentrated *in vacuo*. The residue was triturated with hexane to afford the mesylate **301** (17.1 g) as a solid, which was used directly in the next step. MS (ESI⁺) rt 0.8 min, *m*/*z* 268.1.

**(2S,4S)-1-*tert*-Butyl 2-methyl 4-azidopyrrolidine-1,2-dicarboxylate.** (2S,4R)-1-*tert*-Butyl 2-methyl 4-((methylsulfonyl)oxy)pyrrolidine-1,2-dicarboxylate **(301;** 53.0 mmol, 17.2 g) was dissolved in DMF (80 mL) and sodium azide (80 mmol, 5.17 g) was added to the mixture. The reaction was heated to 70 °C. Upon completion of the reaction, the mixture was cooled to rt, poured into H₂O (100 mL) and extracted with EtOAc (3 x 100 mL). The combined organic extracts were washed sequentially with sat. aq. NaHCO₃ soln. (70 mL), 10% aq. LiCl soln. (4 x 100 mL) and brine. The organics were dried over MgSO₄, filtered and concentrated *in vacuo* (TLC - 1:1 EtOAc:Hexanes, R_{f} = 0.6). The resulting oil was purified by silica gel chromatography, 0-100 % EtOAc in hexanes, to afford **302** (12.3 g, 85%). MS (ESI+) rt 1.57 min, *m*/*z* 293.2 (M + Na); 171.3 (M+1 - BOC). MS (ESI+) rt 0.87 min, *m*/*z* 215.1; 171.1 - consistent for 270.13.

**(2S,4S)-4-Azido-1-(*tert*-butoxycarbonyl)pyrrolidine-2-carboxylic acid.** (2S,4S)-1-*tert*-Butyl 2-methyl 4-azidopyrrolidine-1,2-dicarboxylate **(302;** 45.5 mmol; 12.3 g) was dissolved in THF (50 mL) / MeOH (50 mL) and the resulting solution was treated with 2N LiOH (114 mmol, 56.9 mL). The reaction mixture was stirred at rt until complete conversion was observed at which point the mixture was made acidic (pH 3) with IN HCl and the solution was extracted with CH₂Cl₂ (3x). The combined organic extracts were dried, filtered and concentrated *in vacuo*. Et₂O was added to the residue and the mixture was concentrated 3x to afford **303** (11.3 g, 96%) as a white solid. MS (ESI+) rt 0.73 min, *m*/*z* 257.2.

**(2S,4S)-*tert*-Butyl 4-azido-2-(((S)-1-methoxy-3-(naphthalen-2-yl)-1-oxopropan-2-yl)carbamoyl)pyrrolidine-l-carboxylate.** To a 0 °C solution of (2S,4S)-4-azido-1-(*tert*-butoxycarbonyl)pyrrolidine-2-carboxylic acid **(303;** 39.8 mmol, 10.2 g) in CH₂Cl₂ (379 mL) was added EDC (45.5 mmol, 8.72 g) followed by 3*H*-[1,2,3]-triazolo[4,5-*b*]pyridin-3-ol (HOAt, 45.5 mmol, 6.19 g). After 0.5 h, *N-*methylmorpholine (114 mmol, 12.5 mL) and (S)-methyl 2-amino-3-(naphthalen-2-yl)propanoate **(304;** 37.9 mmol, 8.69 g) were added to the mixture and the resulting reaction was stirred while warming to rt over overnight. The reaction mixture was poured into EtOAc, washed with sat. aq. NaHCO₃ soln., and the aqueous layer was extracted EtOAc (3x). The combined organic extracts were washed with IN HCl and brine. The organic extracts were dried over Na₂SO₄, filtered and concentrated *in vacuo* to give an oil. The residue was purified by silica gel chromatography (ISCO, 25 g column, 20% hexanes/EtOAc - 100% EtOAc) to afford **305** (14.3 g, 81%) as an oil. MS (ESI+) rt 1.04 min, *m*/*z* 468.4.

**(S)-Methyl2-((2S,4S)-4-azidopyrrolidine-2-carboxamido)-3-(naphthalen-2-yl)propanoate, TFA.** To (2S,4S)-*tert*-butyl 4-azido-2-(((S)-1-methoxy-3-(naphthalen-2-yl)-1-oxopropan-2-yl)carbamoyl)pyrrolidine-1-carboxylate

**(305;** 30.7 mmol, 14.3 g) in CH₂Cl₂ (150 mL) was added TFA (50 mL). Reaction monitoring by TLC (1:1 EtOAc/hexanes) demonstrated conversion over 2 h, which was confirmed by LCMS (MS (ESI+) rt 0.72 min, *m*/*z* 368.3). The solvent was removed *in vacuo* and the residue was concentrated from toluene (2x) to remove the residual TFA. The crude oil **306** was taken on directly in the next step without further purification.

**(S)-Methyl2-((2S,4S)-4-azido-1-((S)-2-((tert-butoxycarbonyl)amino)-3,3-dimethylbutanoyl)pyrrolidine-2-carboxamido)-3-(naphthalen-2-yl)propanoate.** To a solution of (S)-2-((*tert*-butoxycarbonyl)amino)-3,3-dimethylbutanoic acid **(307;** 32.2 mmol, 7.44 g) in CH₂Cl₂ (306 mL) at 0 °C was added EDC (36.8 mmol, 7.05 g) followed by 3*H*-[1,2,3]-triazolo[4,5-*b*]pyridin-3-ol (HOAt; 36.8 mmol; 5.01 g). After 0.5 h, a solution of N-methylmorpholine (92 mmol, 10.1 mL) and (S)-methyl 2-((2S,4S)-4-azidopyrrolidine-2-carboxamido)-3-(naphthalen-2-yl)propanoate, TFA **(306;** 30.6 mmol, 11.26 g) was poured into the initial reaction mixture and the resulting solution was stirred while warming to rt overnight. Upon confirmation of reaction completion, (MS (ESI+) rt 1.12 min, *m*/*z* 581.4), the reaction mixture was poured into CH₂Cl₂ and washed with sat. aq. NaHCO₃ soln. The aqueous layer was extracted with EtOAc (3x) and the combined organic extracts were washed with IN HCl and sat. aq. NaCl soln. The organic extracts were dried over Na₂SO₄, filtered and concentrated *in vacuo*. The crude oil was purified by silica gel chromatography (ISCO 300 g column, hexanes - EtOAc) to afford **308** (16.3 g, 91%) as a white foam. MS (ESI+) rt 1.12 min, *m*/*z* 581.4.

**(S)-Methyl2-((2S,4S)-1-((S)-2-amino-3,3-dimethylbutanoyl)-4-azidopyrrolidine-2-carboxamido)-3-(naphthalen-2-yl)propanoate.** To (S)-methyl 2-((2S,4S)-4-azido-1-((S)-2-((*tert*-butoxycarbonyl)amino)-3,3-dimethylbutanoyl)pyrrolidine-2-carboxamido)-3-(naphthalen-2-yl)propanoate **(308;** 28 mmol; 16.3 g) in CH₂Cl₂ (150 mL) was added TFA (50 mL). After stirring the reaction mixture for 1 h (LCMS showed removal of the BOC group was complete), the solvent was removed *in vacuo*, and the resulting residue was taken up in CH₂Cl₂ solution. The organic layer was washed with sat. aq. NaHCO₃ soln., washed with 2 N HCl, dried over Na₂SO₄, filtered and concentrated *in vacuo* to give an oil. The crude product **309** was taken forward as the free base into the next step. MS (ESI+) rt 0.79 min, *m*/*z* 481.4.

**(S)-Methyl 2-((2S,4S)-4-azido-1-((S)-2-((S)-2-((*tert-*butoxycarbonyl)(methyl)amino)-propanamido)-3,3-dimethylbutanoyl)pyrrolidine-2-carboxamido)-3-(naphthalen-2-yl)propanoate.** To a solution of (S)-2-((*tert*-butoxycarbonyl)(methyl)amino)propanoic acid **(310;** 29.4 mmol, 5.98 g) in CH₂Cl₂ (280 mL) at 0 °C was added EDC (33.6 mmol , 6.45 g) followed by 3*H*-[1,2,3]-triazolo[4,5-*b*]pyridin-3-ol (HOAt, 33.6 mmol, 4.58 g). After 30 min, a solution of *N*-methylmorpholine (84 mmol, 9.24 ml) and (S)-methyl 2-((2S,4S)-1-((S)-2-amino-3,3-dimethylbutanoyl)-4-azidopyrrolidine-2-carboxamido)-3-(naphthalen-2-yl)propanoate **(309;** 28 mmol, 13.47 g) and the resulting reaction mixture was stirred overnight while warming to rt. Upon completion of the reaction (MS (ESI+) rt 1.12 min, *m*/*z* 666.5), the mixture was poured into CH₂Cl₂ and sat. aq. NaHCO₃ soln. The aqueous layer was extracted with EtOAc (3x). The combined organic extracts were washed with IN HCl and brine, dried over Na₂SO₄, filtered and concentrated *in vacuo* to give an oil. The crude residue was purified by silica gel chromatography (ISCO 300 g column, hexanes - EtOAc) to afford **311** (16.6 g, 89%) as a white foam. MS (ESI+) rt 1.12 min, *m*/*z* 666.4.

**(S)-2-((2S,4S)-4-Azido-1-((S)-2-((S)-2-((*tert-*butoxycarbonyl)(methyl)amino)-propanamido)-3,3-dimethylbutanoyl)pyrrolidine-2-carboxamido)-3-(naphthalen-2-yl)propanoic acid.** (S)-Methyl 2-((2S,4S)-4-azido-1-((*S*)-2-((*S*)-2-((*tert-*butoxycarbonyl)(methyl)amino)propan-amido)-3,3-dimethylbutanoyl)pyrrolidine-2-carboxamido)-3-(naphthalen-2-yl)propanoate **(311,** 24.99 mmol 16.64 g) was dissolved in THF (22.6 mL) / MeOH (22.6 mL) and treated with 2M LiOH (62.5 mmol, 31.2 mL). The resulting reaction mixture was stirred at rt. Upon complete conversion of the reaction (MS (ESI+) rt 1.02 min, *m*/*z* 652.5), the mixture was made acidic (pH 3) with IN HCl and the solution extracted with CH₂Cl₂ (3x). The combined organic extracts were dried over Na₂SO₄, filtered, and concentrated *in vacuo* to give **312** (16.04 g, 89%) as a white solid. MS (ESI+) rt 1.03 min, *m*/*z* 652.4. ¹H NMR (400 MHz, DMSO-*d₆*) δ 8.10 (d, *J* = 7.5 Hz, 1H), 7.92 - 7.69 (m, 3H), 7.56 - 7.28 (m, 3H), 4.68 - 4.52 (m, 2H), 4.49 - 4.21 (m, 2H), 4.07 (dd, *J* = 10.6, 6.6 Hz, 1H), 3.64 - 3.50 (m, 2H), 3.39 (dd, *J* = 10.6, 6.2 Hz, 1H), 3.22 - 3.01 (m, 2H), 2.81 - 2.63 (m, 3H), 2.45 - 2.33 (m, 1H), 1.89 - 1.65 (m, 2H), 1.48 - 1.29 (m, 9H), 1.20 (d, *J* = 6.8 Hz, 3H), 0.90 (s, 9H). Parallel HPLC: 10 to 100% over 12 min with a 3 min hold time, Solvent A = 0.05% TFA in H₂O:MeCN (95:5) to Solvent B = 0.05% TFA in H₂O:MeCN (5:95); flow = 1 mL/min Sunfire C18 3.5 um, 3.0 x 150 mm; 12.323 min 89.5% @ 220 nM 89.8% 254 nM; Xbridge Phenyl 3.5um, 3.0 x 150 mm 11.371 min 91.6% @ 220 nM 91.4% @ 254.

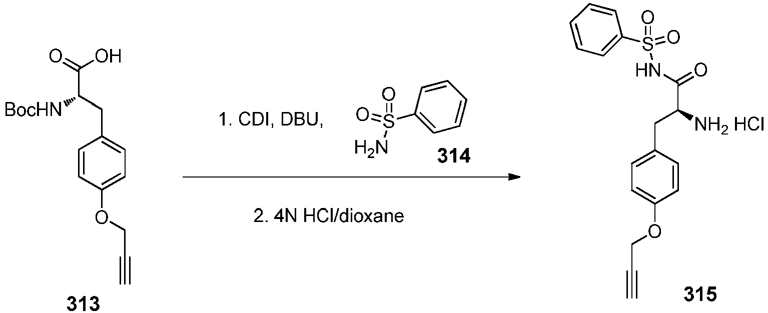

Compound **313** (319 mg, 1.0 mmol) and CDI (178 mg, 1.1 equiv., 1.1 mmol) were dissolved in THF (10 mL) and stirred at 40 °C for 1 h. A solution of benzenesulfonamide **(314,** Aldrich, 236 mg, 1.5 equiv., 1.5 mmol) in THF (2 mL) and DBU (0.3 mL, 2 equiv., 2.0 mmol) was then added to the mixture and the resulting solution was stirred at room temperature for 2 h. The reaction was then quenched with aq. HCl (1N, 10 mL) and extracted with ethyl acetate. The organic layers were concentrated *in vacuo* and the resulting residue was purified by flash column chromatography (ISCO; 40 g column eluting with 0-5% MeOH /DCM).

To the resulting acylsulfonamide intermediate was added HCl/dioxane (4N, 5 mL). The resulting mixture was stirred at room temperature for 2 h and then concentrated *in vacuo* to give intermediate **315** (312 mg, 87%) as a HCl salt. MS(ESI⁺) *m*/*z* 359.1 (M + H)⁺.

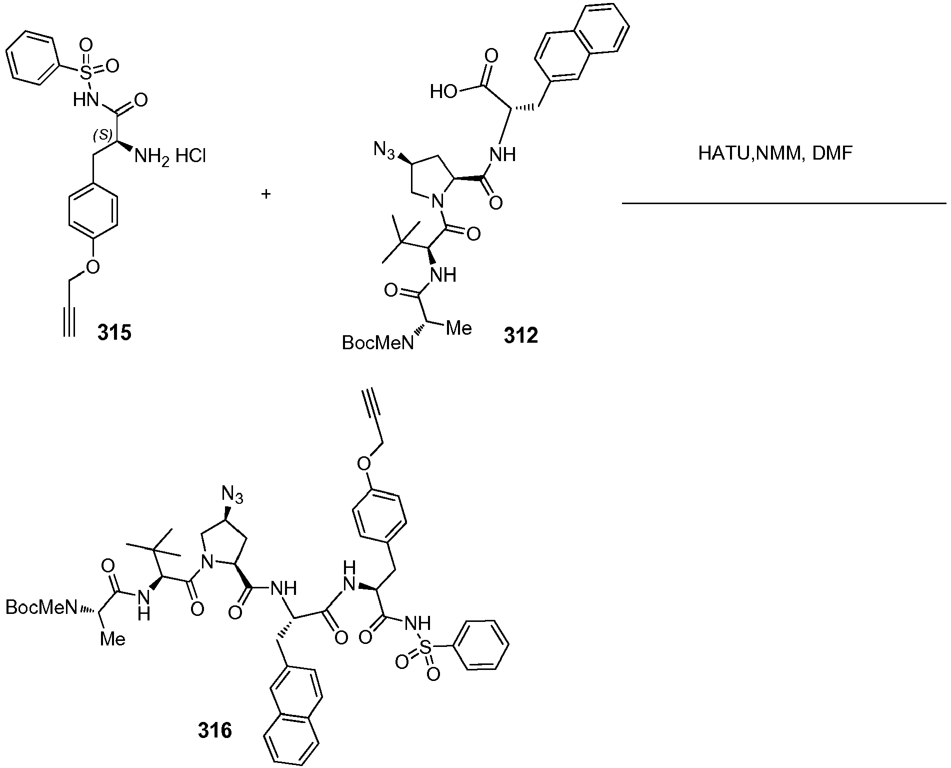

To a solution of peptide **312** (65 mg, 0.1 mmol) in DMF (1 mL) was added compound **315** (36 mg, 0.1 mmol), *N*-methylmorpholine (0.033 mL, 0.3 mmol), and HATU (38 mg, 0.1 mmol). The reaction mixture was stirred at room temperature for 2 h before it was then quenched with 10% aq. lithium chloride solution and extracted with ethyl acetate. The organic layers were washed with brine, dried over sodium sulfate, and concentrated *in vacuo*. The residue was purified by flash column chromatography (ISCO; 12 g column eluting with 0-8 % MeOH / DCM) to give intermediate **316** (25 mg, 25%) as a yellow oil. MS(ESI⁺) *m*/*z* 922.5 (M + H)⁺.

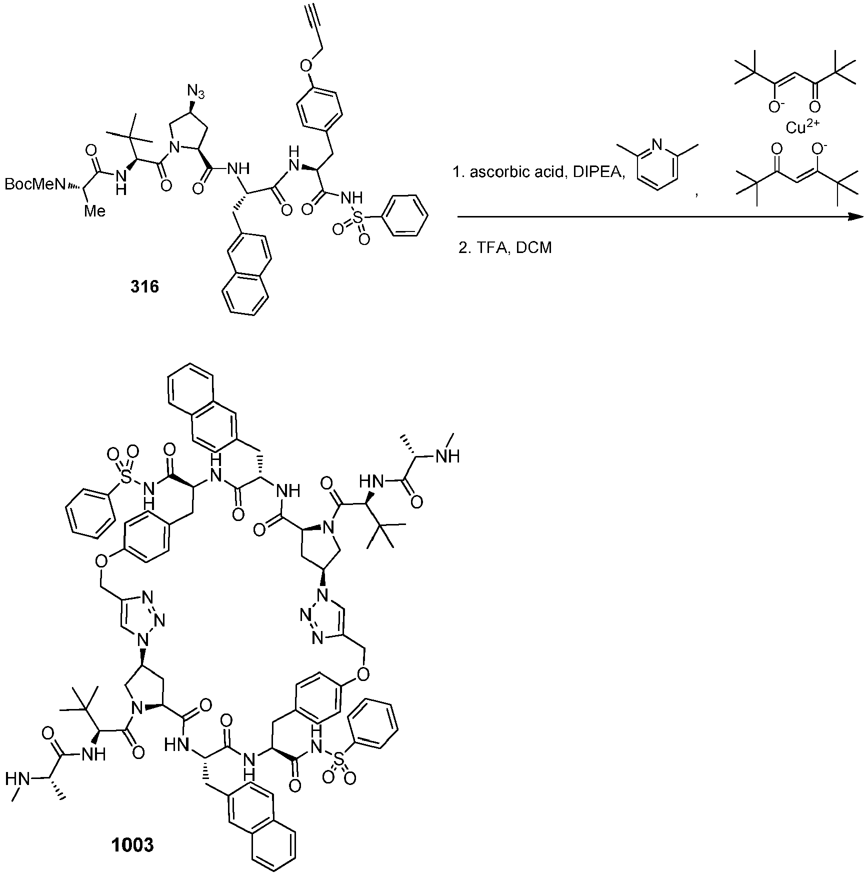

To compound **316** (25 mg, 0.025 mmol) was added a freshly made solution of copper(II) (Z)-2,2,6,6-tetramethyl-5-oxohept-3-en-3-olate (0.004 mmol, 2 mg), ascorbic acid (0.003 mmol, 5 mg), DIPEA (0.003 mmol, 10 mg), 2,6-dimethylpyridine (0.003 mmol, 12 mg) in DMF (0.8 mL) and THF (0.8 mL). The resulting mixture was stirred at room temperature for 24 h and then concentrated *in vacuo*. The residue was dissolved in 50% TFA/DCM (2 mL) and stirred at room temperature for 1 h.

The reaction mixture was then concentrated *in vacuo* and purified on a Shimadzu HPLC with a Phenomenex AXIA 5 um, 21.2 x 100 nm column, using a solvent gradient from 10% acetonitrile/water with 0.1 % TFA increasing to 40% acetonitrile/water with 0.1% TFA, followed by a rapid column flush with 100% acetonitrile/water with 0.1% TFA. The desired macrocyclic compound **1003** (3.4 mg, 15%) was isolated as a white solid. MS(ESI⁺) *m*/*z* 892.9 (M + 2H)⁺.

Macrocyclic compounds (Table Entries) **1004** to **1007** were prepared in a similar manner as described above.

### EXAMPLE 7 -- SYNTHESIS OF COMPOUND 1013 & OTHER EXEMPLARY MACROCYCLIC COMPOUNDS

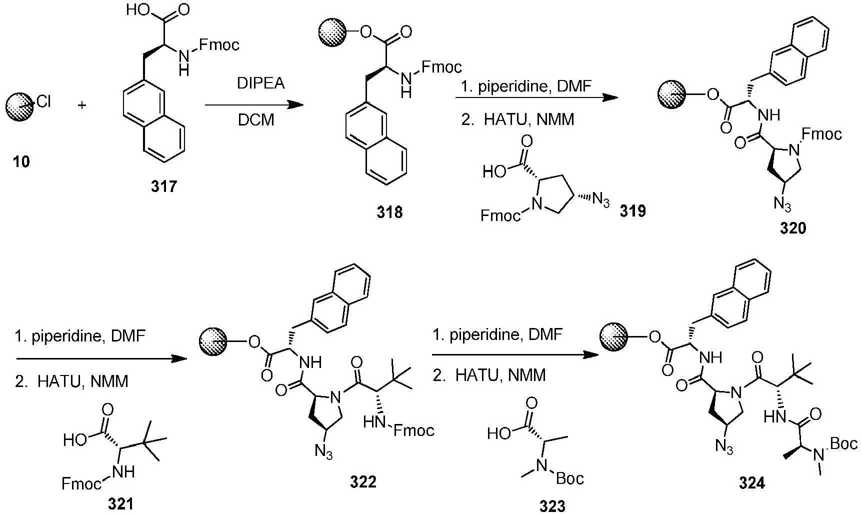

Following a procedure analogous to that for the synthesis of Compound 82 of Example 1, (S)-2-((((9*H*-fluoren-9-yl)methoxy)carbonyl)amino)-3-(naphthalen-2-yl)propanoic acid (43 mg, 0.1 mmol) was converted to the resin-linked peptide 324 using standard Fmoc solid phase peptide synthesis on the Protein Technologies' Prelude peptide synthesizer.

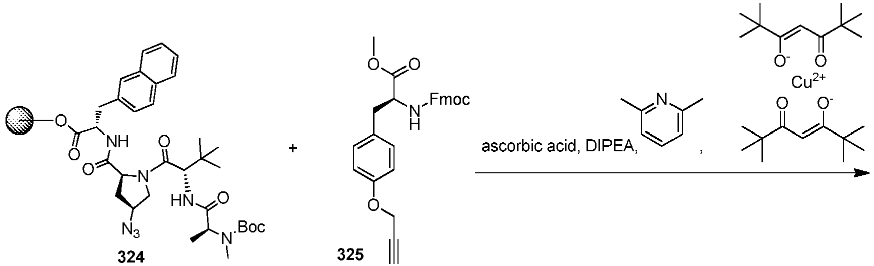

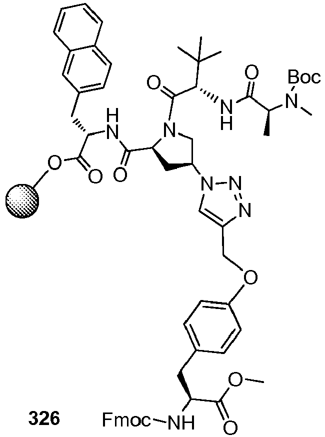

A freshly made solution of copper(II) (Z)-2,2,6,6-tetramethyl-5-oxohept-3-en-3-olate (32 mg, 0.5 equiv., 0.075 mmol), ascorbic acid (79 mg, 3 equiv., 0.45 mmol), DIPEA (0.160 g, 10 equiv., 1.5 mmol), 2,6-dimethylpyridine (0.194 g, 10 equiv., 1.5 mmol) and Fmoc tyrosine propargyl ether methylester **325** (66 mg, 1.5 equiv., 0.15 mmol) in DMF (2 mL) and THF (2 mL) was added to peptide resin **324.** The reaction mixture was agitated by a gentle stream of nitrogen for 5 h. The reagents were drained from the reaction vessel and the resin **326** was washed with DMF (15 mL x 5 min).

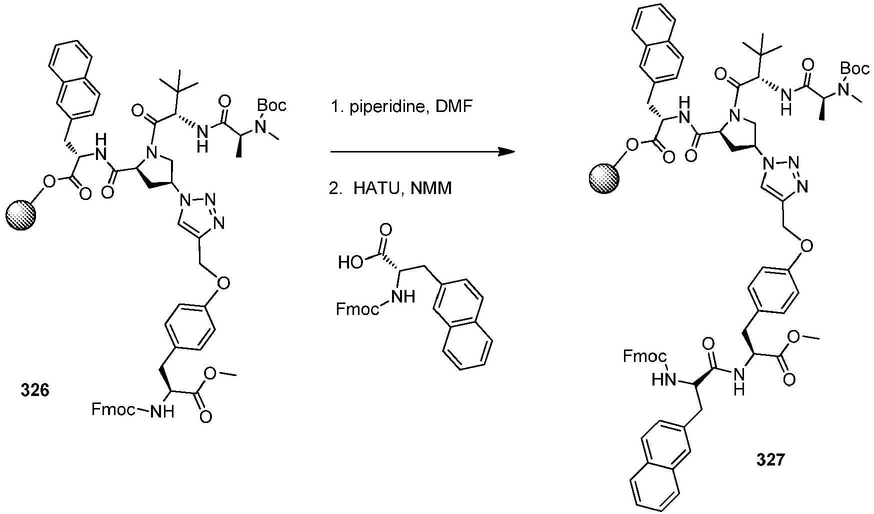

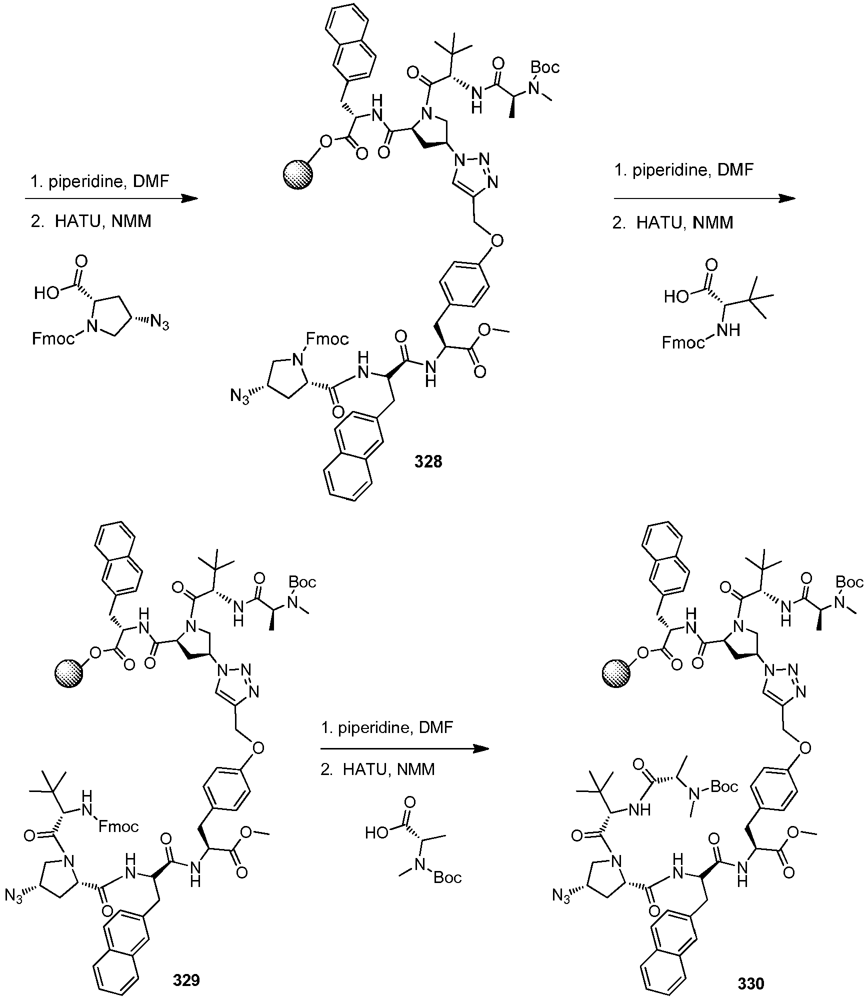

Following a procedure analogous to that for the synthesis of Compound **82** of Example 1, resin-linked peptide **326** was converted to the resin-linked peptide **330** using standard Fmoc solid phase peptide synthesis on the Protein Technologies' Prelude peptide synthesizer.

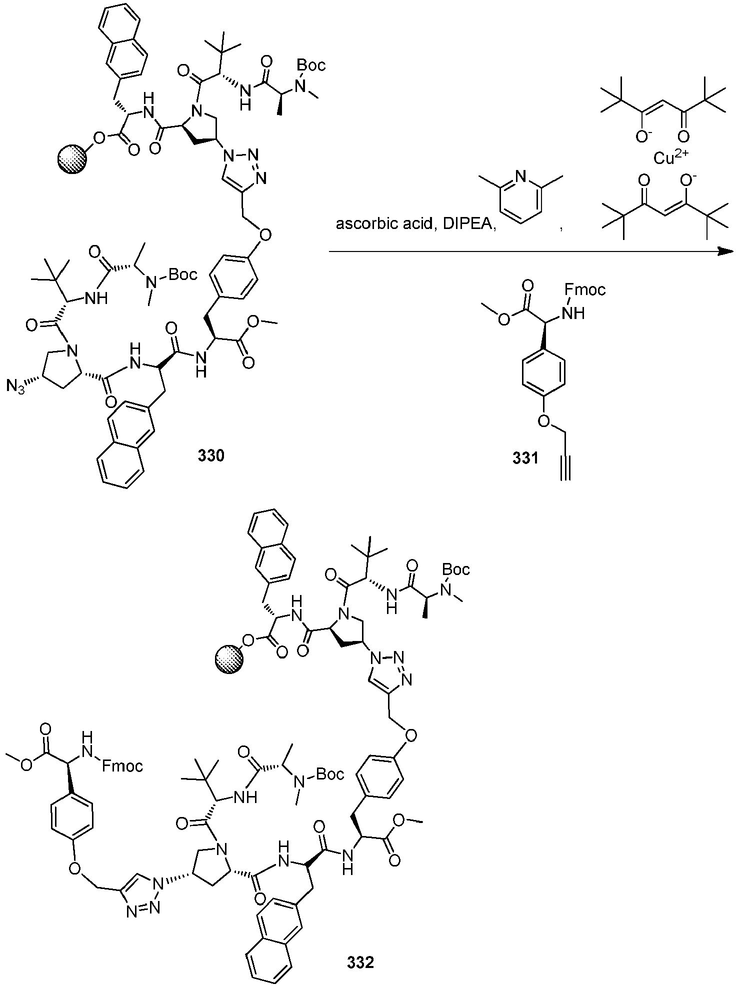

A freshly made solution of copper(II) (Z)-2,2,6,6-tetramethyl-5-oxohept-3-en-3-olate (32 mg, 0.5 equiv., 0.075 mmol), ascorbic acid (79 mg, 3 equiv., 0.45 mmol), DIPEA (0.160 g, 10 equiv., 1.5 mmol), 2,6-dimethylpyridine (0.194 g, 10 equiv., 1.5 mmol) and Fmoc amino acid **331** (66 mg, 1.5 equiv, 0.15 mmol) in DMF (2 mL) and THF (2 mL) was added to peptide resin **330.** The reaction mixture was agitated by a gentle stream of nitrogen for 5 h. The reagents were drained from the reaction vessel, and the resin **332** was washed with DMF (15 mL x 5 min).

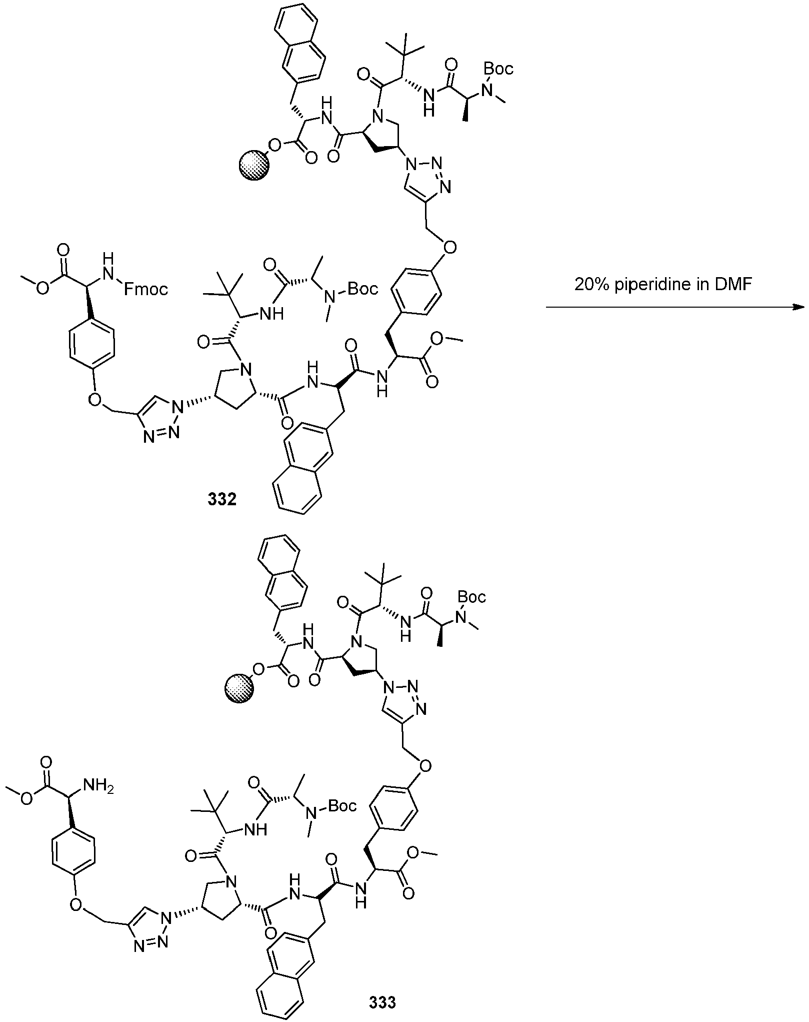

The Fmoc group was removed from the resin-supported building block **332** by mixing the resin twice with a solution of 20% piperidine in DMF (8 mL and 5 minutes per wash) while agitating with a gentle stream of N₂. The resin was washed four times with DMF (8 mL and 30 seconds per wash) to afford resin **333.**

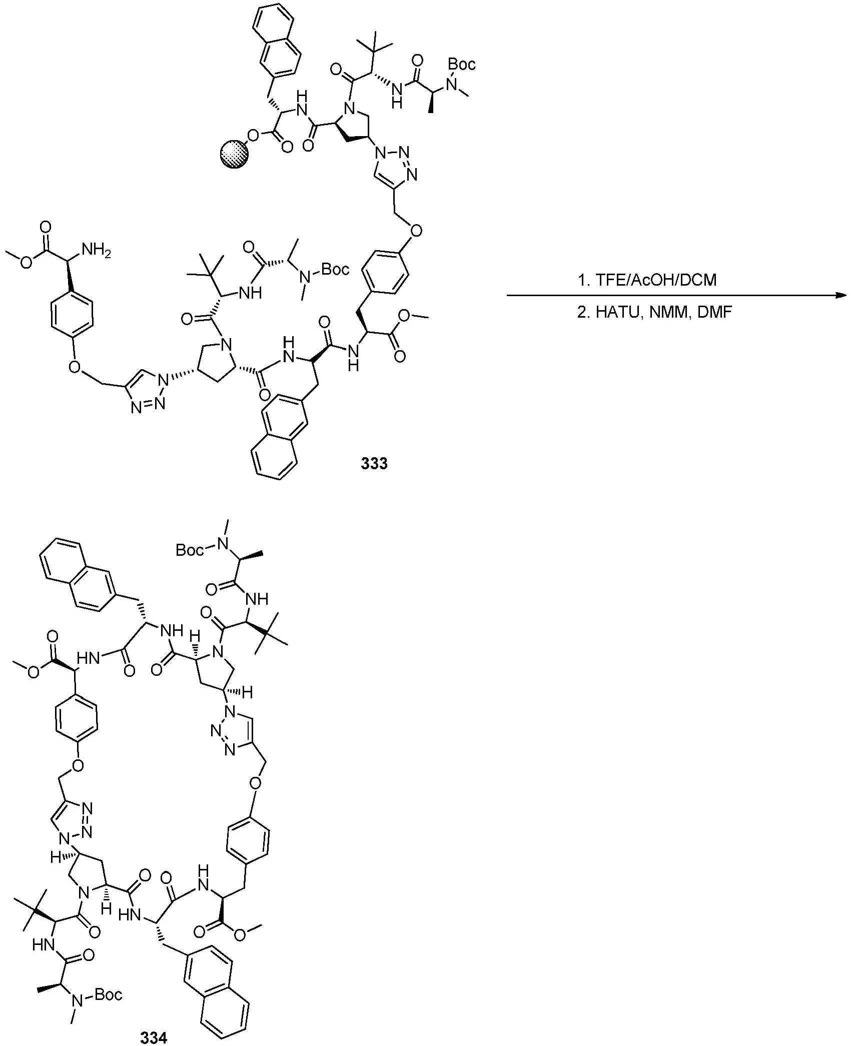

The resin **333** was washed three times with CH₂Cl₂ (15 mL and 30 seconds per wash), and then with a mixture of CH₂Cl₂:AcOH:CF₃CH₂OH (3:1:1, 20 mL). The reaction mixture was rocked for 2 h, followed by a second wash with CH₂Ch:AcOH:CF₃CH₂OH (3:1:1, 20 mL) for 30 seconds. The AcOH washings were combined and the solvents were removed *in vacuo*.

To the resulting linear peptide (15 mg, 0.009 mmol) in acetonitrile (20 mL) was added HATU (9.8 mg, 3 equiv., 0.026 mmol) and Hunig's base (0.045 mL, 3 equiv., 0.026 mmol). The reaction mixture was stirred at room temperature for 2 h and concentrated *in vacuo* to afford macrocyclic peptide **334.** MS(ESI⁺) *m*/*z* 1719.7 (M + H)⁺.

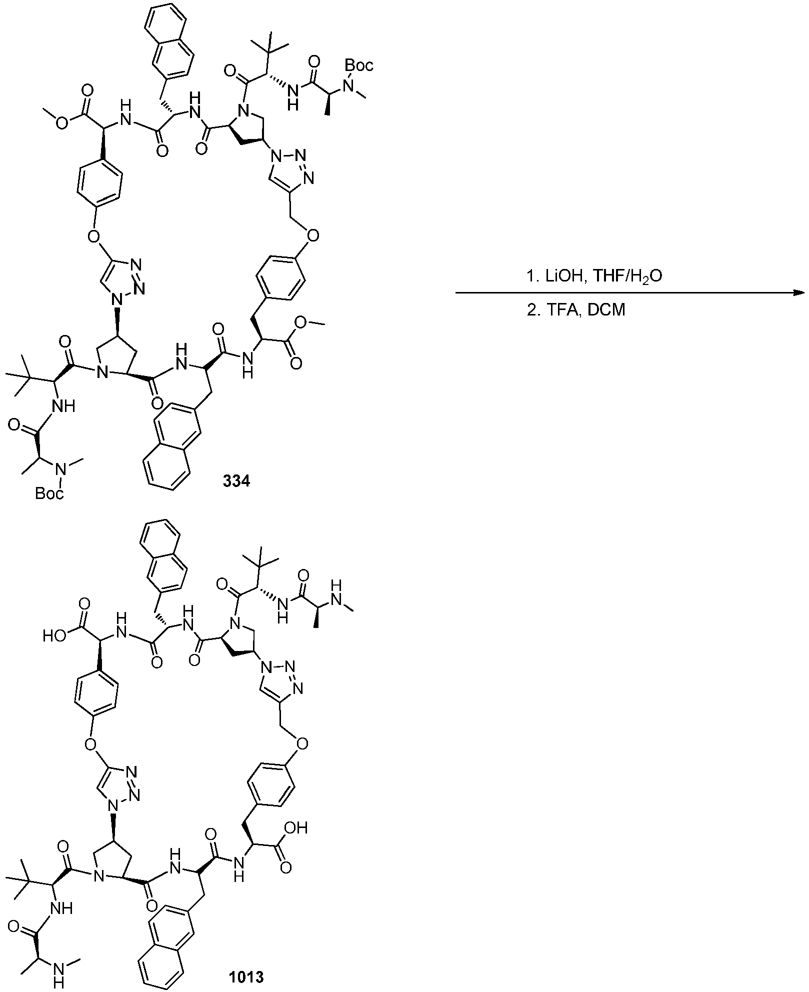

To a solution of compound **334** (8 mg) in THF (2 mL) was added aq. LiOH solution (1M, 0.3 mL). The reaction was stirred at room temperature for 3 h and then concentrated *in vacuo*. The residue was dissolved in 30% TFA in DCM (2 mL) and stirred at room temperature for 2 h. The reaction mixture was then concentrated *in vacuo* and purified on a Shimadzu HPLC with a Phenomenex AXIA 5 um, 21.2 x 100 nm column, using a solvent gradient from 10% acetonitrile/water with 0.1 % TFA increasing to 40% acetonitrile/water with 0.1% TFA, followed by a rapid column flush with 100% acetonitrile/water with 0.1% TFA to afford the desired compound **1013.** MS(ESI⁺) *m*/*z* 746.5 (M + 2H)⁺.

### EXAMPLE 8 -- SYNTHESIS OF COMPOUND 147 & OTHER EXEMPLARY MACROCYCLIC COMPOUNDS

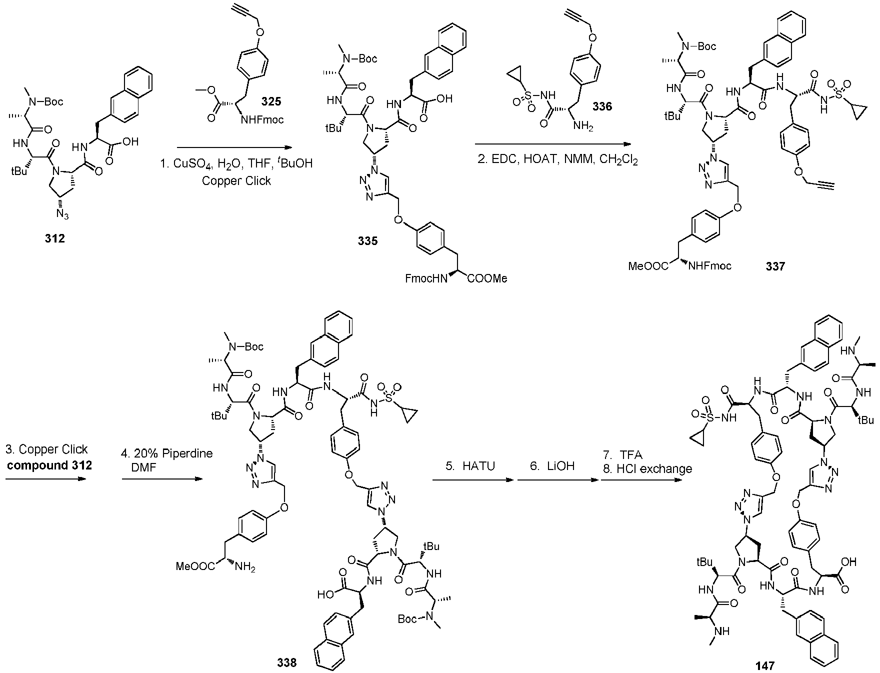

**(S)-2-((2S,4S)-4-(4-((4-((S)-2-((((9*H*-Fluoren-9-yl)methoxy)carbonyl)amino)-3-methoxy-3-oxopropyl)phenoxy)methyl)-1*H*-1,2,3-triazol-1-yl)-1-((S)-2-((S)-2-((*tert*-butoxycarbonyl)(methyl)-amino)propanamido)-3,3-dimethylbutanoyl)pyrrolidine-2-carboxamido)-3-(naphthalen-2-yl)propanoic acid.** To (S)-2-((2S,4S)-4-azido-1-((S)-2-((S)-2-((*tert-*butoxycarbonyl)(methyl)amino)-propanamido)-3,3-dimethylbutanoyl)pyrrolidine-2-carboxamido)-3-(naphthalen-2-yl)propanoic acid **(312,** 1.565 mmol, 1020 mg), (S)-methyl 2-((((9H-fluoren-9-yl)methoxy)carbonyl)amino)-3-(4-(prop-2-yn-1-yloxy)phenyl)propanoate **(325, 1.64** mmol, 749 mg) and sodium (4R,5S,Z)-2,3,4,5,6-pentahydroxyhex-2-enoate (0.391 mmol, 85 mg) in a 100 mL round bottom flask equipped with a magnetic stirrer was added *t*-BuOH (18 mL) and H₂O (9 mL) containing copper(II) sulfate pentahydrate (0.078 mmol, 19.54 mg). The solution became yellow instantly and was stirred for 5 min. THF (18 mL) was added to the mixture to solubilize the insoluble material. The reaction mixture was capped, stirred at rt with monitoring by LCMS. After 3 h, the reaction mixture was diluted with H₂O (20 mL) and extracted with EtOAc (3 x 75 mL). The organics were dried over Na₂SO₄, filtered and concentrated *in vacuo*. The residue was purified by preparative HPLC (MeCN/H₂O/TFA). The pooled fractions containing the desired product were concentrated *in vacuo* and the resulting residue was taken up in EtOAc, washed with brine, dried over Na₂SO₄, and concentrated under vacuum overnight to afford **335** (1.43 g, 83%) as white foam. MS (ESI+) rt 1.16 min, *m*/*z* 1107.6.

**(S)-Methyl2-((((9*H*-fluoren-9-yl)methoxy)carbonyl)amino)-3-(4-((1-((3S,5S)-1-((S)-2-((S)-2-((*tert*-butoxycarbonyl)(methyl)amino)propanamido)-3,3-dimethylbutanoyl)-5-(((S)-1-(((S)-1-(cyclopropanesulfonamido)-1-oxo-3-(4-(prop-2-yn-1-yloxy)phenyl)propan-2-yl)amino)-3-(naphthalen-2-yl)-1-oxopropan-2-yl)carbamoyl)pyrrolidin-3-yl)-1*H*-1,2,3-triazol-4-yl)methoxy)phenyl)propanoate.** To a solution of (S)-2-((2S,4S)-4-(4-((4-((S)-2-((((9H-fluoren-9-yl)methoxy)carbonyl)amino)-3-methoxy-3-oxopropyl)phenoxy)methyl)-1*H*-1,2,3-triazol-1-yl)-1-((S)-2-((S)-2-((*tert*-butoxycarbonyl)-(methyl)-amino)propanamido)-3,3-dimethylbutanoyl)pyrrolidine-2-carboxamido)-3-(naphthalen-2-yl)propanoic acid (**335**, 1.291 mmol, 1430 mg) in CH₂Cl₂ (12.9 mL) at 0 °C was added EDC (1.55 mmol, 297 mg) followed by 3*H*-[1,2,3]-triazolo[4,5-*b*]pyridin-3-ol (HOAt, 1.550 mmol, 211 mg). After 15 min, a solution of *N*-methylmorpholine (3.87 mmol, 0.426 ml) and (S)-*tert*-butyl (1-(cyclopropanesulfonamido)-1-oxo-3-(4-(prop-2-yn-1-yloxy)phenyl)propan-2-yl)carbamate **(336**, 1.55 mmol, 655 mg,) in CH₂Cl₂ (2 mL) was added. The reaction mixture was stirred at 0 °C and monitored by LCMS. The reaction was diluted with 2N HCl and extracted with CH₂Cl₂ (3 x 50 mL). The organic layers were combined, dried over Na₂SO₄ and concentrated *in vacuo*. Purification of the resulting waxy solid (2.5 g, crude) by preparative HPLC (C18 50 x 250 column) afforded **337** (1.51 g, 83%) as a white foam. MS (ESI+) rt 1.3 min, *m*/*z* 1412.3.

**(S)-2-((2S,4R)-4-(4-((4-((S)-2-((S)-2-((2S,4R)-4-(4-((4-((S)-2-Amino-3-methoxy-3-oxopropyl)phenoxy)methyl)-1*H*-1,2,3-triazol-1-yl)-1-((S)-2-((S)-2-((*tert*-butoxycarbonyl)(methyl)-amino)propanamido)-3,3-dimethylbutanoyl)pyrrolidine-2-carboxamido)-3-(naphthalen-2-yl)propanamido)-3-(cyclopropanesulfonamido)-3-oxopropyl)phenoxy)methyl)-1*H*-1,2,3-triazol-1-yl)-1-((S)-2-((S)-2-((*tert-*butoxycarbonyl)(methyl)amino)propanamido)-3,3-dimethylbutanoyl)-pyrrolidine-2-carboxamido)-3-(naphthalen-2-yl)propanoic acid.** To (S)-2-((2S,4S)-4-azido-1-((S)-2-((S)-2-((*tert*-butoxycarbonyl)(methyl)amino)propanamido)-3,3-dimethylbutanoyl)pyrrolidine-2-carboxamido)-3-(naphthalen-2-yl)propanoic acid **(312**, 0.326 mmol, 212 mg) and (S)-methyl 2-((((9*H*-fluoren-9-yl)methoxy)carbonyl)amino)-3-(4-((1-((3S,5*S*)-1-((S)-2-((S)-2-((*tert-*butoxycarbonyl)-(methyl)amino)propanamido)-3,3-dimethylbutanoyl)-5-(((S)-1-(((S)-1-(cyclopropanesulfonamido)-1-oxo-3-(4-(prop-2-yn-1-yloxy)phenyl)propan-2-yl)amino)-3-(naphthalen-2-yl)-1-oxopropan-2-yl)carbamoyl)pyrrolidin-3-yl)-1*H-*1,2,3-triazol-4-yl)methoxy)phenyl)propanoate **(337,** 0.326 mmol, 460 mg) and sodium (4R,5S,Z)-2,3,4,5,6-pentahydroxyhex-2-enoate (0.081 mmol, 17.61 mg) was added *t*-BuOH (10.86 mL) and H₂O (5.431 mL) containing copper(II) sulfate pentahydrate (0.016 mmol, 4.07 mg). The solution became yellow instantly. THF (18 mL) was added to solubilize the insoluble material. The reaction mixture was placed under a blanket of N₂ and stirred at rt. Additional CuSO₄-H₂O (0.5 eq) and acerbate (0.25 eq) in H₂O (1 mL) water were added to the mixture to drive the reaction to completion. Upon consumption of the starting material, the reaction was diluted with 2 N HCl and extracted with EtOAc (4 x). The combined organics were dried over Na₂SO₄, filtered, and concentrated. Purification of crude oil by preparative HPLC (21.2 x 250 column 50 - 100 % H₂O/MeCN/TFA) afforded the desired compound (600 mg, 89%) as a white solid.

The isolated material was dissolved in DMF (6 mL) and treated with piperidine (0.5 mL) to remove the FMOC group. After 3 min, the solvent was removed *in vacuo* and the resulting oil was dissolved in 2 N HCl and extracted with CH₂Cl₂ (3x). The combined organic layers were washed with 10% LiCl (2x), brine (1x), and dried over Na₂SO₄. The resulting waxy product was purified by preparative HPLC to afford the desired product **338** (320 mg, 60%). MS (ESI+) rt 1.06 min, *m*/*z* 1841.1.

**Compound 147.** To a round bottom flask charged with (S)-2-((2S,4S)-4-(4-((4-((S)-2-((S)-2-((2S,4S)-4-(4-((4-((S)-2-amino-3-methoxy-3-oxopropyl)phenoxy)-methyl)-1*H*-1,2,3-triazol-1-yl)-1-((S)-2-((S)-2-((*tert*-butoxycarbonyl)-(methyl)amino)propanamido)-3,3-dimethylbutanoyl)pyrrolidine-2-carboxamido)-3-(naphthalen-2-yl)propanamido)-3-(cyclopropane-sulfonamido)-3-oxopropyl)phenoxy)methyl)-1*H*-1,2,3-triazol-1-yl)-1-((R)-2-((S)-2-((*tert*-butoxycarbonyl)(methyl)amino)propanamido)-3,3-dimethylbutanoyl)pyrrolidine-2-carboxamido)-3-(naphthalen-2-yl)propanoic acid **(338,** 0.174 mmol, 320 mg) in CH₂Cl₂ (790 mL) was added DMF (79 mL) containing *N*-isopropyl-*N*-methylpropan-2-amine (0.521 mmol 60.1 mg) and 2-(3*H*-[1,2,3]triazolo[4,5-*b*]pyridin-3-yl)-1,1,3,3-tetramethylisouronium hexafluorophosphate(V) (0.521 mmol, 198 mg). The mixture was stirred under a blanket of N₂ overnight. Upon completion of the reaction, the solvent was removed *in vacuo* and the residue was dissolved in EtOAc. The solution was washed with 10% aq. LiCl (3x), 1 N HCl and brine, dried over Na₂SO₄, filtered and concentrated *in vacuo*. The crude oil was purified by preparative HPLC (50 to 100% H₂O/MeCN/TFA to MeCN/TFA). The fractions containing the product were combined and concentrated *in vacuo* to afford the desired intermediate (110 mg, 35%) as a white solid; MS (ESI+) rt 1.20 min, *m*/*z* 1824.8.

The isolated material was dissolved in THF (1500 µL) and MeOH (1500 µL) and treated with 2M LiOH (0.200 mmol, 100 µl). The resulting solution was stirred for 30 min and concentrated *in vacuo* to afford a solid, which was immediately taken up in IN HCl (5 mL). The resulting solution was extracted with EtOAc (3 x 15 mL), and the combined organics were concentrated *in vacuo*. The residue was immediately taken up in 40% TFA/CH₂Cl₂ and stirred at rt. Upon completion of the deprotection step, the solvent was removed *in vacuo*. The residue was purified by preparative HPLC (10% to 100 over 45 min; 30 x 100 Phenominex Luna column; MeCN/H₂O/TFA). The fractions containing the desired product were combined and lyophilized in the presence of IN HCl to afford **147** (68 mg, 94%) as a white solid. LCMS: rt 0.80 min, M+2H = 805.4 M+1 1609.4 consistent with MW 1607.7 (Aquity, BEH C18 2.1 x 50 mm 1.7u); HPLC: YMC Pack ODS-AQ 3 um 150 x 4.6 mm (0415172016(W)), 0-100% B, 15 min gradient, 1.25 mL/min flow rate (rt 8.261min, 94.1%); mobile A (0.1% TFA water), mobile B (0.1% TFA MeCN).

### EXAMPLE 9 -- SYNTHESIS OF INTERMEDIA 343 AND EXEMPLARY MACROCYLIC COMPOUNDS

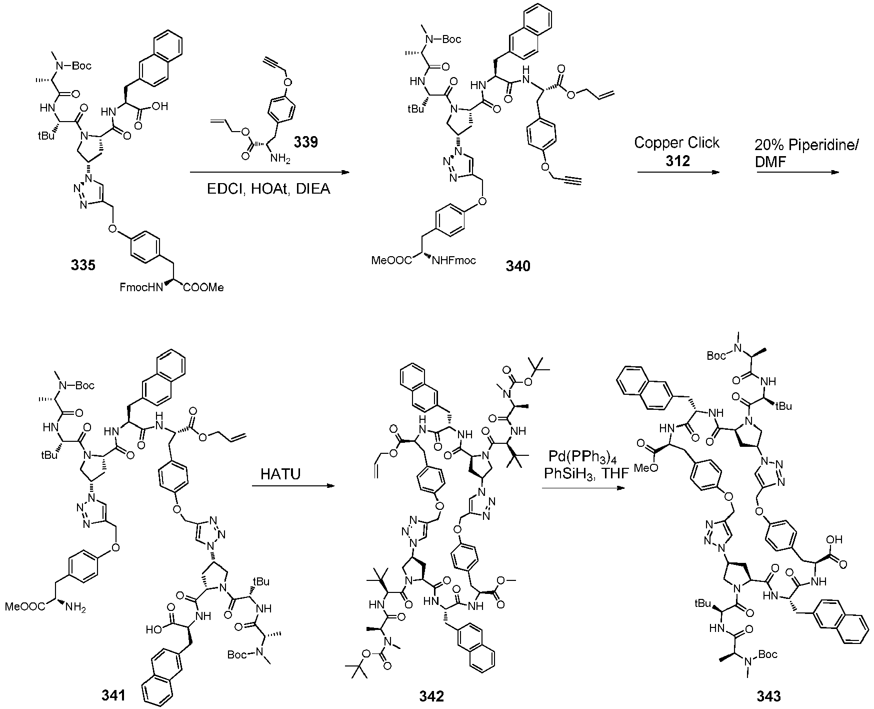

**(S)-2-((2S,4S)-4-(4-((4-((S)-2-((((9*H*-Fluoren-9-yl)methoxy)carbonyl)amino)-3-methoxy-3-oxopropyl)phenoxy)methyl)-1*H*-1,2,3-triazol-1-yl)-1-((S)-2-((S)-2-((*tert*-butoxycarbonyl)(methyl)-amino)propanamido)-3,3-dimethylbutanoyl)pyrrolidine-2-carboxamido)-3-(naphthalen-2-yl)propanoic** acid. A suspension of copper(II) sulfate pentahydrate (0.038 mmol, 9.58 mg) and sodium (4R,5S,Z)-2,3,4,5,6-pentahydroxyhex-2-enoate (0.192 mmol, 41.4 mg) in H₂O (3.00 mL) was shaken until the solution became dark brown and a white solid precipitated. This premade-slurry was added to a *t*-BuOH (6 mL) and THF (6 mL) solution with (S)-2-((2S,4S)-4-azido-1-((S)-2-((S)-2-((*tert-*butoxycarbonyl)(methyl)amino)propanamido)-3,3-dimethylbutanoyl)pyrrolidine-2-carboxamido)-3-(naphthalen-2-yl)propanoic acid **(312,** 0.767 mmol, 500 mg) and (S)-methyl 2-((((9*H*-fluoren-9-yl)methoxy)carbonyl)amino)-3-(4-(prop-2-yn-1-yloxy)phenyl)propanoate **(325,** 0.806 mmol, 367 mg). The reaction mixture was allowed to stir at rt for 40 min (LC-MS showed 80% conversion). In order to drive the reaction to completion, additional copper (II) sulfate pentahydrate (0.038 mmol, 9.58 mg) was added, and the reaction mixture was allowed to stir at rt for an additional hour (LCMS shows completed conversion). The mixture was poured into EtOAc (100 mL) and washed with cold 0.5N HCl. The organic layer was then concentrated *in vacuo* and purified by reverse phase preparative HPLC (Column: Phen-Luna Axia C18 5 u 30 x 250 mm; Solvent A: 95%H₂O/5%MeCN/0.05%TFA; Solvent B: 95%MeCN/ 5%H₂O/0.05%TFA; 45%-100% gradient in 25 min, stop at 32 min) to afford **335** as a white solid (655 mg, 76%). MS (ESI+) rt 1.15 min, *m*/*z* 1107.6.

**(S)-Allyl 2-((S)-2-((2S,4S)-4-(4-((4-((S)-2-((((9*H*-fluoren-9-yl)methoxy)carbonyl)amino)-3-methoxy-3-oxopropyl)phenoxy)methyl)-1*H*-1,2,3-triazol-1-yl)-1-((S)-2-((S)-2-((*tert*-butoxycarbonyl)(methyl)amino)propanamido)-3,3-dimethylbutanoyl)pyrrolidine-2-carboxamido)-3-(naphthalen-2-yl)propanamido)-3-(4-(prop-2-yn-1-yloxy)phenyl)propanoate.** To a solution of (S)-2-((2S,4S)-4-(4-((4-((S)-2-((((9*H*-fluoren-9-yl)methoxy)carbonyl)amino)-3-methoxy-3-oxo-propyl)phenoxy)methyl)-1*H*-1,2,3-triazol-1-yl)-1-((S)-2-((S)-2-((*tert-*butoxycarbonyl)(methyl)amino)-propanamido)-3,3-dimethylbutanoyl)pyrrolidine-2-carboxamido)-3-(naphthalen-2-yl)propanoic acid **(335,** 0.592 mmol, 655 mg) in CH₂Cl₂ (12 mL) at 0 °C was added EDC (0.887 mmol, 170 mg) followed by 3H-[1,2,3]-triazolo[4,5-*b*]pyridin-3-ol (HOAt, 0.887 mmol, 121 mg). After 15 min, a solution of *N*-methylmorpholine (2.37 mmol, 0.260 ml) and (S)-allyl 2-amino-3-(4-(prop-2-yn-1-yloxy)phenyl)propanoate **(339**, 0.769 mmol, 199 mg) in CH₂Cl₂ (6 mL) was added. The solution was stirred at 0 °C, allowed to warm to rt, and stirred at rt for 3 h (MS (ESI+) rt 1.24 min, *m*/*z* 1349.5). The crude material was poured into EtOAc (100 mL), washed with cold 0.5N HCl, and the organics were concentrated to dryness. The resulting residue was purified by reverse phase preparative HPLC (Phen-Luna Axia C18 5 u 21 x 250 mm, Solvent A: 95%H₂O/5%MeCN/0.05%TFA; Solvent B: 95%MeCN/ 5%H₂O/0.05%TFA, 45%-100% gradient in 25 min) to afford **340** (750 mg, 94%) as a white solid. MS (ESI+) rt 1.24 min, *m*/*z* 1349.8.

**(S)-2-((2S,4S)-4-(4-((4-((S)-2-((S)-2-((2S,4S)-4-(4-((4-((S)-2-((((9*H-*Fluoren-9-yl)methoxy)-carbonyl)amino)-3-methoxy-3-oxopropyl)phenoxy)methyl)-1*H*-1,2,3-triazol-1-yl)-1-((S)-2-((S)-2-((*tert-*butoxycarbonyl)(methyl)amino)propanamido)-3,3-dimethylbutanoyl)pyrrolidine-2-carboxamido)-3-(naphthalen-2-yl)propanamido)-3-(allyloxy)-3-oxopropyl)phenoxy)methyl)-1*H*-1,2,3-triazol-1-yl)-1-((S)-2-((S)-2-((tert-butoxycarbonyl)(methyl)amino)propanamido)-3,3-dimethylbutanoyl)pyrrolidine-2-carboxamido)-3-(naphthalen-2-yl)propanoic acid.** A suspension of copper (II) sulfate pentahydrate (0.093 mmol, 0.023g) and sodium (4R,5S,Z)-2,3,4,5,6-pentahydroxyhex-2-enoate (0.463 mmol, 100 mg) in H₂O (5 mL) was shaken until the mixture turned dark brown and a white solid precipitated. This premade slurry was added to a *t*-BuOH (10 mL) and THF (10 mL) solution of starting (S)-2-((2S,4S)-4-azido-1-((S)-2-((S)-2-((*tert*-butoxycarbonyl)(methyl)amino)-propanamido)-3,3-dimethylbutanoyl)pyrrolidine-2-carboxamido)-3-(naphthalen-2-yl)propanoic acid (**312,** 1.27 mmol, 829 mg) and (S)-allyl 2-((S)-2-((2S,4S)-4-(4-((4-((S)-2-((((9*H*-fluoren-9-yl)methoxy)carbonyl)amino)-3-methoxy-3-oxopropyl)phenoxy)methyl)-1*H*-1,2,3-triazol-1-yl)-1-((S)-2-((S)-2-((tert-butoxycarbonyl)(methyl)amino)propanamido)-3,3-dimethylbutanoyl)pyrrolidine-2-carboxamido)-3-(naphthalen-2-yl)propanamido)-3-(4-(prop-2-yn-1-yloxy)phenyl)propanoate (**340,** 1.16 mmol, 1.56 g). The reaction mixture was allowed to stir at rt for 40 min before additional copper (II) sulfate pentahydrate (0.093 mmol, 23 mg) was added. The reaction mixture was stirred at rt for and 1 h. Upon completion of the reaction, the mixture was poured into EtOAc (100 mL) and washed with cold 0.5N HCl. The organic layer was concentrated *in vacuo* and the crude material was purified by reverse phase preparative HPLC (Column: Phen-Luna Axia C18 5 u 30 x 250 mm, Solvent A: 95%H₂O/5%MeCN/0.05%TFA; Solvent B: 95%MeCN/ 5% H₂O /0.05%TFA, 45%-100% gradient in 25 min) to afford the desired intermediate (2.3 g, 80%) as a colorless oil, which was used directly in the next step. MS (ESI+) rt 1.28 min, *m*/*z* 1001.1 (M+2).

The above intermediate (0.98 mmol, 2.3 g) was dissolved into CH₂Cl₂ (30 mL) and treated with piperidine (6 mL) at rt. The resulting mixture was allowed to stir at rt for 2 h. The solvent was evaporated and the crude material was purified by reverse phase preparative HPLC (Column: Phen-Luna Axia C18 5 u 30 x 250 mm; Solvent A: 95% H₂O/5%MeCN/0.05%TFA; Solvent B: 95%MeCN/ 5% H₂O/0.05%TFA; 15%-100% gradient in 25 min) to afford **341** (1.5 g, 85%) as a white solid. MS (ESI+) rt 1.12 min, *m*/*z* 890.0 (M+2).

**Compound 342.** Linear amino acid compound **341** (0.422 mmol, 750 mg) was dissolved into CH₂Cl₂ (1600.0 mL). To this solution was added *N*-ethyl-*N-*isopropylpropan-2-amine (1.27 mmol, 164 mg) and a solution of 2-(3*H-*[1,2,3]triazolo[4,5-*b*]pyridin-3-yl)-1,1,3,3-tetramethylisouronium hexafluorophosphate(V) (1.27 mmol, 481 mg) in DMF (160 mL). The resulting mixture was allowed to stir at rt for 36 h. The solvent was evaporated and the residue was dissolved in EtOAc (300mL). The organics were washed with cold 0.5N HCl and brine. The aqueous layer was subsequently extracted with EtOAc one more time, and the combined organic layers were concentrated *in vacuo*. The crude, poorly soluble material was purified by reverse phase preparative HPLC (Column: Phen-Luna Axia C18 5 u 30 x 100 mm; Solvent A: 95% H₂O/5%MeCN/0.05%TFA; Solvent B: 95%MeCN/ 5% H₂O/0.05%TFA; 65%-100% gradient in 10 min) to afford **342** (500 mg, 67%) as a white solid.

**Compound 343.** A solution of macrocyclic product **342** (0.131 mmol, 230 mg) in anhydrous THF (4 mL) was placed under nitrogen, cooled to 0 °C, and treated with phenylsilane (0.78 mmol, 0.097 ml) and tetrakis(triphenylphosphine)palladium(0) (0.013 mmol, 15.10 mg). The reaction mixture was allowed to warm to rt and stirred at rt for 2 h, at which point the starting material was consumed and the desired product was observed by LCMS - MS (ESI+) rt 1.23 min, *m*/*z* 1721.3. The crude material was poured into EtOAc (150 mL) and washed with cold 0.5N HCl. The organic layer was concentrated *in vacuo* and the crude material was purified by reverse phase preparative HPLC (Column: Phen-Luna Axia C18 5 u 30 x 100 mm; Solvent A: 95%H₂O/5%MeCN/0.05%TFA; Solvent B: 95%MeCN/ 5%H₂O/0.05%TFA; 55%-100% gradient in 12 min) to afford **343** (126 mg, 56%) as a white solid. MS (ESI+) rt 1.23 min, *m*/*z* 1721.3.

**Compounds 1018, 1035-1037, 1039, and 1045-1046. CDI Method:** Compound **343** (1.0 equiv) and CDI (1.1 equiv., 1.1 mmol) was dissolved in THF (0.11 M) and stirred at rt to -40 °C for 1-2 h. A solution of amide (1.5 equiv.) in THF (0.75 M) and DBU (2 equiv.) was then added and the resulting solution was stirred at room temperature for 2 h to 2 d. The reaction was then quenched with aq. IN HCl (0.11 M) and extracted with EtOAc. The organic layers were concentrated *in vacuo* and 0.5 mL of 2M aq. LiOH soln. was added to the residue. The mixture was allowed to stir overnight at room temperature then was evaporated to dryness. The resulting solids were dissolved in 3 mL of 40% TFA/DCM, agitated on a platform for 3 h at room temperature and evaporated to dryness. The solids were redissolved in DMF and purified by preparative HPLC.

**Compounds 1019-1034, 1038, 1042. HATU Method:** A vial was charged with 500 uL of a stock solution of the cyclic peptide **343** in DMF, 100 uL of a stock solution of HATU in DMF and 50 uL of DIEA. The reaction mixture was agitated on a platform shaker overnight at room temperature then 0.5 mL of 2M aq. LiOH soln. was added to the mixture. This mixture was allowed to stir overnight at room temperature then was evaporated to dryness. The resulting solids were dissolved in 3 mL of 40% TFA/DCM, agitated on a platform for 3 h at room temperature and evaporated to dryness. The solids were redissolved in DMF and purified by preparative HPLC.

### EXAMPLE 10-- SYNTHESIS OF COMPOUNDS (TABLE ENTRIES) 1040-1041

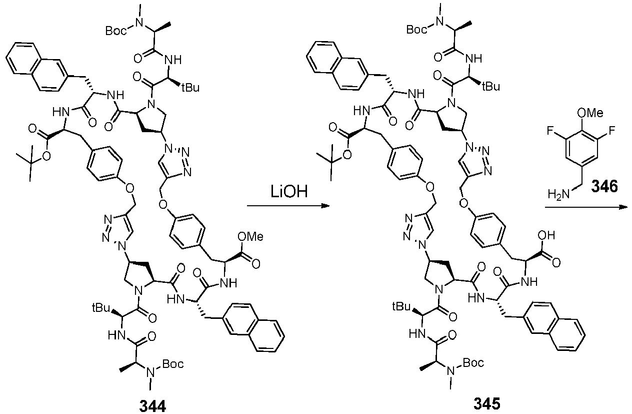

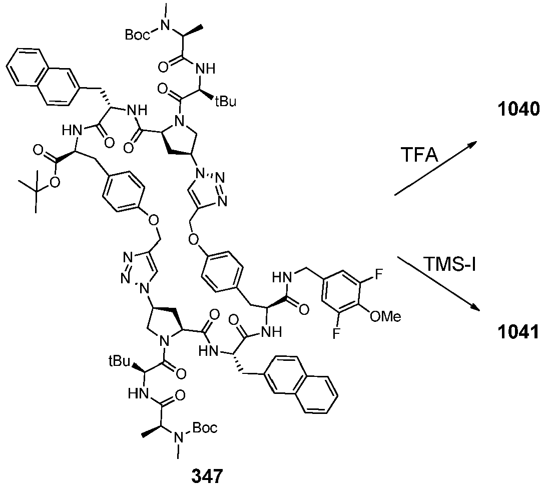

**Acid 345.** The macrocycle **344** (0.141 mmol, 250 mg) was dissolved in THF (4 mL) and MeOH (1 mL), and treated with aq. LiOH solution (0.352 mmol, 0.176 mL). The mixture was stirred at rt for 20 min to achieve selective hydrolysis of the methyl ester. The reaction was quenched with cold IN HCl and extracted with EtOAc. The organic layer was dried over Na₂SO₄, filtered and concentrated *in vacuo* to provide the **345** (70 mg, 28%) as a yellow oil. This material was used directly in the next step without further purification.

**Amide 347.** A solution of acid **345** (0.020 mmol, 35 mg) in DMF (1.5 mL) was treated with 3,5-difluoro-4-methoxyphenyl)methanamine **(346,** 0.060 mmol, 10.32 mg), HATU (0.040 mmol, 0.099 mL) and Hunig's Base (0.099 mmol, 0.017 mL). The reaction mixture was stirred at rt for 2 d, poured into EtOAc (60 mL) and washed with cold 0.5N HCl. The organic layer was dried over Na₂SO₄, filtered, and concentrated under vacuum to provide **347** (36 mg, 76%).

**Compound 1040.** The amide **347** (4.17 µmol, 8 mg) was dissolved in CH₂Cl₂ (1.2 mL) and treated with TFA (7.79 mmol, 0.6 mL). The reaction mixture was stirred for 3 h at rt and concentrated *in vacuo*. The residue was purified by reverse phase preparative HPLC (Column: Phen-Luna Axia C18 5 u 30 x 100 mm; Solvent A: 95%H₂O/5%MeCN/0.05%TFA; Solvent B: 95%MeCN/ 5%H₂O/0.05%TFA; 5%-100% gradient in 15 min) to afford the desired product **1040** (1.12 mg, 13%) as a white solid. MS (ESI+) rt 0.81 min, *m*/*z* 831.4 (M+2).

**Compound 1041.** The amide **347** (5.22 µmol, 10 mg) was dissolved in CH₂Cl₂ (1.5 mL) and treated with TMS-I (0.052 mmol, 7.10 µl). The reaction mixture was stirred at rt for 2 d and treated with cold 0.5N HCl (5 mL). The mixture was stirred for 30 min and extracted with EtOAc (2x). The organic layers were concentrated *in vacuo* and the residue was purified by reverse phase preparative HPLC (Column: Phen-Luna Axia C18 5 u 30 x 100 mm; Solvent A: 95%H₂O/5%MeCN/0.05%TFA; Solvent B: 95%MeCN/ 5%H₂O/0.05%TFA; 5%-100% gradient in 15 min) to afford the desired product **1041** (0.76 mg, 7%) as a white solid. MS (ESI+) rt 0.76 min, *m*/*z* 824.1 (M+2).

### EXAMPLE 11-- SYNTHESIS OF COMPOUND 1043

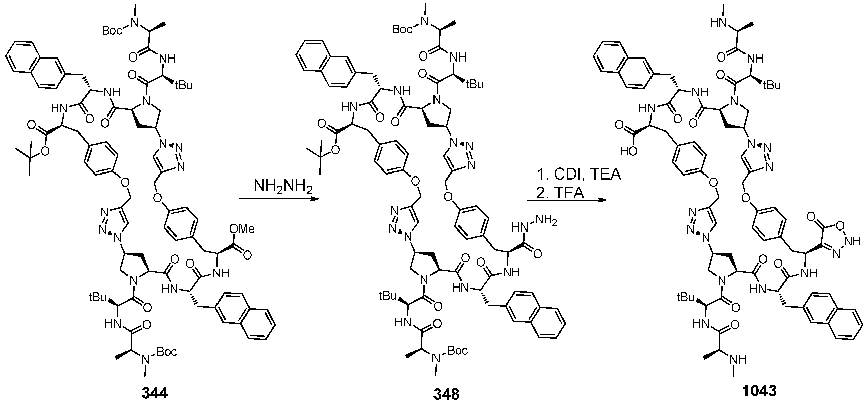

Acyl hydrazide 348. (based on J. Med. Chem. 2008, 51, 4430-4448). Intermediate **344** (0.016 mmol, 28 mg) was treated with hydrazine-H₂O (0.236 mmol, 11.84 mg) and the resulting mixture was warm to 85 °C for 4 h, cooled to rt, and stirred overnight. The crude mixture was poured into EtOAc (4 mL), stirred well and concentrated *in vacuo*. The crude residue was purified by reverse phase preparative HPLC (Column: Phen-Luna Axia C18 5 u 30 x 100 mm; Solvent A: 95%H₂O/5%MeCN/0.05%TFA; Solvent B: 95%MeCN/ 5%H₂O/0.05%TFA, 10%-100% gradient in 15 min) to afford the desired product **348** (9.3 mg, 33%) as a white solid.

**Compound 1043.** The acyl hydrazide **348** (5.12 µmol, 9.1 mg) was dissolved in THF (1.0 mL) and treated with CDI (0.023 mmol, 3.74 mg) and TEA (0.015 mmol, 2.142 µl). The mixture was heated to 83°C for 5 h (LCMS - MS (ESI+) rt 1.20 min, *m*/*z* 1802.3). The reaction mixture was poured into EtOAc (20 mL) and washed with cold 0.5N HCl. The organic layer was dried over Na₇SO₄ and concentrated *in vacuo*. The residue was redissolved in CH₂Cl₂ (1.0 mL) and TFA (0.6 mL) was added to the solution. The reaction mixture was stirred overnight at rt and concentrated *in vacuo*. The residue was purified by reverse phase preparative HPLC (Column: Phen-Luna Axia C18 5 u 30 x 100 mm, Solvent A: 95%H₂O/5%MeCN/0.05%TFA; Solvent B: 95%MeCN/ 5%H₂O/0.05%TFA; 5%-100% gradient in 15 min) to afford 1043 (1.61 mg, 17%) as a white solid. MS (ESI+) rt 0.74 min, *m*/*z* 1546.6.

### EXAMPLE 12-- SYNTHESIS OF COMPOUND 1044

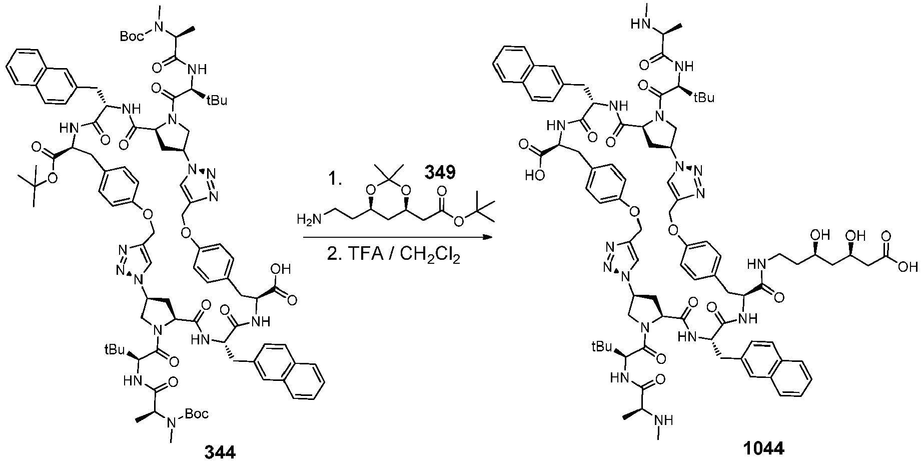

Compound 1044. Compound 344 (0.014 mmol, 25 mg), *tert*-butyl 2-((4R,6R)-6-(2-aminoethyl)-2,2-dimethyl-1,3-dioxan-4-yl)acetate (0.021 mmol, 5.82 mg), HATU (0.043 mmol, 16.18 mg) and Hunig's Base (0.071 mmol, 0.012 ml) in DMF (1.5 mL) were combined in a reaction vial. The reaction mixture was stirred overnight at rt. The solution was poured into EtOAc and washed with 0.5N HCl. The organic layer was dried over Na₇SO₄ and concentrated *in vacuo*. The resulting residue was dissolved in CH₂Cl₂ (0.8 mL), TFA (0.8 mL) and H₂O (0.2 mL). The mixture was stirred overnight at rt and concentrated *in vacuo*. The resulting residue was purified by reverse phase preparative HPLC (Column: Phen-Luna Axia C185 u 30 x 100 mm; Solvent A: 95%H₂O/5%MeCN/0.05%TFA; Solvent B: 95%MeCN/ 5%H₂O/0.05%TFA; 5%-100% gradient in 15 min) to afford **1044** (2.49 mg, 8%) as a white solid. MS (ESI+) rt 0.72 min, *m*/*z* 1646.3.

### EXAMPLE 13-- SYNTHESIS OF COMPOUND 356

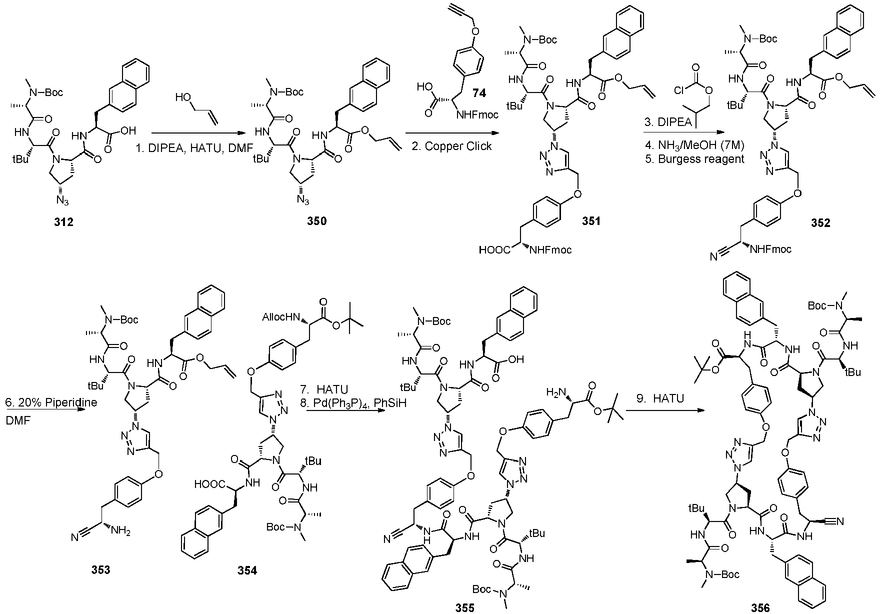

**(S)-Allyl 2-((2S,4S)-4-azido-1-((S)-2-((S)-2-((*tert-*butoxycarbonyl)(methyl)amino)-propanamido)-3,3-dimethylbutanoyl)pyrrolidine-2-carboxamido)-3-(naphthalen-2-yl)propanoate.** (S)-2-((2S,4S)-4-Azido-1-((S)-2-((S)-2-((*tert-*butoxycarbonyl)(methyl)amino)propanamido)-3,3-dimethylbutanoyl)pyrrolidine-2-carboxamido)-3-(naphthalen-2-yl)propanoic acid **(312,** 1.949 mmol, 1.27 g) and prop-2-en-1-ol (29.2 mmol, 1.99 mL) were dissolved in DMF (8.58 mL) and the resulting solution was cooled to 0 °C. The solution was treated with *N,N*-diisopropylethylamine (3.90 mmol, 0.679 mL) and O-(7-azabenzotriazol-1-yl)-*N*,*N*,*N*',*N*'-tetramethyluronium hexafluorophosphate (2.14 mmol, 0.815 g), stirred at 0 °C, and slowly warmed to rt. Upon completion of the reaction, the mixture was poured into aq. NaHCO₃ soln. and extracted with EtOAc (2x). The organics were washed with IN HCl and 10% aq. LiCl (2x), dried over MgSO₄, filtered and concentrated *in vacuo*. The crude oil was purified by flash column chromatography (ISCO 24 g column, hexanes to EtOAc gradient) to afford the desired product **350** (1.087g, 81%). MS (ESI+) rt 1.52 min, *m*/*z* 692.4.

**(S)-2-((((*9*H-Fluoren-9-yl)methoxy)carbonyl)amino)-3-(4-((1-((3S,5S)-5-(((S)-1-(allyloxy)-3-(naphthalen-2-yl)-1-oxopropan-2-yl)carbamoyl)-1-((S)-2-((S)-2-((*tert*-butoxycarbonyl)(methyl)-amino)propanamido)-3,3-dimethylbutanoyl)pyrrolidin-3-yl)-1H-1,2,3-triazol-4-yl)methoxy)-phenyl)propanoic acid.** A round bottom flask was charged with (S)-allyl 2-((2S,4S)-4-azido-1-((S)-2-((S)-2-((*tert*-butoxycarbonyl)-(methyl)amino)propanamido)-3,3-dimethylbutanoyl)pyrrolidine-2-carboxamido)-3-(naphthalen-2-yl)propanoate **(350,** 1.561 mmol, 1.08 g), (S)-2-((((9H-fluoren-9-yl)methoxy)carbonyl)-amino)-3-(4-(prop-2-yn-1-yloxy)phenyl)propanoic acid **(74**, 1.561 mmol, 0.689 g), *t*-BuOH (25 mL), THF (25 mL). To this mixture was added H₂O (12.50 mL) containing copper (II) sulfate pentahydrate (0.078 mmol, 0.019 g) and sodium (4R,5S,Z)-2,3,4,5,6-pentahydroxyhex-2-enoate (0.390 mmol, 0.084 g). The yellow reaction mixture was stirred at rt until the azide starting material was consumed. The mixture was poured into 1 N HCl (5 mL) and EtOAc (30 mL). The solution was extracted with EtOAc (3 x 35 mL) and the combined organic layers were dried over MgSO₄, filtered and concentrated *in vacuo*. The crude solid was dissolved in CH₂Cl₂, adhered to silica gel, and purified by flash column chromatography (ISCO, 24 g column, CH₂Cl₂ to 10% MeOH/CH₂Cl₂ gradient) to afford **351** (1.69 g, 96%) as a foam.

**(S)-Allyl 2-((2S,4S)-4-(4-((4-((S)-2-((((9*H*-fluoren-9-yl)methoxy)carbonyl)amino)-2-cyanoethyl)phenoxy)methyl)-1*H*-1,2,3-triazol-1-yl)-1-((S)-2-((S)-2-((tert-butoxycarbonyl)(methyl)-amino)propanamido)-3,3-dimethylbutanoyl)pyrrolidine-2-carboxamido)-3-(naphthalen-2-yl)propanoate.** To a solution of (S)-2-((((9*H*-fluoren-9-yl)methoxy)carbonyl)amino)-3-(4-((1-((3S,5S)-5-(((S)-1-(allyloxy)-3-(naphthalen-2-yl)-1-oxopropan-2-yl)carbamoyl)-1-((S)-2-((S)-2-((*tert*-butoxycarbonyl)(methyl)amino)propanamido)-3,3-dimethylbutanoyl)pyrrolidin-3-yl)-1*H*-1,2,3-triazol-4-yl)methoxy)phenyl)propanoic acid (**351**, 1.44 mmol, 1.63 g) and Hunig's Base (4.31 mmol, 0.754 mL) in THF (13.08 mL) at -10 °C was added drop wise isobutyl carbonochloridate (2.157 mmol, 0.282 mL). The reaction mixture was stirred at -10 °C for 30 min before 7N NH₃ in MeOH (12.94 mmol, 1.849 mL) was added via syringe and the mixture was slowly warmed to rt. Upon full conversion to the carboxamide, the reaction was quenched with brine (40 mL), washed with 1 N HCl (10 mL), and extracted with EtOAc (3 x 50 mL). The combined organic extracts were dried over MgSO₄, filtered, and concentrated *in vacuo* to give the primary amide as an oil. The intermediate amide was dissolved in CH₂Cl₂/HF(1:1, 1 mL), Burgess reagent (2.157 mmol, 0.514 g) was added in batches over 15 min, and the reaction was stirred for an additional 2 h. The reaction was quenched with brine, extracted with EtOAc, and concentrated *in vacuo*. The resulting residue was purified by flash column chromatography (ISCO, 4 g column, hexanes to EtOAc) to afford **352** (1.23 g, 77%) as a white solid. MS (ESI+) rt 1.21 min, *m*/*z* 1114.6.

**(S)-Allyl 2-((2S,4S)-4-(4-((4-((S)-2-amino-2-cyanoethyl)phenoxy)methyl)-1H-1,2,3-triazol-1-yl)-1-((S)-2-((S)-2-((*tert-*butoxycarbonyl)(methyl)amino)propanamido)-3,3-dimethylbutanoyl)-pyrrolidine-2-carboxamido)-3-(naphthalen-2-yl)propanoate.** A solution of Fmoc-protected **352** (1.077 mmol, 1.2 g) in DMF (5 mL) was treated with piperidine (6.06 mmol, 0.6 mL) and the resulting mixture was stirred at rt for 30 min. The crude reaction was loaded directly onto a gold C18 100 g column and the material was purified by reverse phase chromatography (ISCO; Solvent A: H₂O; Solvent B: 95%MeCN/5%H₂O/0.01%TFA; Gradient: from 30%-95% Solvent B in 15 min) to afford the desired product **353** (730 mg, 76%) as a colorless oil. MS (ESI+) rt 0.99 min, *m*/*z* 892.4.

**(S)-2-((2S,4S)-4-(4-((4-((S)-2-((S)-2-((2S,4S)-4-(4-((4-((S)-2-Amino-3-(*tert*-butoxy)-3-oxopropyl)phenoxy)methyl)-1H-1,2,3-triazol-1-yl)-1-((S)-2-((S)-2-((*tert*-butoxycarbonyl)(methyl)-amino)propanamido)-3,3-dimethylbutanoyl)pyrrolidine-2-carboxamido)-3-(naphthalen-2-yl)propanamido)-2-cyanoethyl)phenoxy)methyl)-1*H*-1,2,3-triazol-1-yl)-1-((S)-2-((S)-2-((*tert*-butoxycarbonyl)(methyl)amino)propanamido)-3,3-dimethylbutanoyl)pyrrolidine-2-carboxamido)-3-(naphthalen-2-yl)propanoic acid.** To a solution of (S)-2-((2S,4S)-4-(4-((4-((S)-2-(((allyloxy)-carbonyl)amino)-3-(*tert*-butoxy)-3-oxopropyl)phenoxy)methyl)-1*H*-1,2,3-triazol-1-yl)-1-((S)-2-((S)-2-((*tert*-butoxy-carbonyl)(methyl)amino)propanamido)-3,3-dimethylbutanoyl)pyrrolidine-2-carboxamido)-3-(naphthalen-2-yl)propanoic acid (**354,** 1.064 mmol, 1076 mg) and (S)-allyl 2-((2S,4S)-4-(4-((4-((S)-2-amino-2-cyanoethyl)phenoxy)methyl)-1H-1,2,3-triazol-1-yl)-1-((S)-2-((S)-2-((*tert-*butoxycarbonyl)-(methyl)amino)propanamido)-3,3-dimethylbutanoyl)pyrrolidine-2-carboxamido)-3-(naphthalen-2-yl)propanoate (**353**, 0.818 mmol, 730 mg) in CH₂Cl₂ (9 mL) and DMF (3 mL) was added Hunig's base (3.27 mmol, 0.572 mL) and HATU (2.046 mmol, 778 mg). The reaction mixture was stirred at rt for 3 h, poured into EtOAc (250 mL) and washed with cold 0.5N HCl. The organic layer was concentrated *in vacuo*, and the crude residue was purified by reverse phase preparative HPLC (Column: Phen-Luna Axia C18 5 u 30 x 250 mm; Solvent A: 95%H₂O/5%MeCN/0.05%TFA; Solvent B: 95%MeCN/ 5%H₂O/0.05%TFA; 30%-100% gradient in 33 min) to afford the desired intermediate (540 mg) as a white solid, which was used directly in the next step.

A solution of (S)-allyl 2-((2S,4S)-4-(4-((4-((S)-2-((S)-2-((2S,4S)-4-(4-((4-((S)-2-(((allyloxy)-carbonyl)amino)-3-(*tert*-butoxy)-3-oxopropyl)phenoxy)methyl)-1*H*-1,2,3-triazol-1-yl)-1-((S)-2-((S)-2-((*tert-*butoxycarbonyl)(methyl)amino)propanamido)-3,3-dimethylbutanoyl)pyrrolidine-2-carboxamido)-3-(naphthalen-2-yl)propanamido)-2-cyanoethyl)phenoxy)methyl)-1*H-*1,2,3-triazol-1-yl)-1-((S)-2-((S)-2-((*tert-*butoxycarbonyl)(methyl)amino)propanamido)-3,3-dimethylbutanoyl)pyrrolidine-2-carboxamido)-3-(naphthalen-2-yl)propanoate (0.286 mmol, 540 mg) in anhydrous THF (20 mL) was placed under nitrogen, cooled to 0 °C and treated with phenylsilane (1.719 mmol, 0.212 mL) and tetrakis(triphenylphosphine)palladium(0) (0.014 mmol, 16.6 mg). The reaction mixture was warmed to rt over 40 min and stirred at rt for 2 h at which point complete consumption of the starting material was observed. The solution was filtered and the solvent was removed. The crude product was purified flash chromatography using a C18 ISCO system (100 g Gold column) to yield **355** (510 mg, 32%) as a pale brown solid. MS (ESI+) rt 1.08 min, *m*/*z* 881.3 (M+2).

**Cyano-substituted Macrocycle 356.** A solution of **355** (0.290 mmol, 510 mg) in CH₂CL₂ (975 mL) was treated with a solution of N-isopropyl-*N*-methylpropan-2-amine (1.16 mmol, 133 mg) and 2-(3*H*-[1,2,3]triazolo[4,5-*b*]pyridin-3-yl)-1,1,3,3-tetramethylisouronium hexafluoro-phosphate(V) (0.869 mmol, 330 mg) in DMF (98 mL). The resulting reaction mixture was allowed to stir at rt for 40 min and concentrated *in vacuo*. The residue was poured onto EtOAc (250 mL), washed with cold 0.5N HCl and 10% LiCl soln., and extracted again with EtOAc. The combined organic layers were concentrated *in vacuo* and purified by reverse phase preparative HPLC (Column: Phen-Luna Axia C18 5 u 30 x 100 mm; Solvent A: 95%H₂O/5%MeCN/0.05%TFA; Solvent B: 95%MeCN/ 5%H₂O/0.05%TFA; 65%-100% gradient in 10 min) to afford compound **356.**

### EXAMPLE 14- SYNTHESIS OF COMPOUND 1047

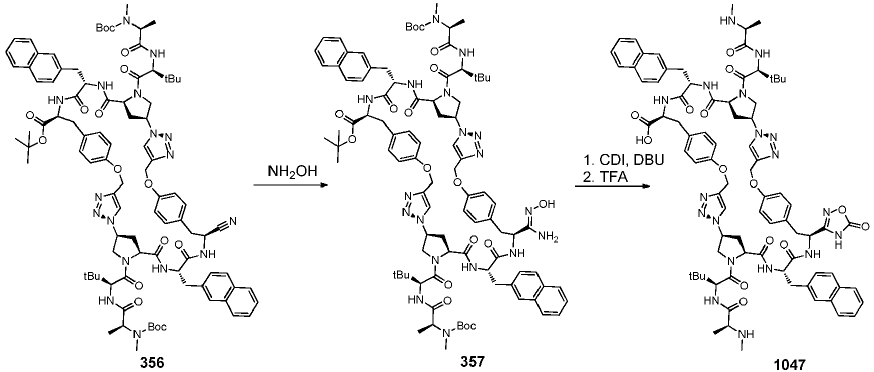

**Hydroxyimidamide 357.** Compound **356** (0.017 mmol, 30 mg) was treated with DMSO (1 mL), DIPEA (0.086 mmol, 0.015 mL) and solid hydroxylamine, HCl (0.086 mmol, 5.98 mg). The reaction mixture was heated to 70 °C for 8 h. The crude product was purified by reverse phase preparative HPLC (Column: Phen-Luna Axia C18 5 u 30 x 100 mm; Solvent A: 95%H₂O/5%MeCN/0.05%TFA; Solvent B: 95%MeCN/5%H₂O/0.05%TFA; 20%-100% gradient in 15 min) to afford **357.**

**Compound 1047.** Intermediate **357** (5.63 µmol, 10 mg) was dissolved in 1,4-dioxane (1 mL) and treated with DBU (6.76 µmol, 1.018 µl) and CDI (7.04 µmol, 1.141 mg) in 1,4-dioxane (100 µL). The mixture was heated to reflux for 1 h, cooled to rt, poured into EtOAc, and washed with cold 0.5 HCl. The organics were concentrated *in vacuo*. The crude residue was dissolved in CH₂Cl₂ (1 mL) and TFA (1 mL) and stirred at rt until product is formed as judged by LCMS. At that point, the solvent was removed and the crude oil was purified by reverse phase preparative HPLC (C18 30 x 100 mm; 0-100% MeCN in H₂O with 0.5% TFA) to afford **1047** as a white solid (2.1 mg, 21%). MS (ESI+) rt 0.75 min, *m*/*z* 1547.0.

### EXAMPLE 15-- SYNTHESIS OF COMPOUND 1048

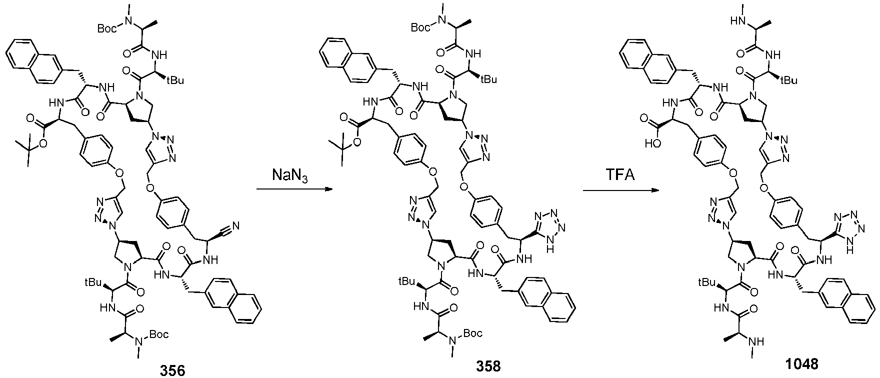

**Compound 1048.** An 8 mL vial equipped with a magnetic stirrer was charged with cyano-substituted macrocycle **356** (0.017 mmol, 30 mg), sodium azide (0.138 mmol, 8.95 mg) and DMF (1.5 mL). To the mixture was added Zn(II)bromide (0.034 mmol, 7.75 mg) and the resulting reaction mixture was heated to 80 °C for 24 h. An additional 1 equiv. of ZnBr₂ was added and the mixture was stirred at 85°C for 3 d, cooled to rt, poured into EtOAc and washed with 10% LiCl. The organic layer was dried over Na₂SO₄, filtered and concentrated *in vacuo*. The residue was treated with 50% TFA in CH₂Cl₂ overnight until complete conversion was observed. The resulting residue was purified by reverse phase preparative HPLC (Column: Phen-Luna Axia C18 5 u 30 x 250 mm; Solvent A: 95%H₂O/5%MeCN/0.05%TFA; Solvent B: 95%MeCN/5%H₂O/0.05%TFA; 5%-100% gradient in 28 min) to afford **1048** (4.5 mg, 14%) as a white solid.

### EXAMPLE 16--ADDITIONAL EVALUATION OF BIOLOGICAL ACTIVITY

Exemplary compounds were tested for inhibition of XIAP BIR3 and XIAP BIR2-3 activity. The experimental procedures for the XIAP BIR2/ SMAC peptide alphascreen and XIAP-BIR3 / SMAC FPA are found in Example 5. Additional experimental procedures and results are provided below.

### A. XIAP-BIR3 / SMAC Homogeneous Time Resolved Fluorescence (HTRF) Assay

Assays were performed in black, flat-bottom, 384-well plates. The final assay volume was 50 µL prepared from additions of His-BIR3 (241-356, XIAP), fluorescein labeled SMAC peptide, and test compounds in assay buffer consisting of 20 mM Sodium Phosphate, 1 mM EDTA, 50 mM NaCl, 50 µg/ml BSA, and 0.05% Pluronic F68. The reaction was incubated at room temperature for 60 minutes, following which 10 µl of mouse anti-6xHis-terbium labeled Fab (Medarex,Cis-bio) was added to the reaction (40 µl) for an additional 30 minute incubation. The HTRF signal, ratio of fluorescence intensities at emission wavelengths for fluorescein acceptor (520 nm) and terbium donor (615 nm), the 520/615 ratio, generated by the reaction was then measured on the Envision Plate Reader. Inhibition data were calculated from the 520/615 ratio generated by the no protein control reactions for 100% inhibition and vehicle-only reactions for 0% inhibition. The final concentration of reagents in the assay was 1 nM N-His -BIR3(241-356, XIAP), 5 nM fluorescein labeled SMAC peptide, 0.25 nM anti-His-Tb-Fab, and 0.1% DMSO. Dose response curves were generated to determine the concentration required for inhibiting 50% of the HTRF signal (IC₅₀)- Compounds were dissolved at 3 mM in dimethylsulfoxide (DMSO) and evaluated at eleven serially diluted concentrations. IC₅₀ and Kᵢ values were derived by non-linear regression analysis.

### B. XIAP-BIR2-3 dimeric SMAC Peptide Homogeneous Time Resolved Fluorescence (HTRF) Assay

Assays were performed in black, flat-bottom, 384-well plates. The final assay volume was 50 µL prepared from additions of His-BIR2-3 (125-356, C202A/C213G, XIAP), fluorescein labeled dimeric SMAC peptide, and test compounds in assay buffer consisting of 20 mM Sodium Phosphate, 1 mM EDTA, 50 mM NaCl, 50 µg/ml BSA, and 0.05% Pluronic F68. The reaction was incubated at room temperature for 60 minutes, following which 10 µl of mouse anti-6xHis-Tb IgG (Medarex,Cis-bio) was added to the reaction (40 µl) for an additional 30 minute incubation. The HTRF signal, ratio of fluorescence intensities at emission wavelengths for fluorescein acceptor (520 nm) and terbium donor (615 nm), the 520/615 ratio, generated by the reaction was then measured on the Envision Plate Reader. Inhibition data were calculated from the 520/615 ratio generated by the no protein control reactions for 100% inhibition and vehicle-only reactions for 0% inhibition. The final concentration of reagents in the assay was 0.5 nM N-His -BIR2-3(125-356, C202A/C213G, XIAP), 20 nM fluorescein labeled dimeric SMAC peptide, 0.25 nM anti-His-Tb-Fab, and 0.1% DMSO. Dose response curves were generated to determine the concentration required for inhibiting 50% of the HTRF signal (IC₅₀). Compounds were dissolved at 3 mM in dimethylsulfoxide (DMSO) and evaluated at eleven serially diluted concentrations. IC₅₀ and Kᵢ values were derived by non-linear regression analysis.

### Results:

Results of the XIAP BIR3, XIAP BIR2 and XIAP BIR2-3 assays are shown in Table 4 below. XIAP BIR3 FPA IC₅₀₃ values are reported as follows: "A" indicates an IC₅₀ value of less than 50 nM; "B" indicates an IC₅₀ value of 50 to 100 nM; "C" indicates an IC₅₀ value of greater than 100 nM. XIAP BIR3 HTRF IC₅₀ values are reported as follows: "A" indicates an IC₅₀ value of less than 10 nM; "B" indicates an IC₅₀ value of 10 to 100 nM; "C" indicates an IC₅₀ value of greater than 100 nM. XIAP BIR2 Alphascreen IC₅₀ values are reported as follows: "A" indicates an IC₅₀ value of less than 2 µM; "B" indicates an IC₅₀ value of 2 to 12 µM; "C" indicates an IC₅₀ value of greater than 12 µM. XIAP BIR2-3 HTRF IC₅₀ values are reported as follows: "A" indicates an IC₅₀ value of less than or equal to 1 nM; "B" indicates an IC₅₀ value of greater than 1 to 10 nM; "C" indicates an IC₅₀ value of greater than 10 nM. "NT" means that the compound was not tested in the assay.

**TABLE 4 - Inhibitory Activity of Compounds of Formula I**

| **Compound No.** | **BIR3 FPA IC₅₀** | **BIR3 HTRF IC₅₀** | **BIR2 Alpha Screen IC₅₀** | **BIR2-3 HTRF IC₅₀** |
|---|---|---|---|---|
| **147** | A | A | A | B |
| **148** | A | A | A | A |
| **149** | A | NT | A | NT |
| **150** | B | NT | A | NT |
| **1000** | B | NT | NT | NT |
| **1001** | NT | C | NT | C |
| **1002** | A | NT | A | NT |
| **1003** | B | NT | A | NT |
| **1004** | B | NT | A | NT |
| **1005** | A | NT | A | NT |
| **1006** | C | NT | A | NT |
| **1007** | B | NT | B | NT |
| **1008** | NT | B | NT | A |
| **1009** | NT | A | NT | B |
| **1010** | NT | B | NT | C |
| **1011** | NT | A | NT | B |
| **1012** | NT | A | NT | B |
| **1013** | NT | A | NT | A |
| **1014** | NT | A | NT | A |
| **1015** | NT | B | NT | B |
| **1016** | NT | A | NT | A |
| **1017** | NT | A | NT | A |
| **1018** | NT | A | NT | B |
| **1019** | NT | A | NT | B |
| **1020** | NT | B | NT | B |
| **1021** | NT | A | NT | B |
| **1022** | NT | A | NT | B |
| **1023** | NT | A | NT | B |
| **1024** | NT | A | NT | B |
| **1025** | NT | A | NT | B |
| **1026** | NT | B | NT | B |
| **1027** | NT | B | NT | B |
| **1028** | NT | A | NT | A |
| **1029** | NT | B | NT | B |
| **1030** | NT | B | NT | B |
| **1031** | NT | B | NT | B |
| **1032** | NT | A | NT | A |
| **1033** | NT | A | NT | B |
| **1034** | NT | B | NT | C |
| **1035** | NT | A | NT | A |
| **1036** | NT | B | NT | B |
| **1037** | NT | B | NT | B |
| **1038** | NT | B | NT | C |
| **1039** | NT | B | NT | B |
| **1040** | NT | C | NT | C |
| **1041** | NT | B | NT | B |
| **1042** | NT | B | NT | B |
| **1043** | NT | B | NT | B |
| **1044** | NT | A | NT | B |
| **1045** | NT | A | NT | A |
| **1046** | NT | A | NT | A |
| **1047** | NT | A | NT | A |
| **1048** | NT | A | NT | A |
| **1049** | NT | C | NT | B |
| **1050** | NT | NT | NT | C |
| **1051** | NT | C | NT | C |
| **1052** | NT | A | NT | A |

## Claims

1. A compound of the Formula I: 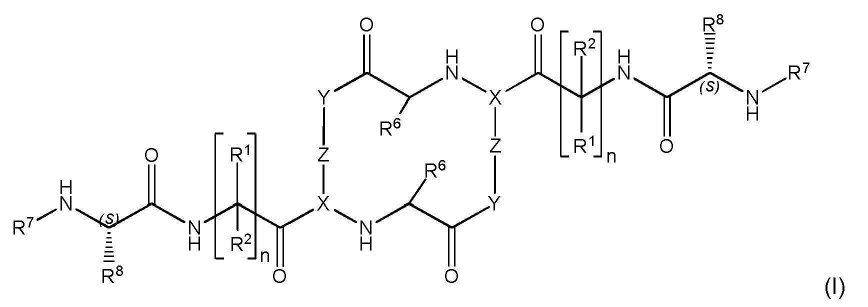 or a pharmaceutically acceptable salt thereof, wherein:
each n is independently 1 or 2;
each R¹ is independently selected from hydrogen, cycloalkyl and -(C₁-C₄ alkylene)-R⁴, wherein each R⁴ is independently selected from hydrogen, aryl, and cycloalkyl, wherein at least one R¹ is other than hydrogen; and
each R² is hydrogen; or
R¹ and R² are taken together to the carbon atom to which they are commonly bound to form a cycloalkyl;
each R⁶ is independently -(C₁-C₄ alkylene)-R⁹, wherein each R⁹ is independently selected from hydrogen, aryl, heteroaryl and cycloalkyl; wherein any aryl, heteroaryl or cycloalkyl portion of R⁶ is optionally substituted with up to two substituents independently selected from halo, CF₃, OH, C₁-C₄ alkoxy, C₁-C₄ alkenyloxy, phenyl, phenyloxy, and phenylmethyloxy; and wherein one -CH₂- in the -(C₁-C₄ alkylene)- portion of R⁶ is optionally replaced with -O-;
each R⁷ is independently selected from hydrogen and methyl;
each R⁸ is independently selected from methyl and ethyl;
each X is independently selected from: 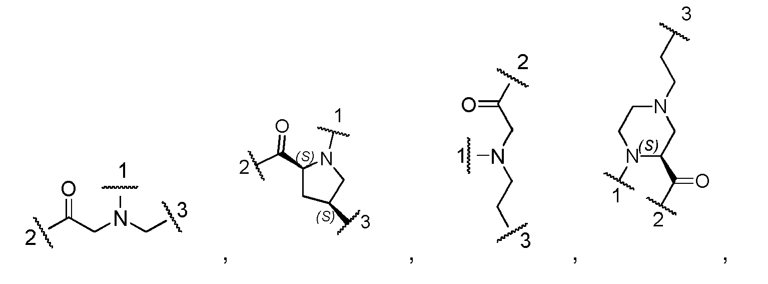 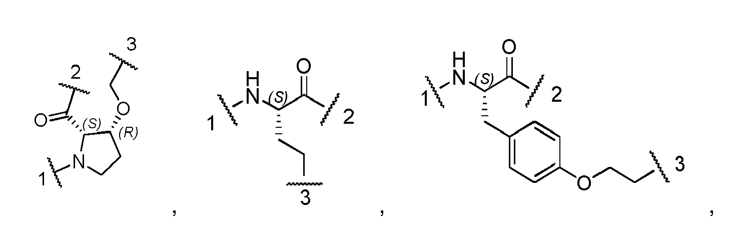 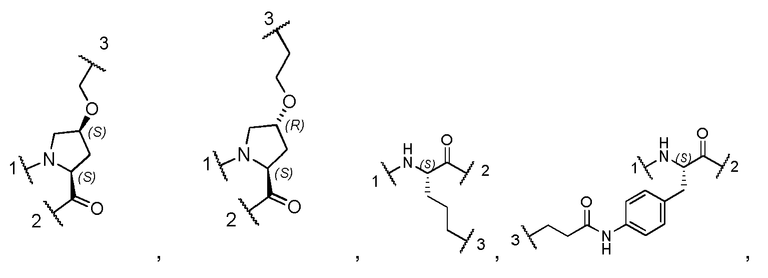 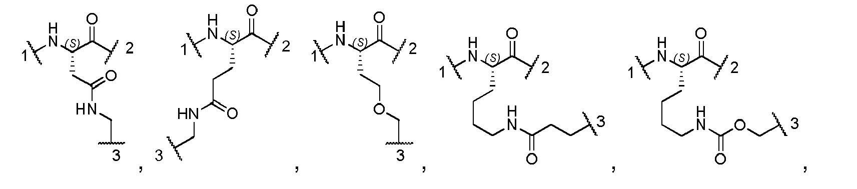 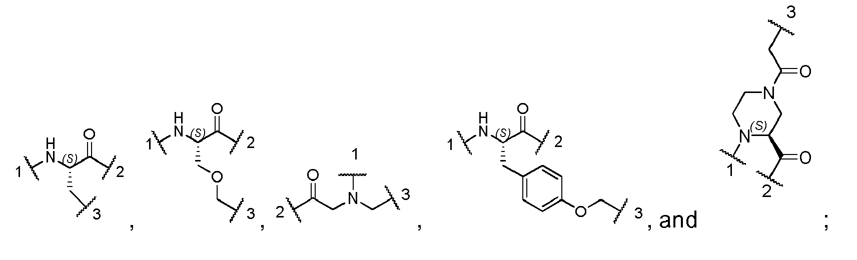
each Z is 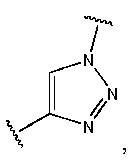 wherein each 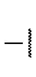 represents a point of attachment to the compound; and
each Y is selected from: 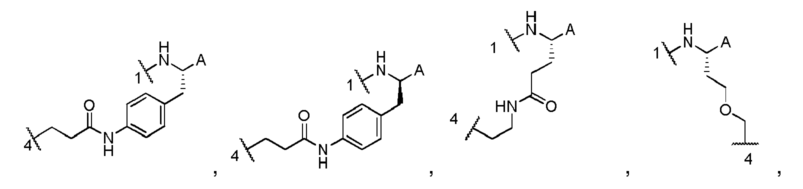 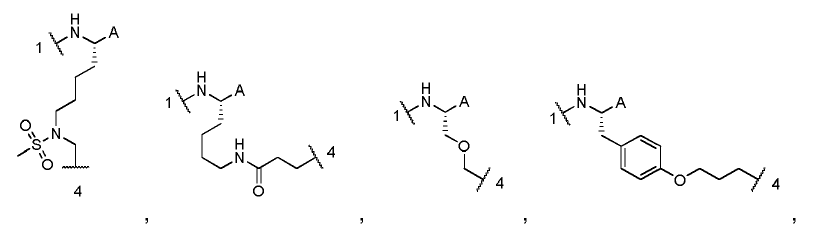 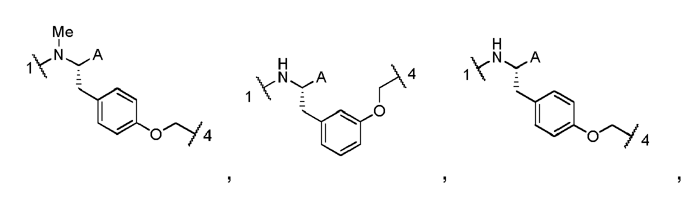 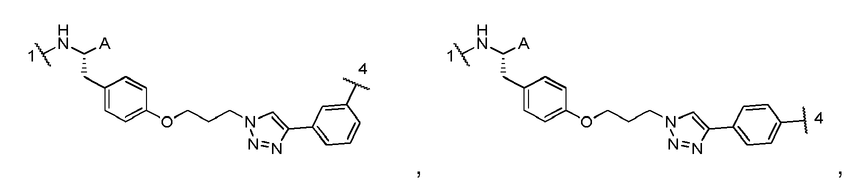 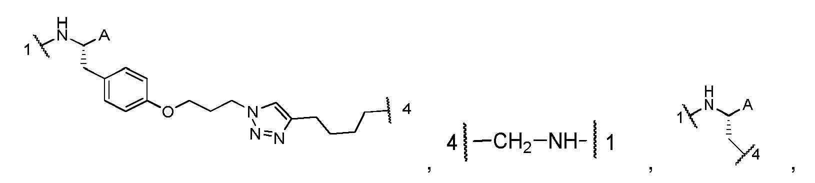 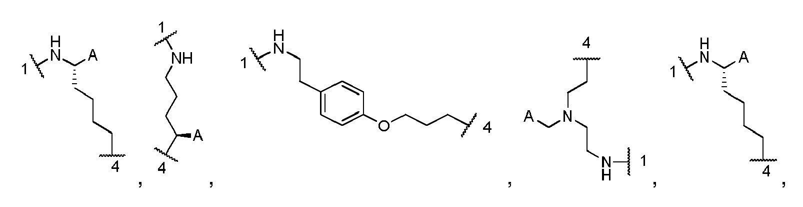 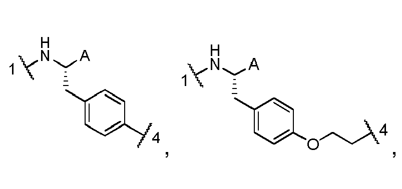 and 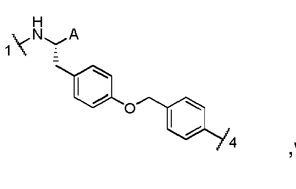 wherein: 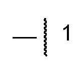
represents a point of attachment to a -C=O portion of the compound; 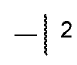
represents a point of attachment to a -NH portion of the compound; 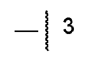
represents a first point of attachment to Z; 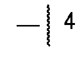
represents a second point of attachment to Z; and
A is selected from -C(O)R³ or 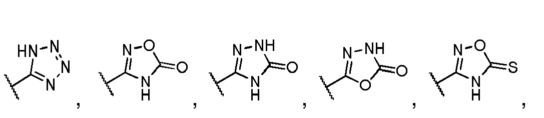 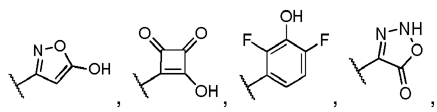 or a tautomeric form of any of the foregoing, wherein:
R³ is OH, -O-(C1-C4 alkyl), NHCN, NHSO₂R¹⁰, NHOR¹¹ or N(R¹²)(R¹³);
R¹⁰ and R¹¹ are selected from -C₁-C₄ alkyl, cycloalkyl, aryl, heteroaryl, or heterocycloalkyl, any of which are optionally substituted, and hydrogen;
each of R¹² and R¹³ are independently selected from hydrogen, -C₁-C₄ alkyl, -(C₁-C₄ alkylene)-NH-(C₁-C₄ alkyl), -(C₁-C₄ alkylene)-O-(C₁-C₄ alkyl) and -(C₁-C₄alkylene)-O-(C₁-C₄ hydroxyalkyl), or R¹² and R¹³ are taken together with the nitrogen atom to which they are commonly bound to form a saturated heterocyclyl optionally comprising one additional heteroatom selected from N, O and S, and wherein the saturated heterocycle is optionally substituted with methyl.

2. The compound of claim 1, wherein R⁷ is methyl and R⁸ is methyl.

3. The compound of claim 1, wherein a portion of the compound represented by -NH-[C(R¹)(R²)]ₙ-C(O)- is selected from: 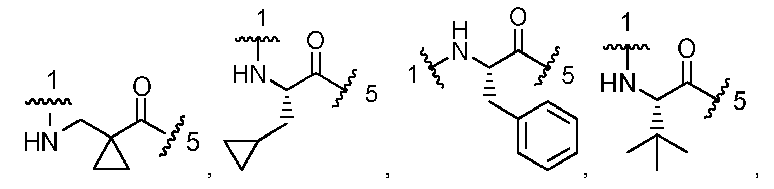 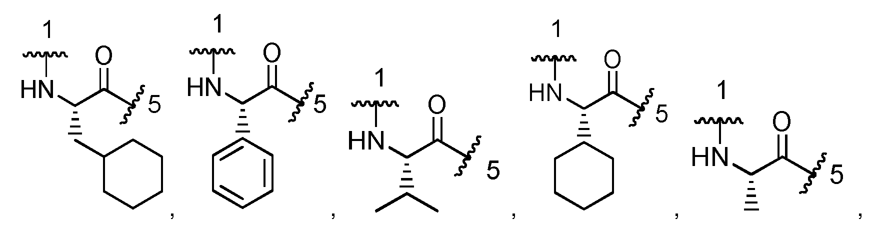 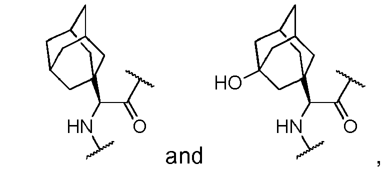 wherein: 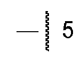
represents a point of attachment to the amino portion of X.

4. The compound of claim 3, wherein a portion of the compound represented by -NH-[C(R¹)(R²)]ₙ-C(O)- is

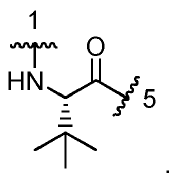

5. The compound of claim 1, wherein a portion of the compound represented by -NH-CH(R⁶)-C(O)- is selected from: 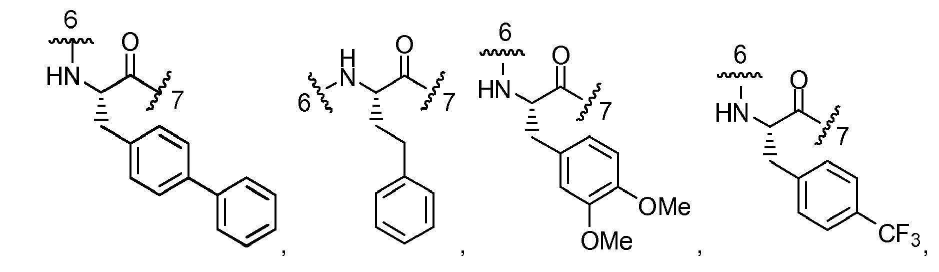 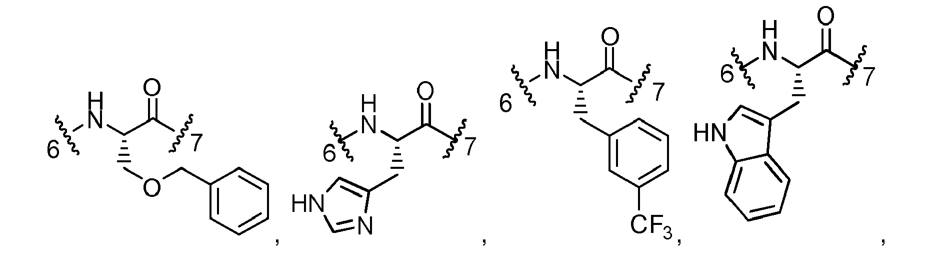 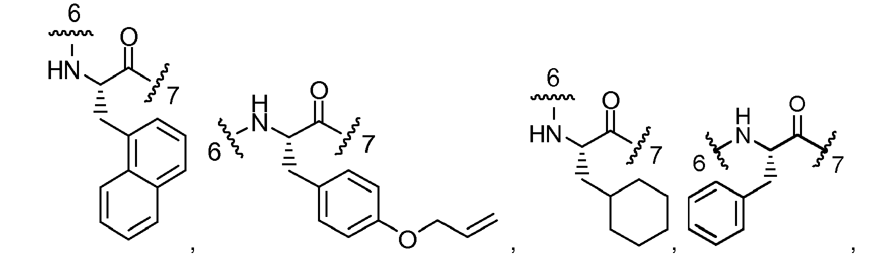 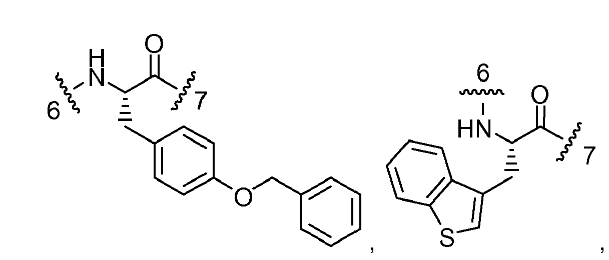 and 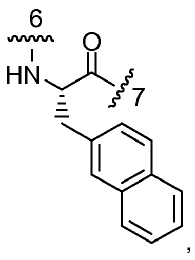 wherein: 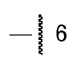
represents a point of attachment to the -C=O portion of X; and 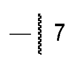 represents a point of attachment to the amino portion of Y.

6. The compound of claim 5, wherein a portion of the compound represented by -NH-CH(R⁶)-C(O)- is

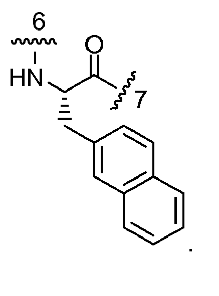

7. The compound of claim 1, wherein X is selected from:

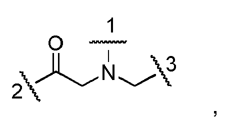

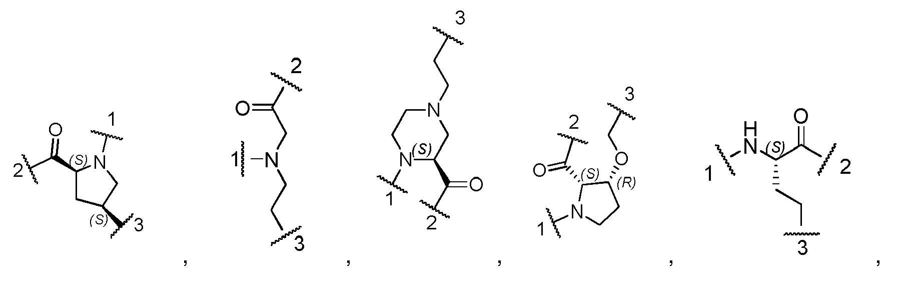

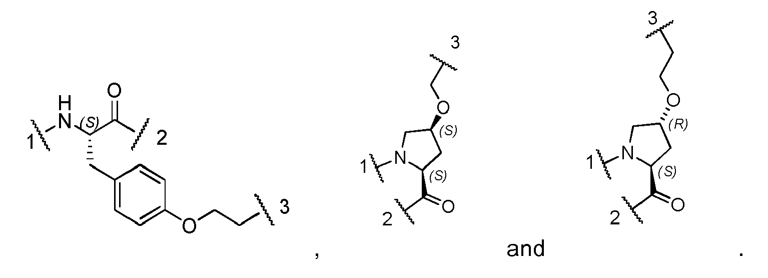

8. The compound of claim 7, wherein X is selected from:

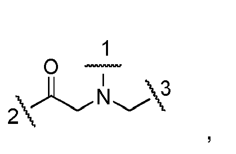

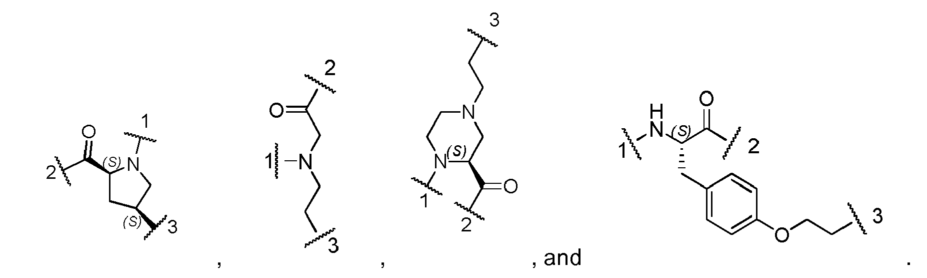

9. The compound of claim 8, wherein X is

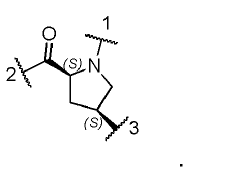

10. The compound of claim 1, wherein Y is selected from

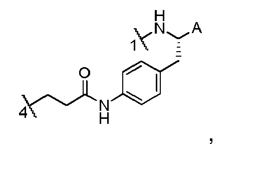

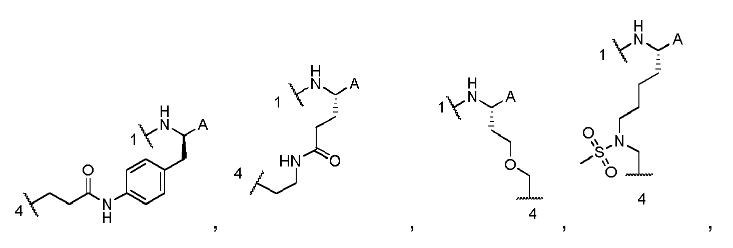

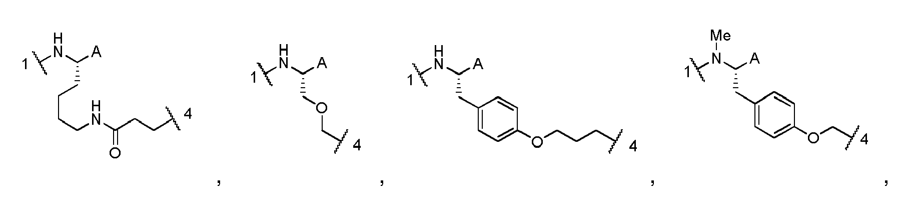

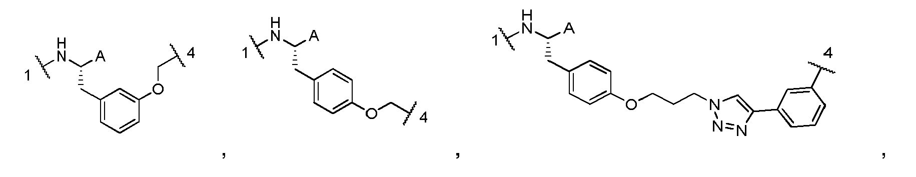

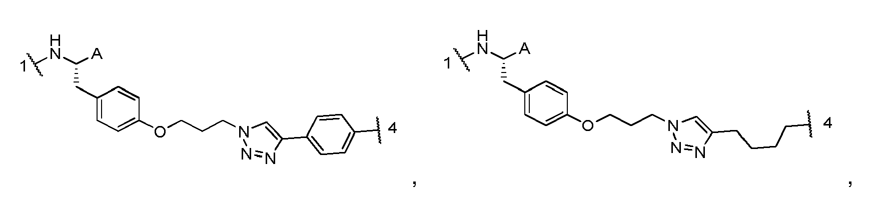

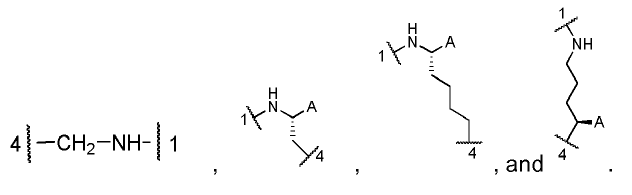

11. The compound of claim 10, wherein Y is

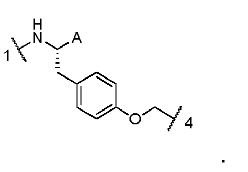

12. The compound of claim 1, wherein:
each R¹ is the same;
each R² is the same;
each R⁶ is the same;
each R⁷ is the same;
each R⁸ is the same;
each X is the same;
each Y is the same;
each Z is the same; and
each n is the same.

13. The compound of claim 1, wherein the compound is selected from the group consisting of:

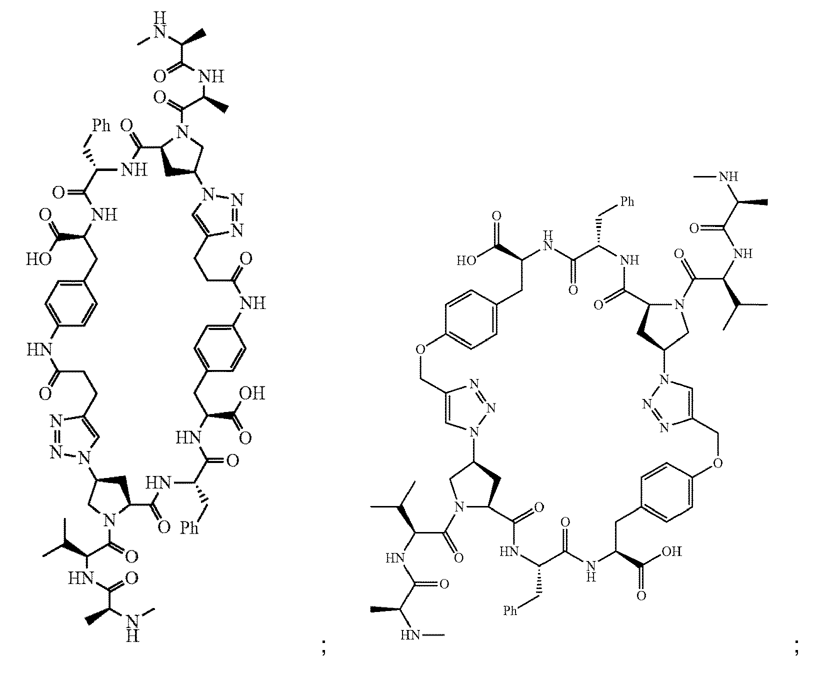

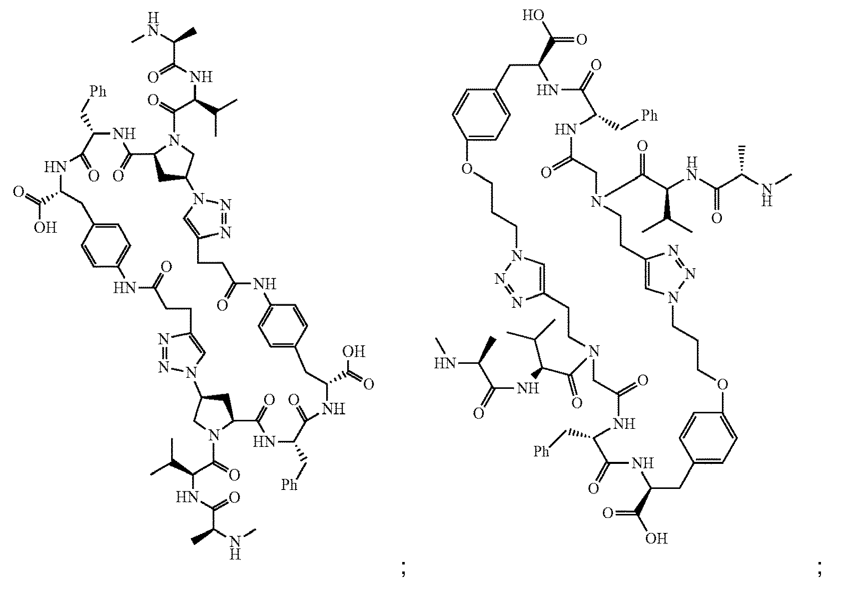

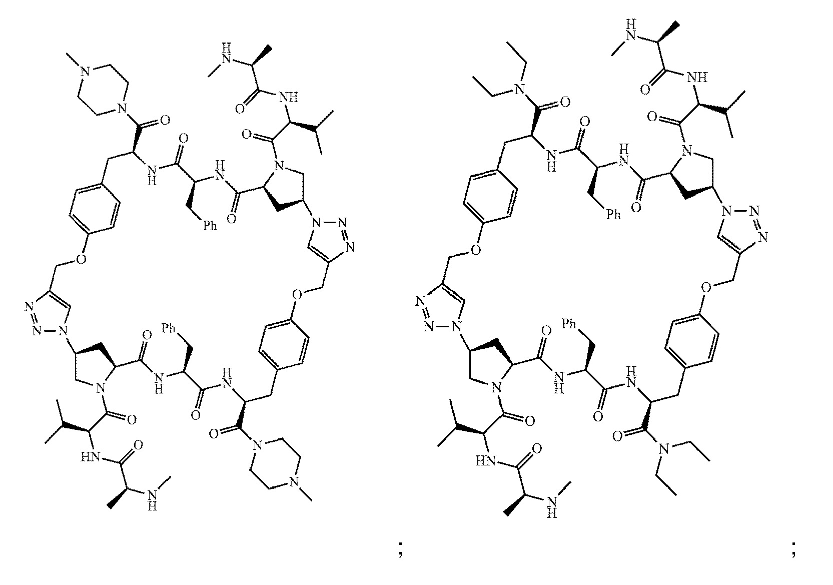

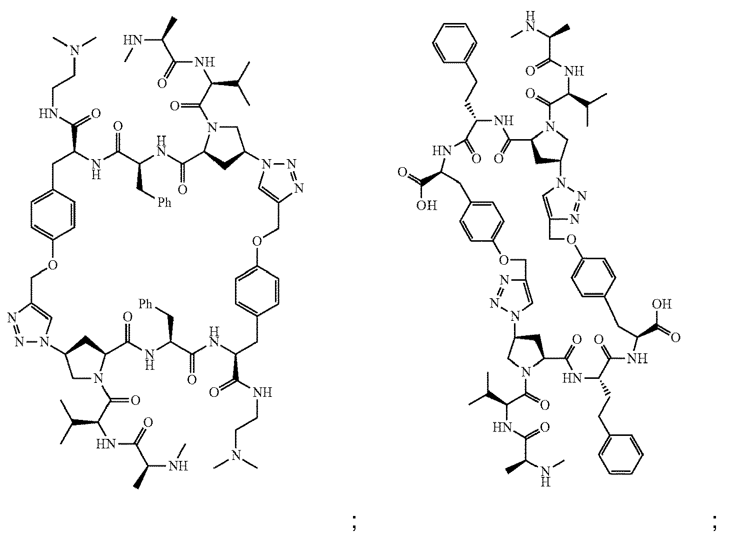

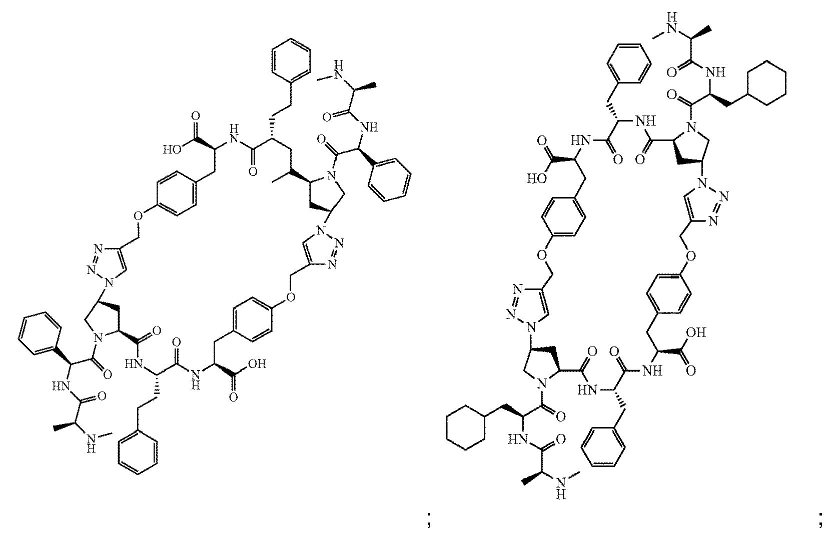

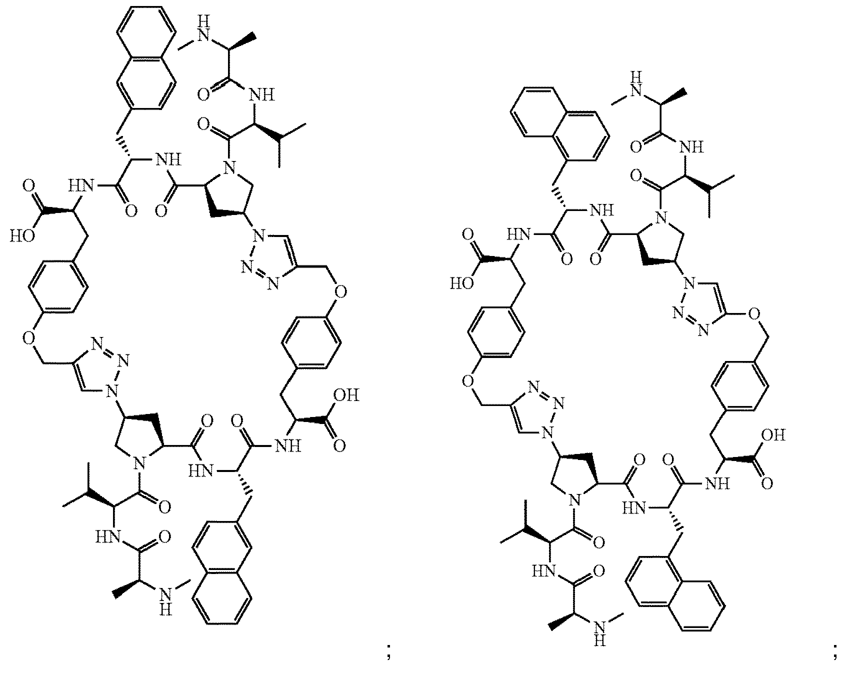

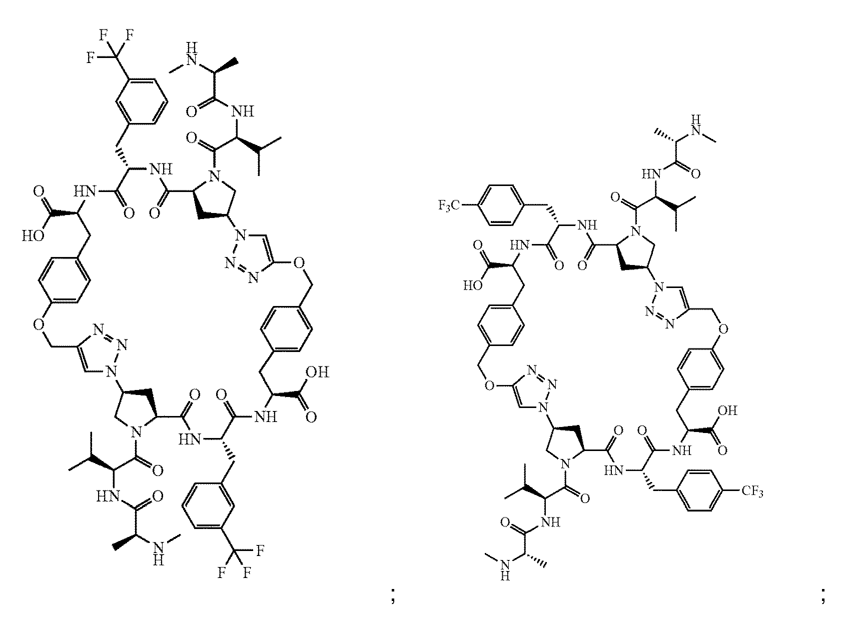

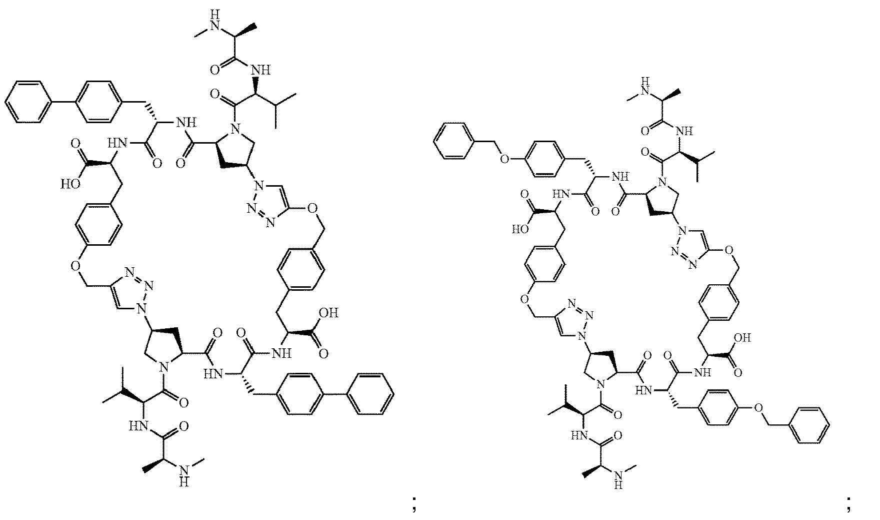

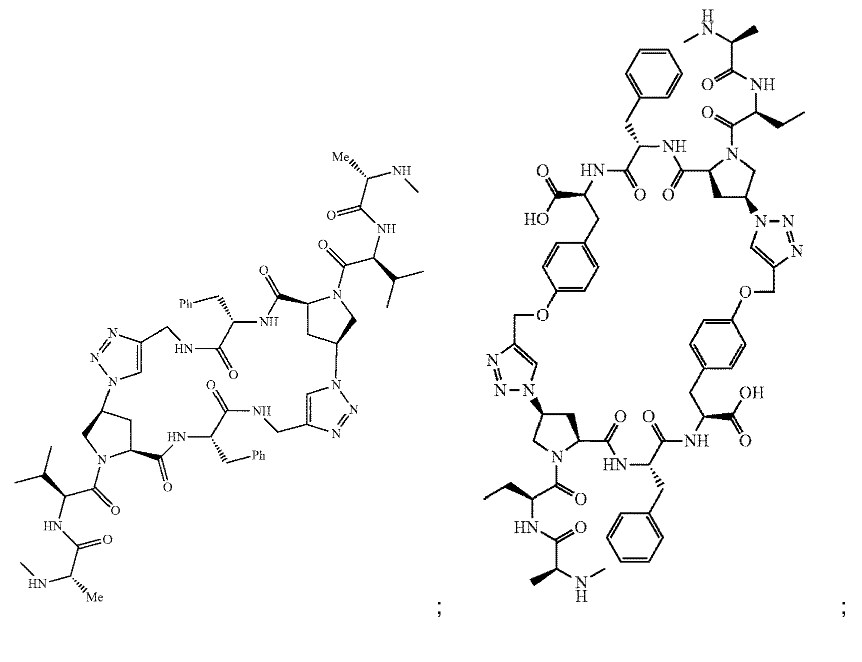

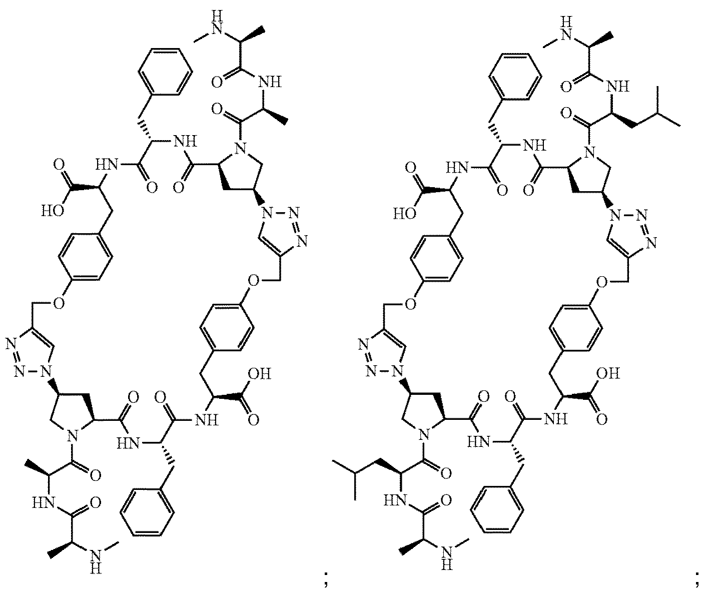

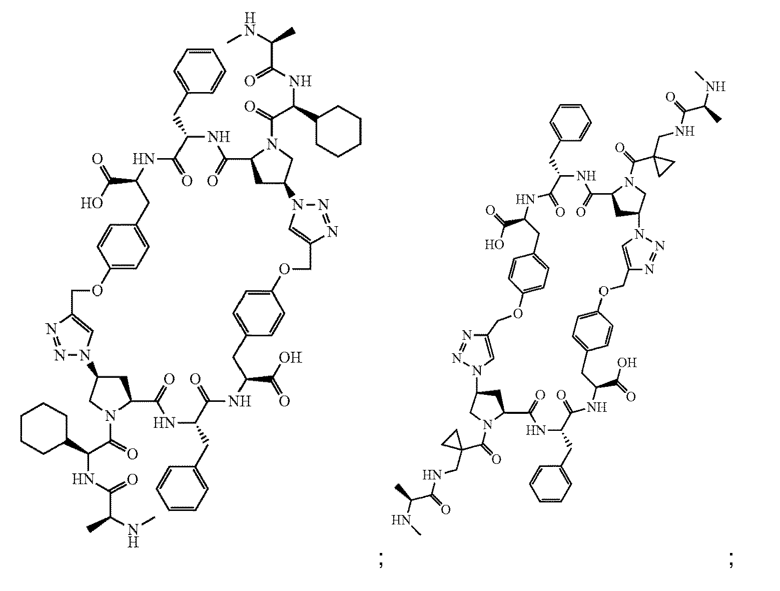

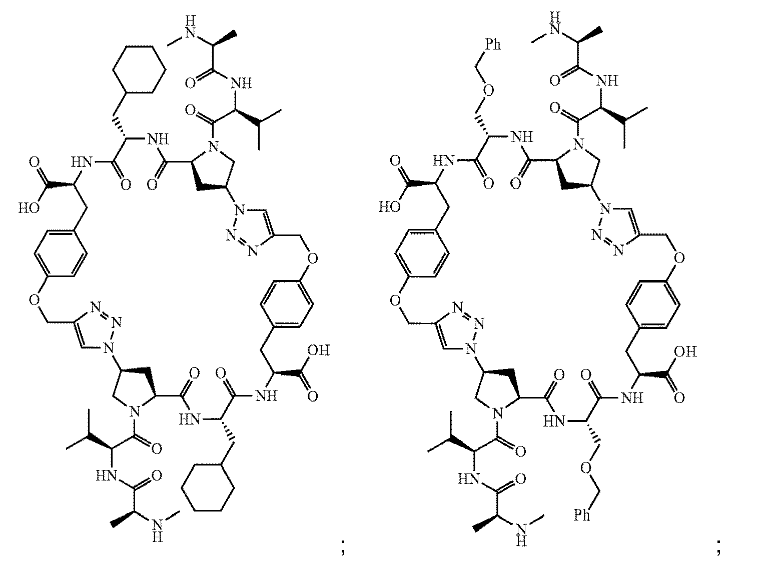

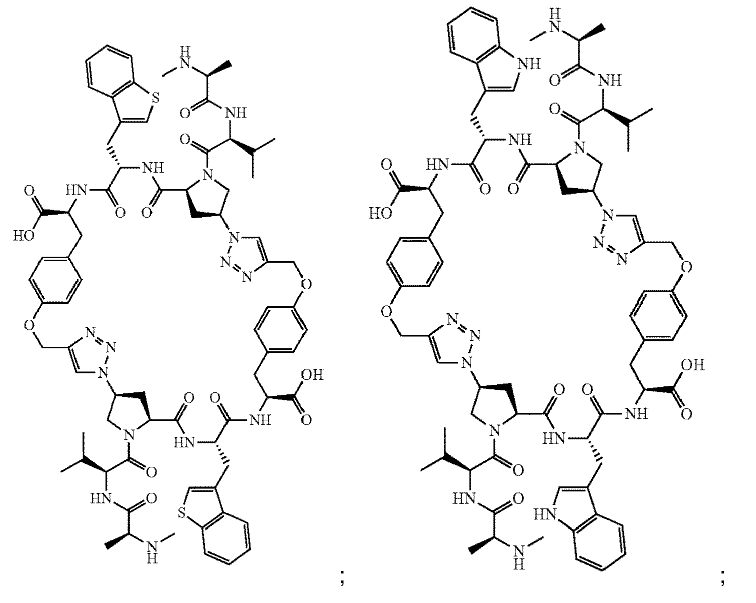

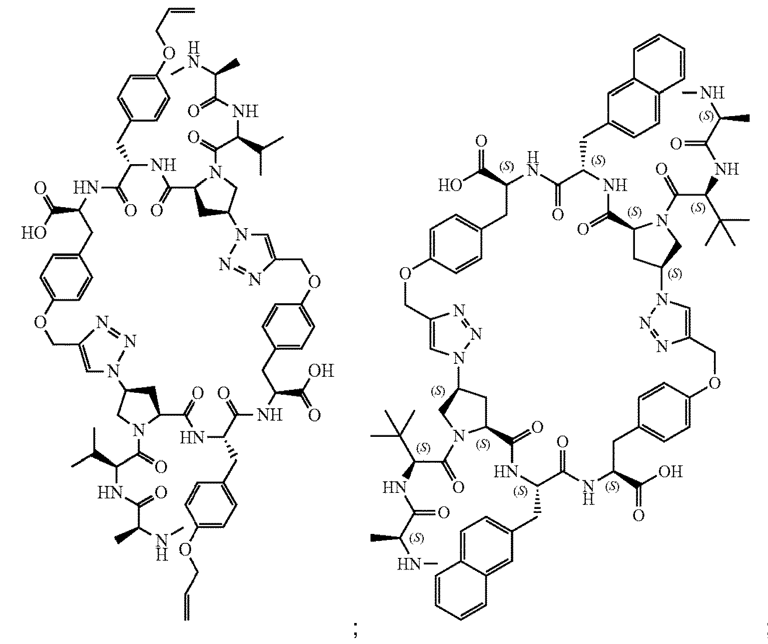

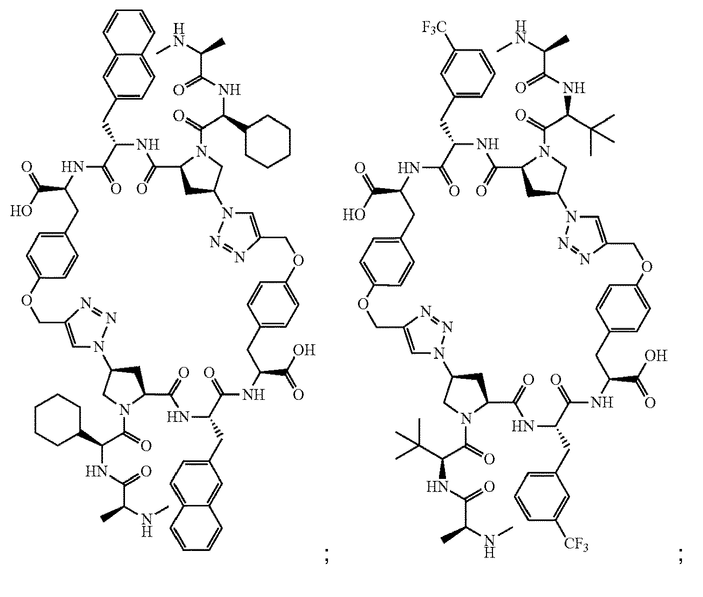

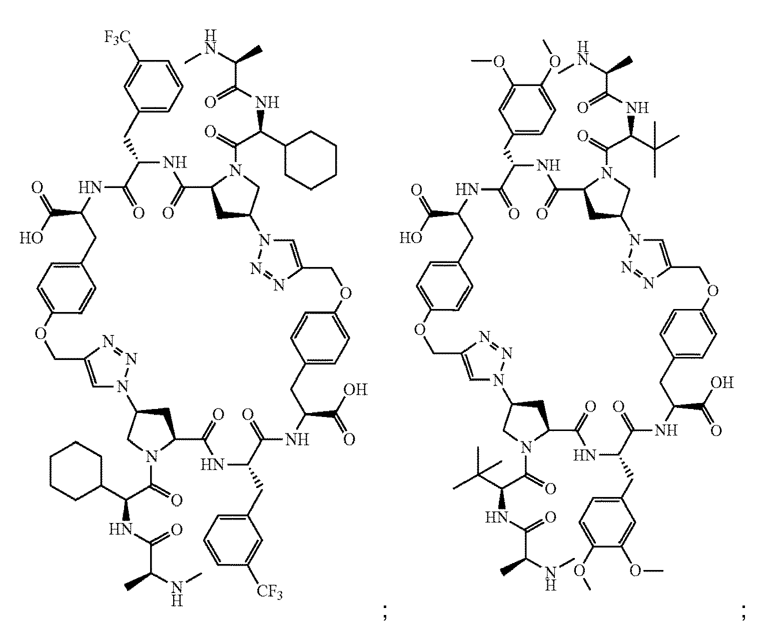

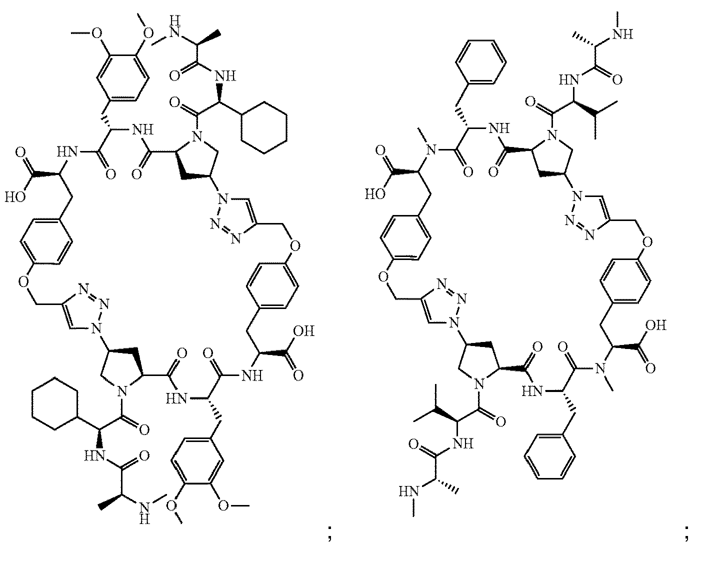

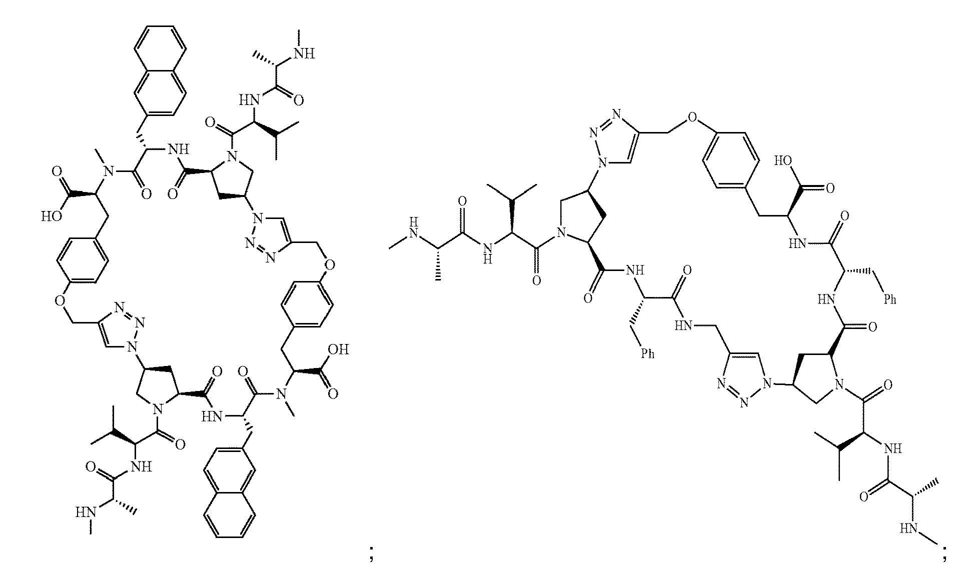

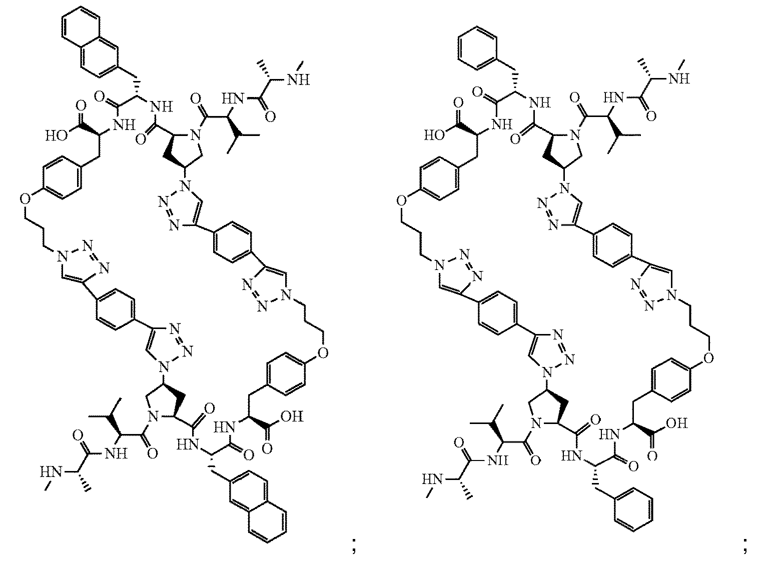

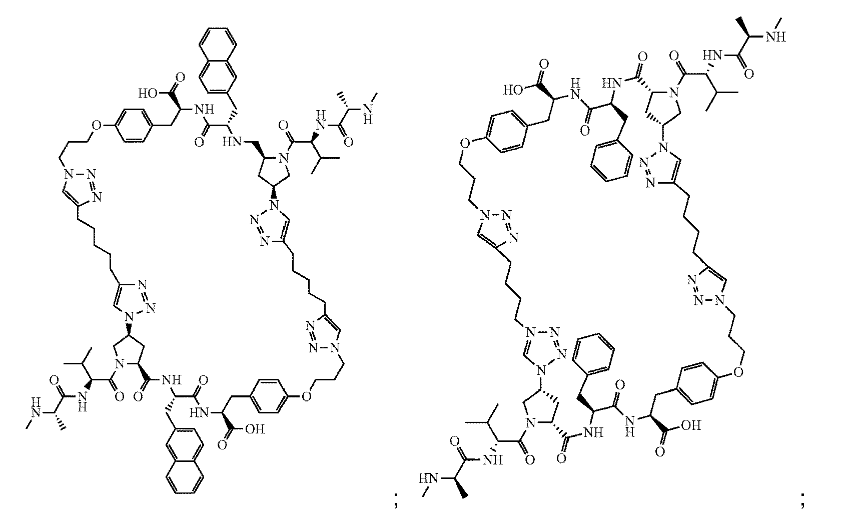

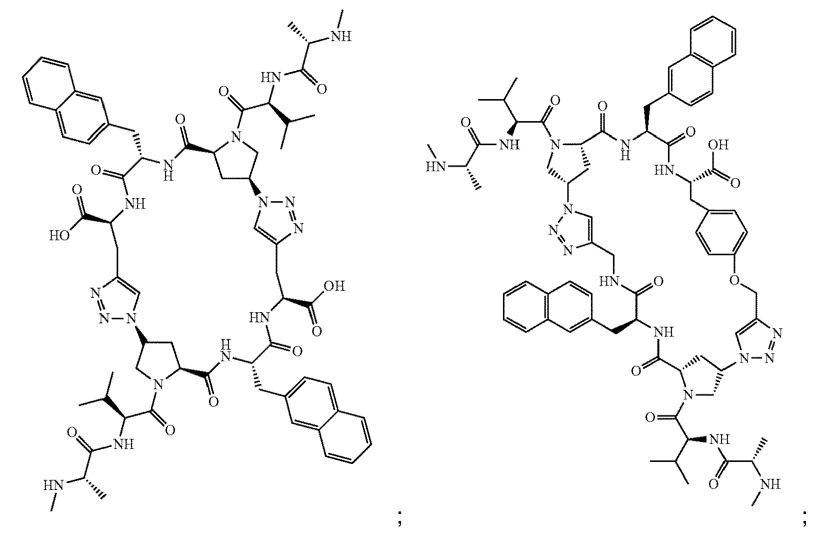

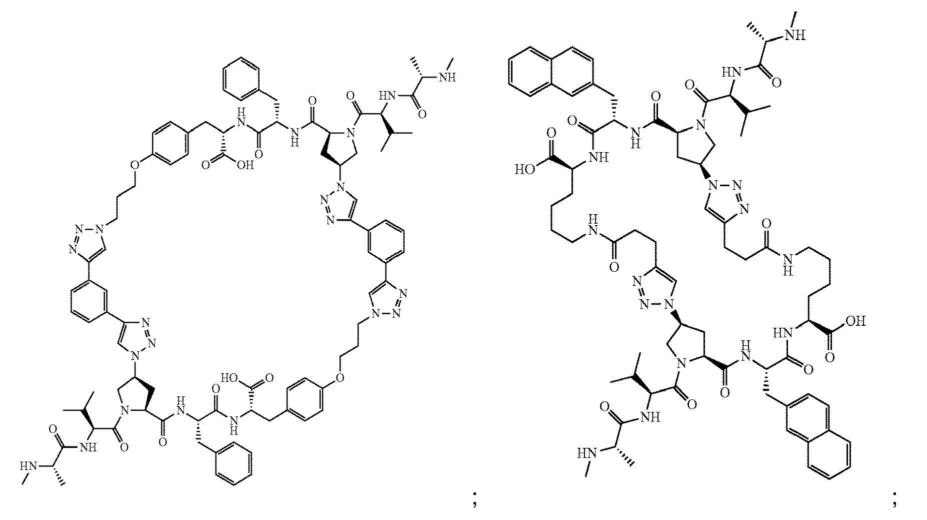

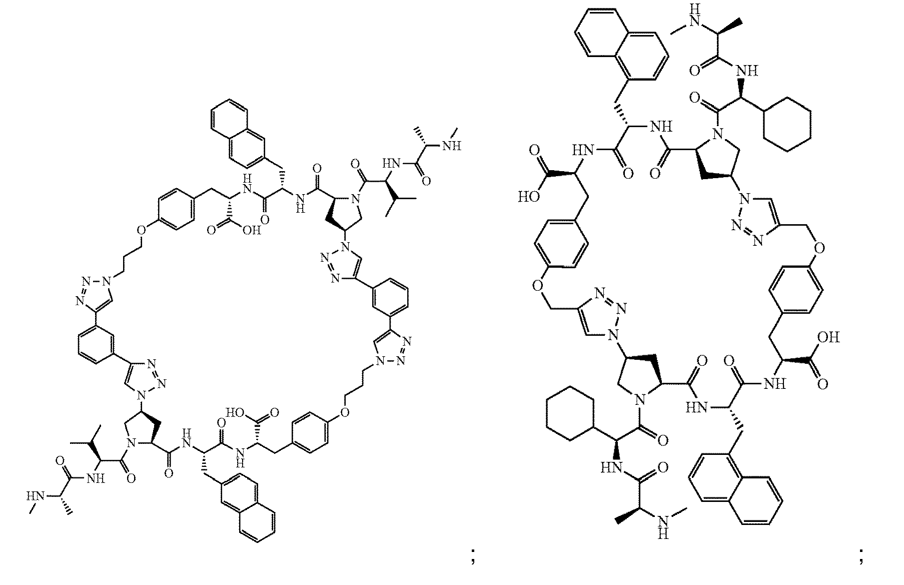

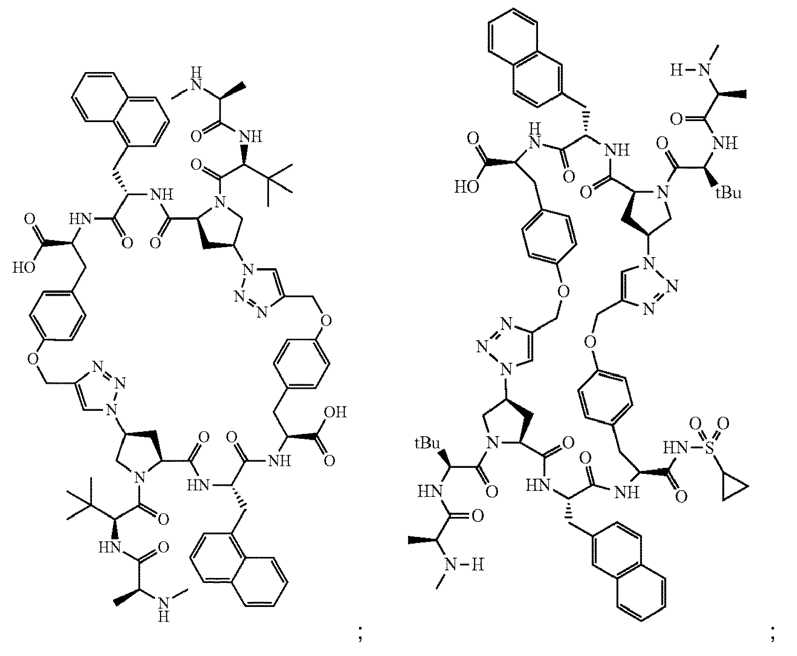

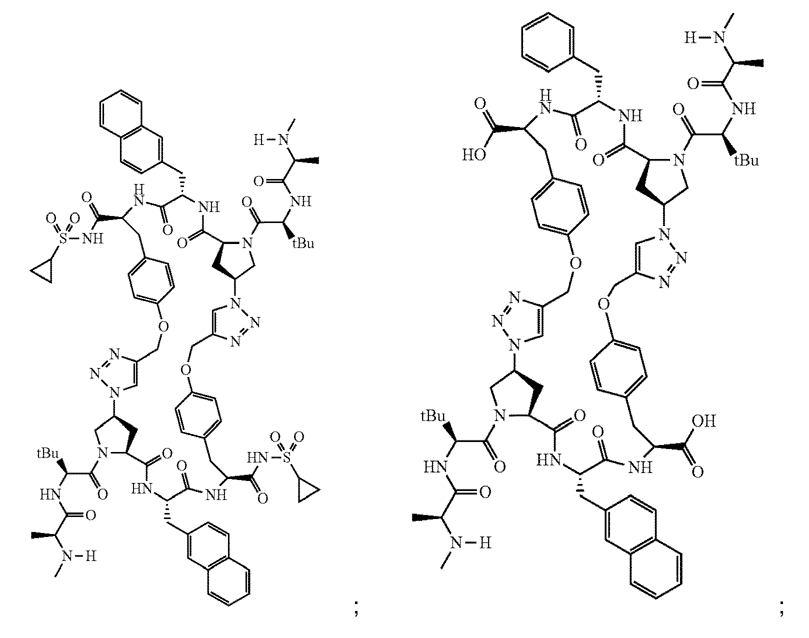

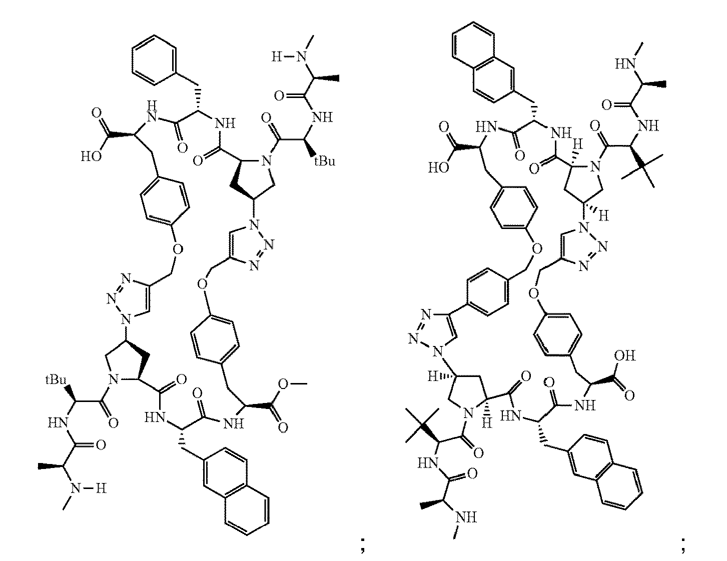

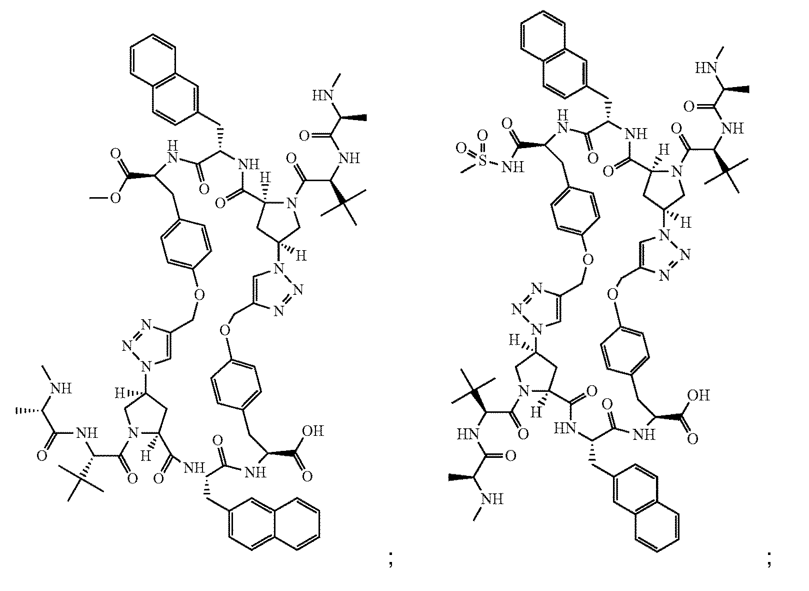

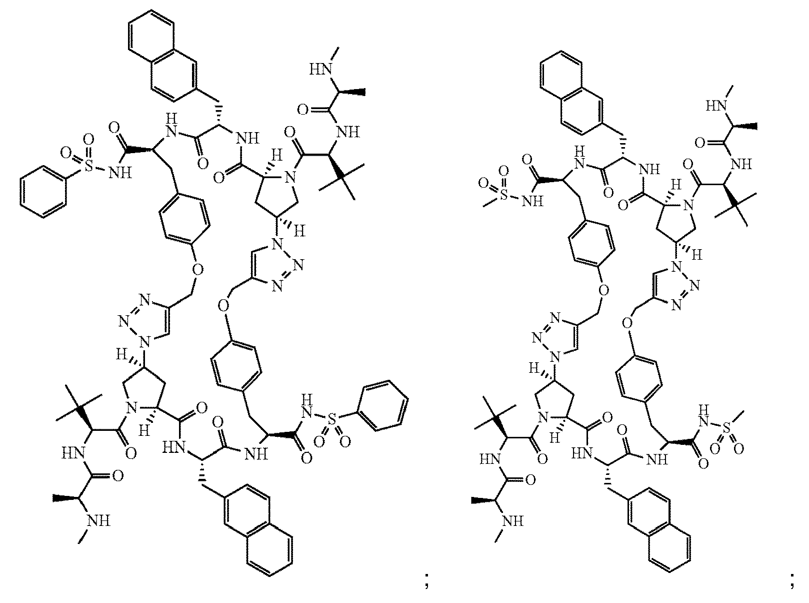

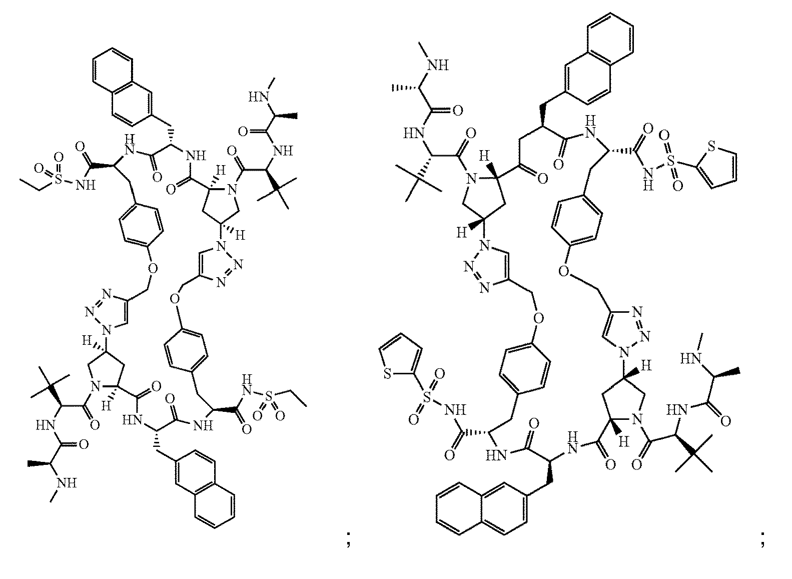

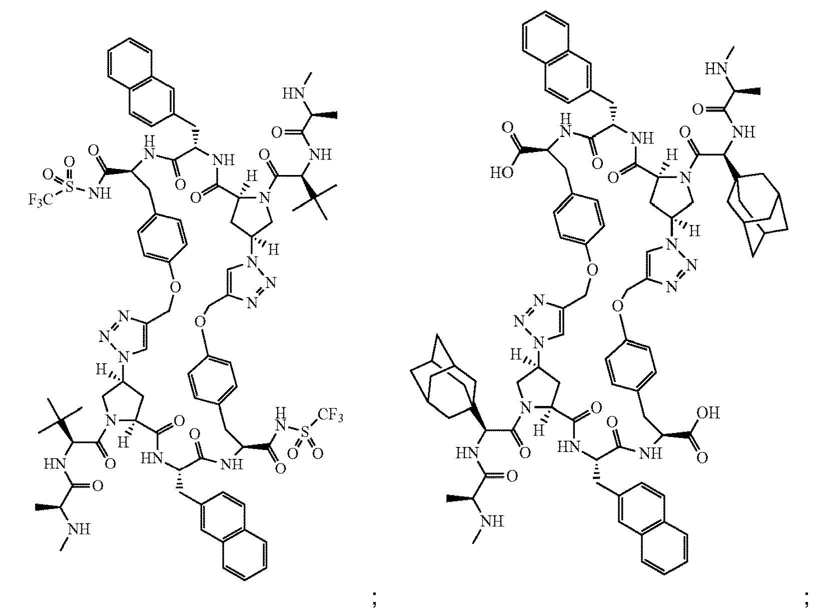

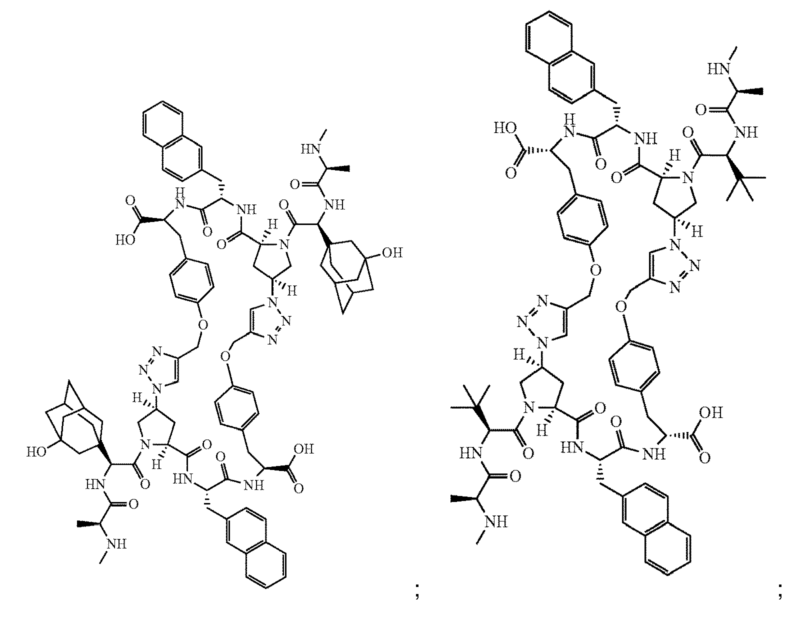

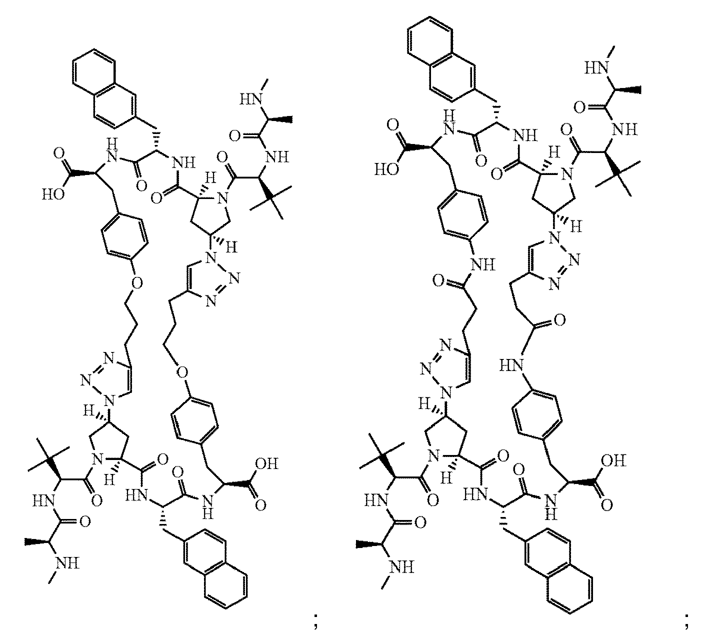

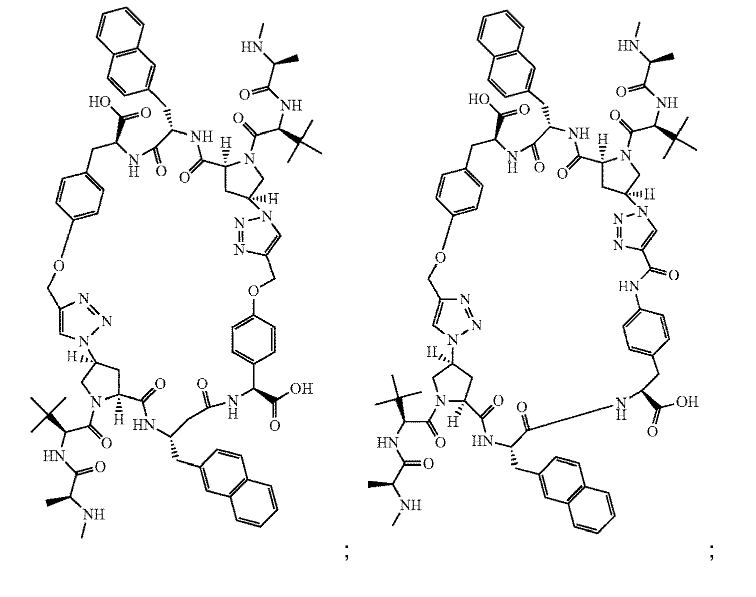

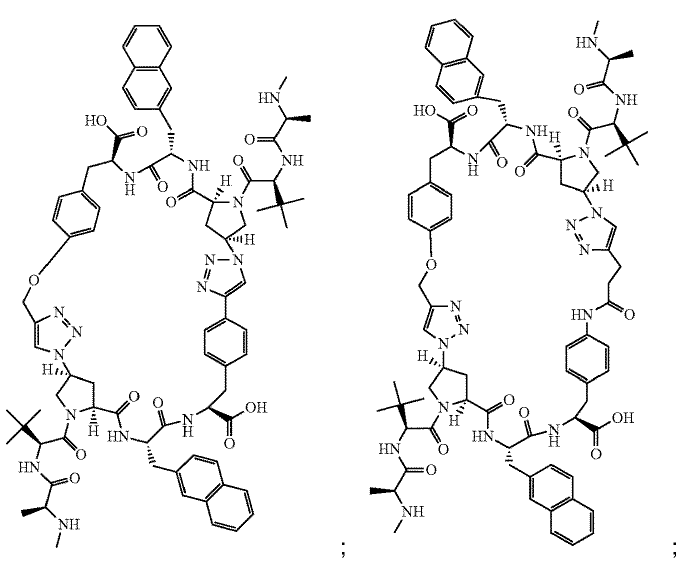

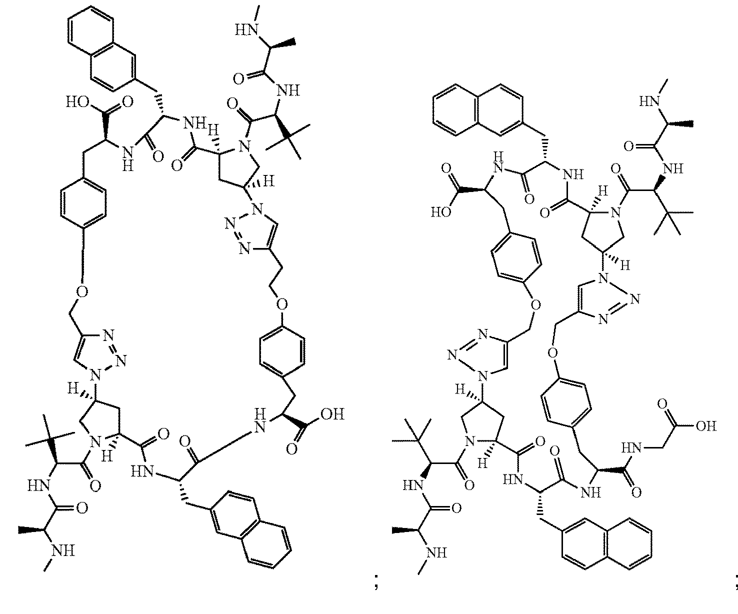

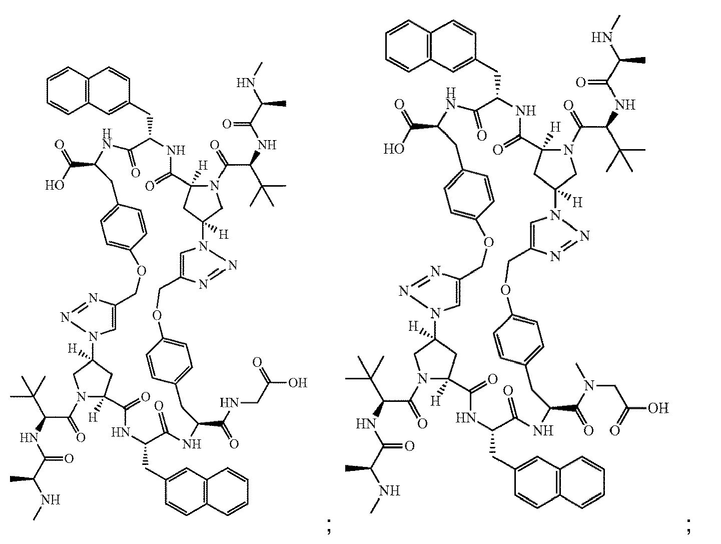

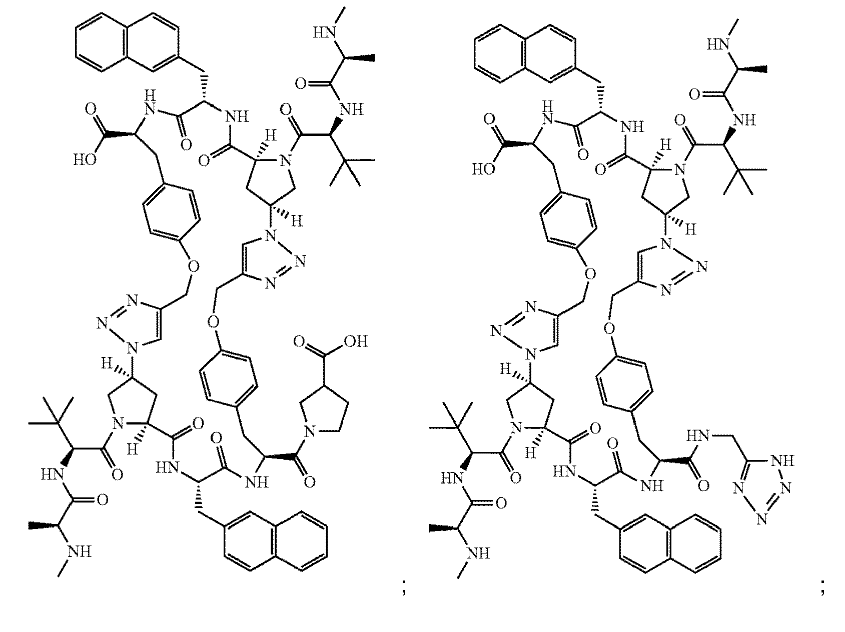

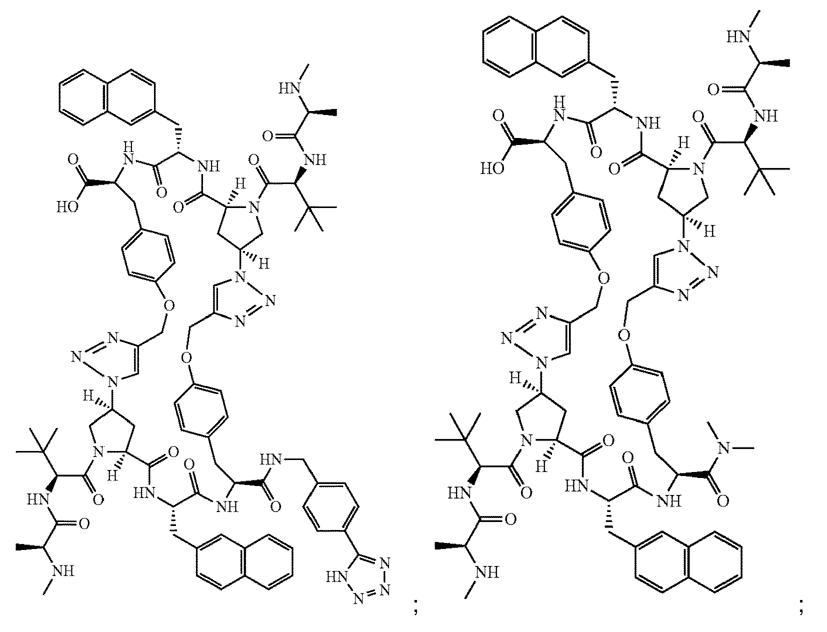

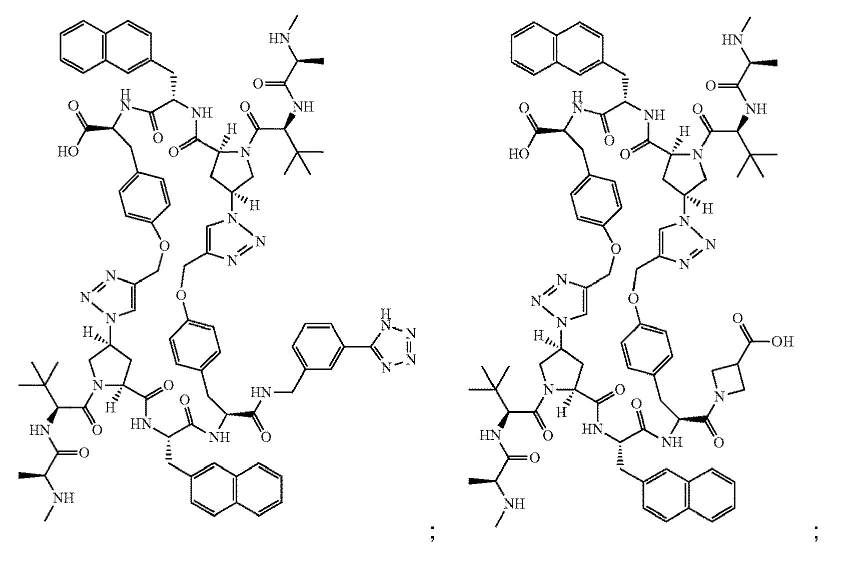

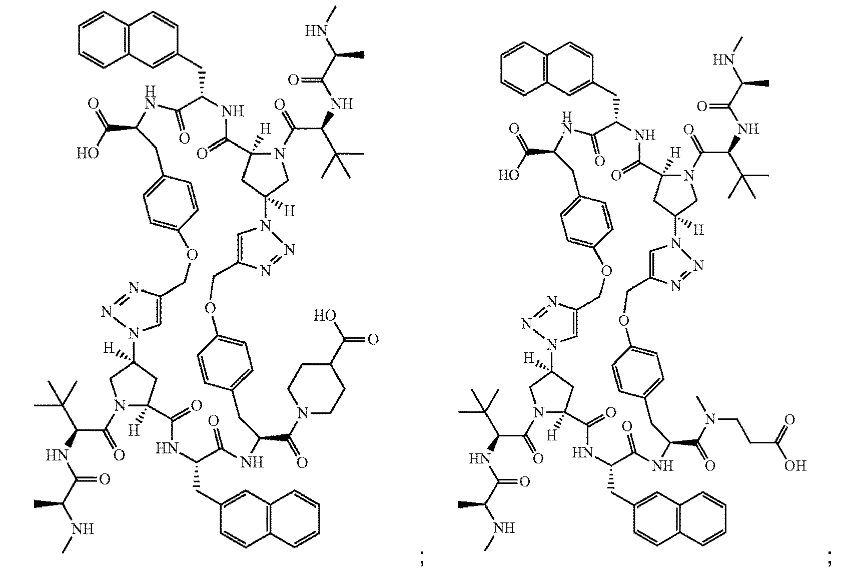

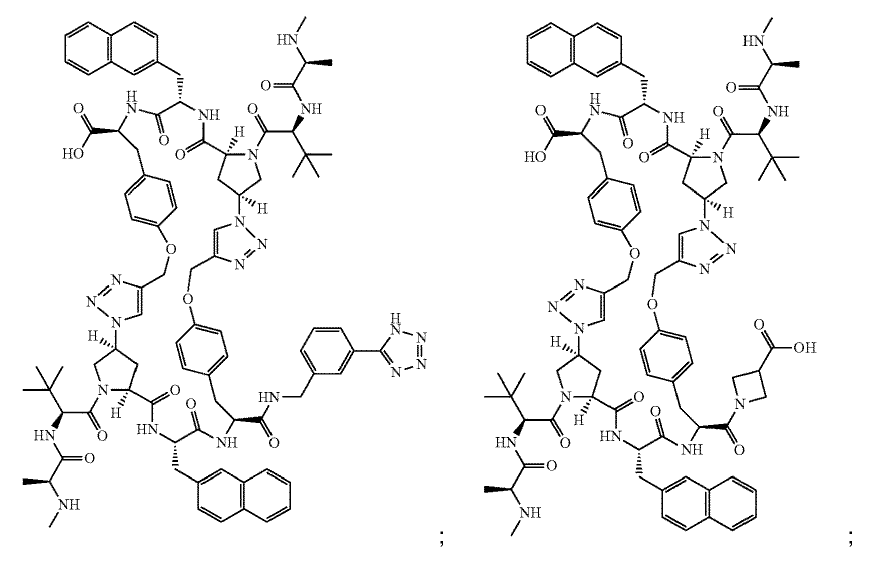

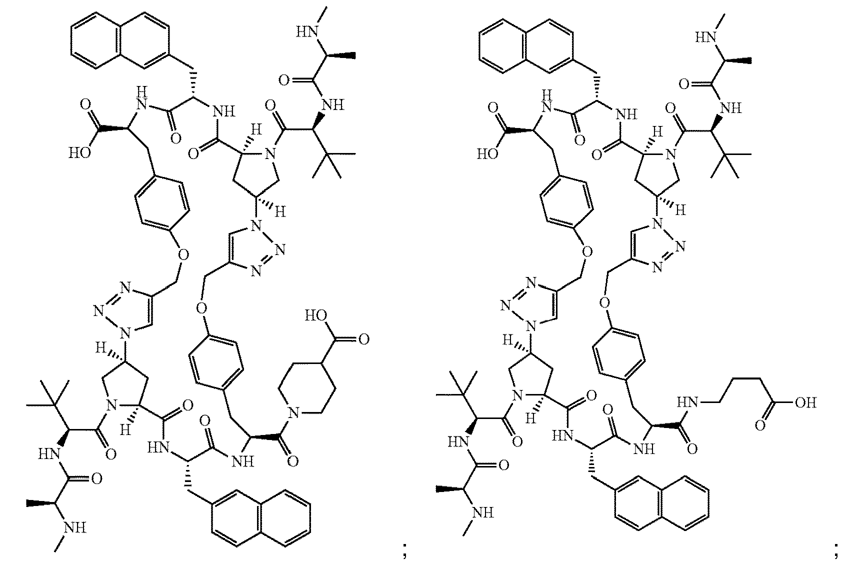

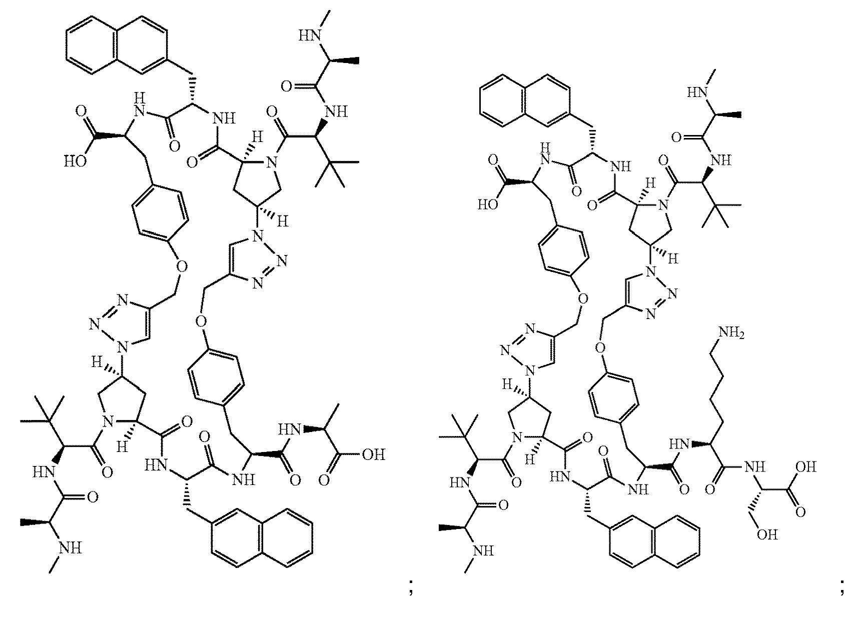

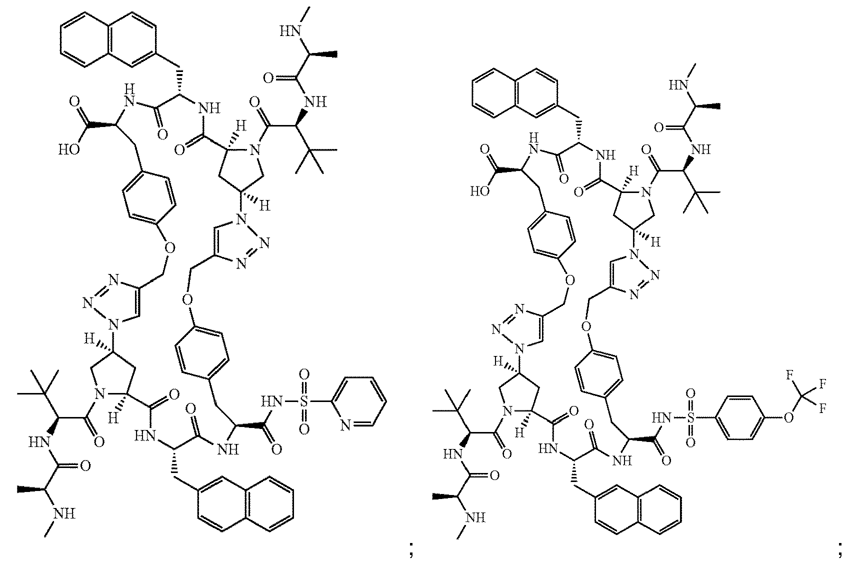

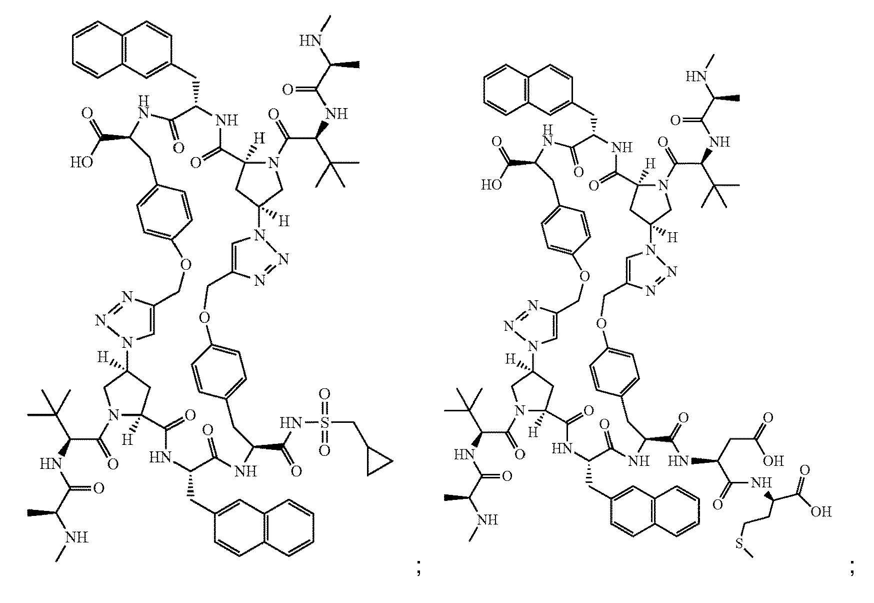

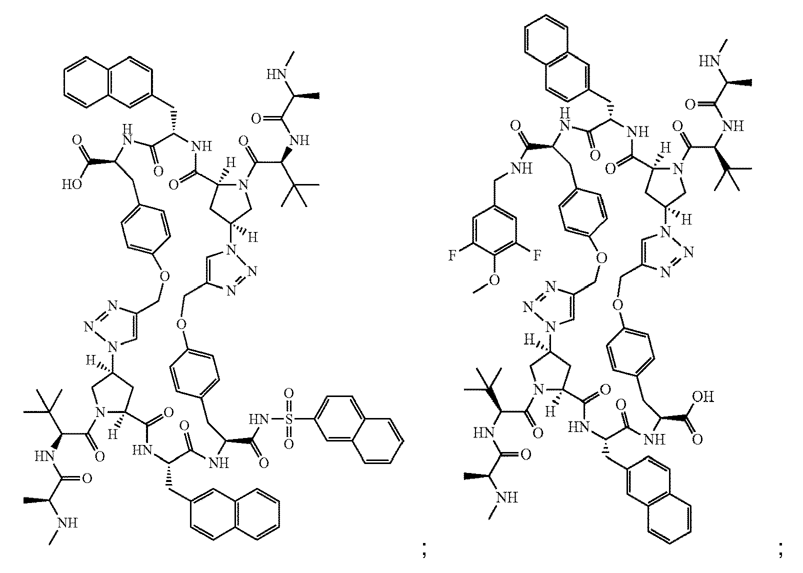

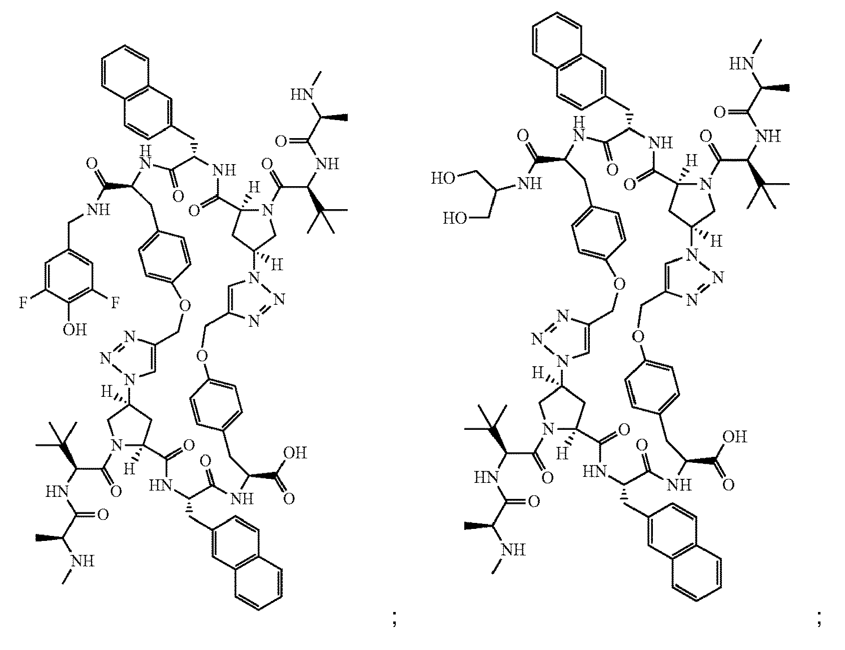

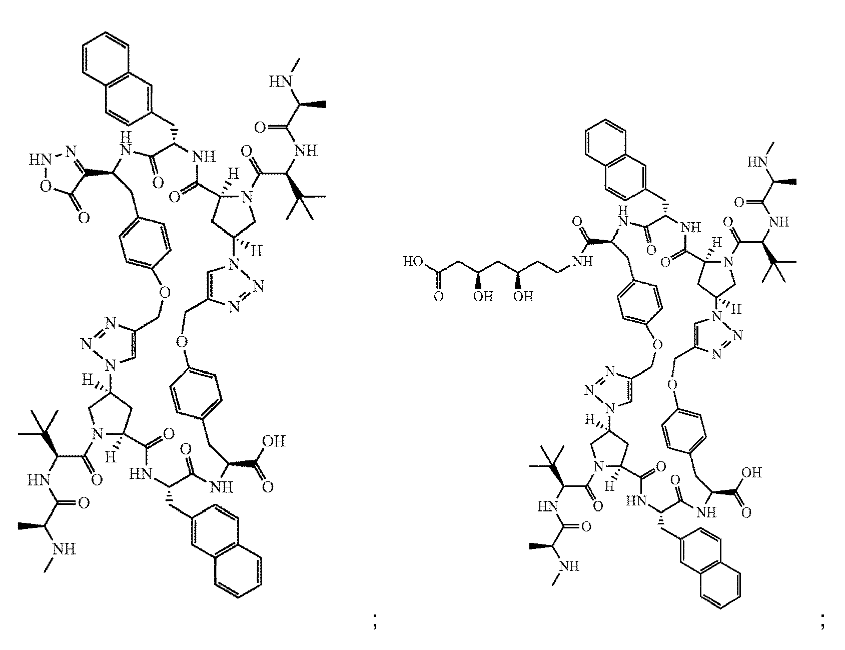

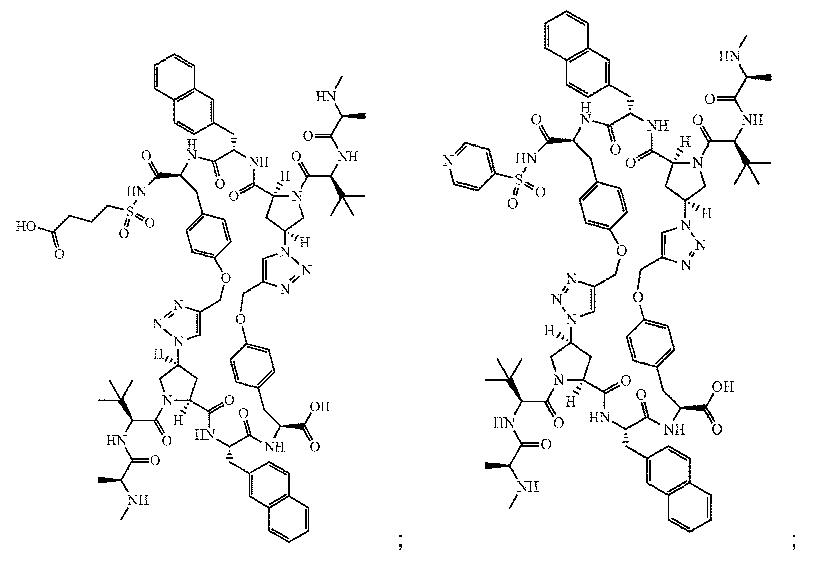

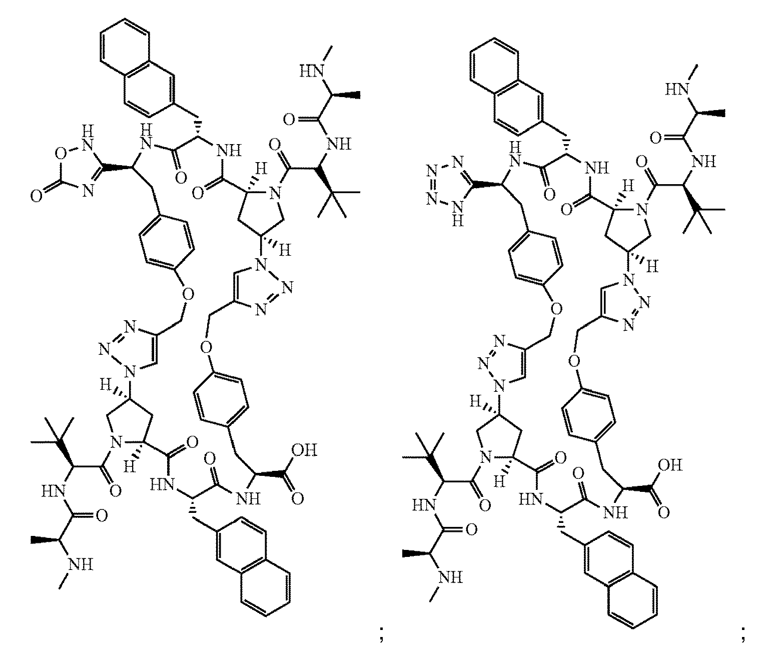

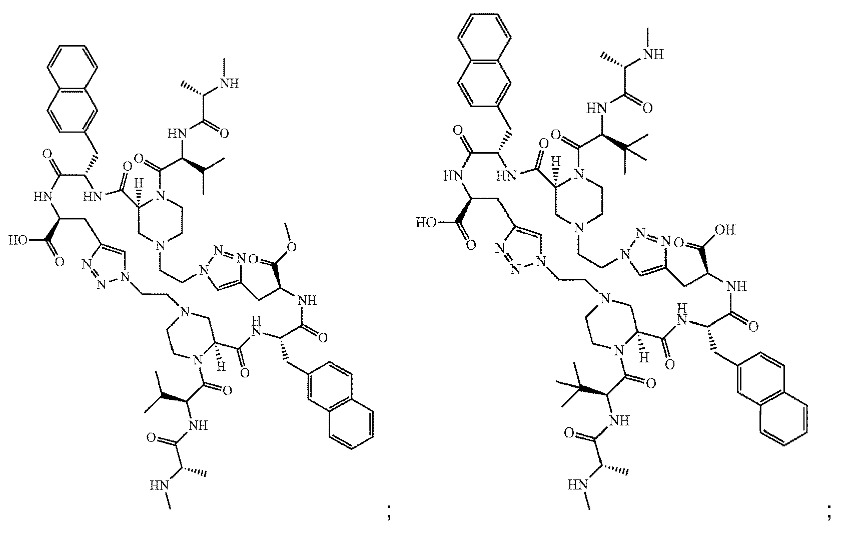

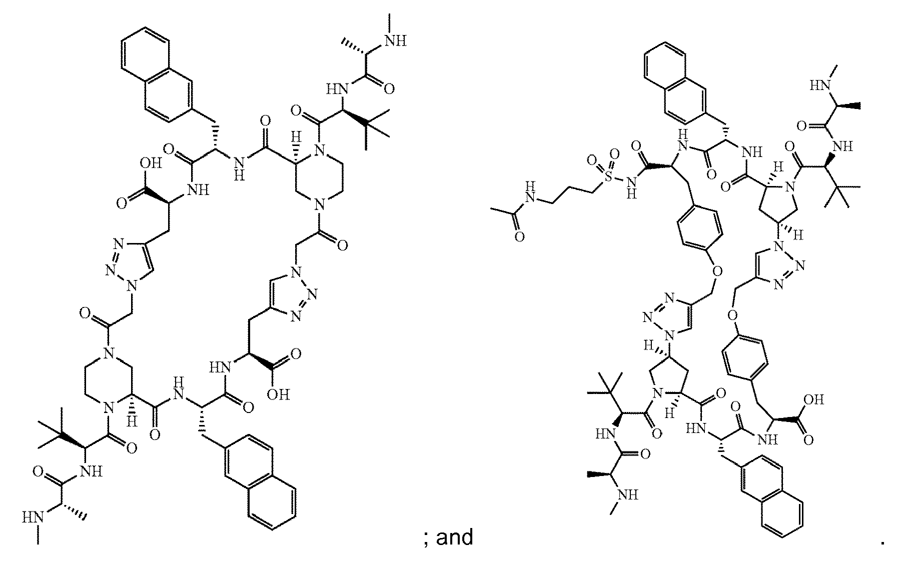

14. A compound according to any preceding claim for use as a medicament.

15. A pharmaceutically acceptable composition comprising a compound according to any of claims 1 to 13 and a pharmaceutically acceptable carrier.

16. A compound according to any of claims 1 to 13 or a pharmaceutical composition according to claim 15 for use in the treatment of cancer.

## Patentansprüche

1. Verbindung mit der Formel I: 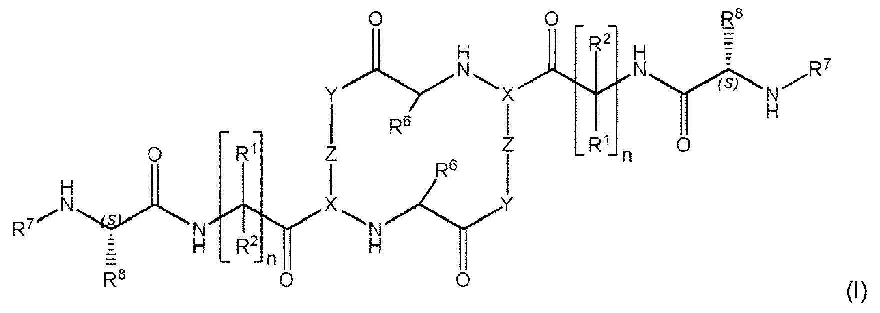 oder pharmazeutisch unbedenkliches Salz davon, wobei:
jedes n unabhängig 1 oder 2 ist;
jeder R¹ unabhängig ausgewählt ist aus Wasserstoff, Cycloalkyl und -(C₁-C₄-Alkylen)-R⁴, wobei jeder R⁴ unabhängig ausgewählt ist aus Wasserstoff, Aryl und Cycloalkyl, wobei wenigstens ein R¹ etwas anderes ist als Wasserstoff; und
jeder R² Wasserstoff ist; oder
R¹ und R² zusammen zum Kohlenstoffatom genommen werden, an das sie gemeinsam binden, um ein Cycloalkyl zu bilden;
jeder R⁶ unabhängig -(C₁-C₄-Alkylen)-R⁹ ist, wobei jeder R⁹ unabhängig ausgewählt ist aus Wasserstoff, Aryl, Heteroaryl und Cycloalkyl; wobei jeder beliebige Aryl-, Heteroaryl- oder Cycloalkyl-Teil von R⁶ optional substituiert ist durch bis zu zwei Substituenten, die unabhängig ausgewählt sind aus Halo, CF₃, OH, C₁-C₄-Alkoxy, C₁-C₄-Alkenyloxy, Phenyl, Phenyloxy und Phenylmethyloxy; und wobei ein -CH₂- im Teil -(C₁-C₄-Alkylen)- von R⁶ optional ersetzt wird durch -O-;
jeder R⁷ unabhängig ausgewählt ist aus Wasserstoff und Methyl;
jeder R⁸ unabhängig ausgewählt ist aus Methyl und Ethyl;
jedes X unabhängig ausgewählt ist aus Folgenden: 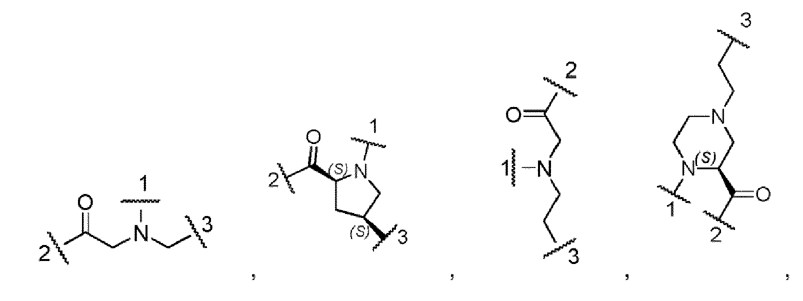 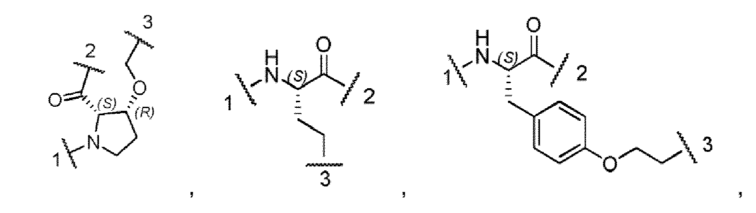 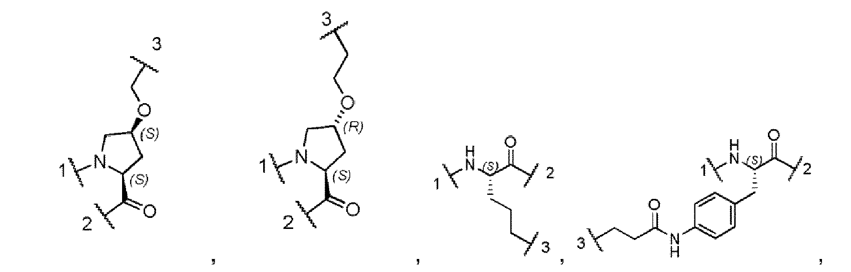 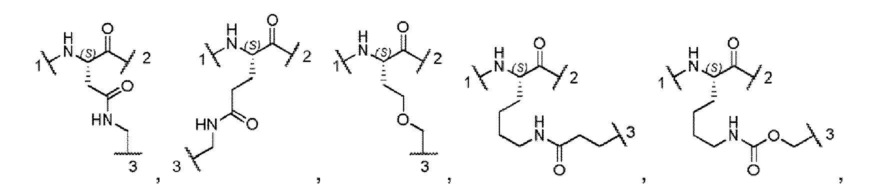 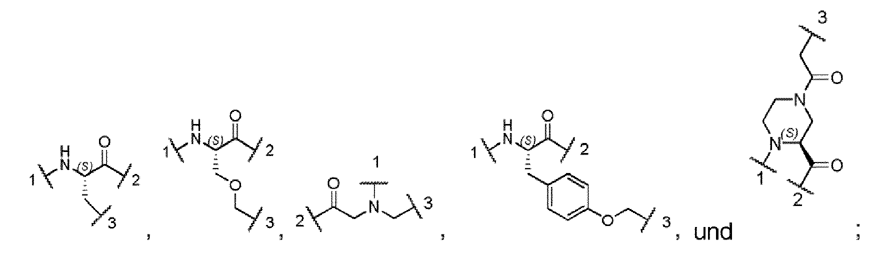
jedes 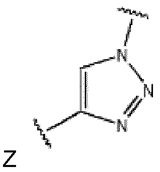 ist, wobei jedes 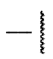 einen Bindungspunkt an die Verbindung repräsentiert; und
jedes Y ausgewählt ist aus Folgenden: 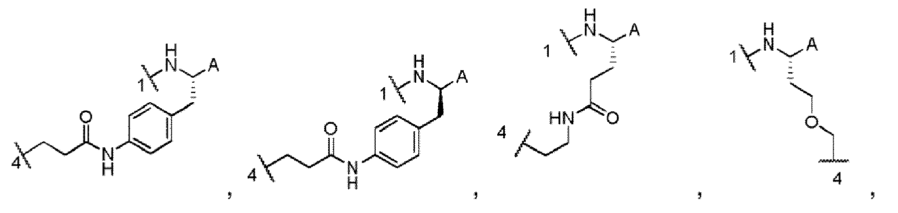 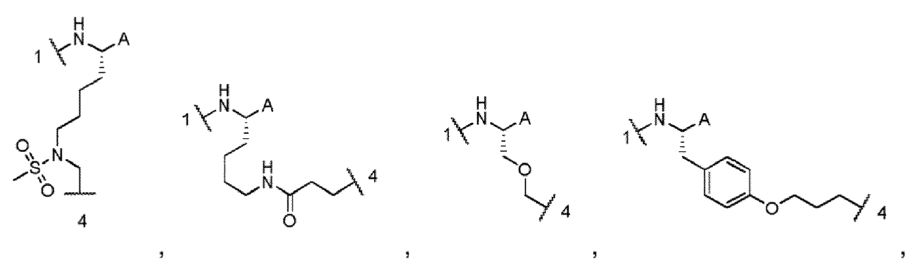 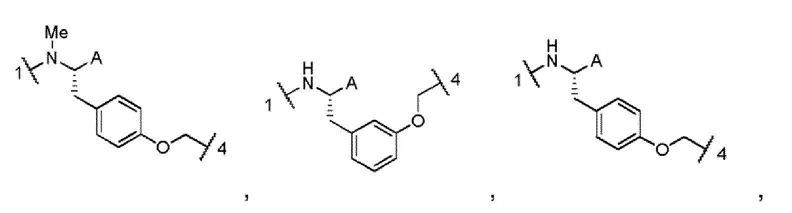 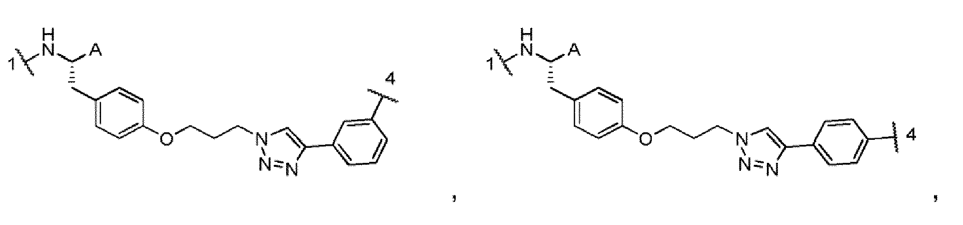 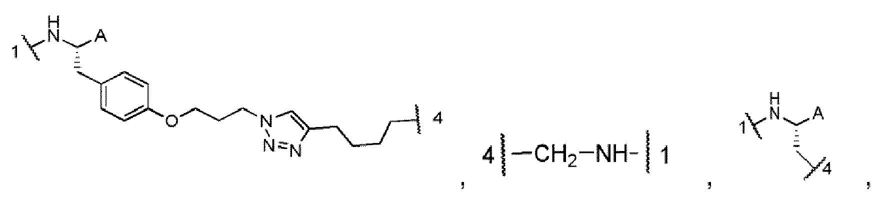 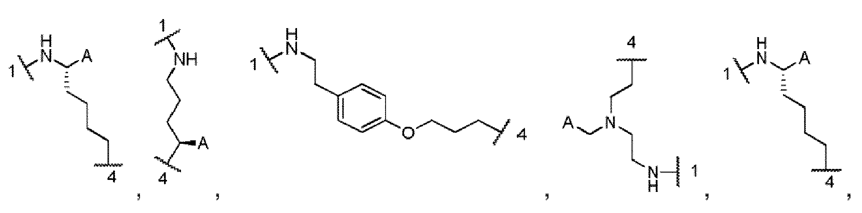 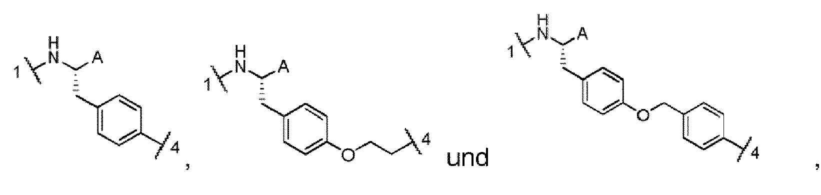 wobei: 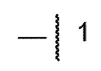
einen Bindungspunkt an einem Teil -C=O der Verbindung repräsentiert; 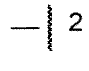
einen Bindungspunkt an einem Teil -NH der Verbindung repräsentiert; 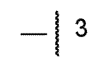
einen ersten Bindungspunkt an Z repräsentiert; 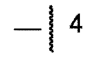
einen zweiten Bindungspunkt an Z repräsentiert; und
A ausgewählt ist aus -C(O)R³ oder 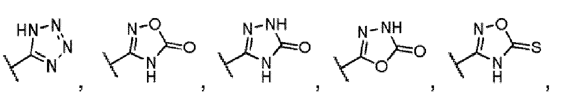 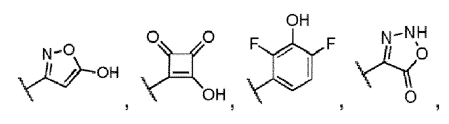 oder einer tautomeren Form eines der Vorhergehenden,
wobei:
R³ OH, -O-(C₁-C₄-Alkyl), NHCN, NHSO₂R¹⁰, NHOR¹¹ oder N(R¹²)(R¹³) ist;
R¹⁰ und R¹¹ ausgewählt sind aus -C₁-C₄-Alkyl, Cycloalkyl, Aryl, Heteroaryl oder Heterocycloalkyl, von denen jegliche optional substituiert sind, und Wasserstoff;
jeder von R¹² und R¹³ unabhängig ausgewählt ist aus Wasserstoff, -C₁-C₄-Alkyl, -(C₁-C₄-Alkylen)-NH-(C₁-C₄-Alkyl), -(C₁-C₄-Alkylen)-O-(C₁-C₄-Alkyl) und -(C₁-C₄-Alkylen)-O-(C₁-C₄-Hydroxyalkyl) oder R¹² und R¹³ mit dem Stickstoffatom zusammengenommen sind, an das sie gemeinsam binden, um ein gesättigtes Heterocyclyl zu bilden, optional umfassend ein zusätzliches Heteroatom, ausgewählt aus N, O und S und wobei das gesättigte Heterocyclyl optional substituiert ist durch Methyl.

2. Verbindung nach Anspruch 1, wobei R⁷ Methyl ist und R⁸ Methyl ist.

3. Verbindung nach Anspruch 1, wobei ein Teil der Verbindung, die durch -NH-[C(R¹)(R²)]ₙ-C(O)- repräsentiert ist, ausgewählt ist aus Folgenden: 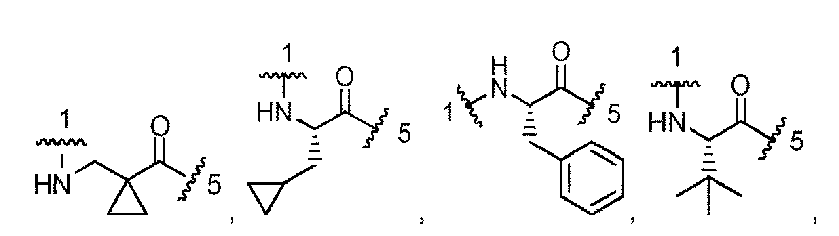 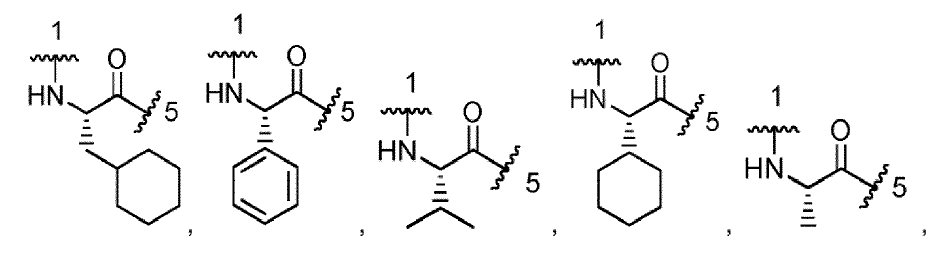 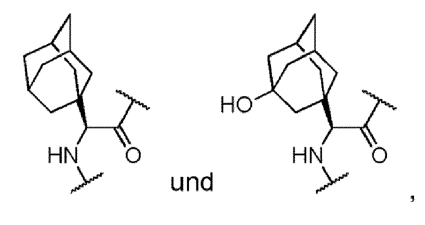 wobei: 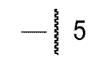
einen Bindungspunkt an den Aminoteil von X repräsentiert

4. Verbindung nach Anspruch 3, wobei ein Teil der Verbindung, die durch-NH-[C(R¹)(R²)]ₙ-C(O)- repräsentiert ist, 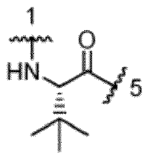 ist.

5. Verbindung nach Anspruch 1, wobei ein Teil der Verbindung, die durch-NH-CH(R⁶)-C(O)- repräsentiert ist, ausgewählt ist aus Folgenden: 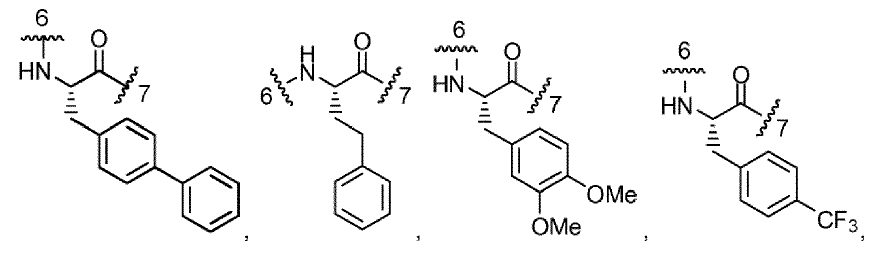 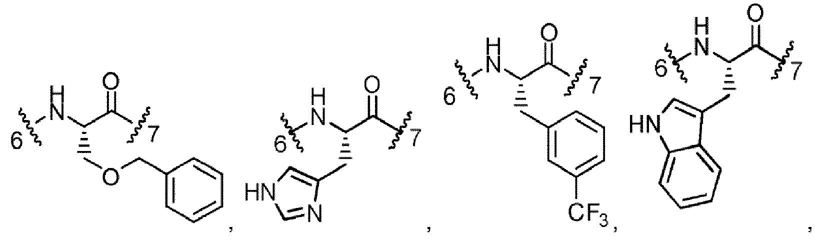 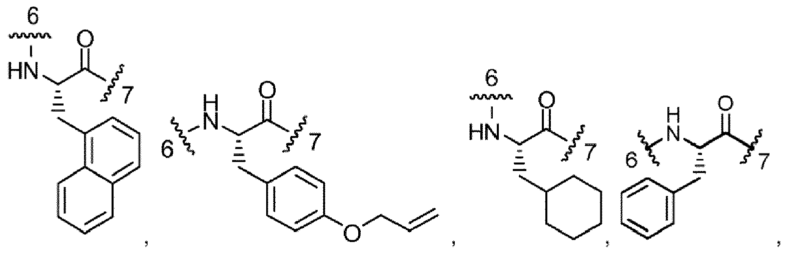 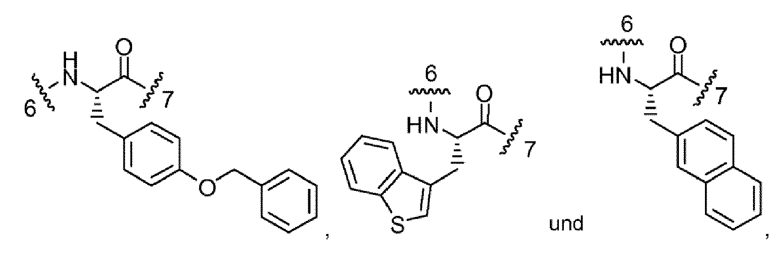 wobei: 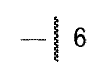
einen Bindungspunkt am Teil -C=O von X repräsentiert und 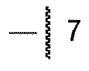
einen Bindungspunkt am Aminoteil von Y repräsentiert.

6. Verbindung nach Anspruch 5, wobei ein Teil der Verbindung, die durch -NH-CH(R⁶)-C(O)- repräsentiert ist, 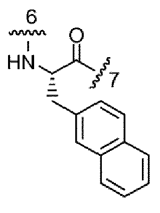 ist.

7. Verbindung nach Anspruch 1, wobei X ausgewählt ist aus Folgenden:

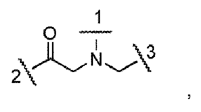

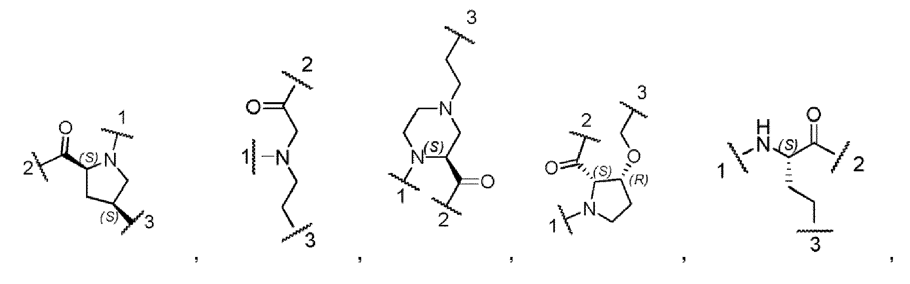

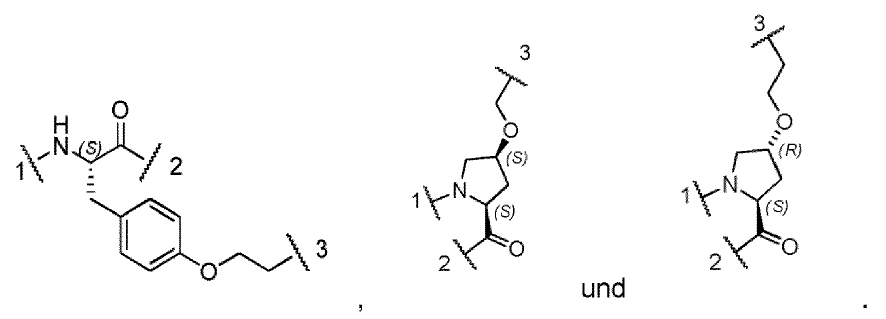

8. Verbindung nach Anspruch 7, wobei X ausgewählt ist aus Folgenden:

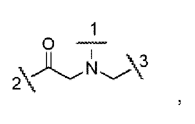

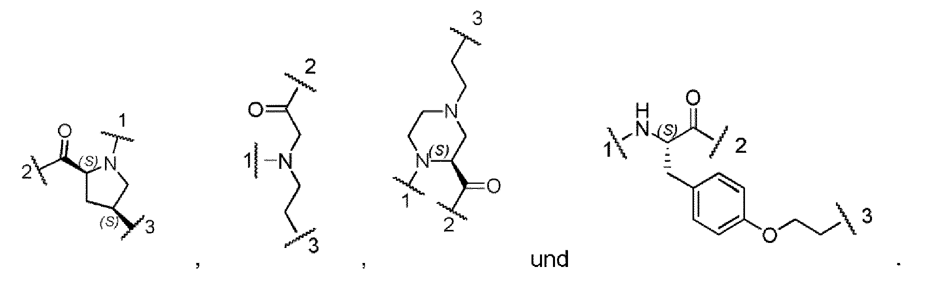

9. Verbindung nach Anspruch 8, wobei X 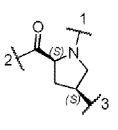 ist.

10. Verbindung nach Anspruch 1, wobei Y ausgewählt ist aus Folgenden:

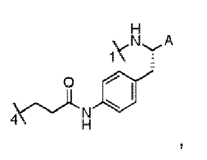

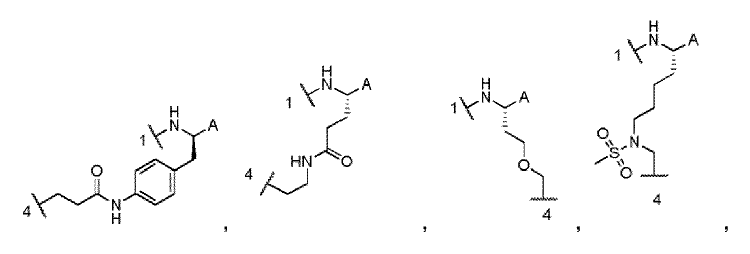

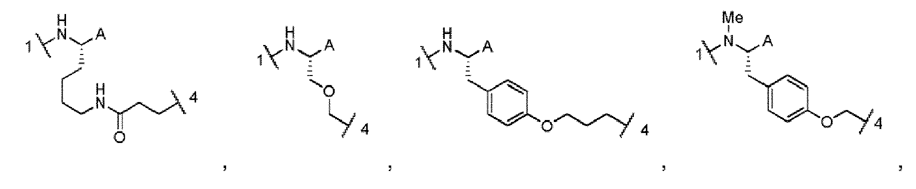

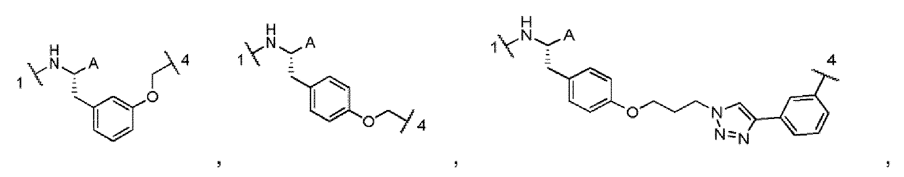

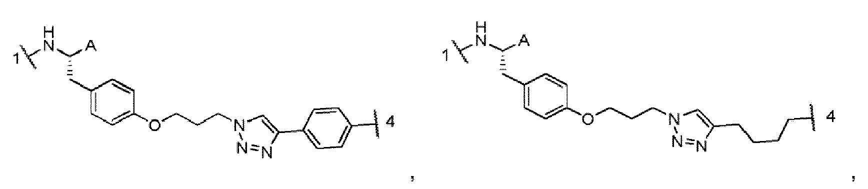

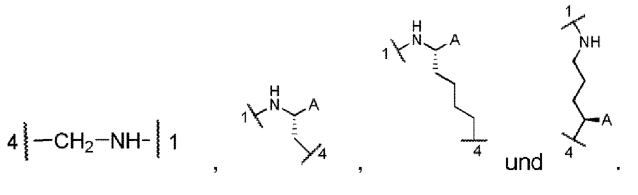

11. Verbindung nach Anspruch 10, wobei Y 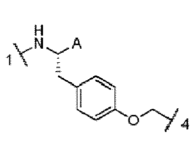 ist.

12. Verbindung nach Anspruch 1, wobei:
jeder R¹ gleich ist,
jeder R² gleich ist,
jeder R⁶ gleich ist,
jeder R⁷ gleich ist,
jeder R⁸ gleich ist,
jedes X gleich ist,
jedes Y gleich ist,
jedes Z gleich ist und
jedes n gleich ist.

13. Verbindung nach Anspruch 1, wobei die Verbindung ausgewählt ist aus der Gruppe bestehend aus Folgenden:

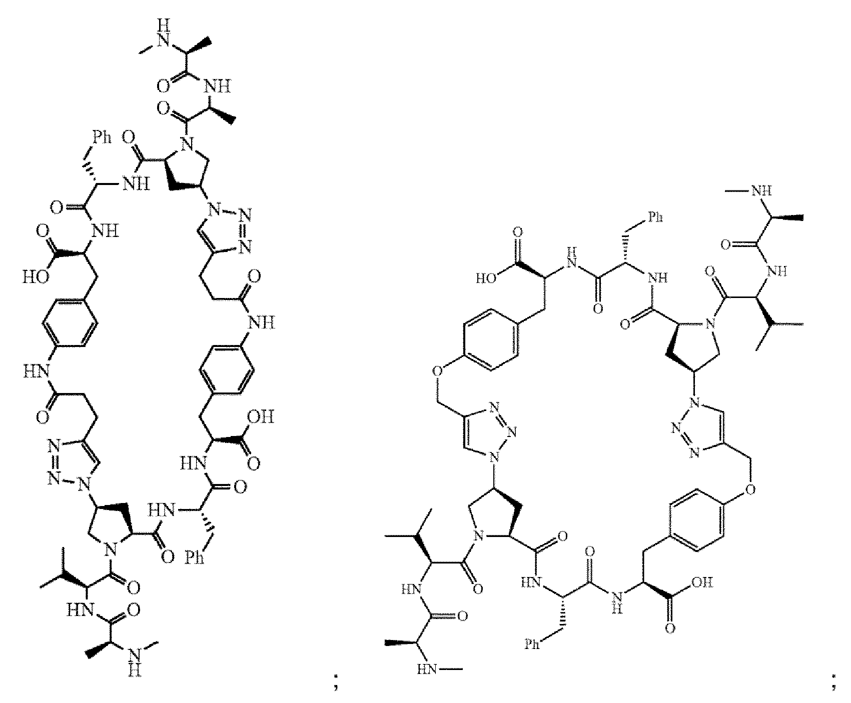

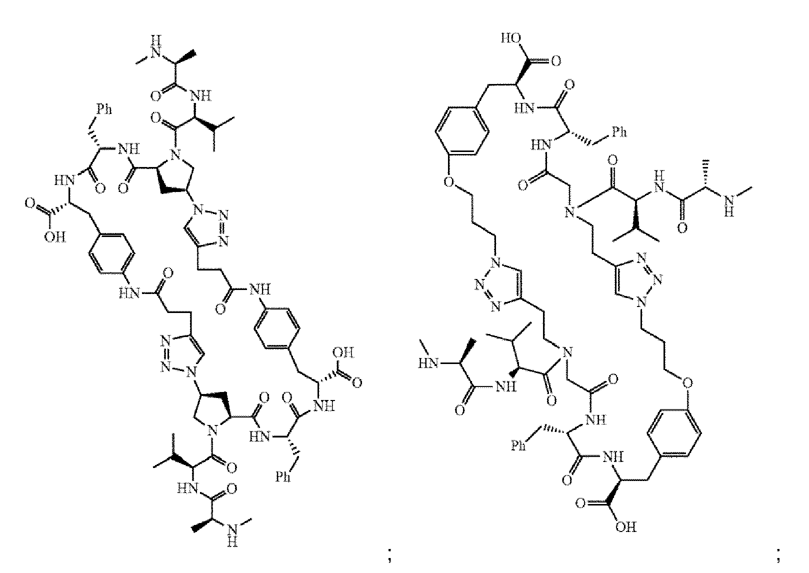

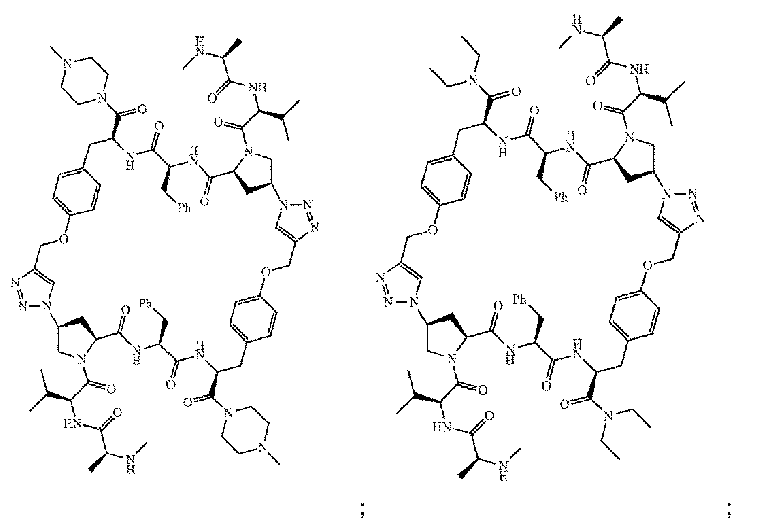

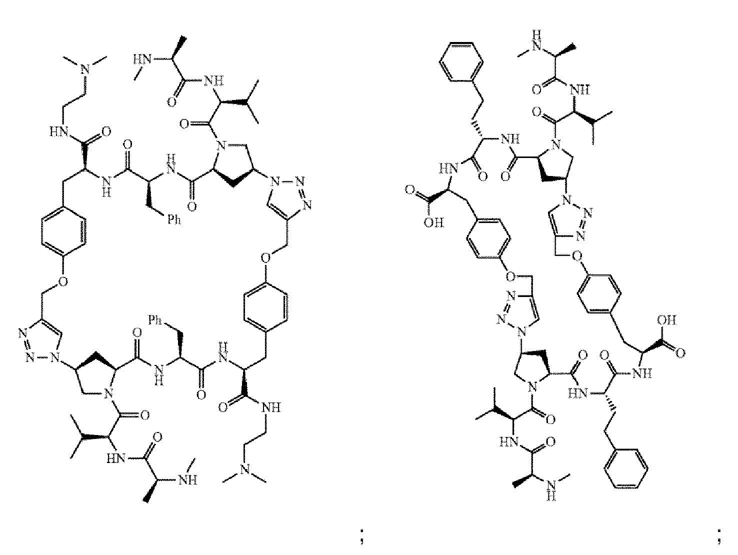

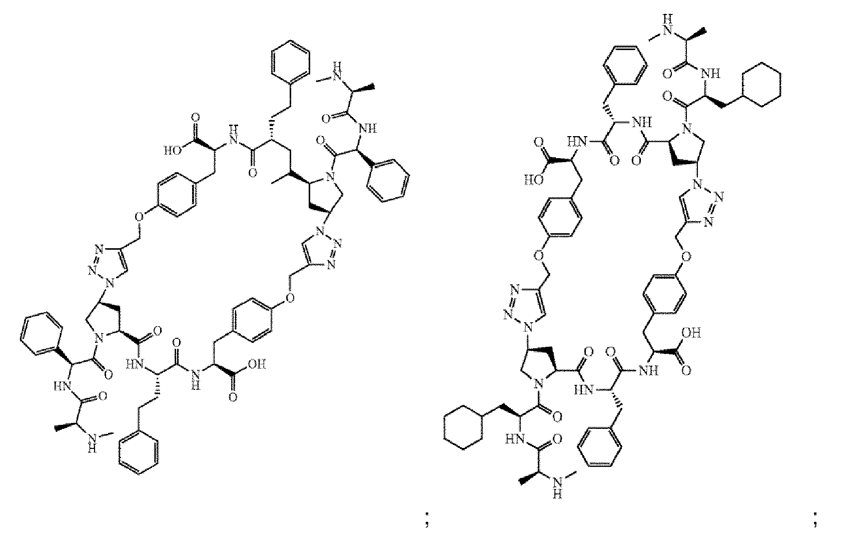

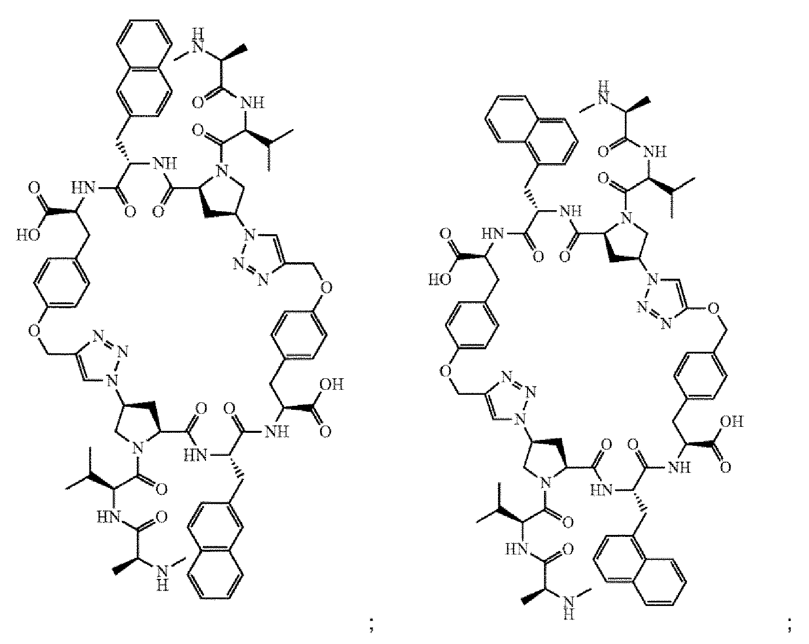

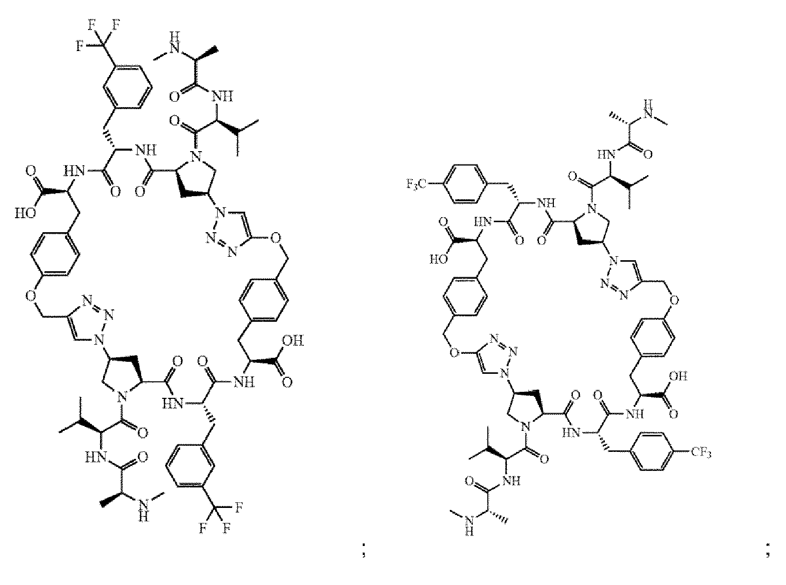

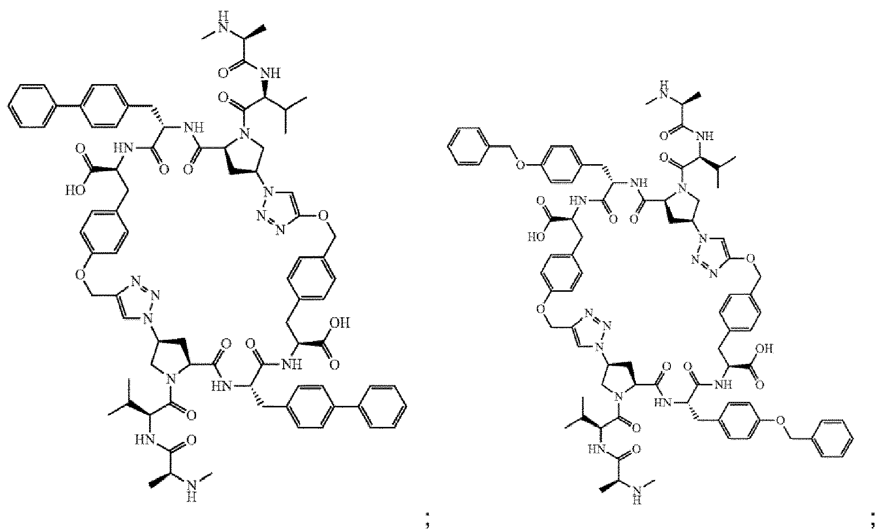

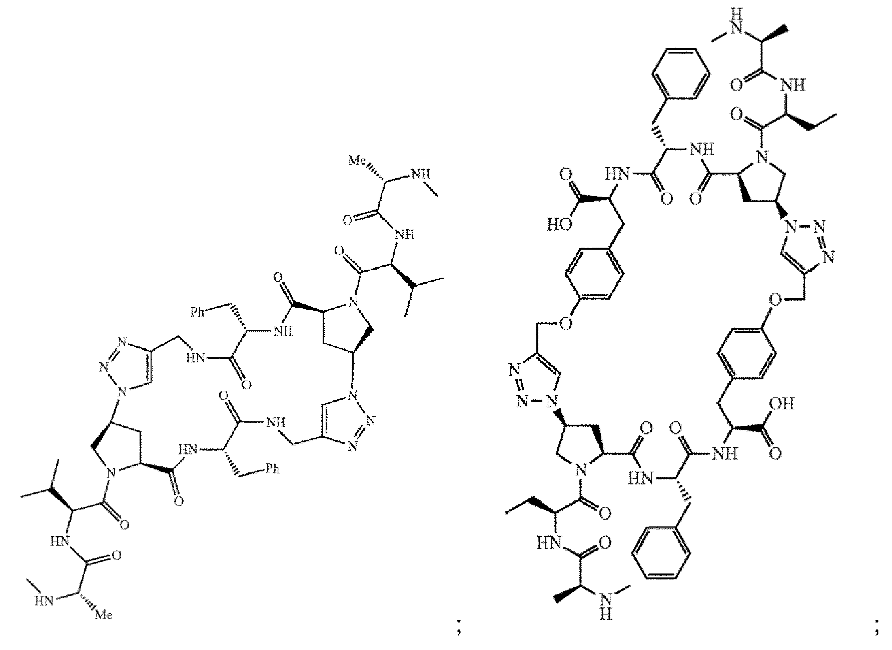

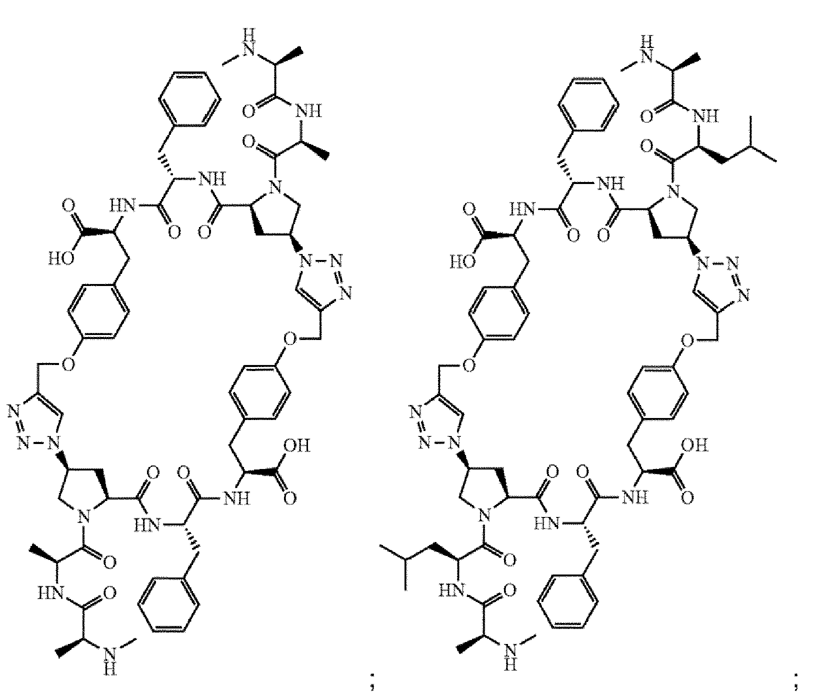

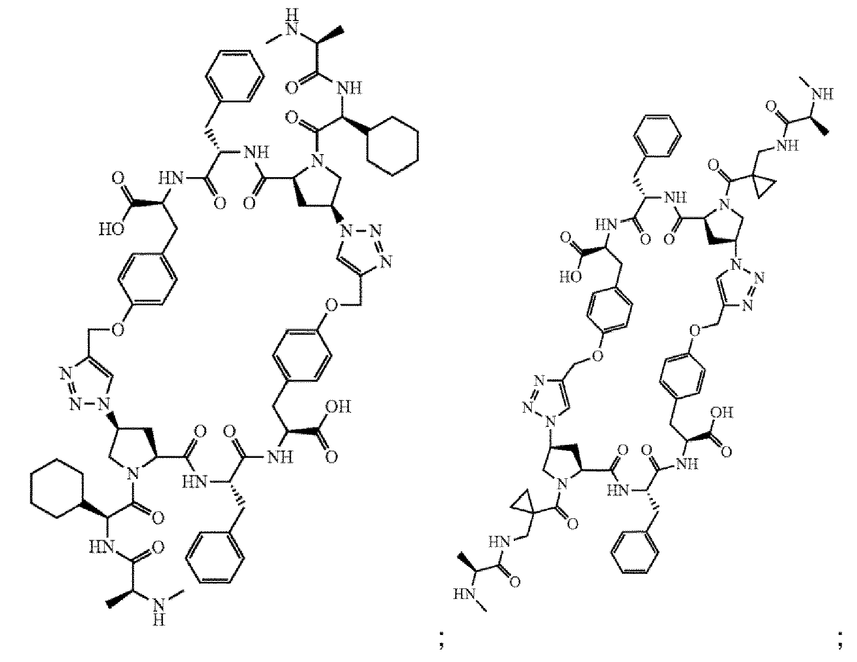

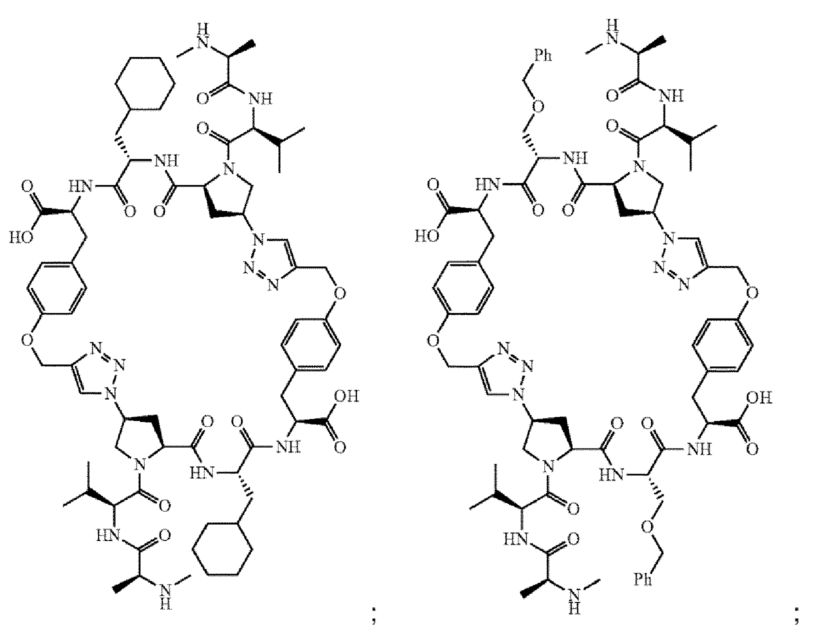

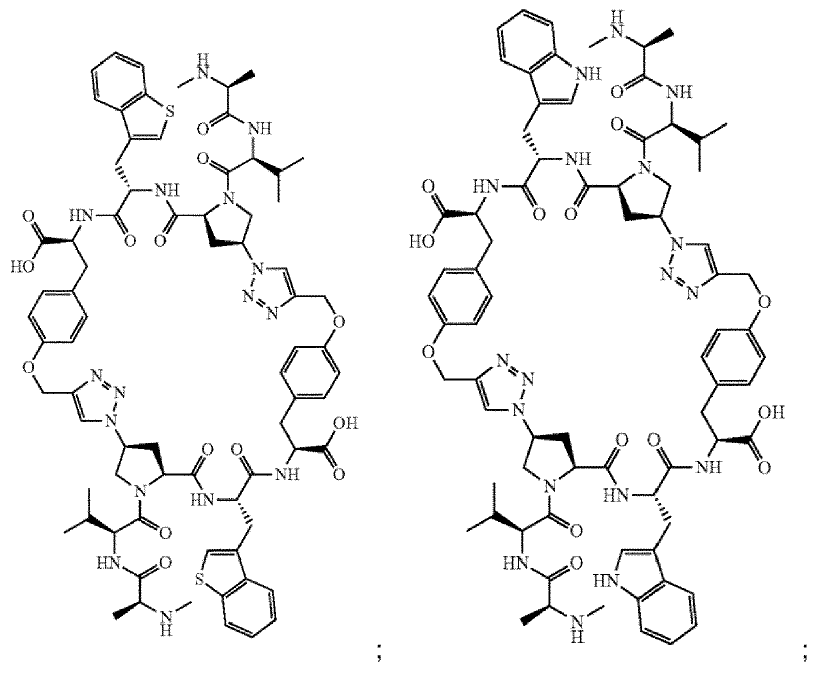

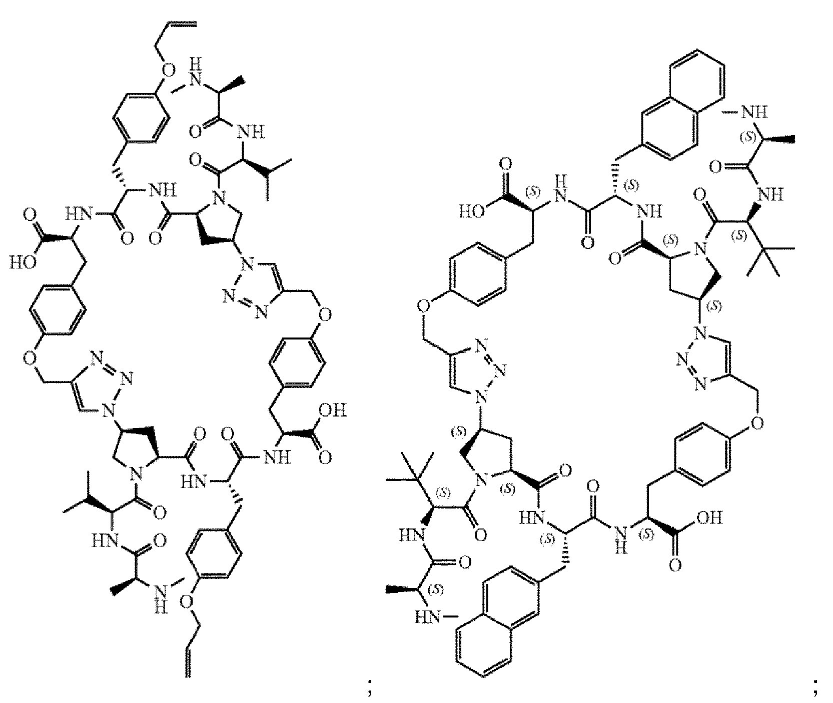

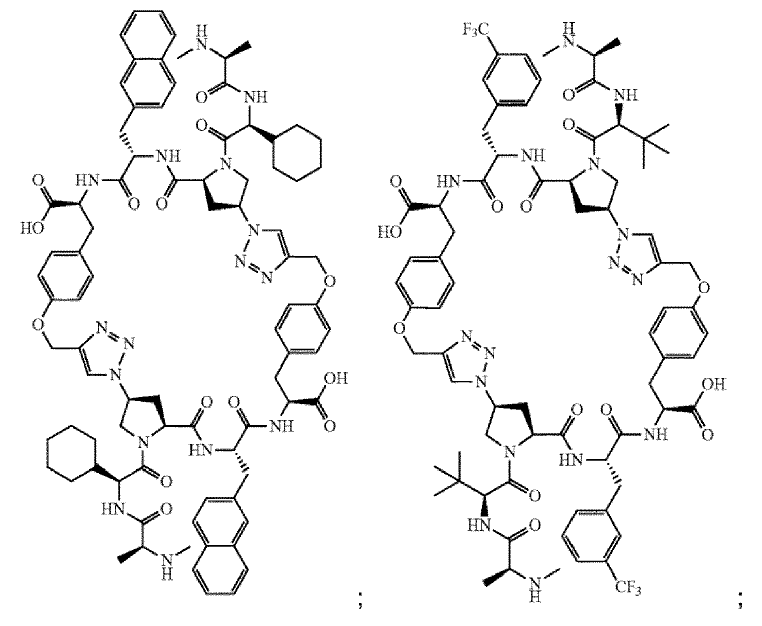

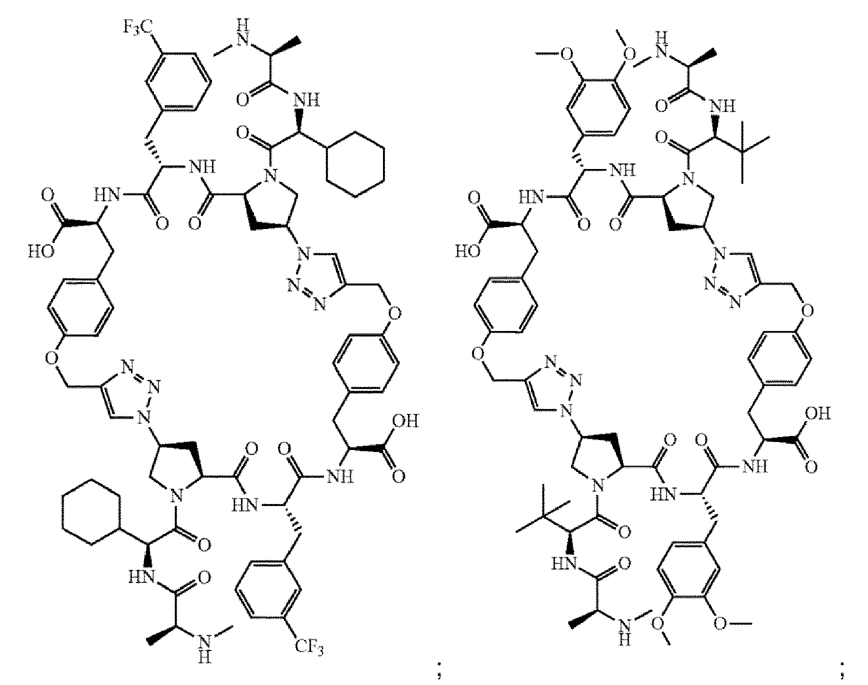

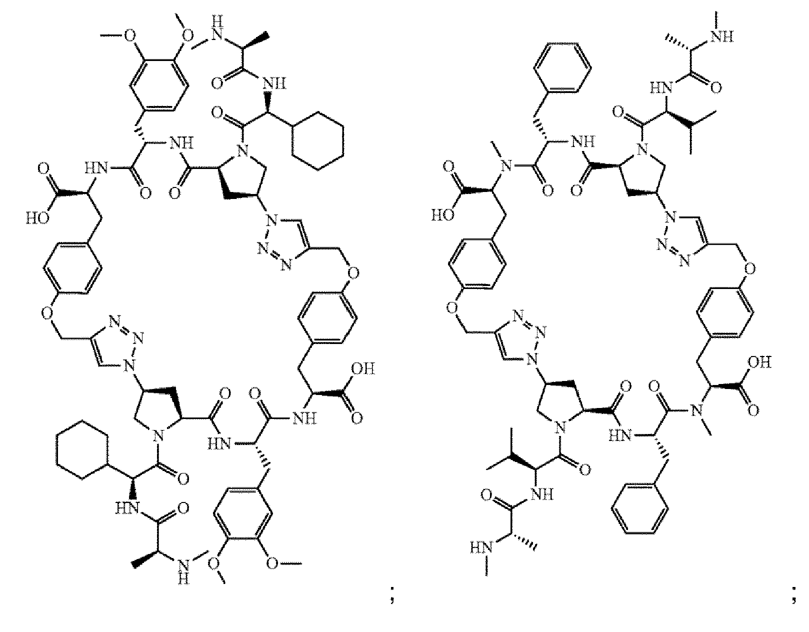

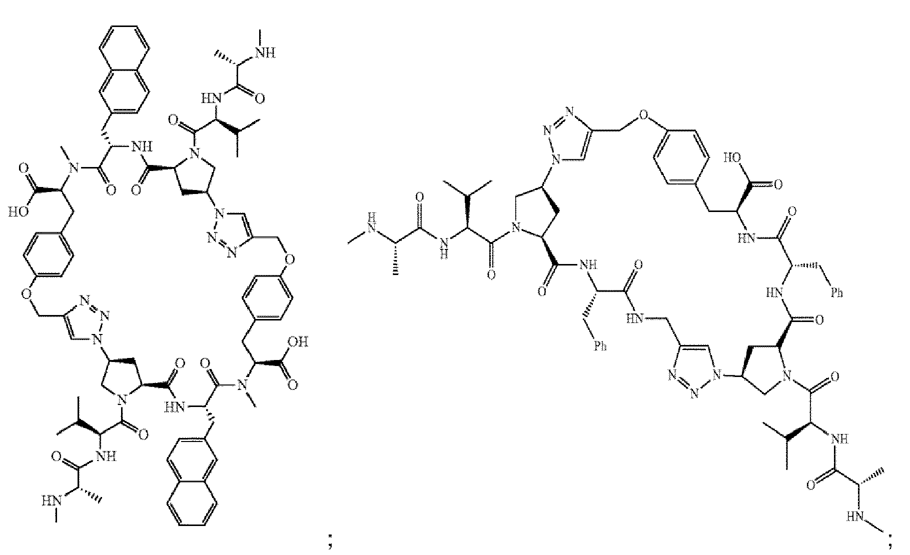

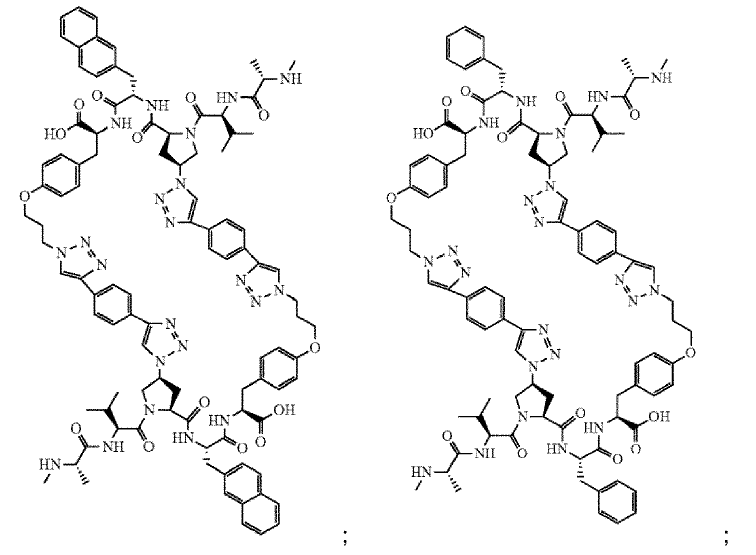

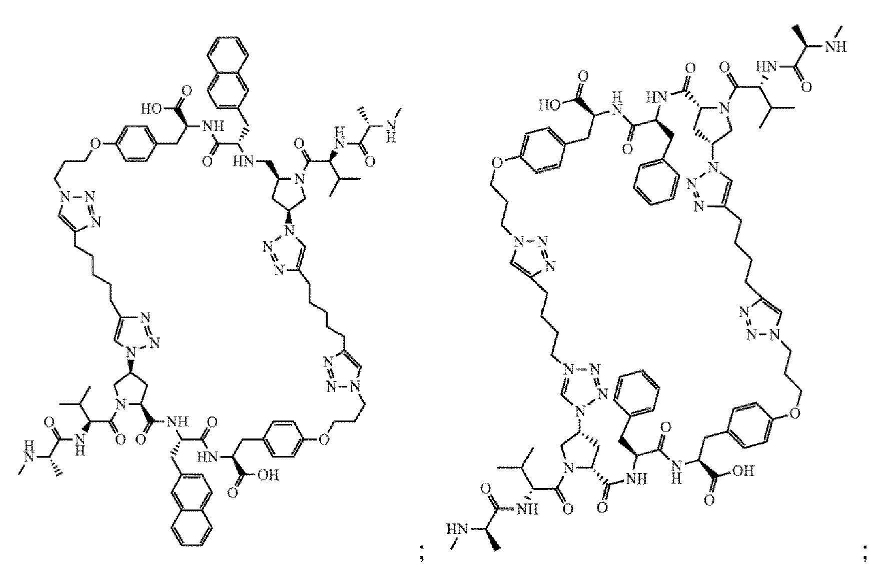

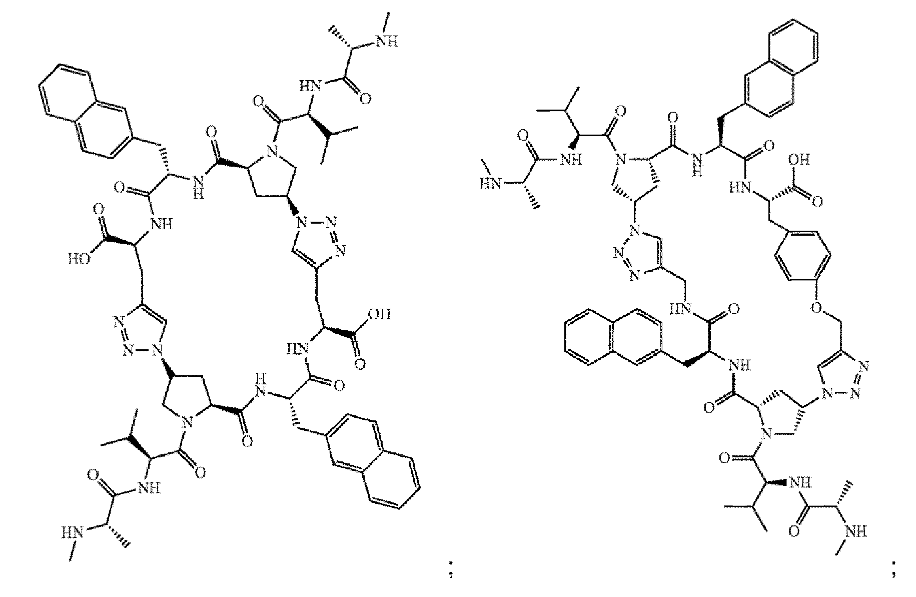

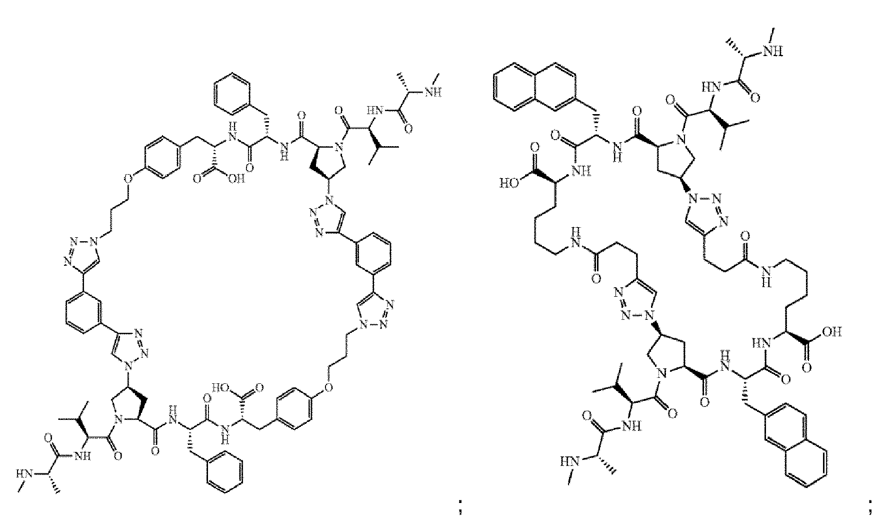

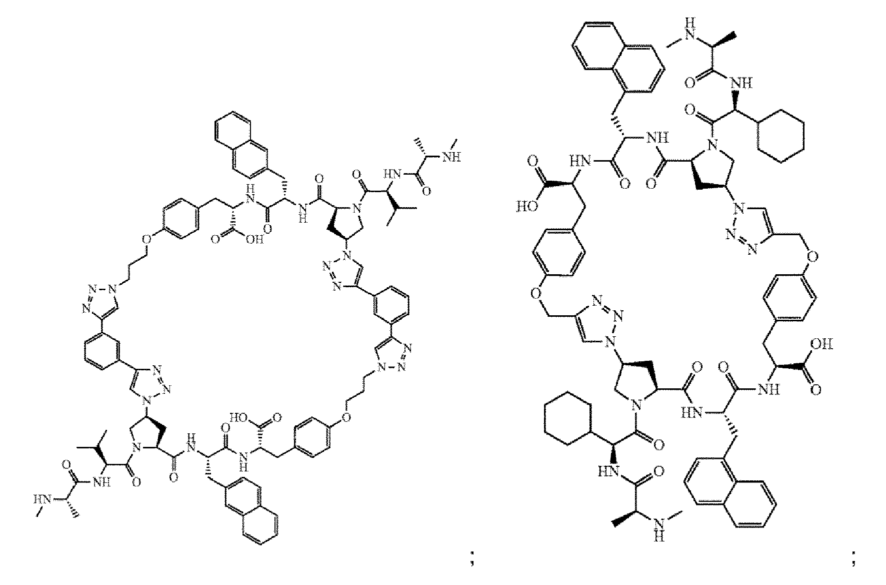

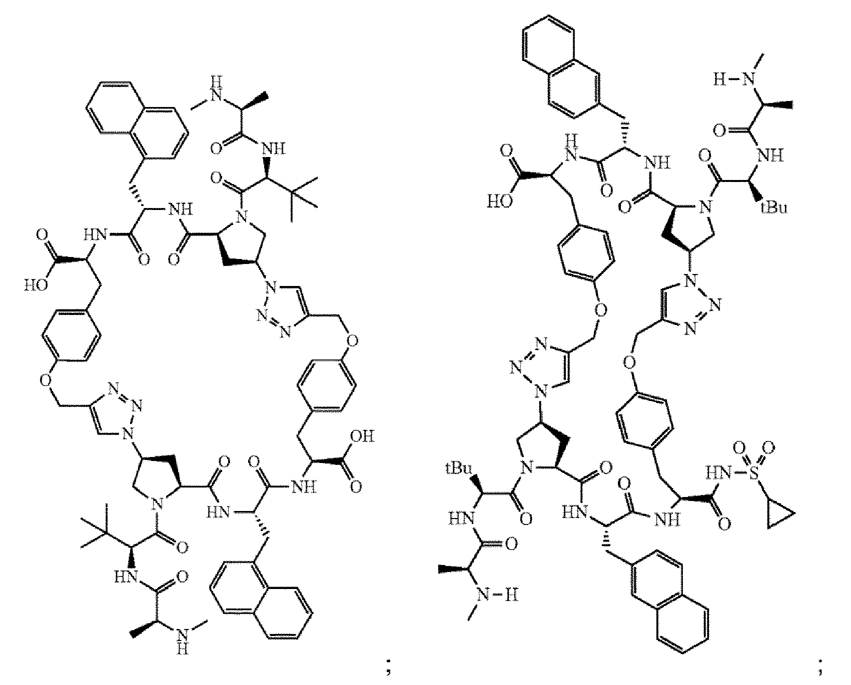

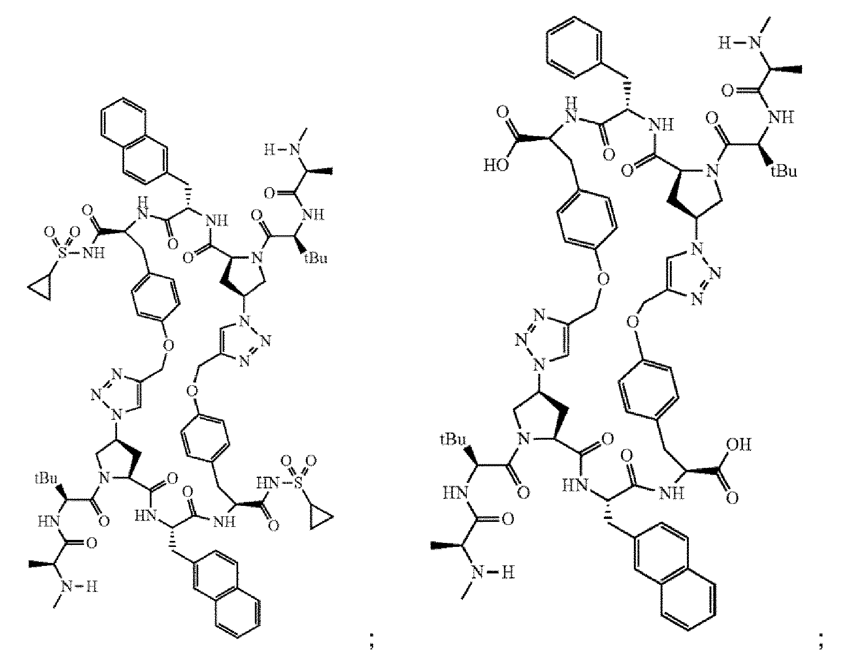

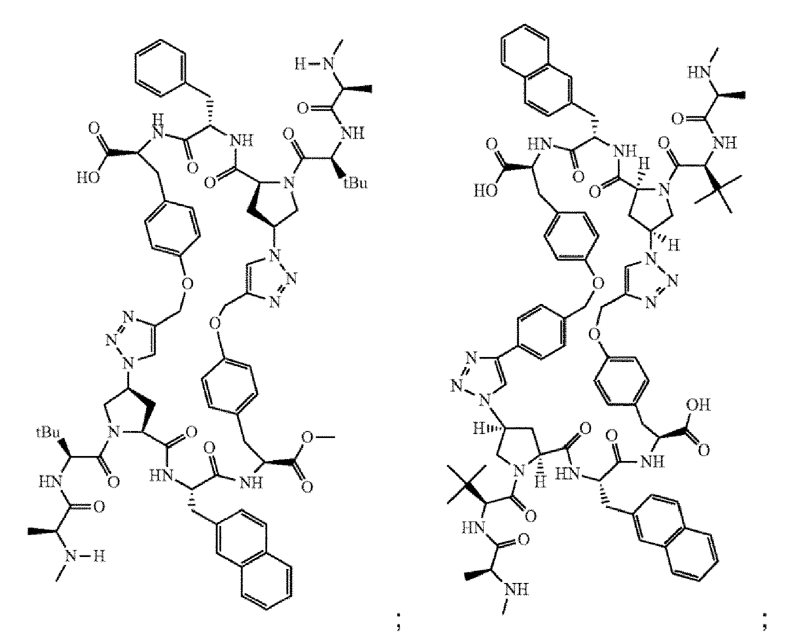

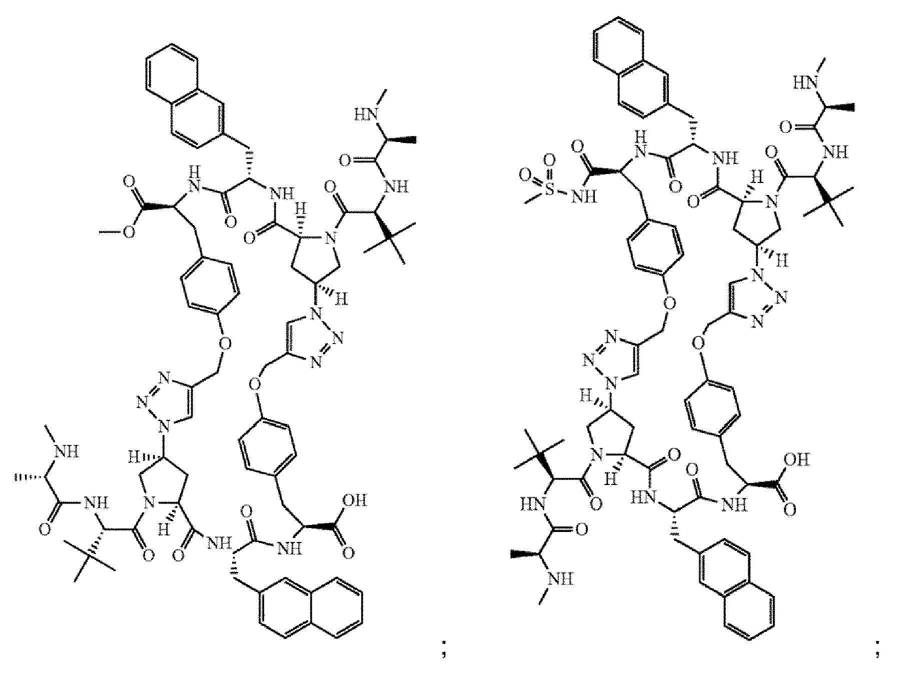

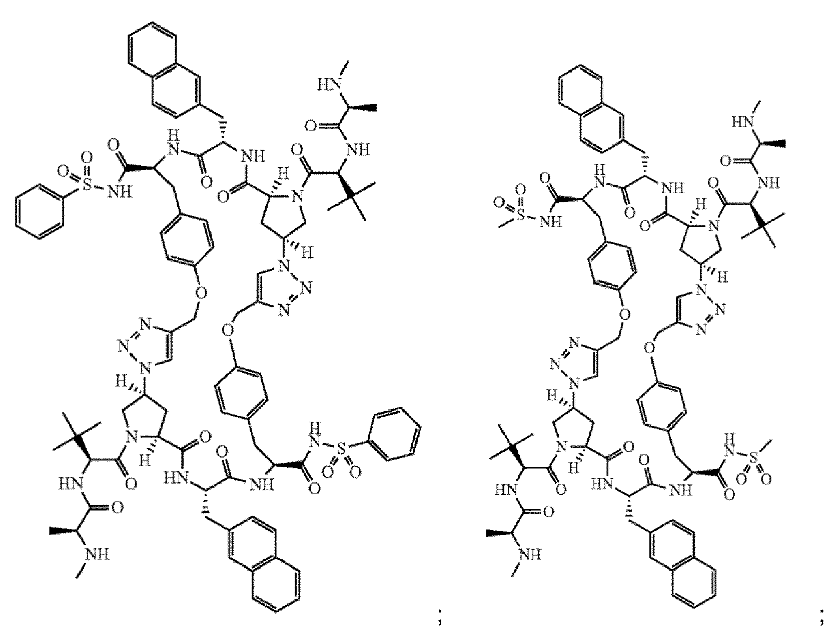

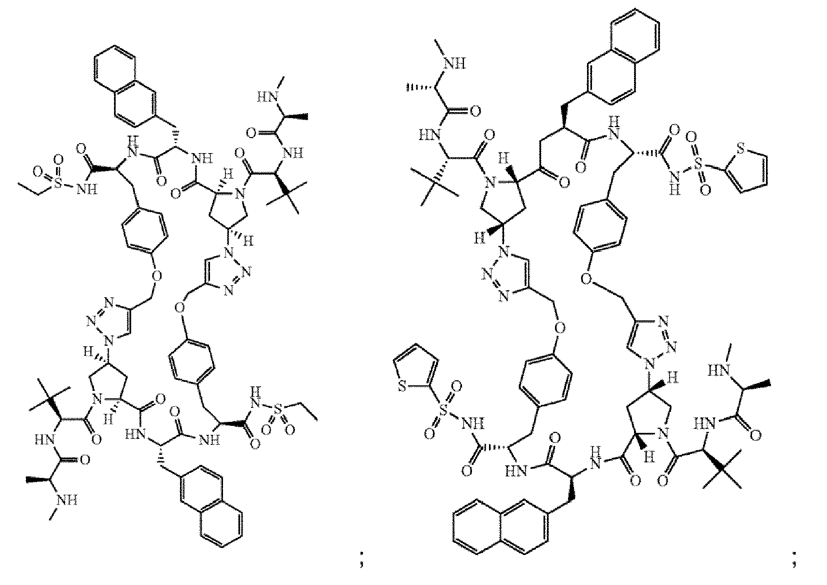

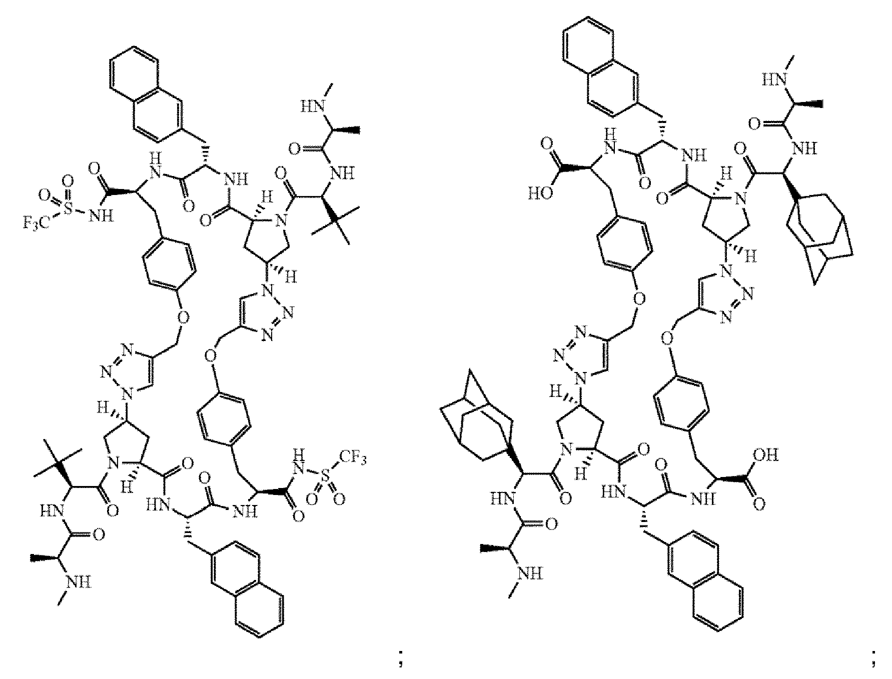

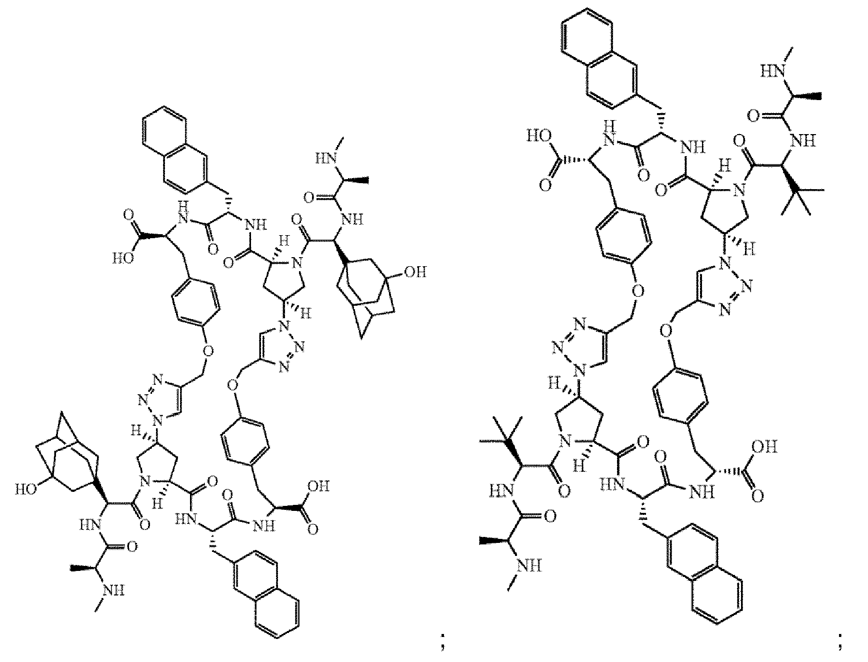

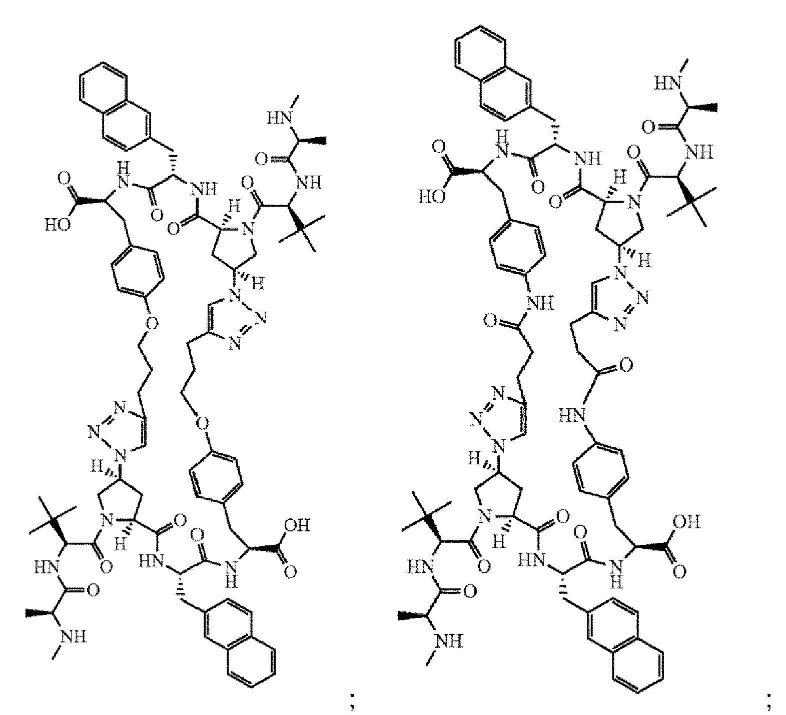

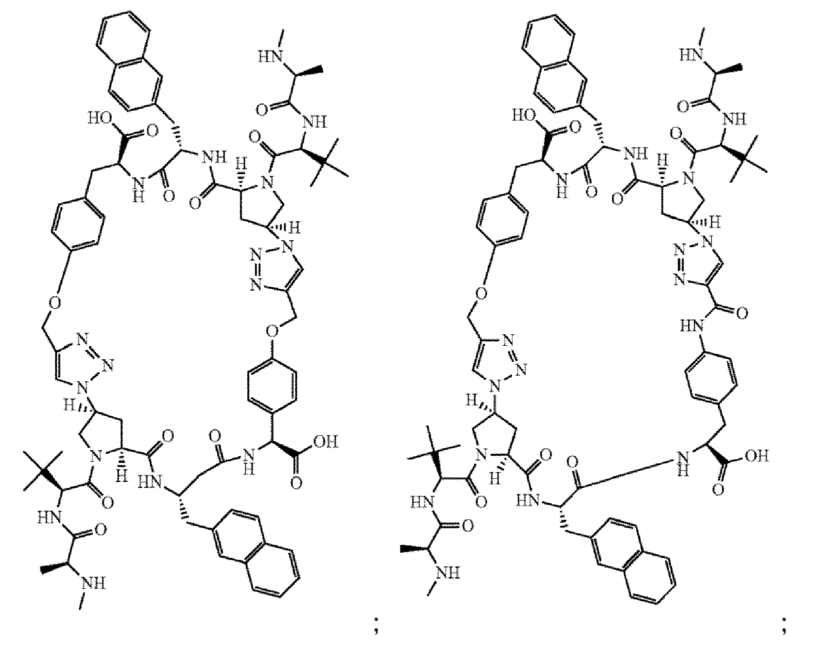

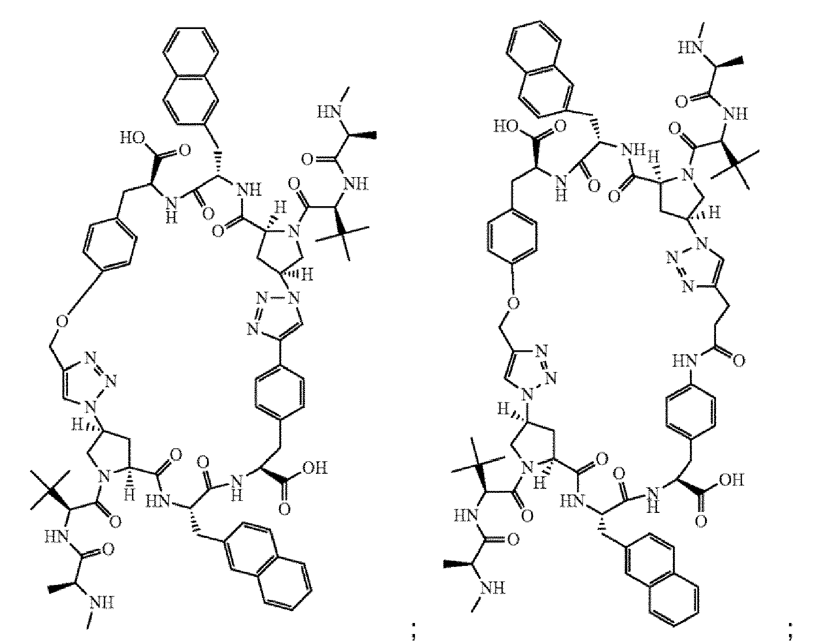

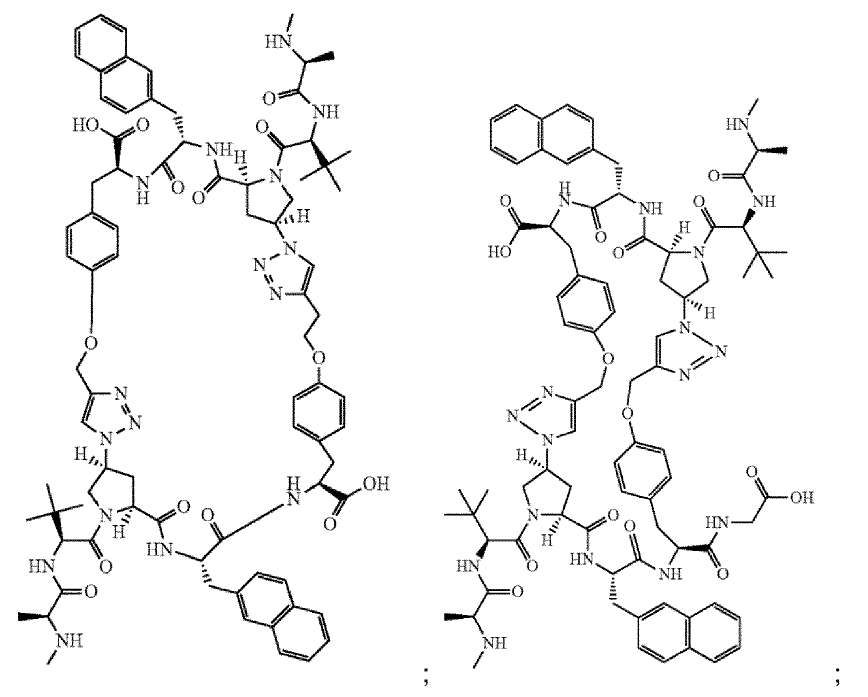

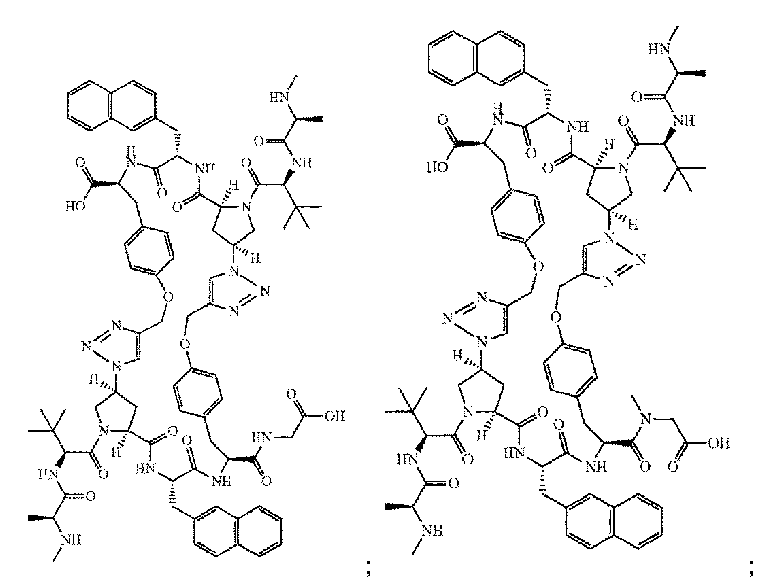

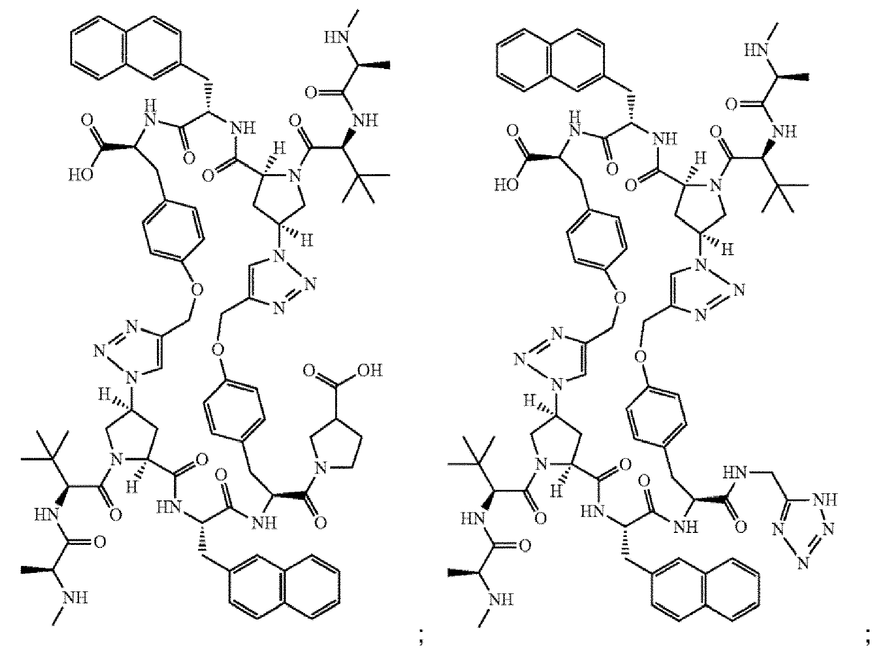

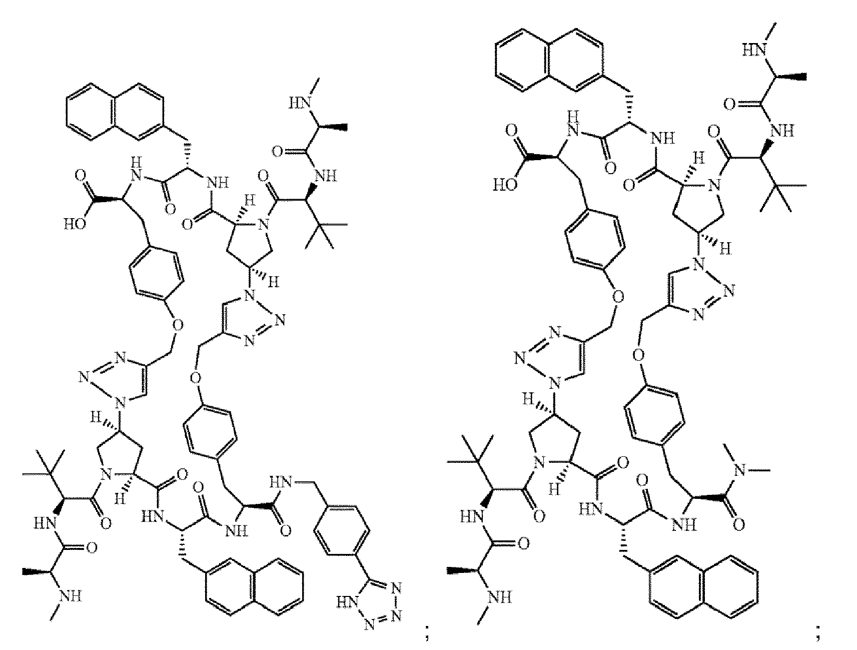

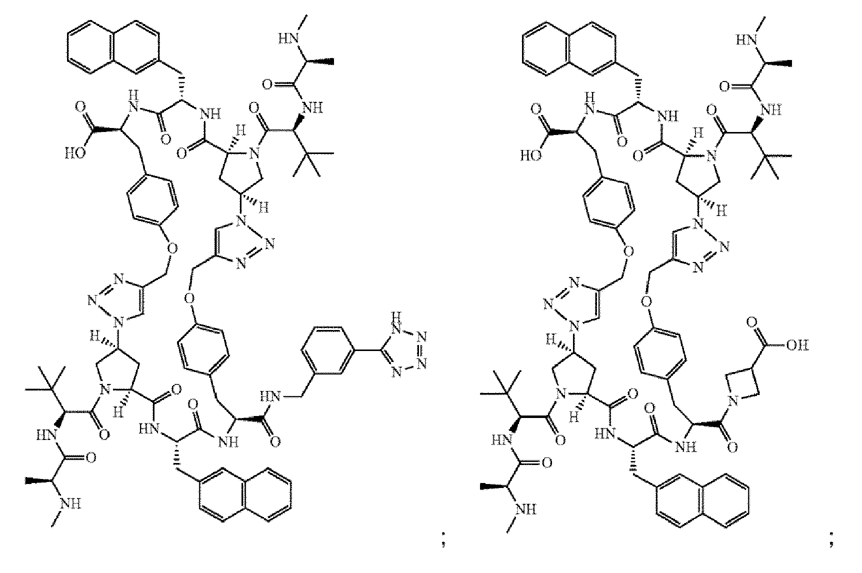

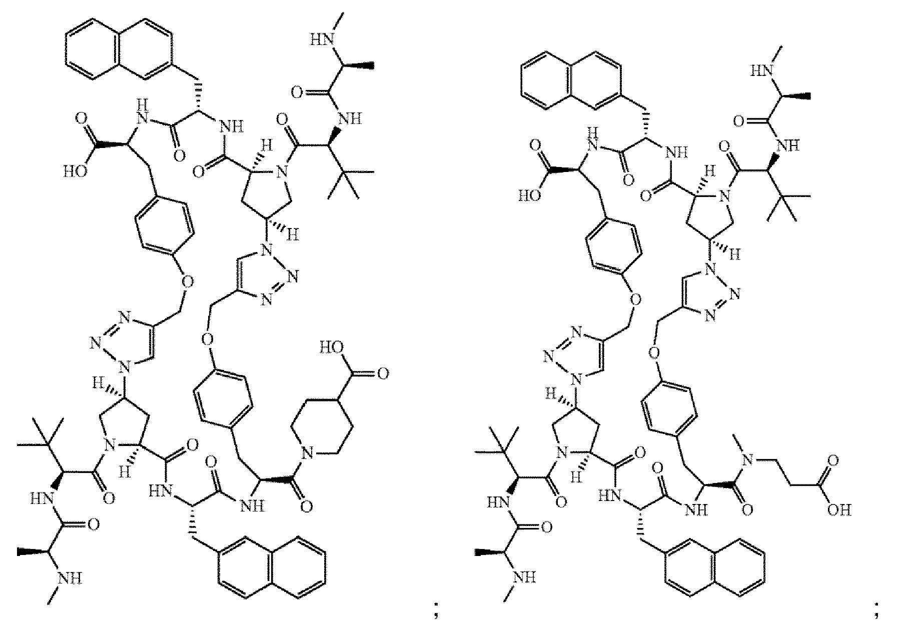

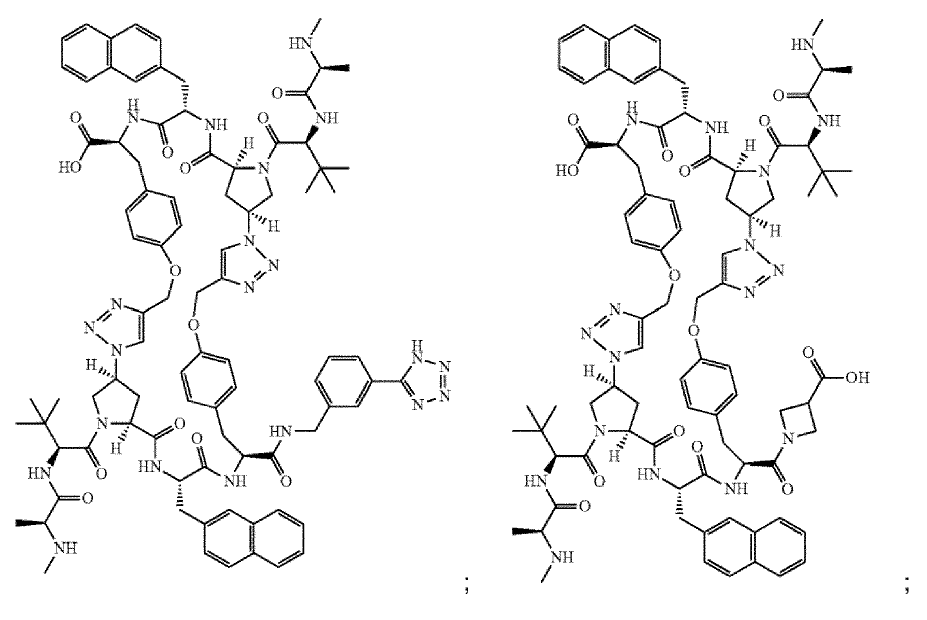

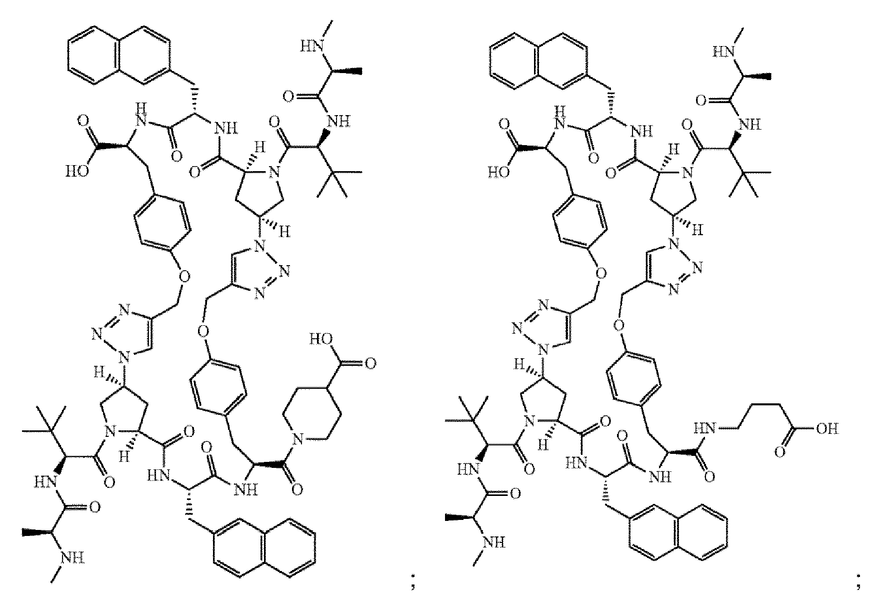

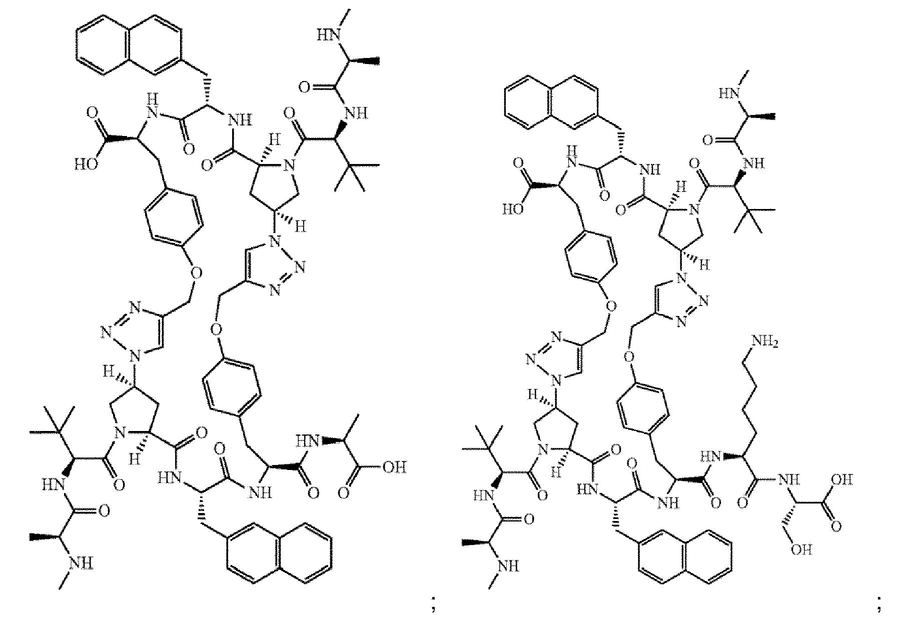

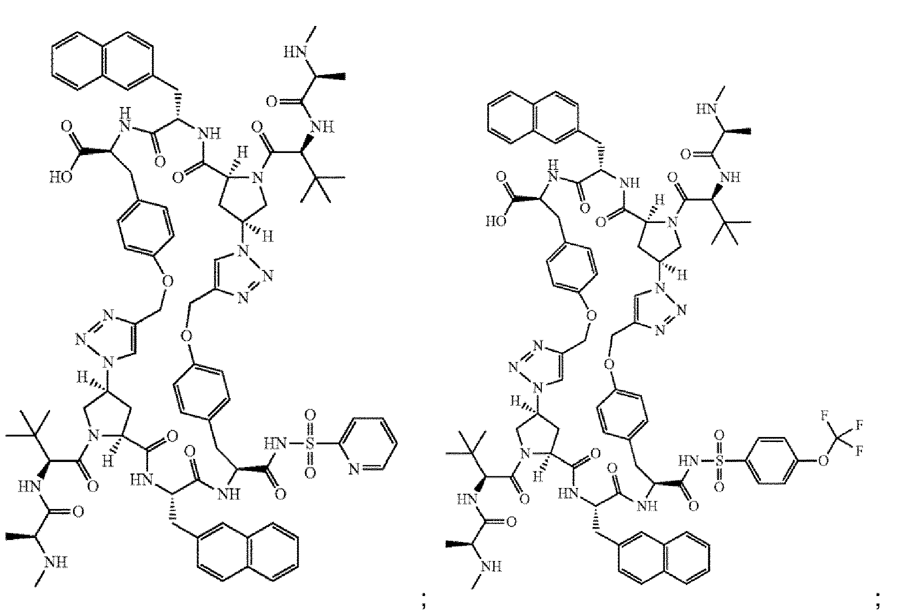

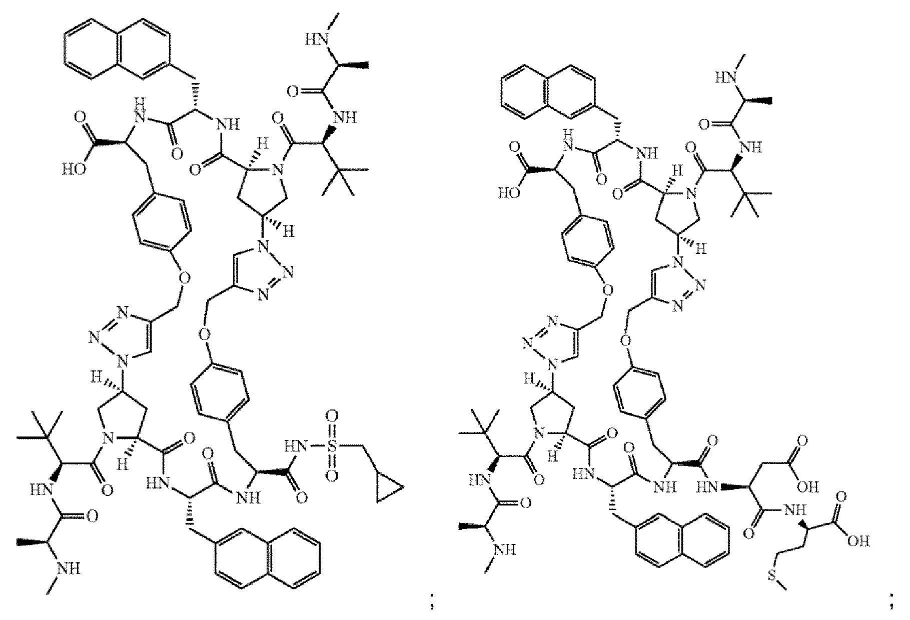

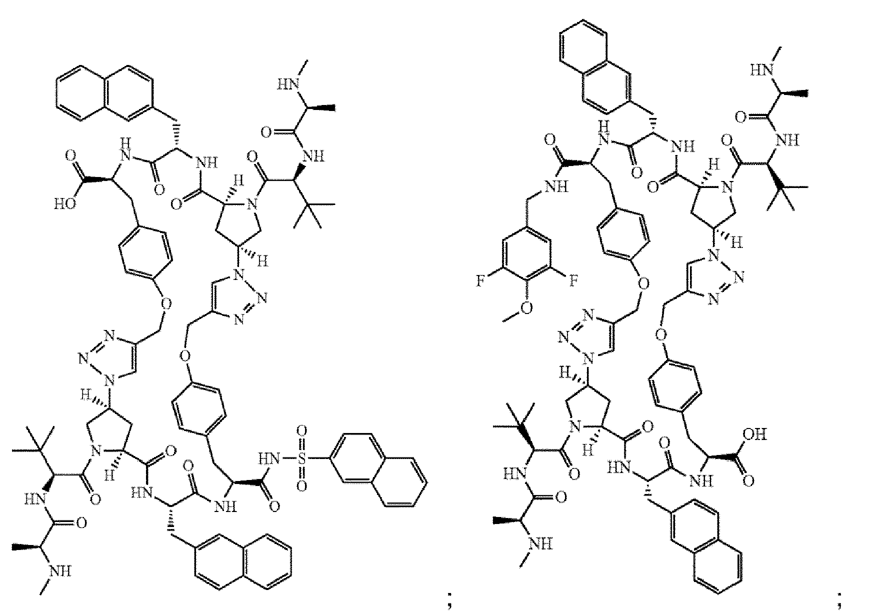

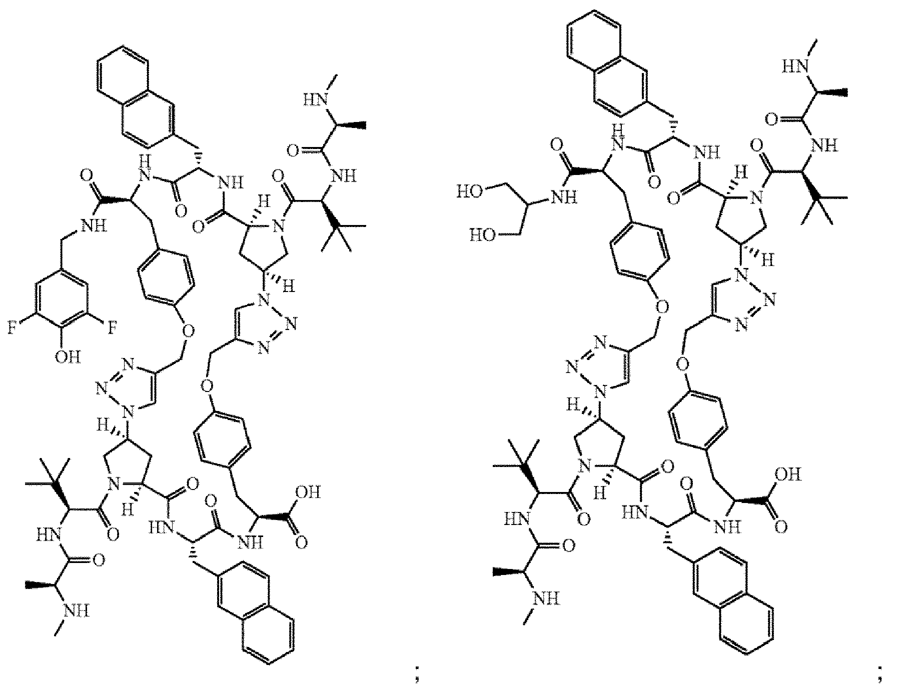

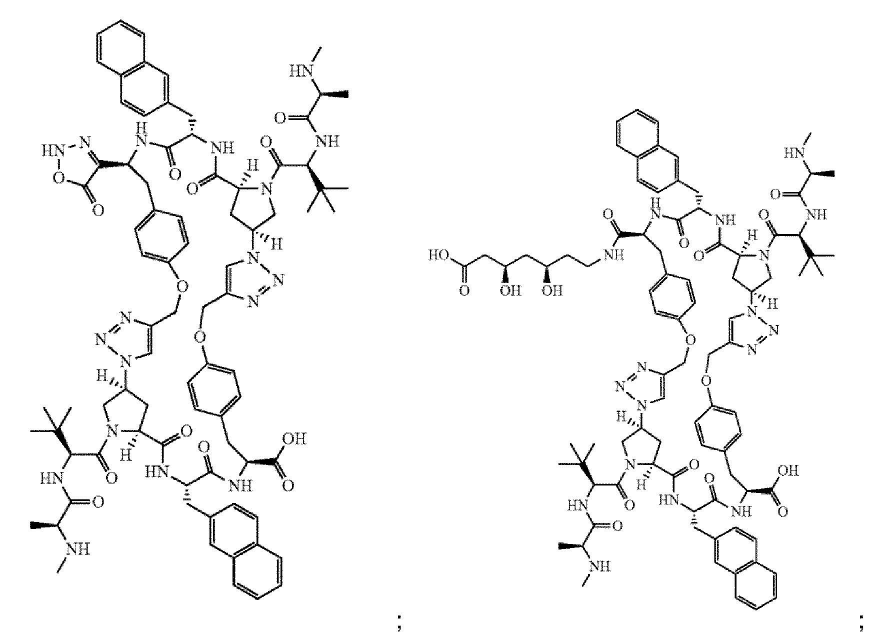

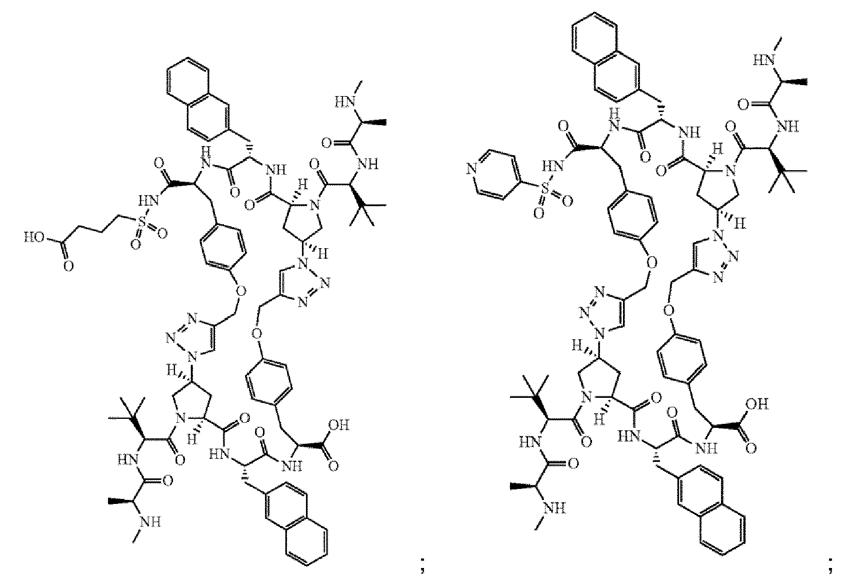

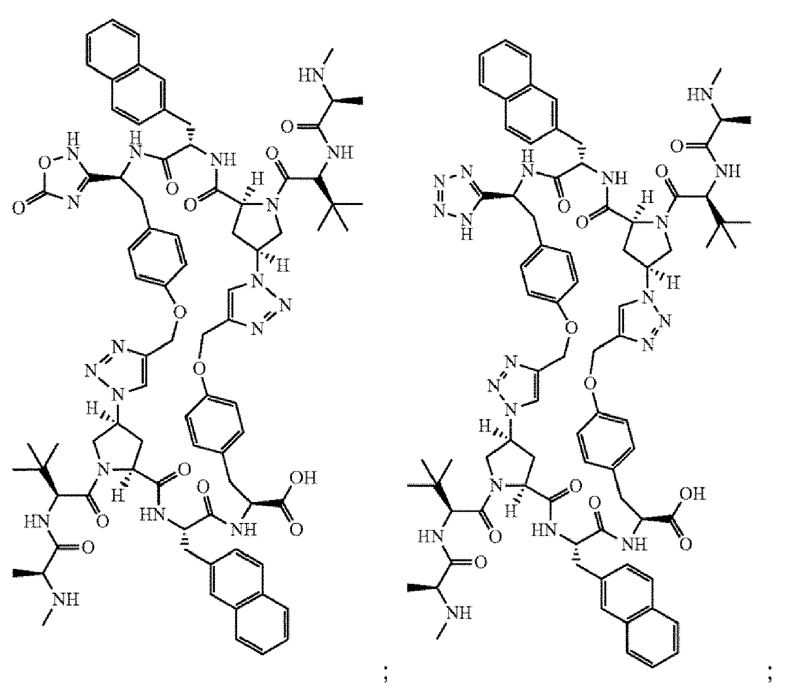

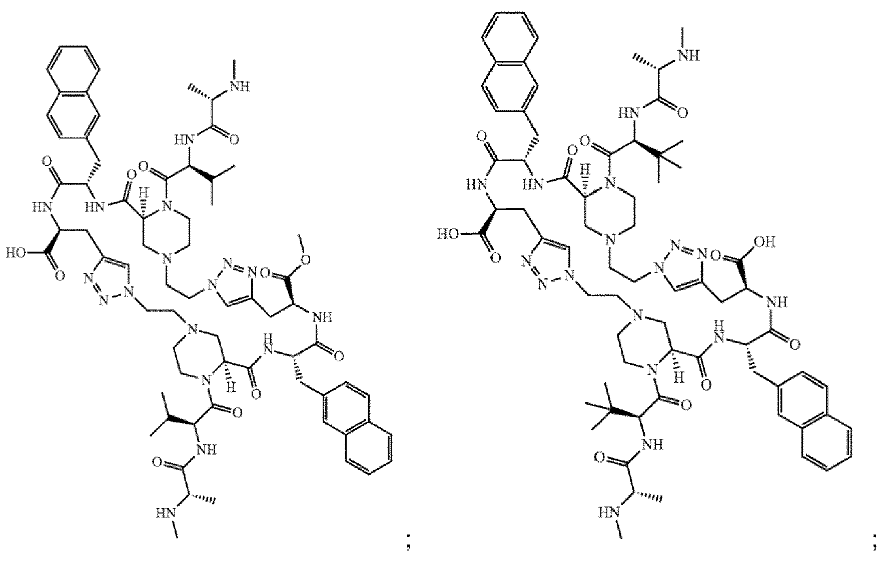

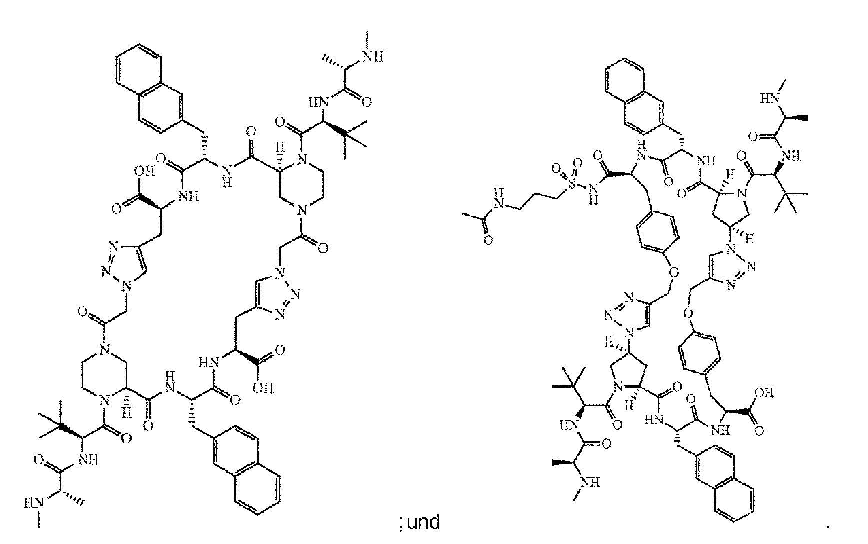

14. Verbindung nach einem der vorherigen Ansprüche zum Gebrauch als ein Medikament.

15. Pharmazeutisch unbedenkliche Zusammensetzung, umfassend eine Verbindung nach einem der Ansprüche 1 bis 13 und einen pharmazeutisch unbedenklichen Träger.

16. Verbindung nach einem der Ansprüche 1 bis 13 oder eine pharmazeutische Zusammensetzung nach Anspruch 15 zum Gebrauch beim Behandeln von Krebs.

## Revendications

1. Composé de Formule I : 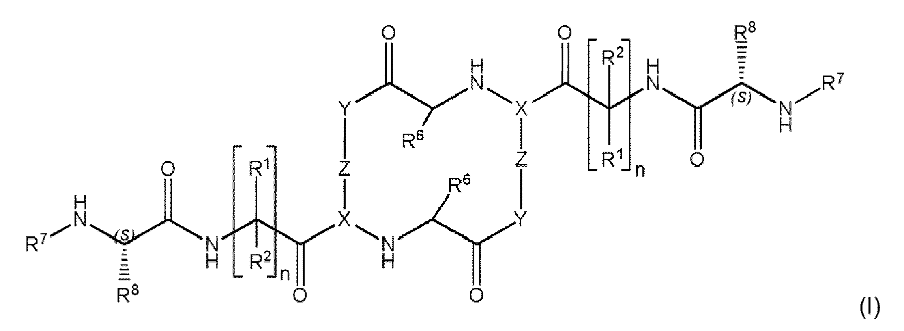 ou sel pharmaceutiquement acceptable de celui-ci, dans lequel :
chaque n vaut indépendamment 1 ou 2 ;
chaque R¹ est indépendamment sélectionné parmi un hydrogène, un cycloalkyle et un -(alkylène en C₁-C₄)-R⁴ chaque R⁴ étant indépendamment sélectionné parmi un hydrogène, un aryle et un cycloalkyle et au moins un R¹ ne représentant pas un hydrogène ; et
chaque R² représente un hydrogène ; ou
R¹ et R², avec l'atome de carbone auquel ils sont l'un et l'autre attachés, forment ensemble un cycloalkyle ;
chaque R⁶ représente indépendamment un -(alkylène en C₁-C₄)-R⁹, chaque R⁹ étant indépendamment sélectionné parmi un hydrogène, un aryle, un hétéroaryle et un cycloalkyle ; tout fragment aryle, hétéroaryle ou cycloalkyle de R⁶ étant facultativement substitué par jusqu'à deux substituants indépendamment sélectionnés parmi un halogéno, CF₃, OH, un alkoxy en C₁-C₄, un alcényloxy en C₁-C₄, un phényle, un phényloxy et un phénylméthyloxy ; et au moins un -CH₂- du fragment alkylène en C₁-C₄ de R⁶ étant facultativement remplacé par -O- ;
chaque R⁷ est indépendamment sélectionné parmi un hydrogène et un méthyle ;
chaque R⁹ est indépendamment sélectionné parmi un méthyle et un éthyle ;
chaque X est indépendamment sélectionné parmi : 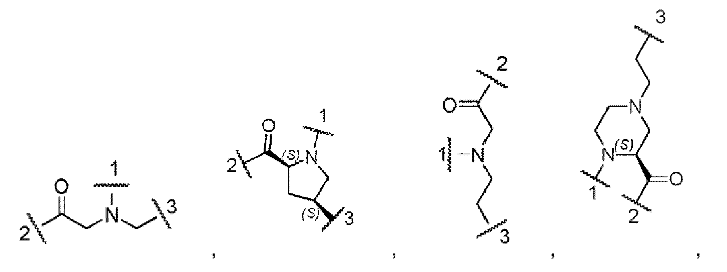 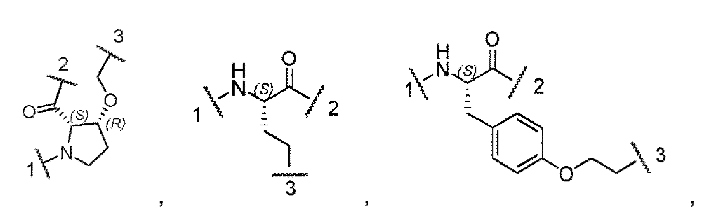 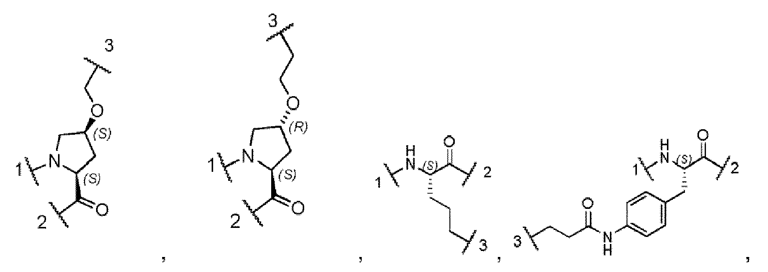 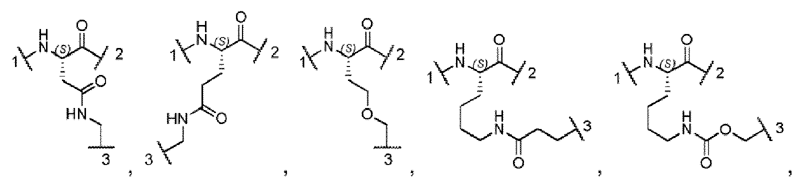 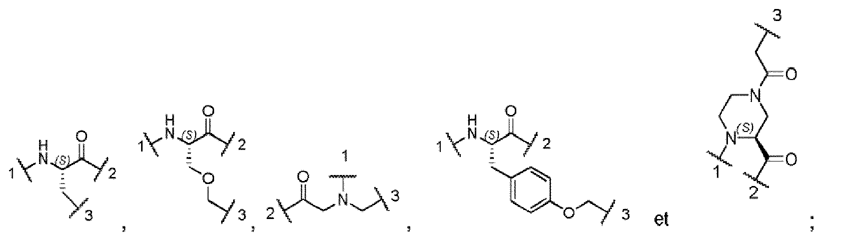
chaque Z représente 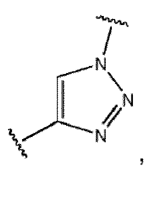 où chaque 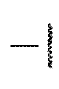 représente un point d'attache au composé; et
chaque Y est sélectionné parmi : 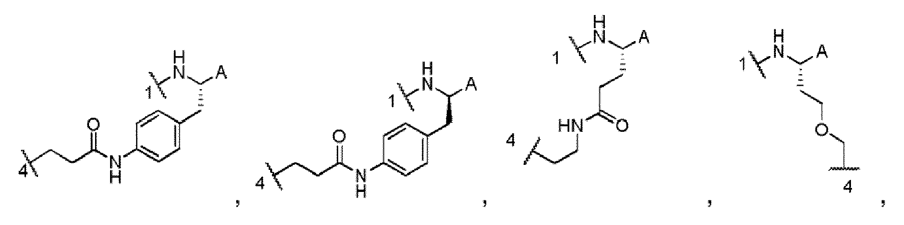 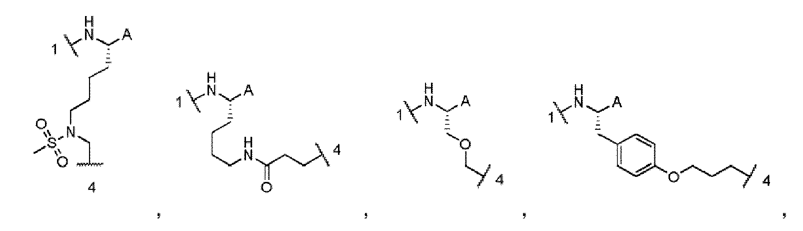 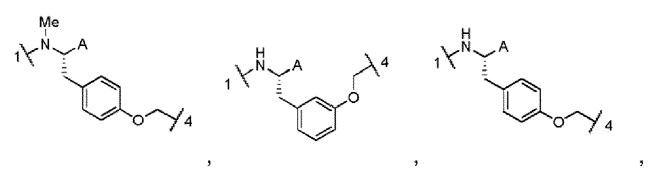 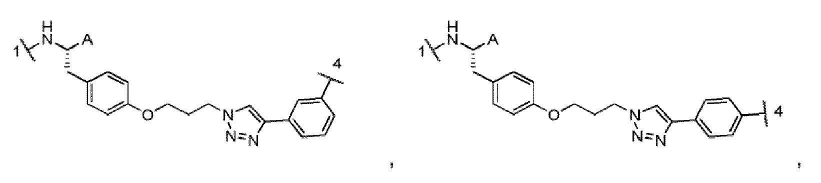 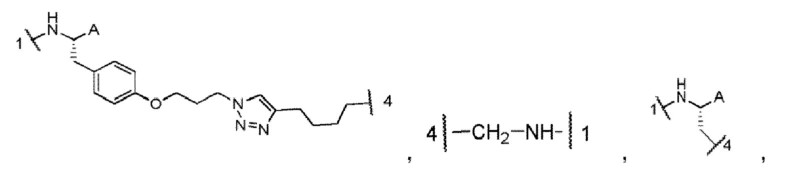 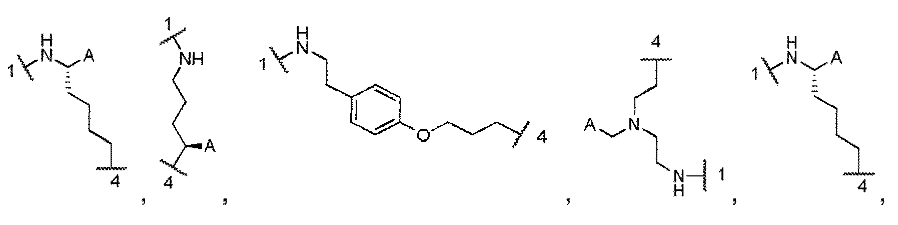 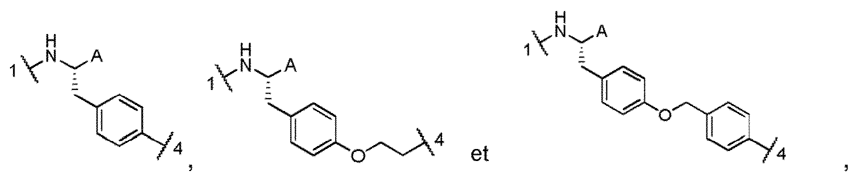 où : 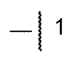
représente un point d'attache à un fragment-C=O du composé ; 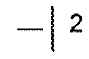
représente un point d'attache à un fragment-NH du composé ; 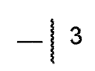
représente un premier point d'attache à Z ; 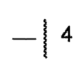
représente un second point d'attache à Z ; et
A est sélectionné parmi un -C(O)R³ ou 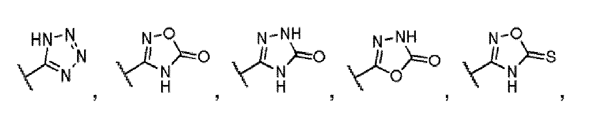 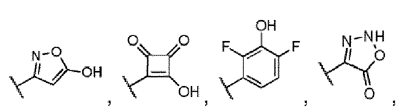 ou une forme tautomère de l'un quelconque de ceux-ci,
où :
R³ représente OH, -O-(alkyle en C₁-C₄), -NHCN, -NHSO₂R¹⁰, -NHOR¹¹ ou -NR¹²R¹³ ;
R¹⁰ et R¹¹ sont sélectionnés parmi un alkyle en C₁-C₄, un cycloalkyle, un aryle, un hétéroaryle ou un hétérocycloalkyle, l'un quelconque de ces groupements étant facultativement substitué, et un hydrogène ;
chacun des radicaux R¹² et R¹³ est indépendamment sélectionné parmi un hydrogène, un alkyle en C₁-C₄, un -(alkylène en C₁-C₄)-NH-(alkyle en C₁-C₄), un -(alkylène en C₁-C₄)-O-(alkyle en C₁-C₄) et un -(alkylène en C₁-C₄)-O-(hydroxyalkyle en C₁-C₄), ou R¹² et R¹³, avec l'atome d'azote auquel ils sont l'un et l'autre attachés, forment ensemble un hétérocyclyle saturé comprenant facultativement un hétéroatome supplémentaire sélectionné parmi N, O et S, l'hétérocyclyle saturé étant facultativement substitué par un méthyle.

2. Composé selon la revendication 1, dans lequel R⁷ représente un méthyle et R⁸ représente un méthyle.

3. Composé selon la revendication 1, dans lequel un fragment du composé représenté par -NH-[C(R¹)(R²)ₙ-C(O)- est sélectionné parmi : 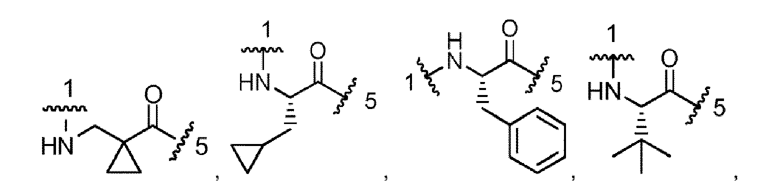 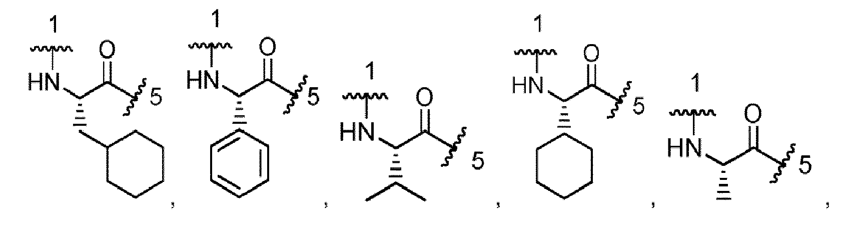 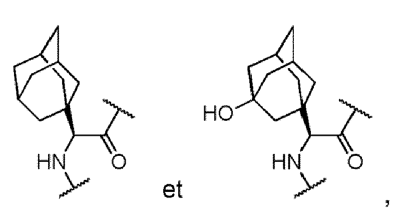 où : 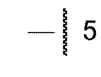
représente un point d'attache au fragment amino de X.

4. Composé selon la revendication 3, dans lequel un fragment du composé représenté: par -NH-[C(R¹)(R²)]ₙ-C(O)- est

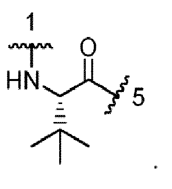

5. Composé selon la revendication 1, dans lequel un fragment du composé représenté par -NH-CH(R⁶)-C(O)- est sélectionné parmi : 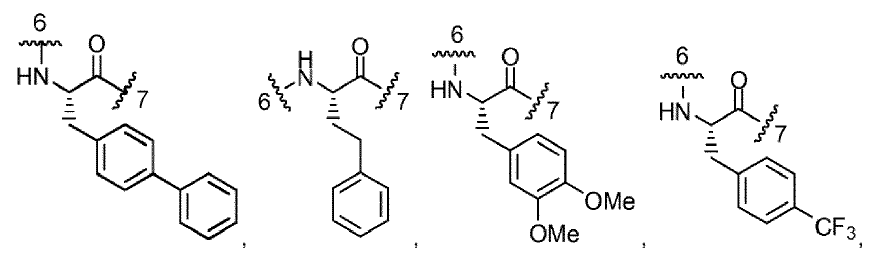 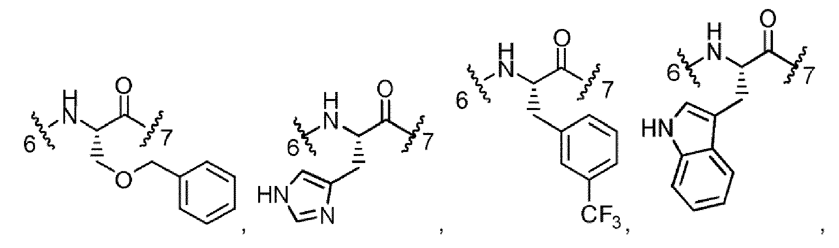 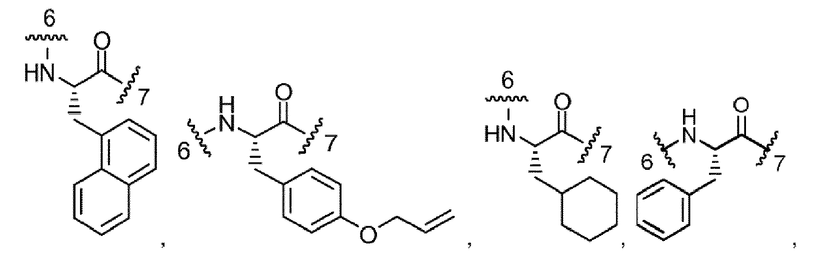 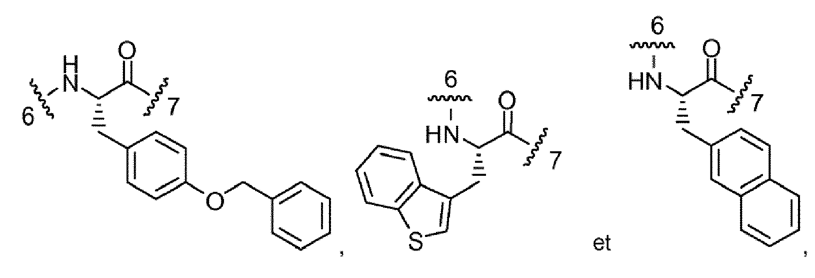 où : 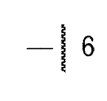
représente un point d'attache au fragment-C=O de X ; et 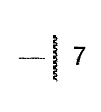
représente un point d'attache au fragment amino de Y.

6. Composé selon la revendication 5, dans lequel un fragment du composé représenté par -NH-CH(R⁶)-C(O)- est

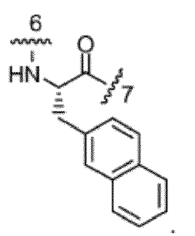

7. Composé selon la revendication 1, dans lequel X est sélectionné parmi :

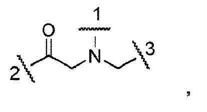

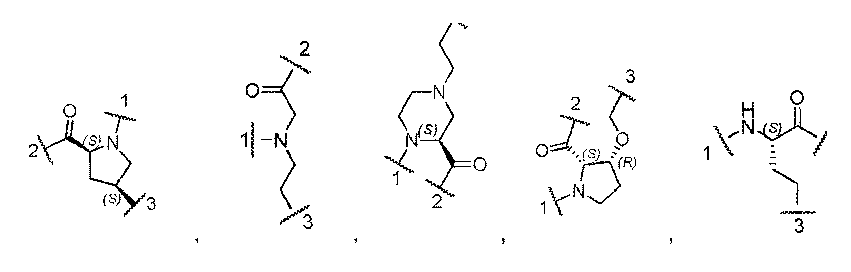

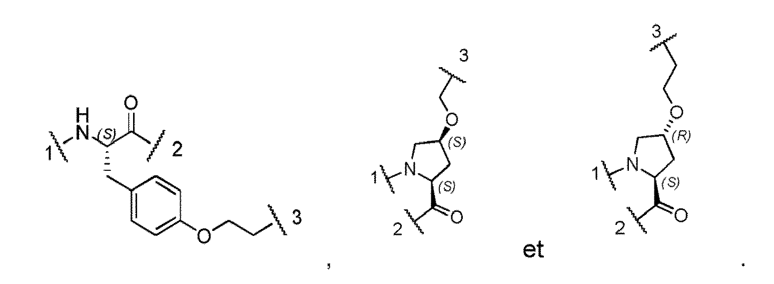

8. Composé selon la revendication 7, dans lequel X est sélectionné parmi :

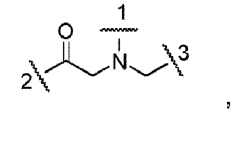

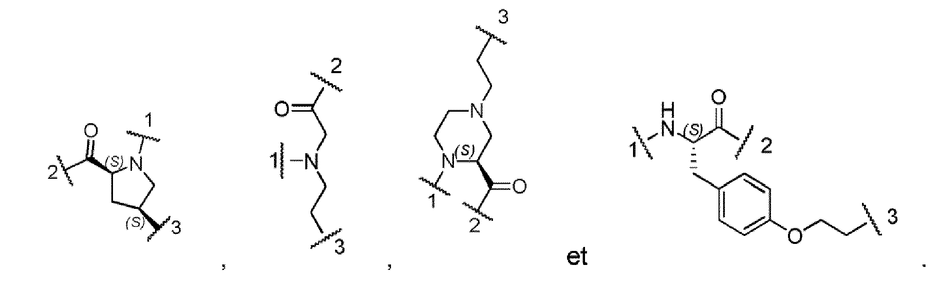

9. Composé selon la revendication 8, dans lequel X représente

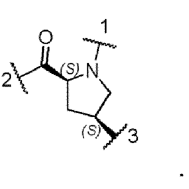

10. Composé selon la revendication 1, dans lequel Y est sélectionné parmi

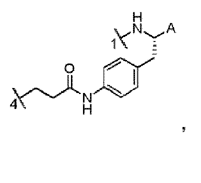

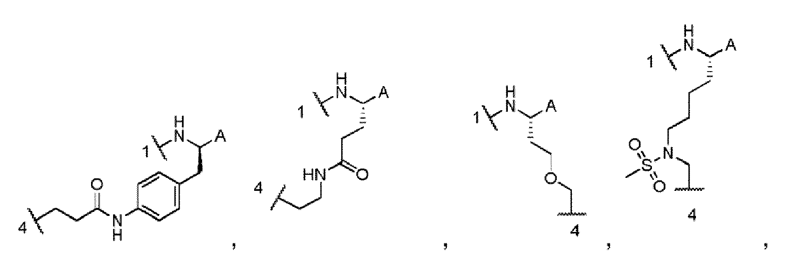

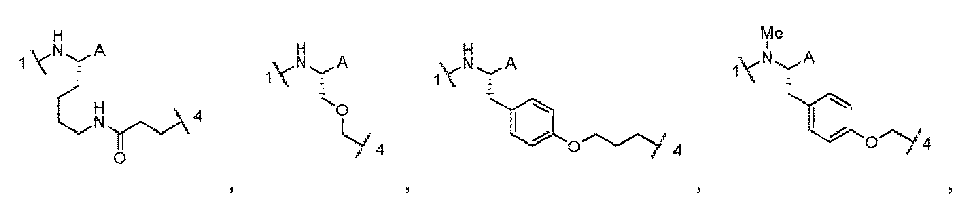

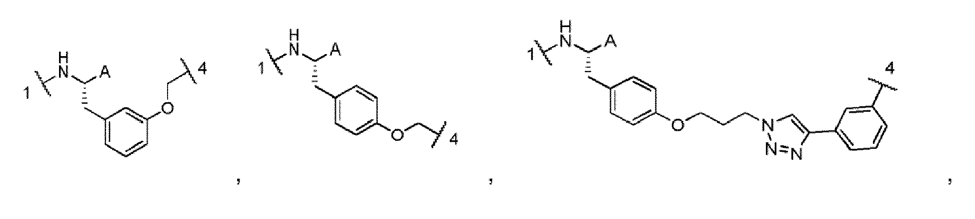

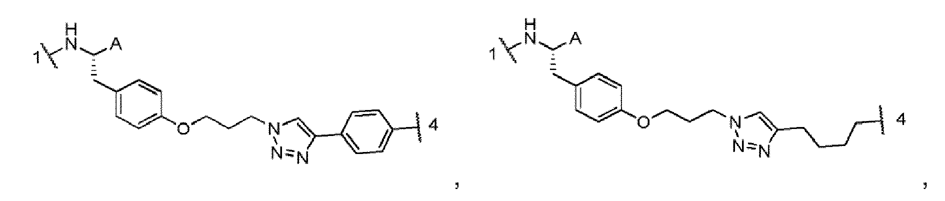

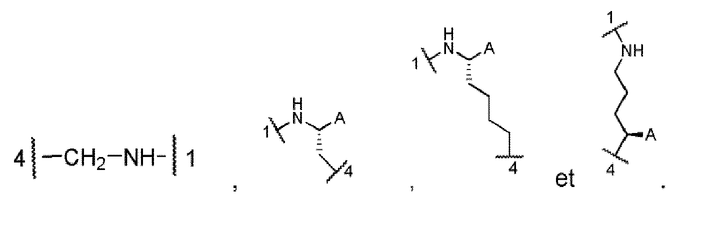

11. Composé selon la revendication 10, dans lequel Y représente

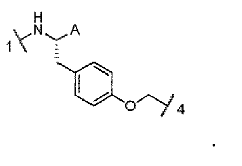

12. Composé selon la revendication 1 dans lequel :
chaque R¹ représente le même radical ;
chaque R² représente le même radical ;
chaque R⁶ représente le même radical ;
chaque R⁷ représente le même radical ;
chaque R⁸ représente le même radical ;
chaque X représente le même radical ;
chaque Y représente le même radical ;
chaque Z représente le même radical ; et
chaque n a la même valeur.

13. Composé selon la revendication 1, qui est sélectionné dans le groupe consistant en les suivants :

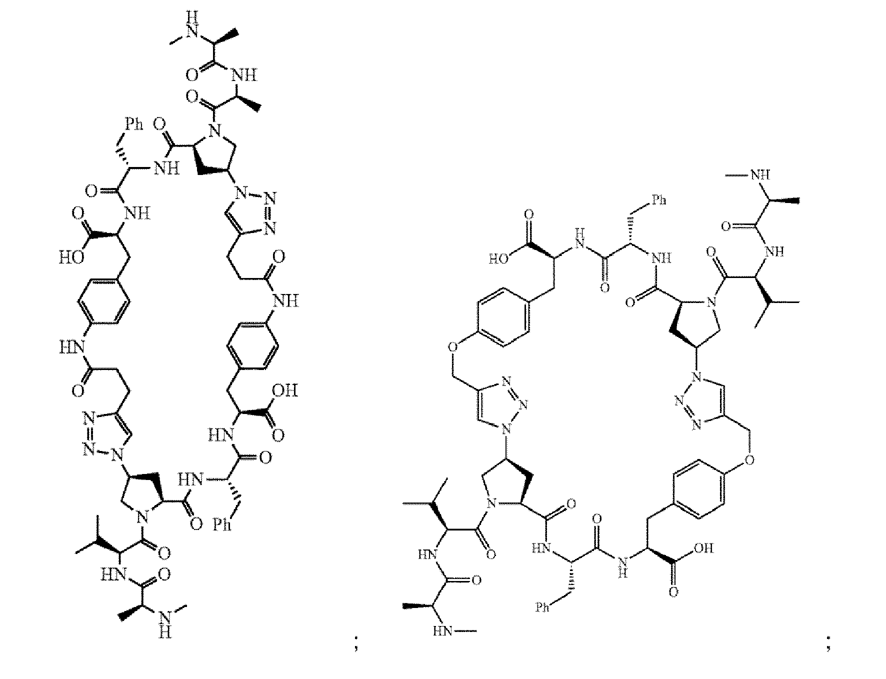

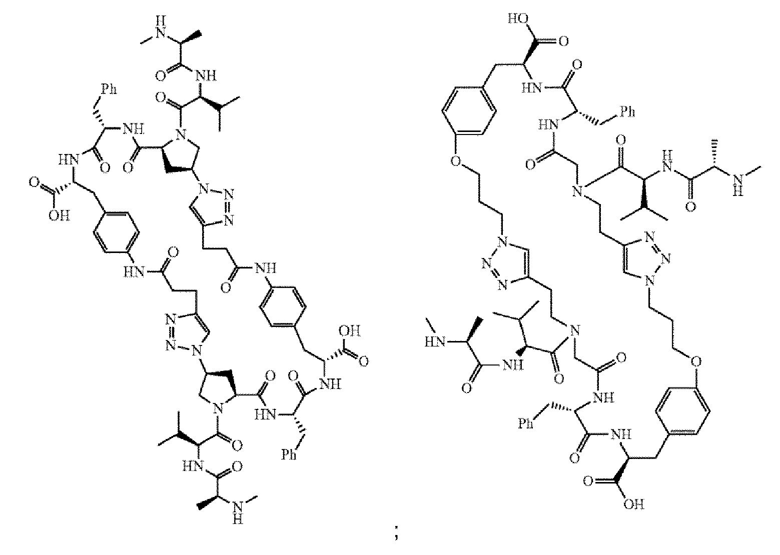

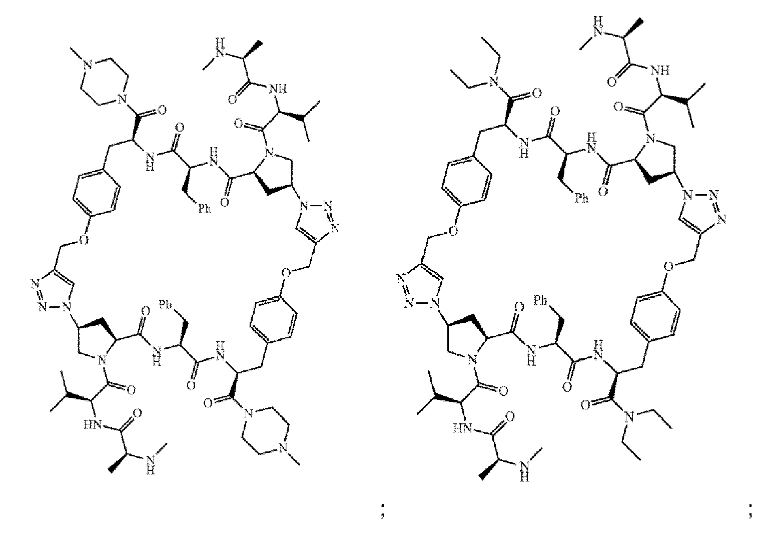

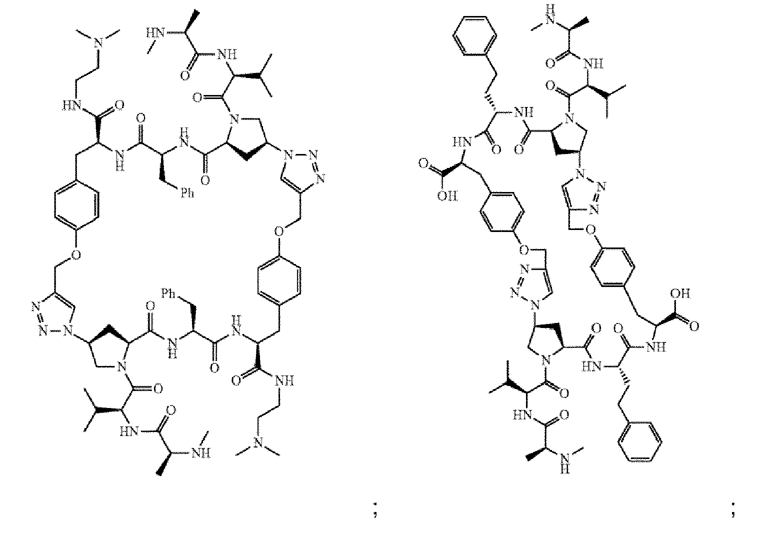

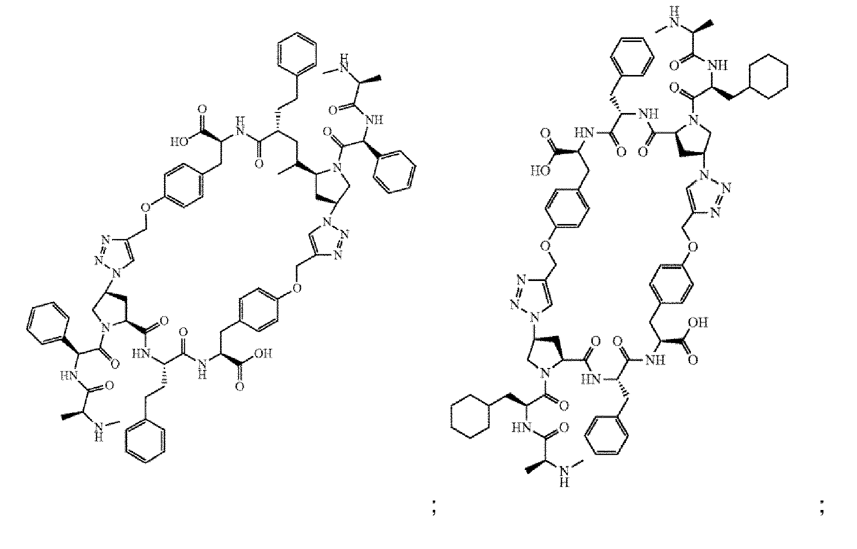

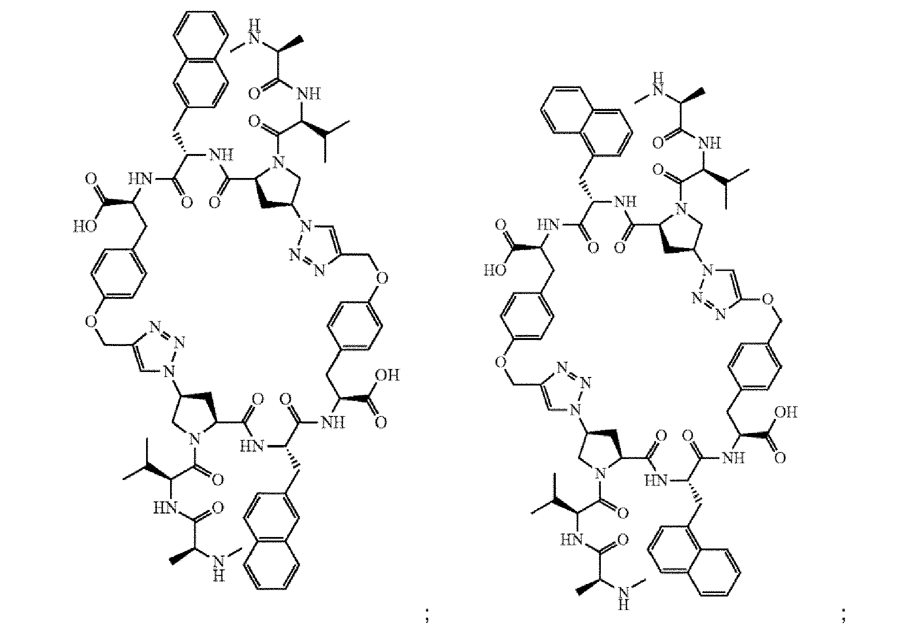

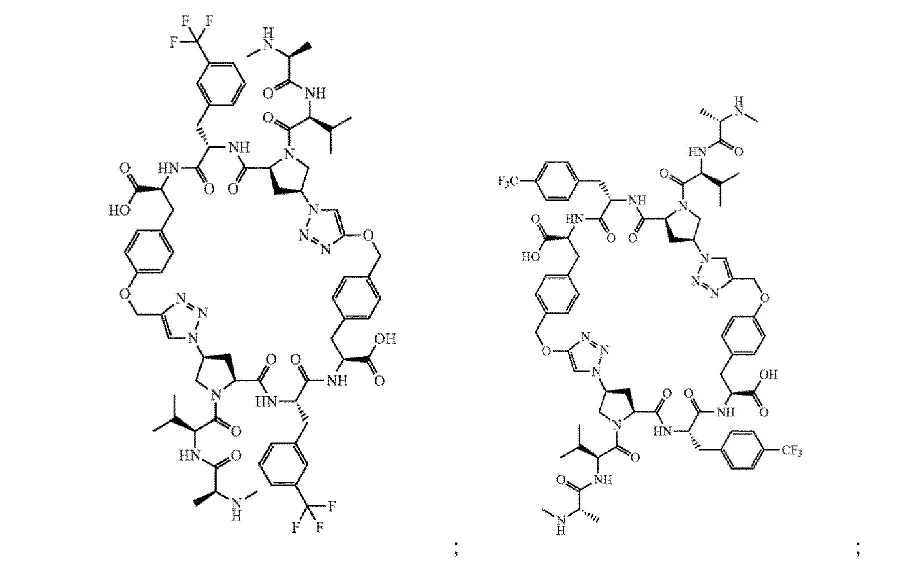

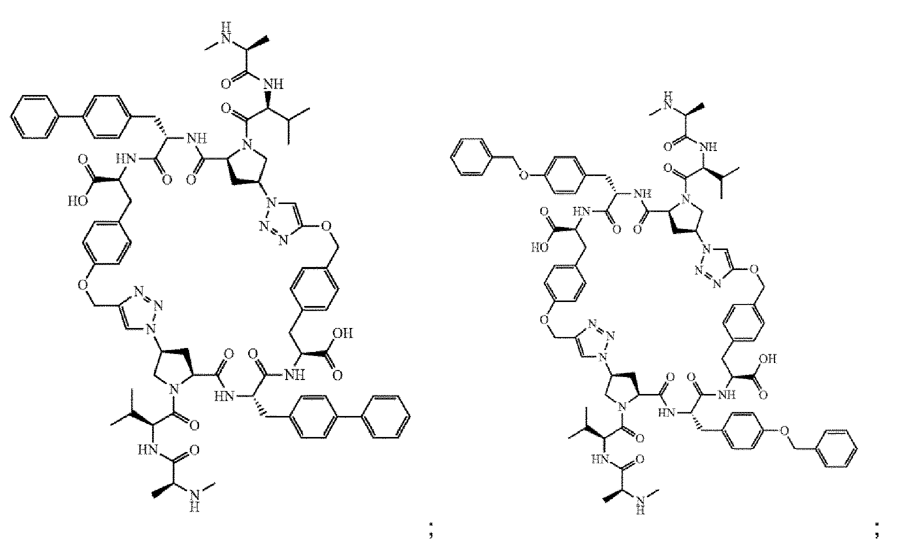

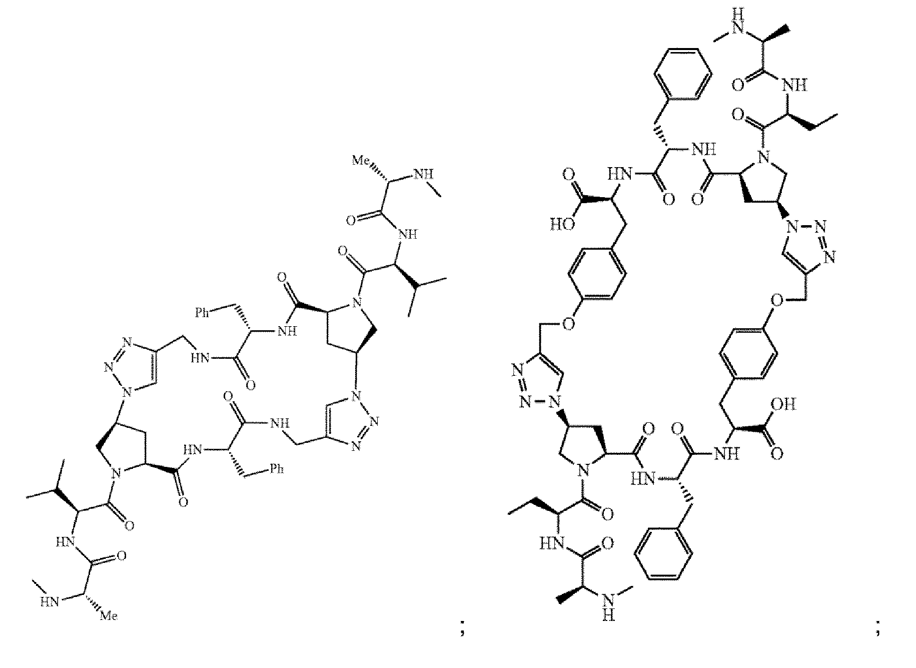

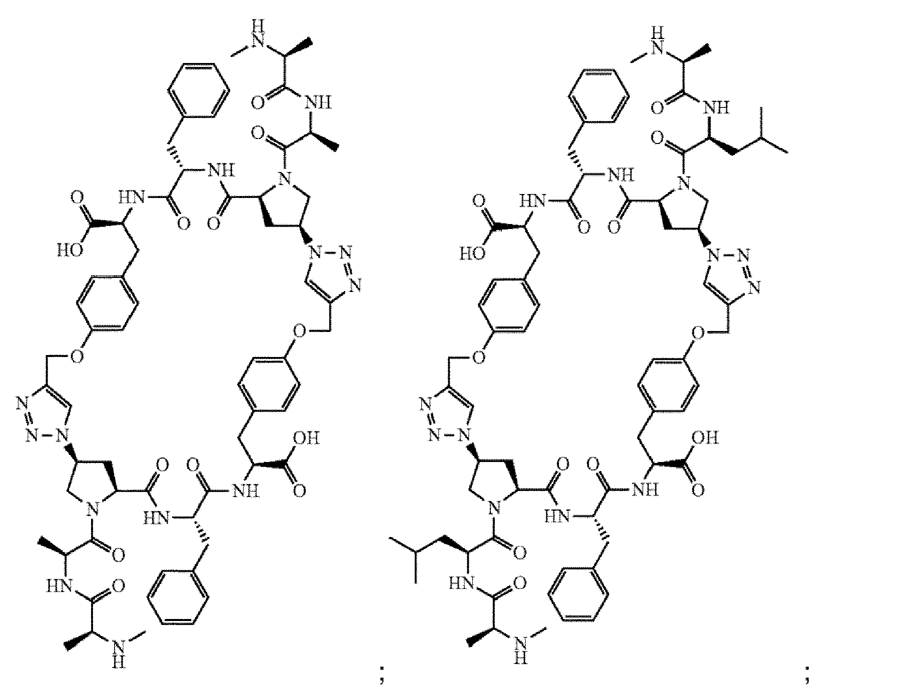

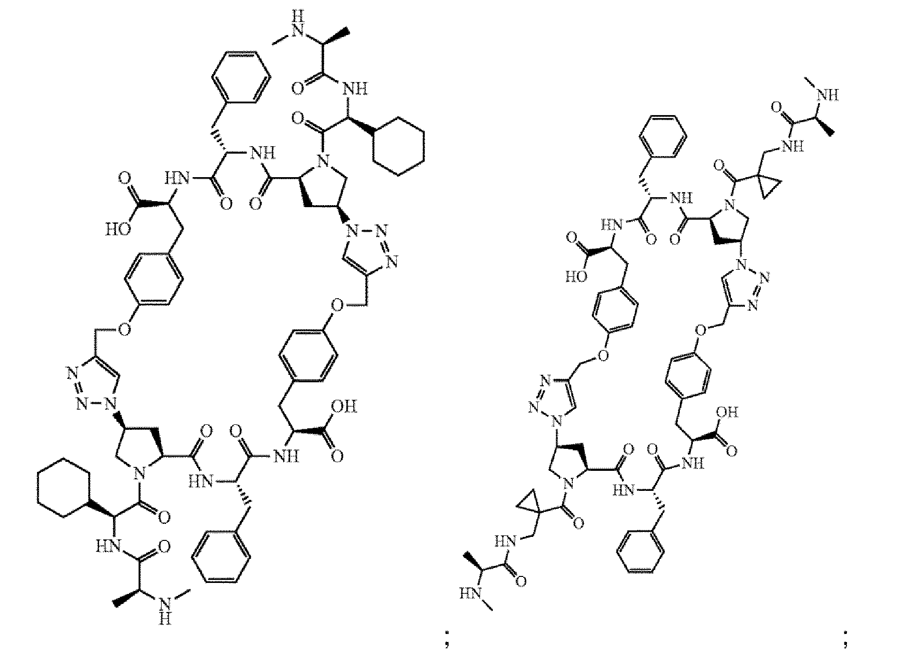

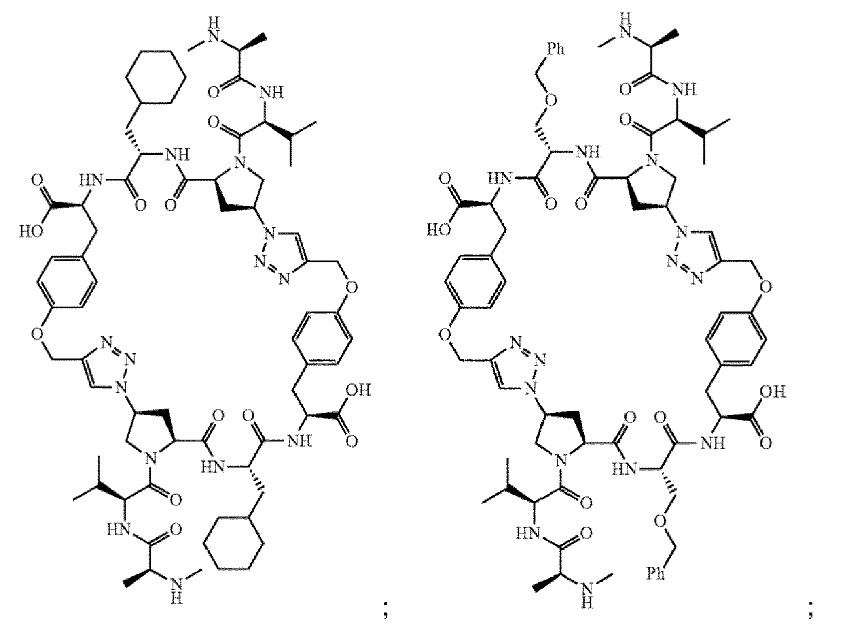

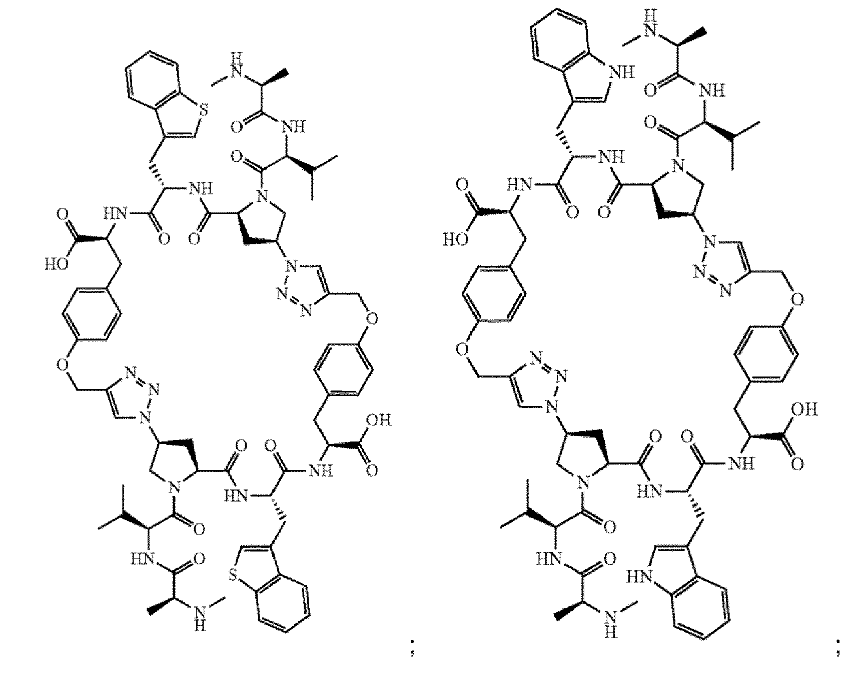

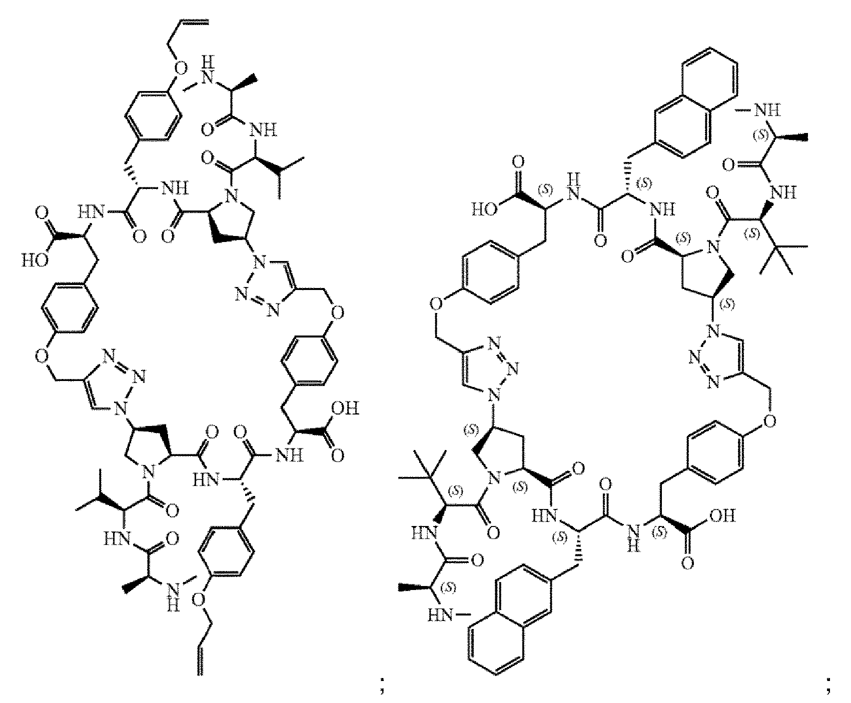

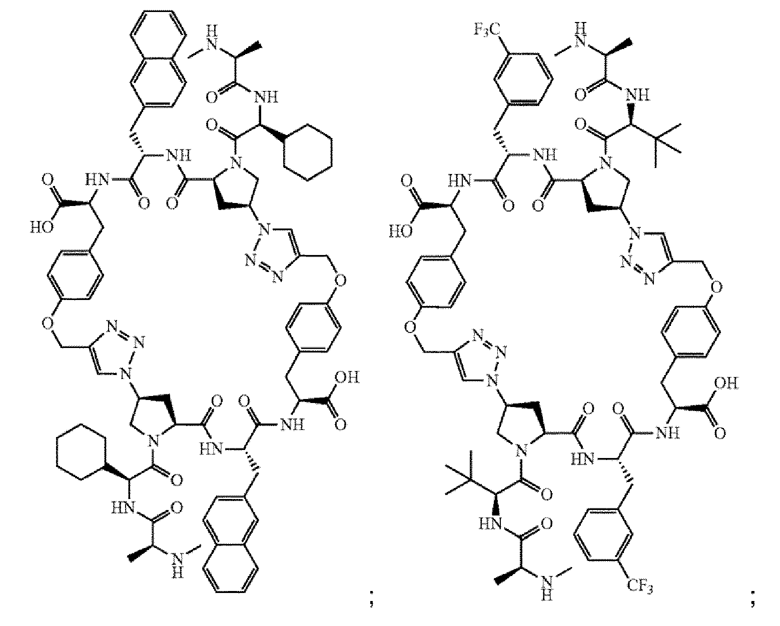

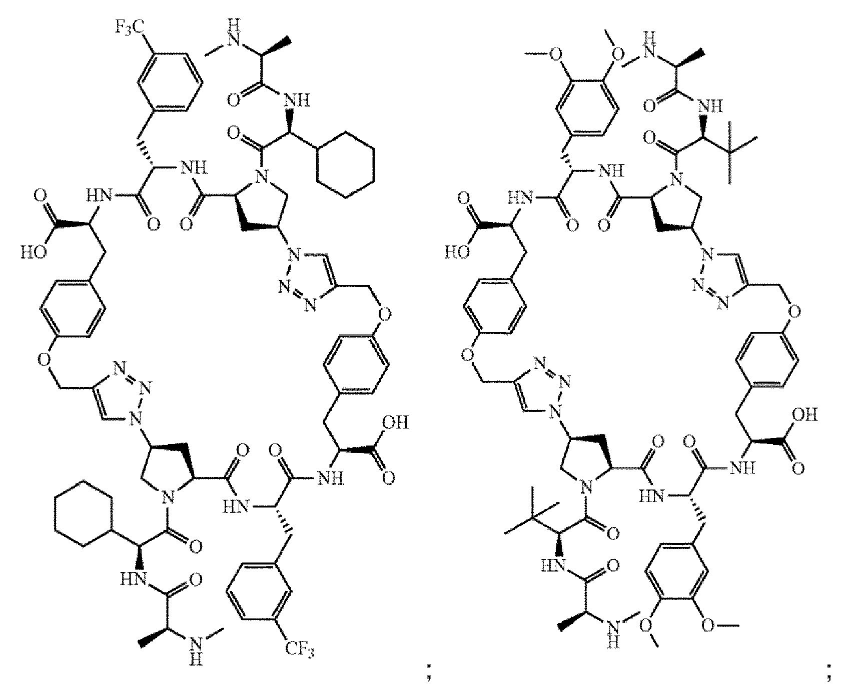

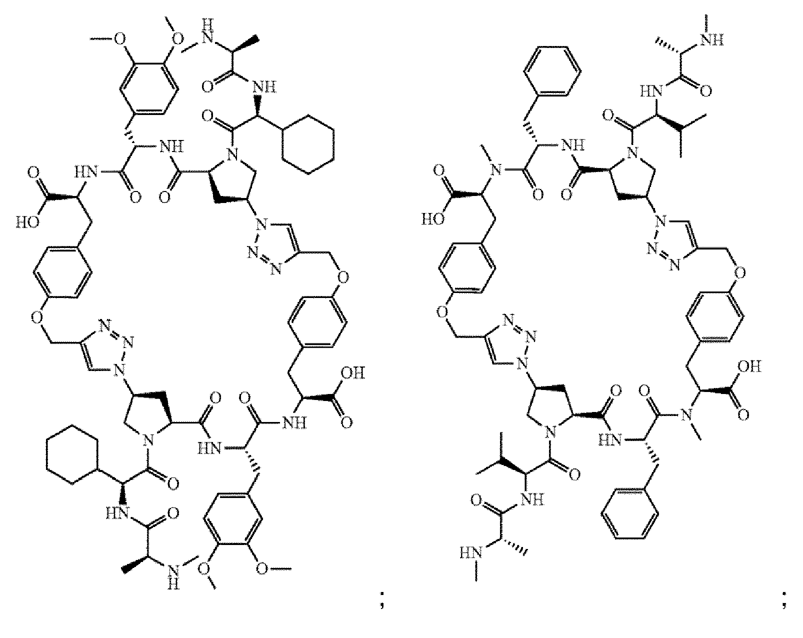

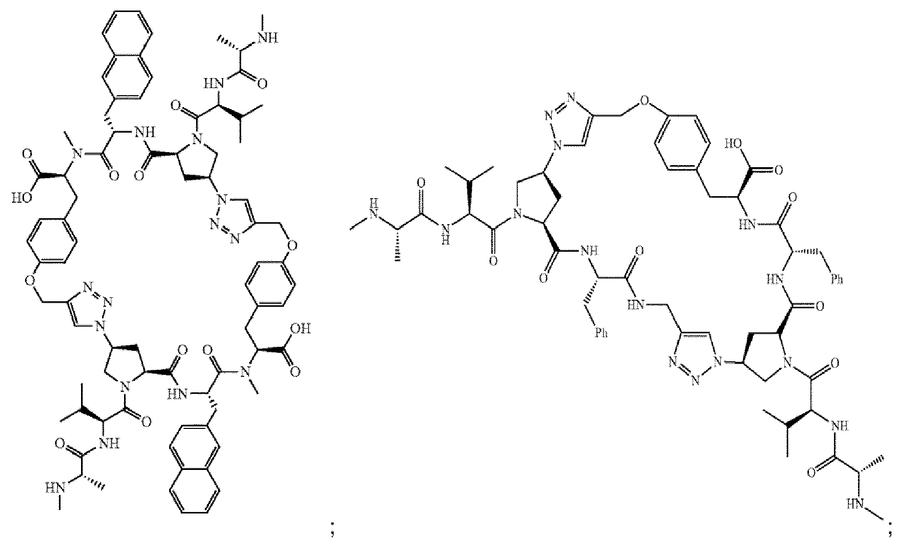

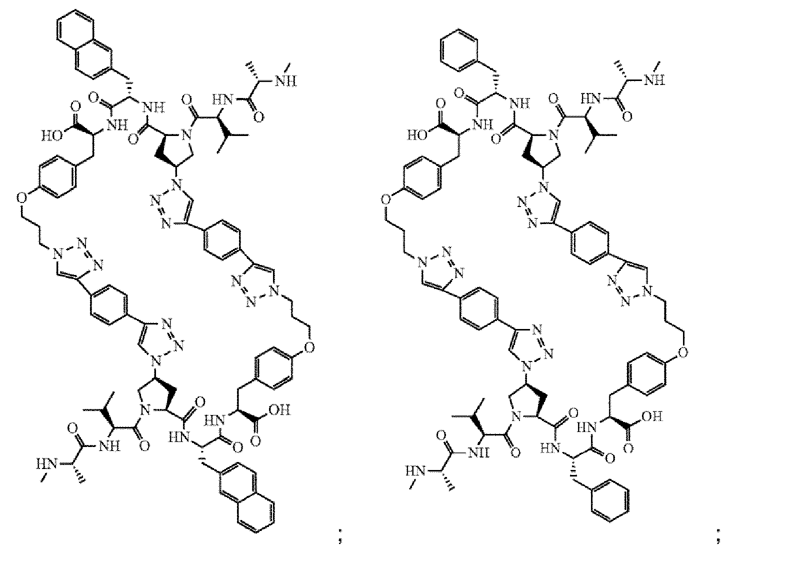

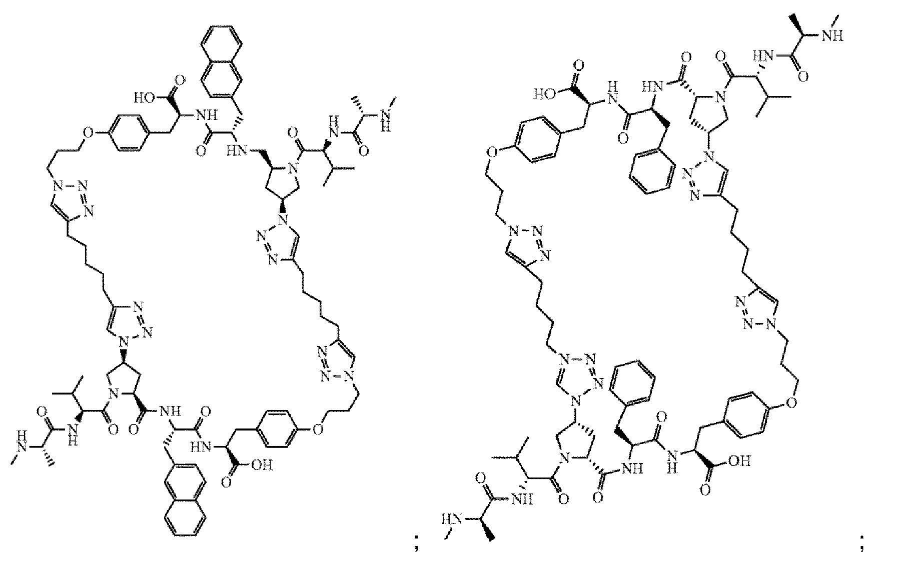

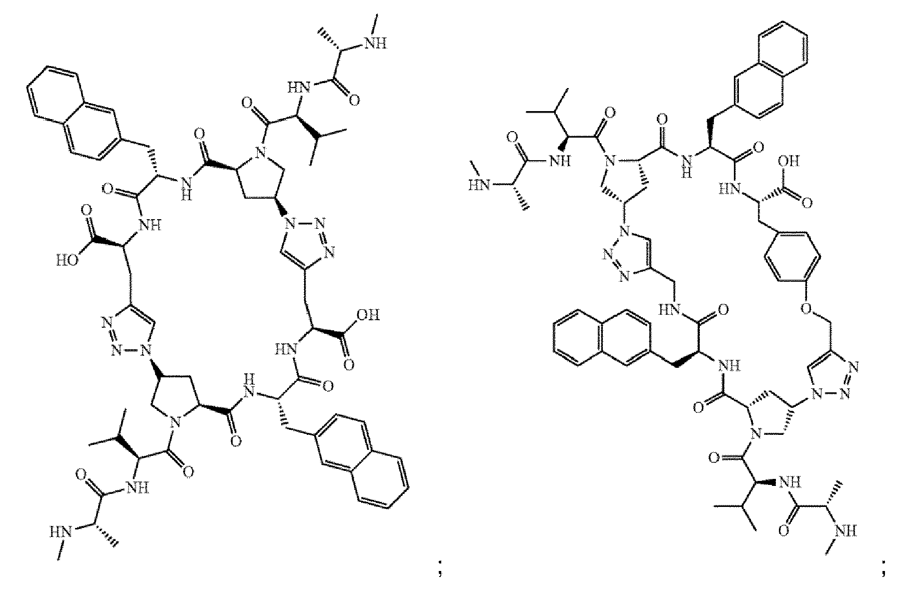

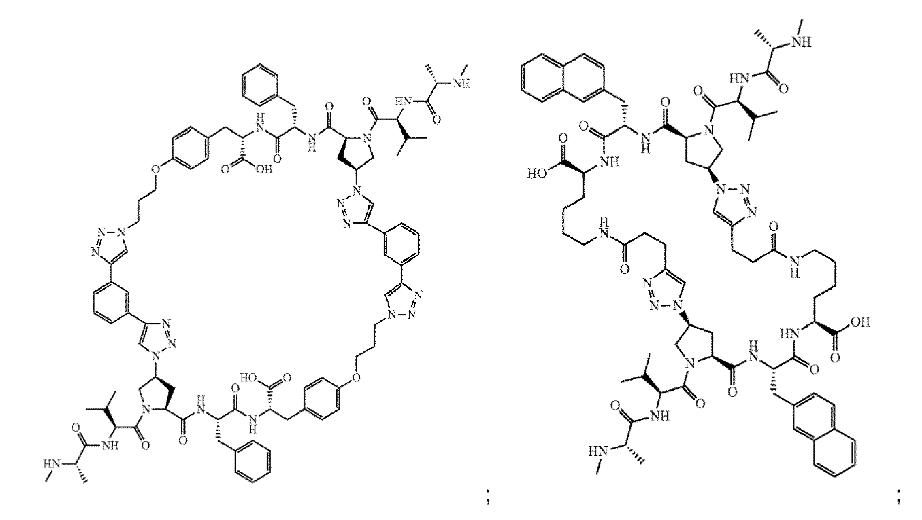

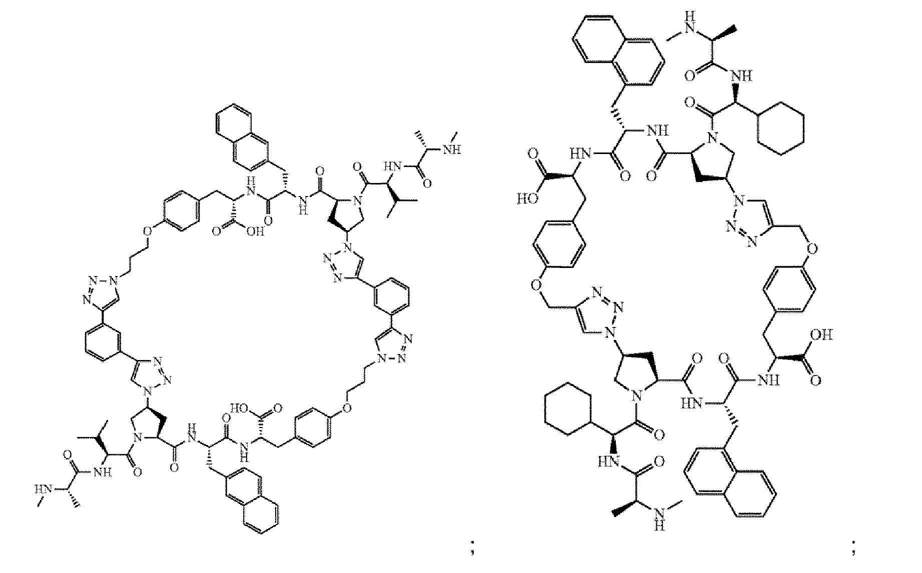

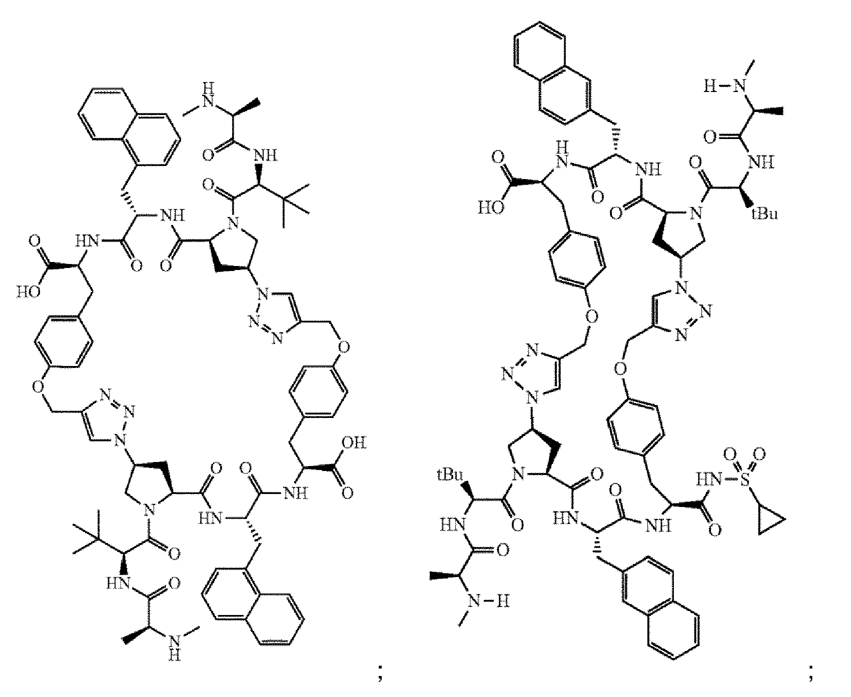

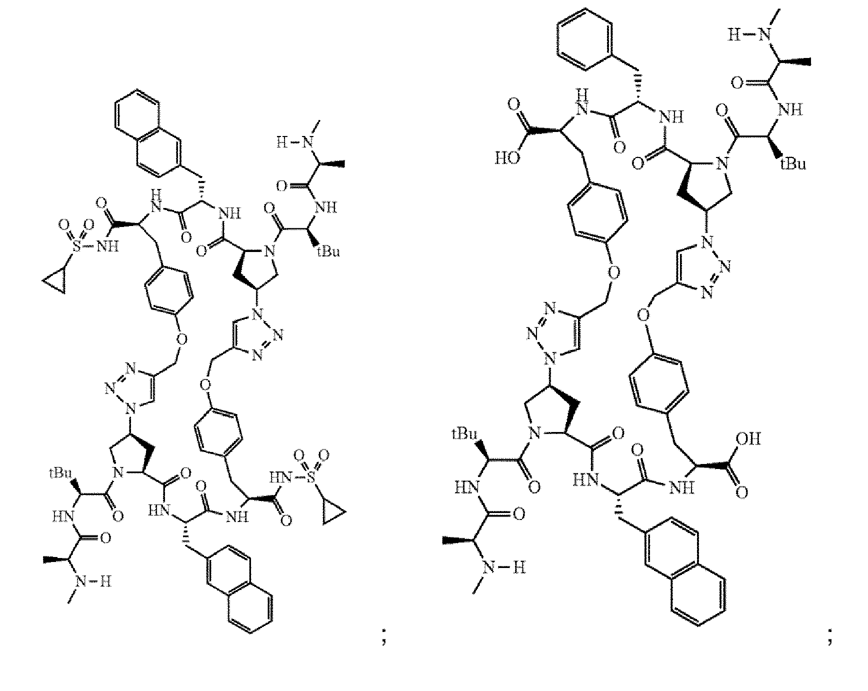

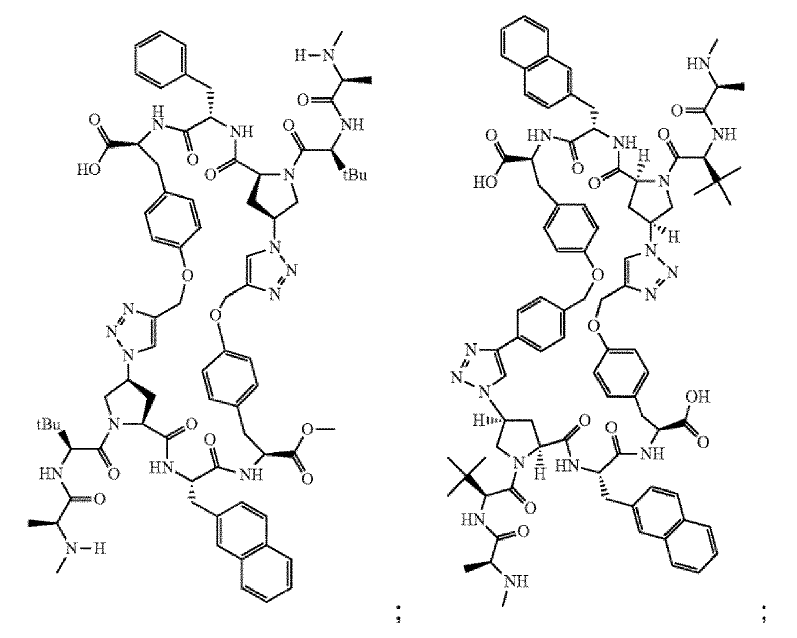

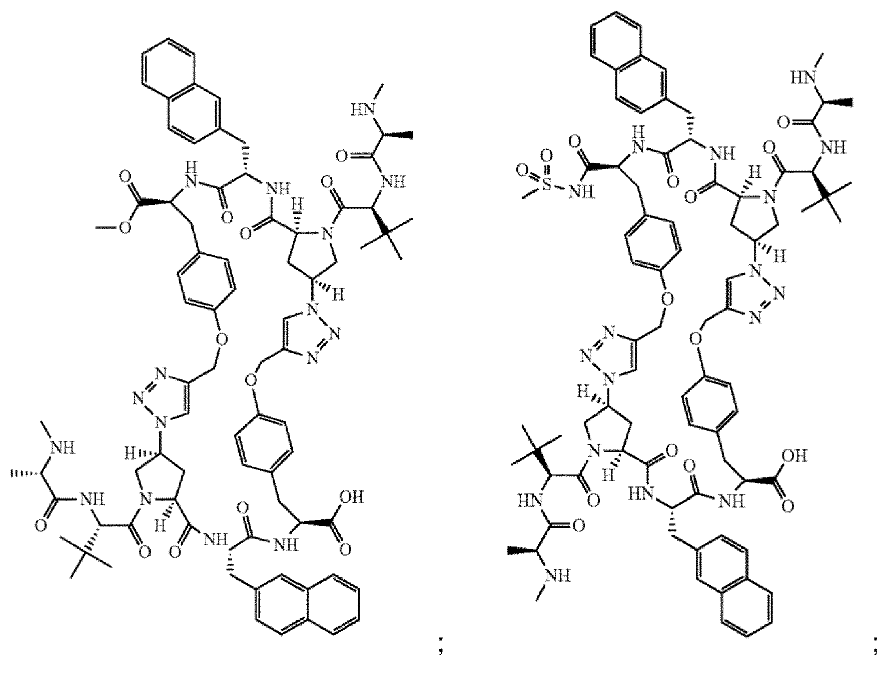

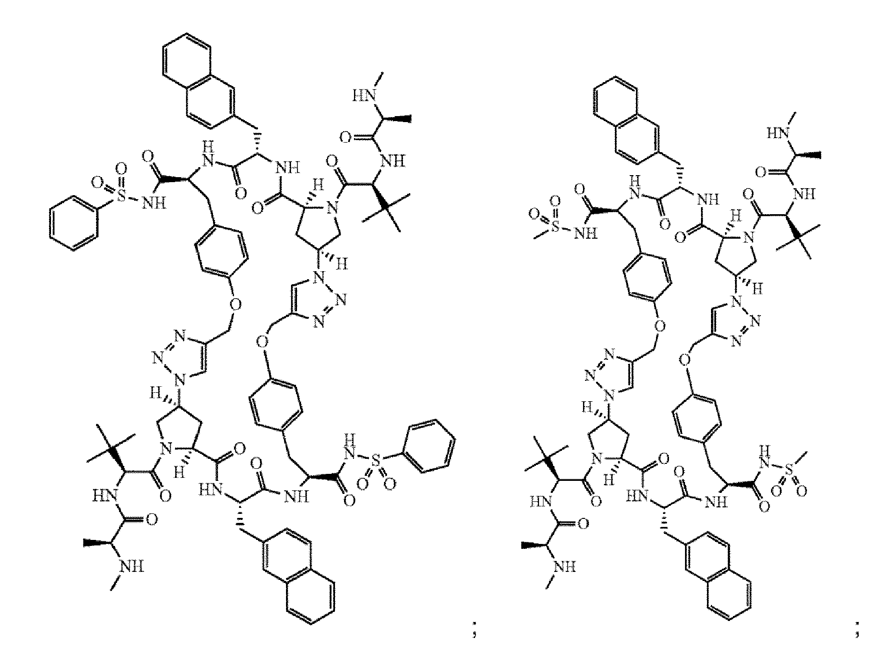

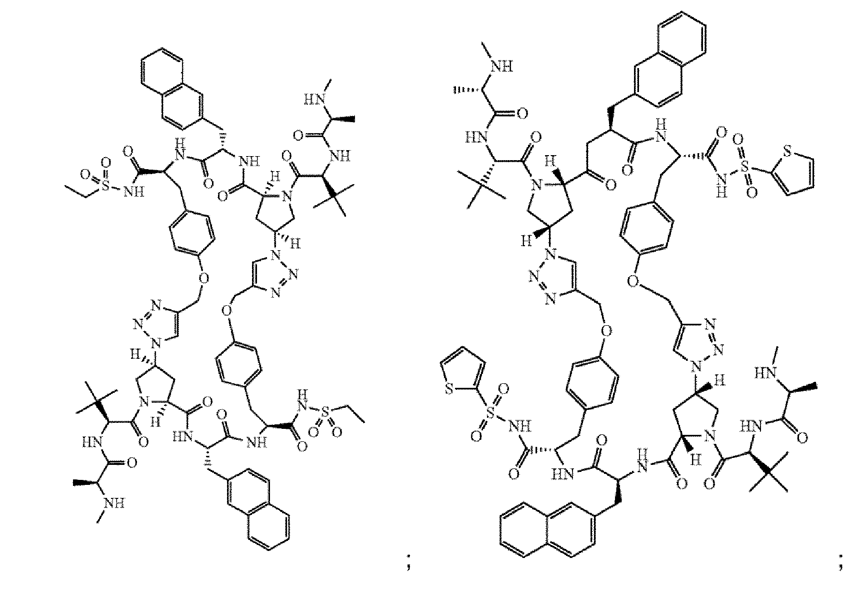

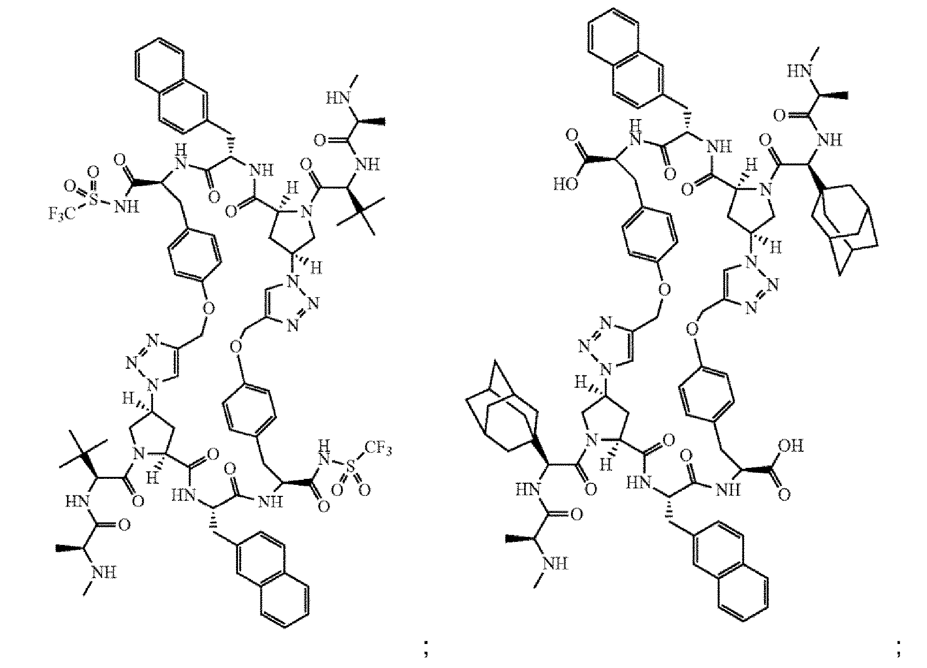

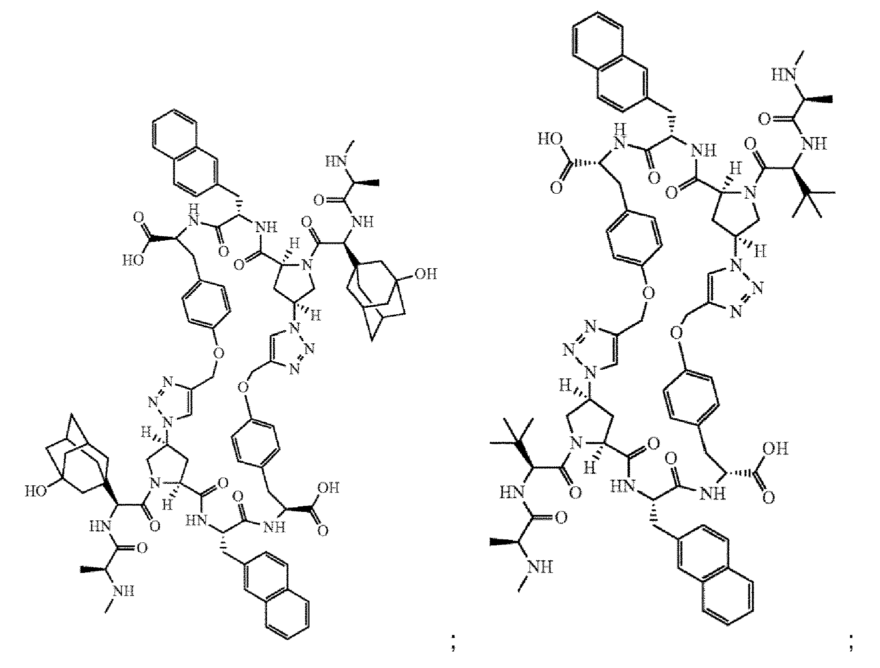

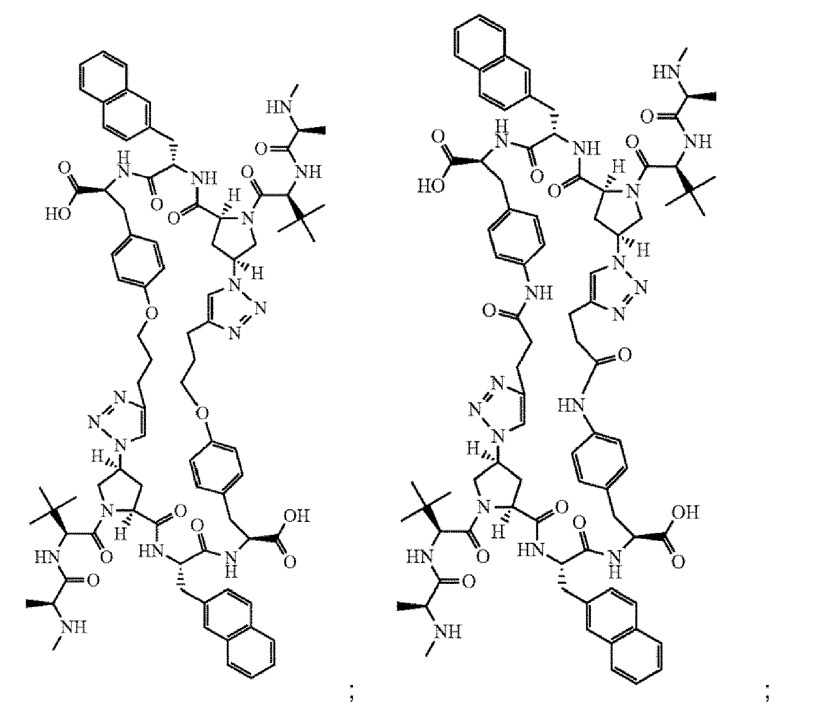

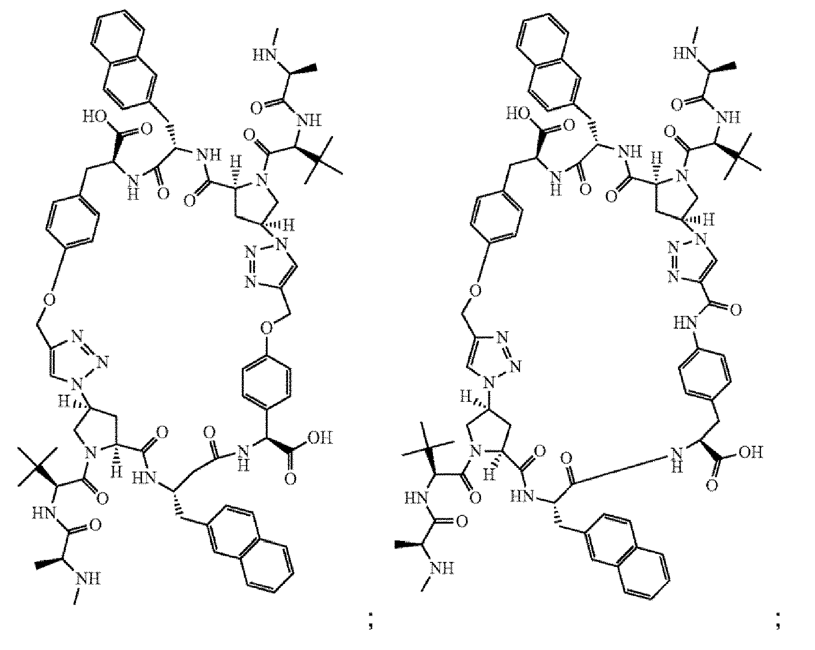

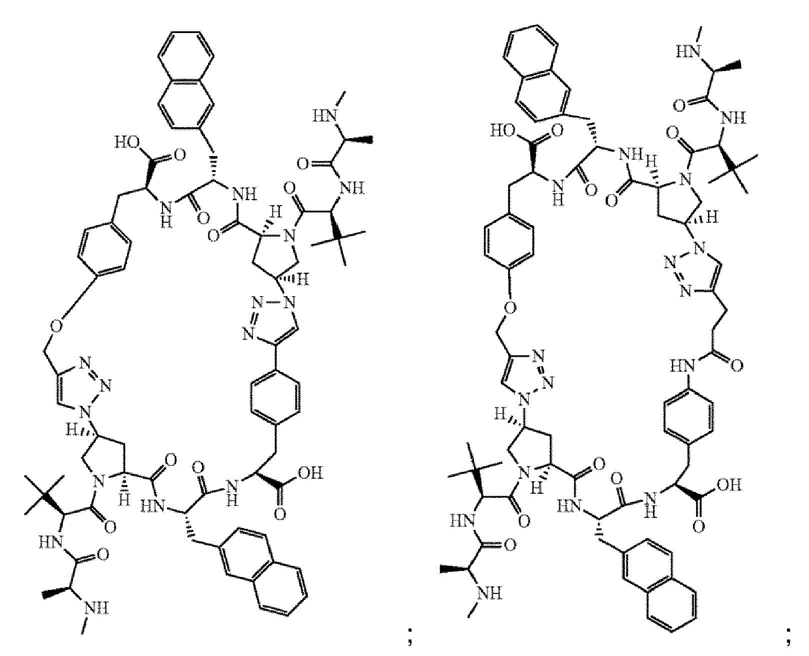

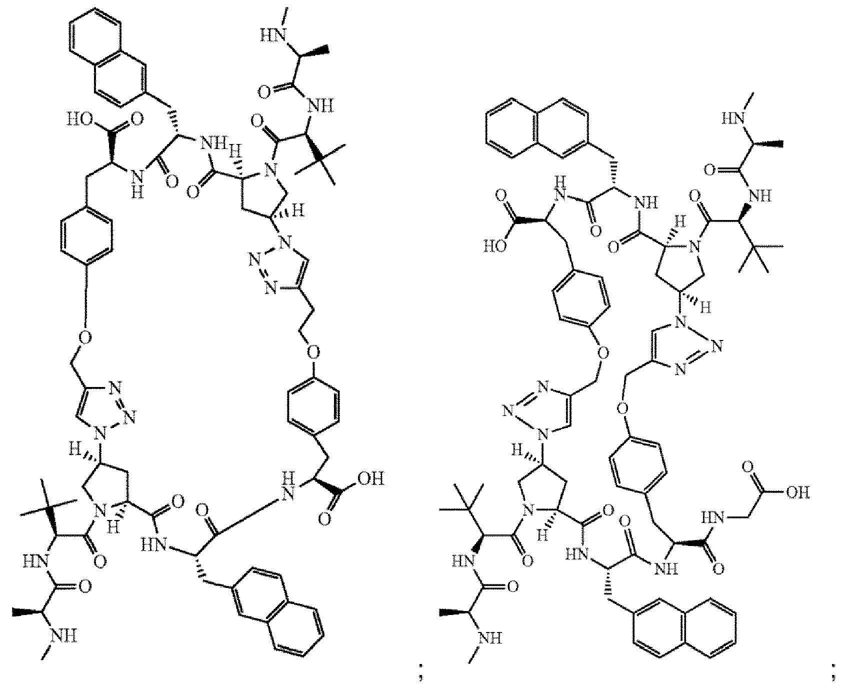

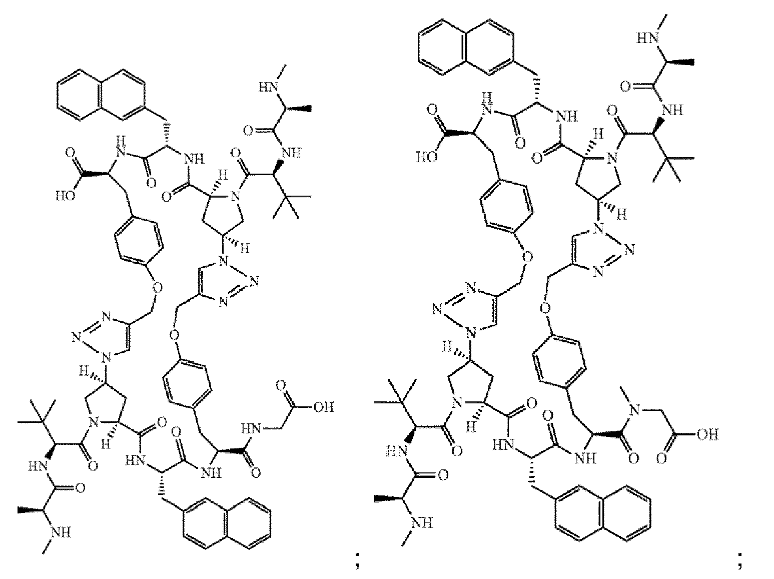

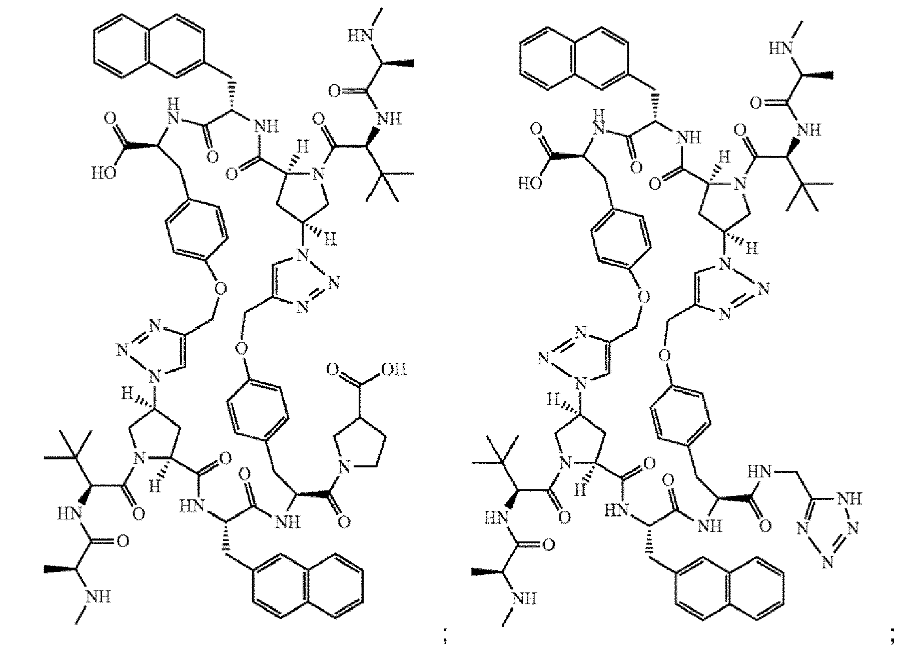

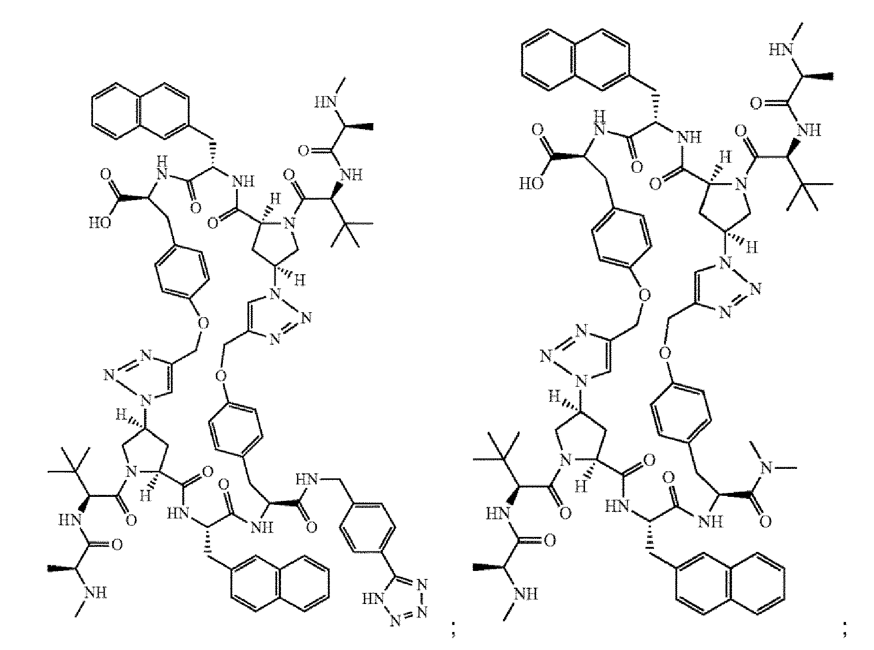

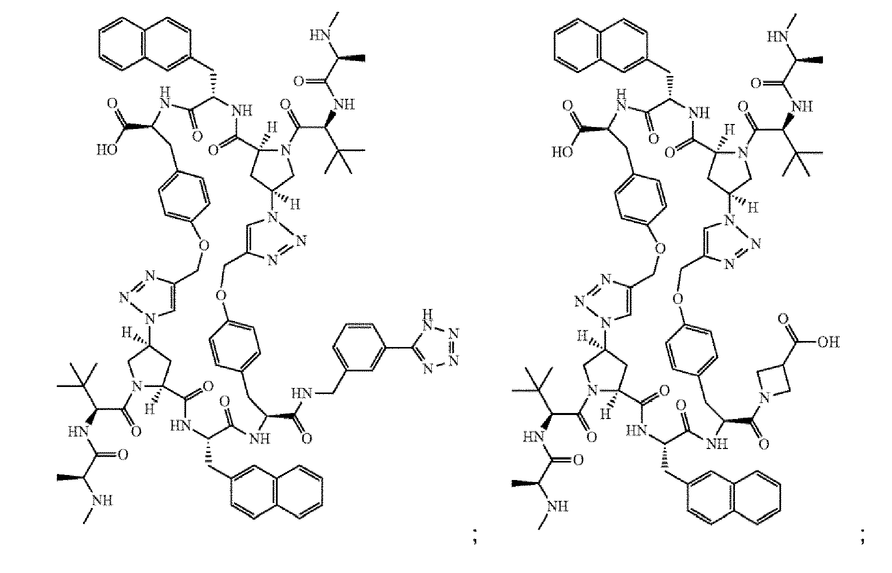

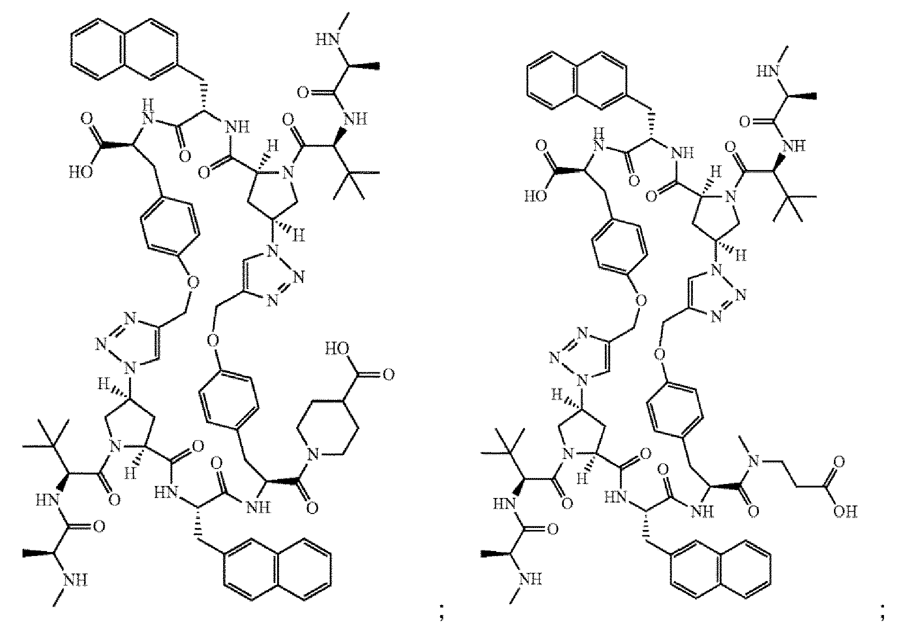

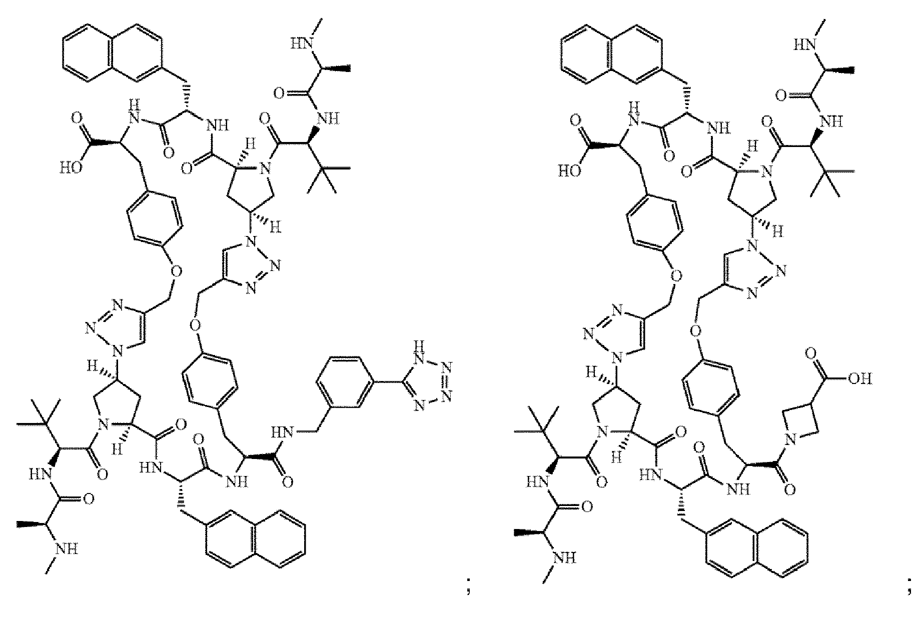

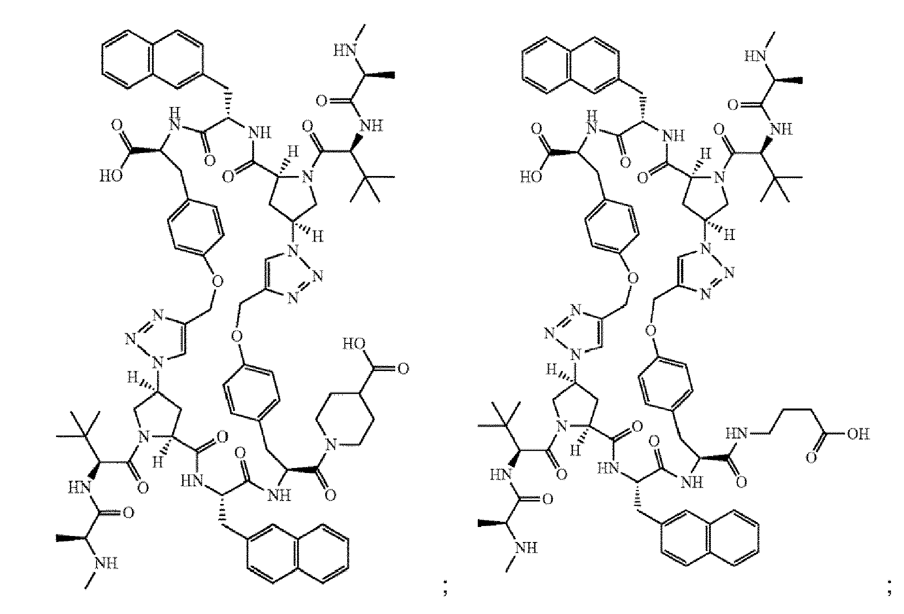

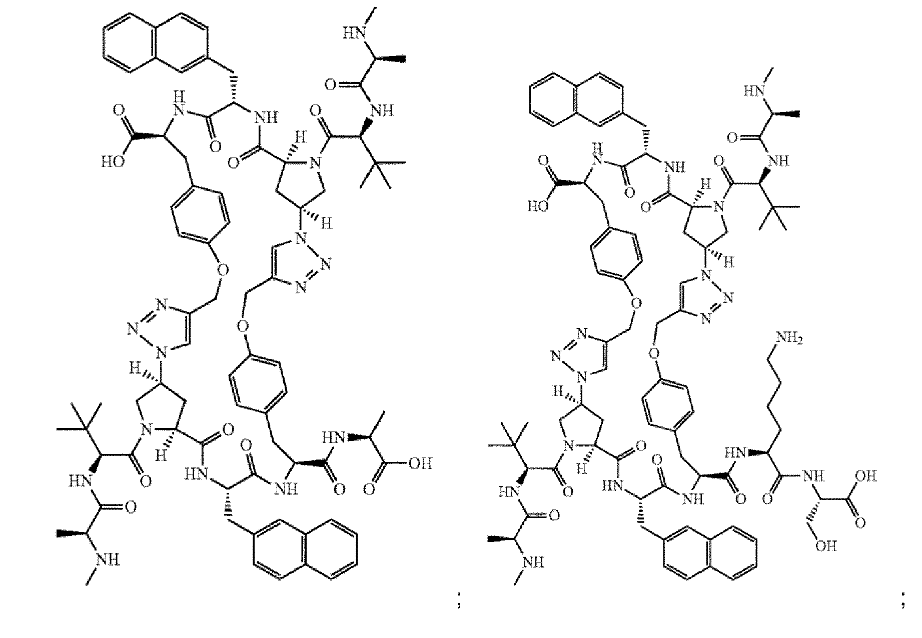

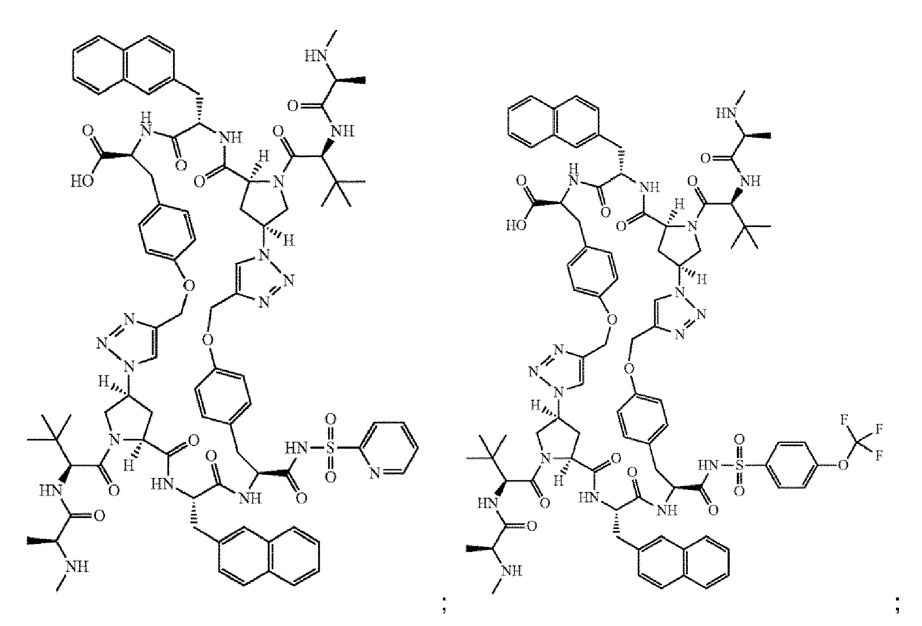

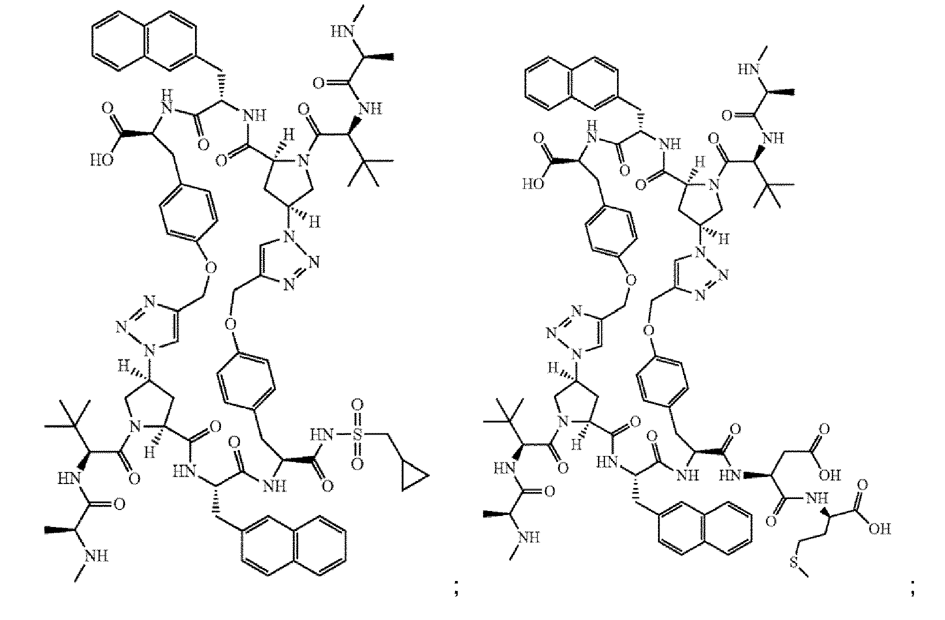

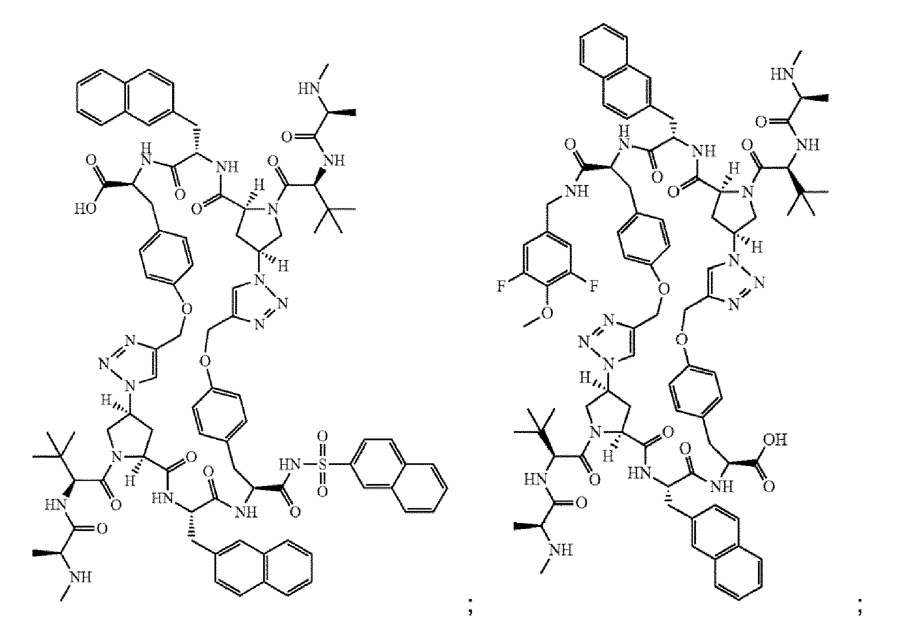

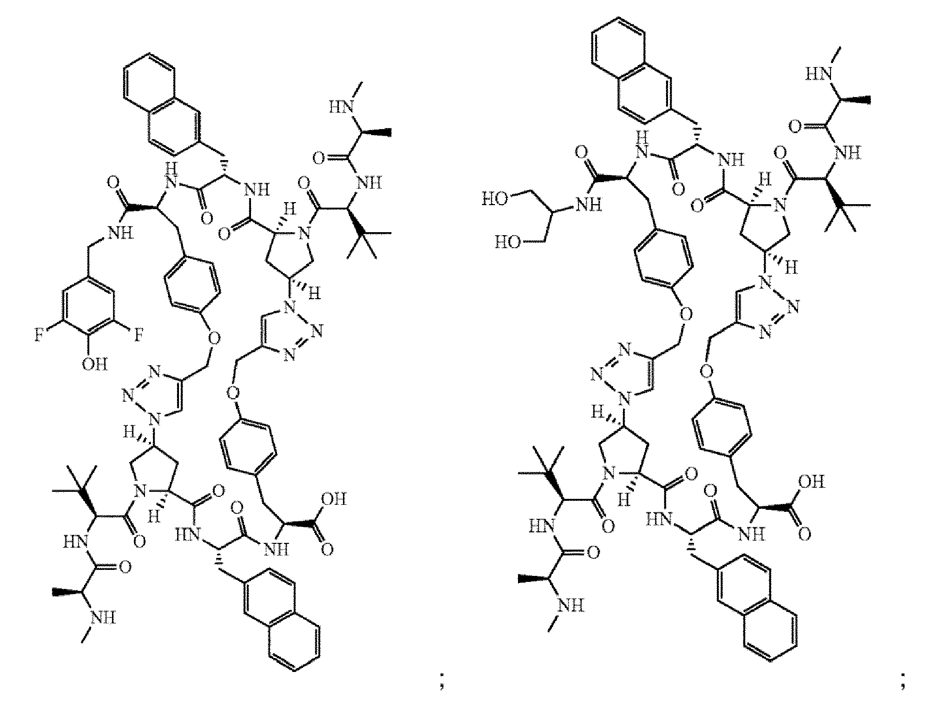

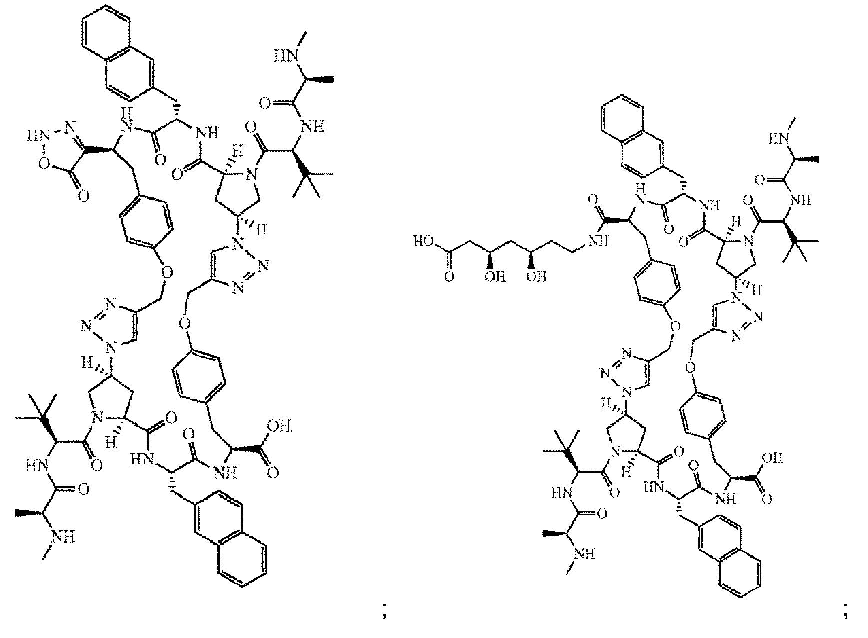

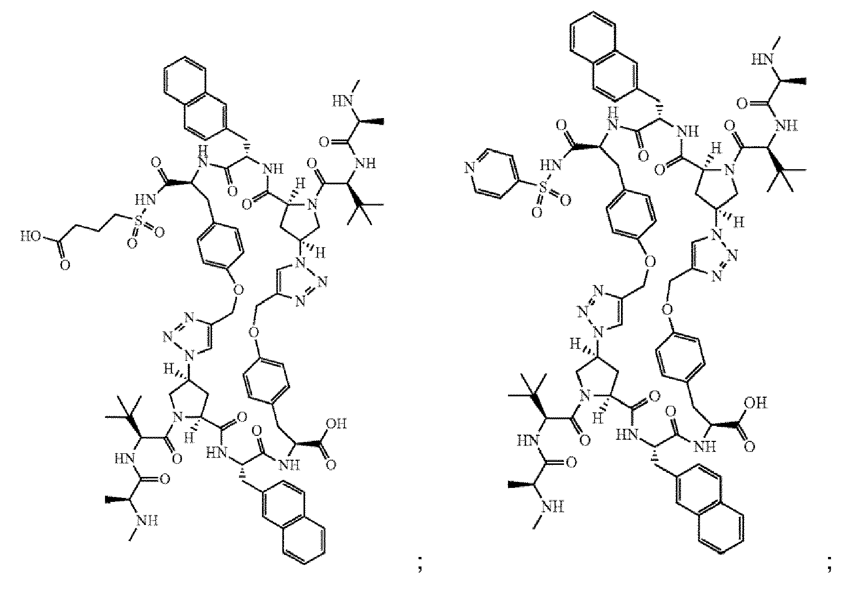

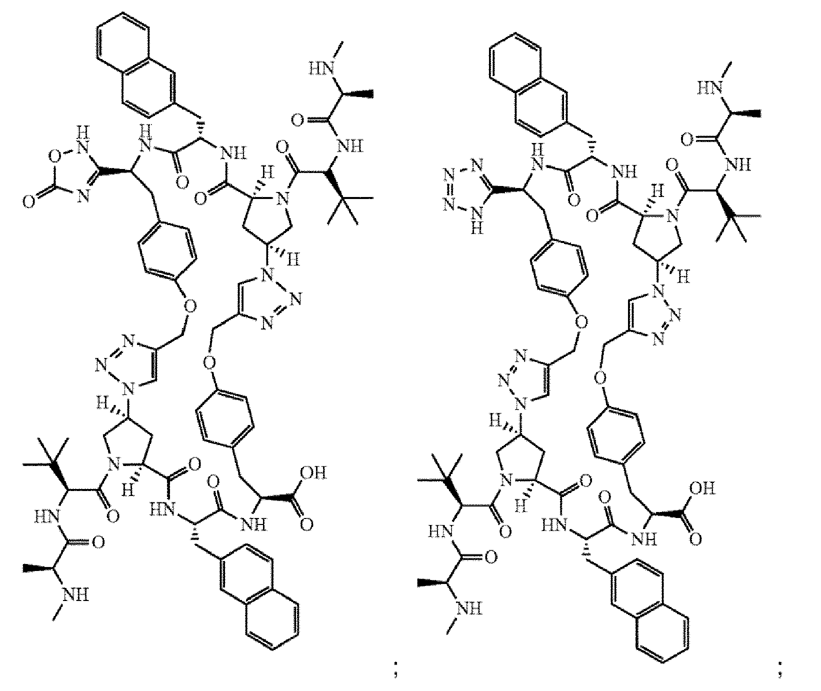

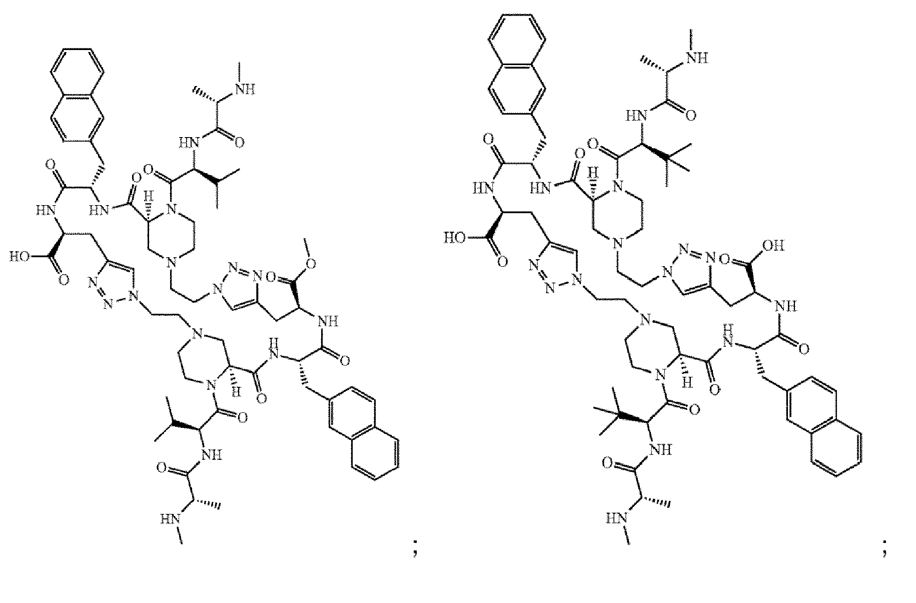

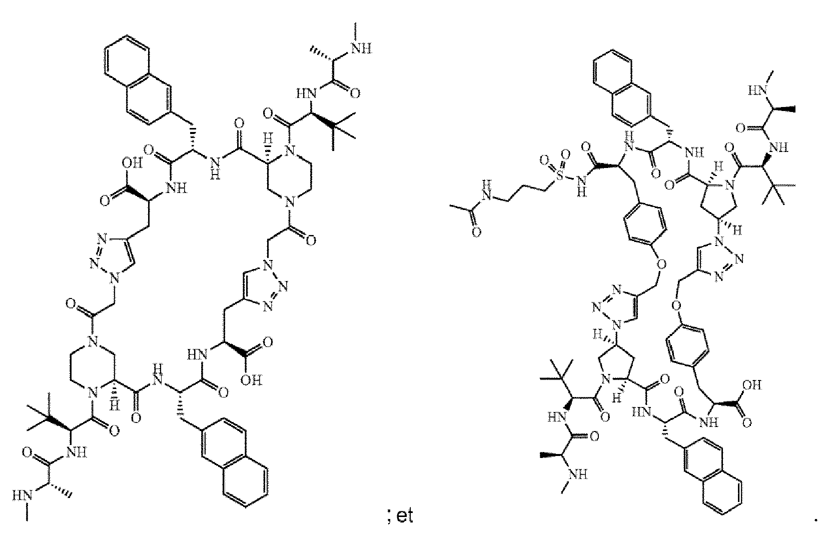

14. Composé selon l'une quelconque des revendications précédentes pour une utilisation en tant que médicament.

15. Composition pharmaceutiquement acceptable comprenant un composé selon l'une quelconque des revendications 1 à 13 et un véhicule pharmaceutiquement acceptable.

16. Composé selon l'une quelconque des revendications 1 à 13 ou composition pharmaceutique selon la revendication 15 pour une utilisation dans le traitement du cancer.
